(19) Europäisches Patentamt
European Patent Office
Office européen des brevets

(11) Veröffentlichungsnummer: **0 398 115 B1**

# (12) EUROPÄISCHE PATENTSCHRIFT

(45) Veröffentlichungstag der Patentschrift: **04.01.95**

(51) Int. Cl.[6]: **C07D 405/10**, C07D 409/10, A01N 43/28, A01N 43/24, A01N 43/32

(21) Anmeldenummer: **90108543.1**

(22) Anmeldetag: **07.05.90**

Die Akte enthält technische Angaben, die nach dem Eingang der Anmeldung eingereicht wurden und die nicht in dieser Patentschrift enthalten sind.

## (54) Herbizide N-Phenyltetrahydrophthalimide, Herstellung und Mittel.

(30) Priorität: **19.05.89 DE 3916292**

(43) Veröffentlichungstag der Anmeldung:
**22.11.90 Patentblatt 90/47**

(45) Bekanntmachung des Hinweises auf die Patenterteilung:
**04.01.95 Patentblatt 95/01**

(84) Benannte Vertragsstaaten:
**CH DE ES FR GB IT LI NL**

(56) Entgegenhaltungen:
**EP-A- 0 207 894**
**DE-A- 3 714 373**
**DE-A- 3 741 272**
**DE-A- 3 741 273**

(73) Patentinhaber: **BASF Aktiengesellschaft**
**Carl-Bosch-Strasse 38**
**D-67063 Ludwigshafen (DE)**

(72) Erfinder: **Rueb, Lothar, Dr.**
**Am Schoeneck 11**
**D-6720 Speyer (DE)**
Erfinder: **Eicken, Karl, Dr.**
**Am Huettenwingert 12**
**D-6706 Wachenheim (DE)**
Erfinder: **Westphalen, Karl-Otto, Dr.**
**Mausbergweg 58**
**D-6720 Speyer (DE)**
Erfinder: **Wuerzer, Bruno, Dr.**
**Ruedigerstrasse 13**
**D-6701 Otterstadt (DE)**

**EP 0 398 115 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft N-Phenyltetrahydrophthalimide der allgemeinen Formel I,

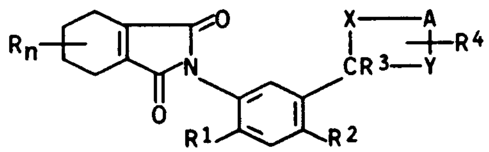

I,

in der die Substituenten und der Index folgende Bedeutung haben:

- R eine $C_1$-$C_4$-Alkylgruppe;
- n 1 oder 2;
- $R^1$ Wasserstoff oder ein Fluoratom;
- $R^2$ ein Halogenatom;
- $R^3$ Wasserstoff oder eine $C_1$-$C_4$-Alkylgruppe;
- $R^4$ Wasserstoff; eine $C_1$-$C_8$-Alkoxycarbonylgruppe, eine $C_3$-$C_6$-Alkenyloxycarbonylgruppe oder eine $C_3$-$C_6$-Alkinyloxycarbonylgruppe, wobei diese Gruppen einen $C_1$-$C_4$-Alkoxyrest tragen können;
- X,Y Sauerstoff oder Schwefel;
- A eine $C_2$-$C_3$-Alkylenkette, welche ein bis drei der folgenden Reste tragen kann: Hydroxyl, Carboxyl, $C_1$-$C_4$-Alkyl und/oder $C_1$-$C_6$-Alkoxycarbonyl, wobei die beiden letztgenannten Reste ihrerseits ein bis fünf Halogenatome oder einen der folgenden Reste tragen können: Cyano, Hydroxy, Mercapto, $C_1$-$C_4$-Alkoxy, $C_3$-$C_6$-Alkenyloxy, $C_3$-$C_6$-Alkinyloxy, $C_1$-$C_6$-Alkylcarbonyloxy, $C_1$-$C_4$-Alkylthio, $C_3$-$C_6$-Alkenylthio, $C_3$-$C_6$-Alkinylthio, $C_1$-$C_6$-Alkoxycarbonyl, $C_1$-$C_6$-Alkoxycarbonyl-$C_1$-$C_4$-alkoxy oder $C_1$-$C_6$-Alkoxycarbonyl-$C_1$-$C_4$-alkylthio,

wobei $R^4$ nicht Wasserstoff bedeutet, wenn X und Y Sauerstoff bedeuten.

Des weiteren betrifft die Erfindung Verfahren zur Herstellung dieser Verbindungen I sowie deren Verwendung als herbizide Mittel.

Aus der Literatur sind N-Phenyltetrahydrophthalimide bekannt, deren allgemeine Struktur bis auf die Reste $R_n$ den vorstehend definierten Verbindungen entspricht (EP-A 207 894, DE-A 3 741 272, DE-A 3 741 273).

Es sind jedoch Verbindungen wünschenswert, die bei geringeren Aufwandmengen gute herbizide Wirkung zeigen.

Der Erfindung lagen daher neue N-Phenyltetrahydrophthalimidderivate mit verbesserter herbizider Wirkung als Aufgabe zugrunde.

Demgemäß wurden die eingangs definierten N-Phenyltetrahydrophthalimide I gefunden.

Außerdem wurden Verfahren zur Herstellung der Verbindungen I, die Verwendung dieser Verbindungen als Herbizide sowie diese enthaltende herbizide Mittel gefunden.

Man erhält die Verbindungen I beispielsweise dadurch, daß man ein Nitrobenzol der allgemeinen Formel II in an sich bekannter Weise (Ferri, Reaktionen der org. Synthese; G. Thieme Verlag Stuttgart, S. 426ff) in einem inerten organischen Lösungsmittel in Gegenwart einer Säure bei Temperaturen von 0 bis 180 °C, vorzugsweise 25 bis 150 °C mit einer Verbindung III acetalisiert, das so erhaltene Acetal bzw. Thioacetal IV anschließend zum Anilinderivat V reduziert und V mit einem Tetrahydrophthalsäureanhydrid zum Derivat I cyclisiert.

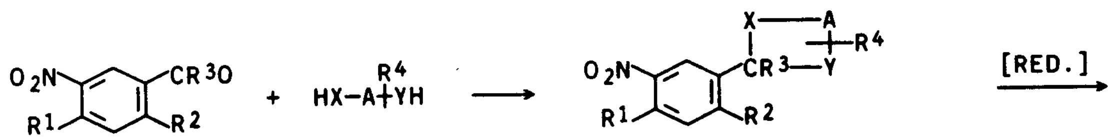


II III IV

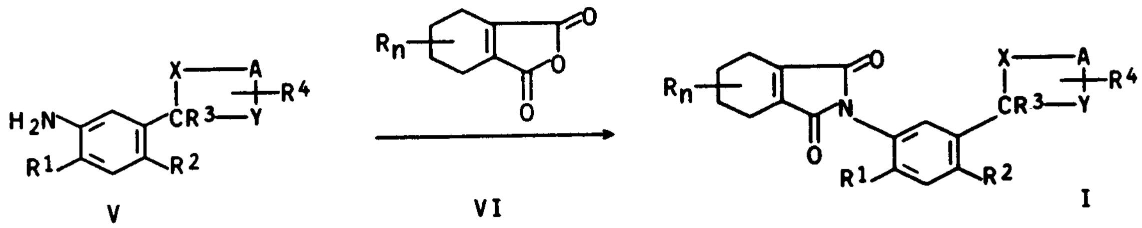

V VI I

Die Acetalisierung von II mit III wird bevorzugt in einem aprotischen organischen Lösungsmittel wie Toluol und Xylol, insbesondere Toluol, in Gegenwart von p-Toluolsulfonsäure als Katalysator durchgeführt.

Die Reduktion des so erhaltenen Acetals IV zum Anilinderivat V wird nach allgemein üblichen Methoden (Houben-Weyl, Bd. 4/1c, S. 507ff und Bd. 4/1d, S. 470ff), beispielsweise in Gegenwart von anorganischen Reduktionsmitteln wie Eisen und Zinn-II-salzen in aprotisch oder protisch polaren Lösungsmitteln oder in Gegenwart von Wasserstoff an Metallkontakten wie Platin, Palladium und Raney-Nickel in aprotisch polaren Lösungsmitteln durchgeführt.

Die anschließende Umsetzung des Anilinderivats V mit dem entsprechenden Tetrahydrophthalsäureanhydrid erfolgt in an sich bekannter Weise in einem inerten organischen Lösungsmittel. Als Lösungsmittel dienen beispielsweise niedere Alkansäuren wie Essigsäure, Propionsäure und Isobuttersäure und Ester dieser Säuren wie Essigsäure-ethylester, desweiteren höhersiedende Kohlenwasserstoffe wie Toluol und Xylol und/oder Dimethylformamid. Die Umsetzung erfolgt in der Regel bei Temperaturen von 0 bis 150 °C, vorzugsweise 20 bis 100 °C. Beim Arbeiten in einem aprotischen Lösungsmittel empfiehlt es sich saure Katalysatoren wie aromatische Sulfonsäuren zuzusetzen und das Reaktionswasser fortlaufend zu entfernen.

Man erhält die N-Phenyltetrahydrophthalimidderivate I aber auch, in dem man ein cyclisches Acetal VII in an sich bekannter Weise in einem inerten organischen Lösungsmittel in Gegenwart einer Säure bei Temperaturen von 0 bis 150 °C, vorzugsweise 20 bis 100 °C mit dem entsprechenden Tetrahydrophthalsäureanhydrid VI unter Acetalspaltung zum Derivat VII cyclisiert und VIII anschließend VIII mit III zu I acetalisiert.

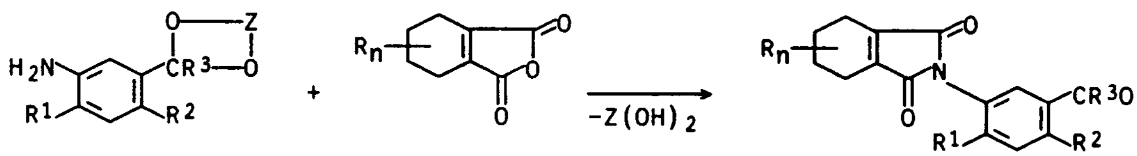


VII VI VIII

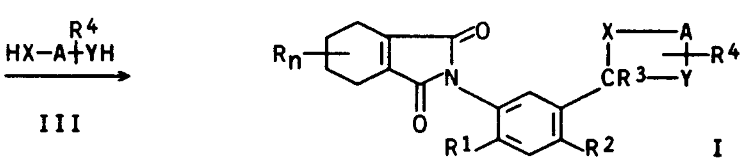

III I

Z in Formel VII bedeutet eine Ethylen- oder Propylenkette, welche ein bis drei $C_1$-$C_3$-Alkylgruppen wie vorzugsweise Methyl und Ethyl tragen kann.

Die Umsetzung von VII mit VI wird in der Regel in protisch polaren Lösungsmitteln analog der Umsetzung von V mit VI durchgeführt.

Die Acetalisierung von VIII mit III verläuft analog den vorstehend geschilderten Bedingungen für die Umsetzung von II mit III.

Im Hinblick auf die bestimmungsgemäße Verwendung der Verbindungen I als Herbizide kommen als Substituenten bevorzugt folgende Reste in Betracht:

R Alkyl wie Methyl, Ethyl, Propyl, 1-Methylethyl, Butyl, 1-Methylpropyl, 2-Methylpropyl und 1,1-Dimethyl, insbesondere Methyl und Ethyl;

n 1 oder 2;

$R^1$ Wasserstoff oder Fluor;

$R^2$ Halogen wie Fluor, Chlor, Brom und Jod, vorzugsweise Fluor, Chlor und Brom;

$R^3$ Wasserstoff oder Alkyl wie bei R genannt, vorzugsweise Wasserstoff, Methyl und Ethyl;

$R^4$ Wasserstoff,

Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl, Pentoxycarbonyl, 1-Methylbutoxycarbonyl, 2-Methylbutoxycarbonyl, 3-Methylbutoxycarbonyl,1,2-Dimethylpropyloxycarbonyl, 1,1-Dimethylpropyloxycarbonyl, 2,2-Dimethylpropyloxycarbonyl, 1-Ethylpropyloxycarbonyl, n-Hexyloxycarbonyl, 1-Methyl-pentyloxycarbonyl, 2-Methylpentyloxycarbonyl, 3-Methylpentyloxycarbonyl, 4-Methylpentyloxycarbonyl, 1,2-Dimethylbutyloxycarbonyl, 1,3-Dimethylbutoxycarbonyl, 2,3-Dimethylbutoxycarbonyl, 1,1-Dimethylbutoxycarbonyl, 2,2-Dimethylbutoxycarbonyl, 3,3-Dimethylbutoxycarbonyl, 1,1,2-Trimethylpropyloxycarbonyl, 1,2,2-Trimethylpropyloxycarbonyl, 1-Ethylbutoxycarbonyl, 2-Ethylbutoxycarbonyl und 1-Ethyl-2-Methylpropyloxycarbonyl;

Alkenyloxycarbonyl wie 2-Propenyloxycarbonyl, 2-Butenyloxycarbonyl, 3-Butenyloxycarbonyl, 1-Methyl-2-propenyloxycarbonyl, 2-Methyl-2-propenyloxycarbonyl, 2-Pentenyloxycarbonyl, 3-Pentenyloxycarbonyl, 4-Pentenyloxycarbonyl, 1-Methyl-2-butenyloxycarbonyl, 2-Methyl-2-butenyloxycarbonyl, 3-Methyl-2-butenyloxycarbonyl, 1-Methyl-3-butenyloxycarbonyl, 2-Methyl-3-butenyloxycarbonyl, 3-Methyl-3-butenyloxycarbonyl, 1,1-Dimethyl-2-propenyloxycarbonyl, 1,2-Dimethyl-2-propenyloxycarbonyl, 1-Ethyl-2-propenyloxycarbonyl, 2-Hexenyloxycarbonyl, 3-Hexenyloxycarbonyl, 4-Hexenyloxycarbonyl, 5-Hexenyloxycarbonyl, 1-Methyl-2-pentenyloxycarbonyl, 2-Methyl-2-pentenyloxycarbonyl, 3-Methyl-2-pentenyloxycarbonyl, 4-Methyl-2-pentenyloxycarbonyl, 1-Methyl-3-pentenyloxycarbonyl, 2-Methyl-3-pentenyloxycarbonyl, 3-Methyl-3-pentenyloxycarbonyl, 4-Methyl-3-pentenyloxycarbonyl, 1-Methyl-4-pentenyloxycarbonyl, 2-Methyl-4-pentenyloxycarbonyl, 3-Methyl-4-pentenyloxycarbonyl, 4-Methyl-4-pentenyloxycarbonyl, 1,1-Dimethyl-2-butenyloxycarbonyl, 1,1-Dimethyl-3-butenyloxycarbonyl, 1,2-Dimethyl-2-butenyloxycarbonyl, 1,2-Dimethyl-3-butenyloxycarbonyl, 1,3-Dimethyl-2-butenyloxycarbonyl, 1,3-Dimethyl-3-butenyloxycarbonyl, 2,2-Dimethyl-3-butenyloxycarbonyl, 2,3-Dimethyl-2-butenyloxycarbonyl, 2,3-Dimethyl-3-butenyloxycarbonyl, 1-Ethyl-2-butenyloxycarbonyl, 1-Ethyl-3-butenyloxycarbonyl, 2-Ethyl-2-butenyloxycarbonyl, 2-Ethyl-3-butenyloxycarbonyl, 1,1,2-Trimethyl-2-propenyloxycarbonyl, 1-Ethyl-1-Methyl-2-propenyloxycarbonyl und 1-Ethyl-2-Methyl-2-propenyloxycarbonyl;

Alkinyloxycarbonyl wie 2-Propinyloxycarbonyl, 2-Butinyloxycarbonyl, 3-Butinyloxycarbonyl, 1-Methyl-2-propinyloxycarbonyl, 2-Pentinyloxycarbonyl, 3-Pentinyloxycarbonyl, 4-Pentinyloxycarbonyl, 1-Methyl-3-butinyloxycarbonyl, 2-Methyl-3-butinyloxycarbonyl, 1-Methyl-2-butinyloxycarbonyl, 1,1-Dimethyl-2-propinyloxycarbonyl, 1-Ethyl-2-propinyloxycarbonyl, 2-Hexinyloxycarbonyl, 3-Hexinyloxycarbonyl, 4-Hexinyloxycarbonyl, 5-Hexinyloxycarbonyl, 1-Methyl-2-pentinyloxycarbonyl,1-Methyl-3-pentinyloxycarbonyl, 1-Methyl-4-pentinyloxycarbonyl, 2-Methyl-3-pentinyloxycarbonyl, 2-Methyl-4-pentinyloxycarbonyl, 3-Methyl-4-pentinyloxycarbonyl, 4-Methyl-2-pentinyloxycarbonyl, 1,1-Dimethyl-2-butinyloxycarbonyl, 1,1-Dimethyl-3-butinyloxycarbonyl, 1,2-Dimethyl-3-butinyloxycarbonyl, 2,2-Dimethyl-3-butinyloxycarbonyl, 1-Ethyl-2-butinyloxycarbonyl, 1-Ethyl-3-butinyloxycarbonyl, 2-Ethyl-3-butinyloxycarbonyl und 1-Ethyl-1-methyl-2-propinyloxycarbonyl;

wobei diese Gruppen zusätzlich einen Alkoxyrest wie Methoxy, Ethoxy, Propyloxy, 1-Methylethoxy, Butyloxy, 1-Methylpropyloxy, 2-Methylpropyloxy und 1,1-Dimethylethoxy, vorzugsweise Methoxy und Ethoxy, tragen können;

X,Y Sauerstoff oder Schwefel;

A Alkylen wie 1,2-Ethylen und 1,3-Propylen, welches ein bis drei der folgenden Reste tragen kann: Hydroxy; Carboxyl;

Alkyl wie bei R oder Alkoxycarbonyl wie bei $R^4$ im allgemeinen und im besonderen genannt, wobei die beiden letztgenannten Reste ihrerseits ein bis fünf Halogenatome wie bei $R^2$ genannt oder einen der folgenden Reste tragen können: Cyano; Mercapto; Alkoxy wie bei $R^4$ genannt; Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-2-butenyloxy, 1,2-Dimethyl-3-butenyloxy, 1,3-Dimethyl-1-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy und 1-Ethyl-2-methyl-2-propenyloxy; Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-3-butinyloxy, 2-Methyl-3-butinyloxy, 1-Methyl-2-butinyloxy, 1,1-Dimethyl-2-propinyloxy, 1-Ethyl-2-propinyloxy, 2-Hexinyloxy, 3-Hexinyloxy, 4-Hexinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-4-pentinyloxy, 4-Methyl-2-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy, 2-Ethyl-3-butinyloxy und 1-Ethyl-1-methyl-2-propinyloxy; Alkylcarbonyloxy wie Methylcarbonyloxy, Ethylcarbonyloxy, Propylcarbonyloxy, 1-Methylethylcarbonyloxy, Butylcarbonyloxy, 1-Methylpropylcarbonyloxy, 2-Methylpropylcarbonyloxy, 1,1-Dimethylethylcarbonyloxy, Pentylcarbonyloxy, 1-Methylbutylcarbonyloxy, 2-Methylbutylcarbonyloxy, 3-Methylbutylcarbonyloxy, 1,2-Dimethylpropylcarbonyloxy, 1,1-Dimethylpropylcarbonyloxy, 2,2-Dimethylpropylcarbonyloxy, 1-Ethylpropylcarbonyloxy, n-Hexylcarbonyloxy, 1-Methyl-pentylcarbonyloxy, 2-Methylpentyl-carbonyloxy,3-Methylpentylcarbonyloxy, 4-Methylpentylcarbonyloxy, 1,2-Dimethylbutylcarbonyloxy, 1,3-Dimethylbutylcarbonyloxy, 2,3-Dimethylbutylcarbonyloxy, 1,1-Dimethylbutylcarbonyloxy, 2,2-Dimethylbutylcarbonyloxy, 3,3-Dimethylbutylcarbonyloxy, 1,1,2-Trimethylpropylcarbonyloxy, 1,2,2-Trimethylpropylcarbonyloxy, 1-Ethylbutylcarbonyloxy, 2-Ethylbutylcarbonyloxy, und 1-Ethyl-2-methylpropylcarbonyloxy; Alkylthio wie Methylthio, Ethylthio, 1-Methylethylthio, Butylthio, 1-Methylpropylthio, 2-Methylpropylthio und 1,1-Dimethylethylthio; Alkenylthio wie 2-Propenylthio, 2-Butenylthio, 3-Butenylthio, 1-Methyl-2-propenylthio, 2-Methyl-2-propenylthio, 2-Pentenylthio, 3-Pentenylthio, 4-Pentenylthio, 1-Methyl-2-butenylthio, 2-Methyl-2-butenylthio, 3-Methyl-2-butenylthio, 1-Methyl-3-butenylthio, 2-Methyl-3-butenylthio, 3-Methyl-3-butenylthio, 1,1-Dimethyl2-propenylthio, 1,2-Dimethyl-2-propenylthio, 1-Ethyl-2-propenylthio, 2-Hexenylthio, 3-Hexenylthio, 4-Hexenylthio, 5-Hexenylthio, 1-Methyl-2-pentenylthio, 2-Methyl-2-pentenylthio, 3-Methyl-2-pentenylthio, 4-Methyl-2-pentenylthio, 1-Methyl-3-pentenylthio, 2-Methyl-3-pentenylthio, 3-Methyl-3-pentenylthio, 4-Methyl-3-pentenylthio, 1-Methyl-4-pentenylthio, 2-Methyl-4-pentenylthio, 3-Methyl-4-pentenylthio, 4-Methyl-4-pentenylthio, 1,1-Dimethyl-2-butenylthio, 1,1-Dimethyl-3-butenylthio, 1,2-Dimethyl-2-butenylthio, 1,2-Dimethyl-3-butenylthio, 1,3-Dimethyl-1-butenylthio, 1,3-Dimethyl-2-butenylthio, 1,3-Dimethyl-3-butenylthio, 2,2-Dimethyl-3-butenylthio, 2,3-Dimethyl-2-butenylthio, 2,3-Dimethyl-3-butenylthio, 1-Ethyl-2-butenylthio, 1-Ethyl-3-butenylthio, 2-Ethyl-2-butenylthio, 2-Ethyl-3-butenylthio, 1,1,2-Trimethyl-2-propenylthio, 1-Ethyl-1-methyl-2-propenylthio und 1-Ethyl-2-methyl-2-propenylthio; Alkinylthio wie 2-Propinylthio, 2-Butinylthio, 3-Butinylthio, 1-Methyl-2-propinylthio, 2-Pentinylthio, 3-Pentinylthio, 4-Pentinylthio, 1-Methyl-3-butinylthio, 2-Methyl-3-butinylthio, 1-Methyl-2-butinylthio, 1,1-Dimethyl-2-propinylthio, 1-Ethyl-2-propinylthio, 2-Hexinylthio, 3-Hexinylthio, 4-Hexinylthio, 5-Hexinylthio, 1-Methyl-2-pentinylthio, 1-Methyl-3-pentinylthio, 1-Methyl-4-pentinylthio, 2-Methyl-3-pentinylthio, 2-Methyl-4-pentinylthio, 3-Methyl-4-pentinylthio, 4-Methyl-2-pentinylthio, 1,1-Dimethyl-2-butinylthio, 1,1-Dimethyl-3-butinylthio, 1,2-Dimethyl-3-butinylthio, 2,2-Dimethyl-3-butinylthio, 1-Ethyl-2-butinylthio, 1-Ethyl-3-butinylthio, 2-Ethyl-3-butinylthiound 1-Ethyl-1-methyl-2-propinylthio oder Alkoxycarbonyl wie bei $R^4$ genannt welches direkt oder über eine der vorstehend genannten Alkoxygruppen oder Alkylthiogruppen gebunden sein kann,

wobei $R^4$ nicht Wasserstoff bedeutet, wenn X und Y Sauerstoff bedeuten.

Insbesondere bevorzugt sind N-henyltetrahydrophthalimide der allgemeinen Formel I, in denen

R Methyl oder Ethyl,
n 1 oder 2
$R^2$ Fluor, Chlor oder Brom und
$R^3$ Wasserstoff, Methyl oder Ethyl

bedeutet.

Beispielhaft sind einige besonders aktive Verbindungen I in den folgenden Tabellen A, B und C aufgeführt.

Tabelle A

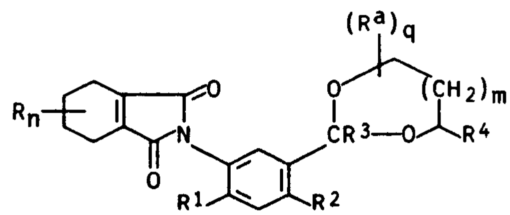

| $R_n$ | $R^1$ | $R^2$ | $R^3$ | m | $(R^a)_q$ | $R^4$ |
|---|---|---|---|---|---|---|
| 3-$CH_3$ | H | F | H | 0 | H | $CO_2CH_3$ |
| 4-$CH_3$ | H | F | H | 0 | H | $CO_2CH_3$ |
| 3-$CH_3$ | F | F | H | 0 | H | $CO_2CH_3$ |
| 4-$CH_3$ | F | F | H | 0 | H | $CO_2CH_3$ |
| 3-$CH_3$ | H | Cl | H | 0 | H | $CO_2CH_3$ |
| 4-$CH_3$ | H | Cl | H | 0 | H | $CO_2CH_3$ |
| 3-$CH_3$ | F | Cl | H | 0 | H | $CO_2CH_3$ |
| 4-$CH_3$ | F | Cl | H | 0 | H | $CO_2CH_3$ |
| 3-$CH_3$ | H | F | $CH_3$ | 0 | H | $CO_2CH_3$ |
| 4-$CH_3$ | H | F | $CH_3$ | 0 | H | $CO_2CH_3$ |
| 3-$CH_3$ | F | F | $CH_3$ | 0 | H | $CO_2CH_3$ |
| 4-$CH_3$ | F | F | $CH_3$ | 0 | H | $CO_2CH_3$ |
| 3-$CH_3$ | H | Cl | $CH_3$ | 0 | H | $CO_2CH_3$ |
| 4-$CH_3$ | H | Cl | $CH_3$ | 0 | H | $CO_2CH_3$ |
| 3-$CH_3$ | F | Cl | $CH_3$ | 0 | H | $CO_2CH_3$ |
| 4-$CH_3$ | F | Cl | $CH_3$ | 0 | H | $CO_2CH_3$ |
| 3-$CH_3$ | H | F | H | 0 | H | $CO_2CH_2CH_3$ |
| 4-$CH_3$ | H | F | H | 0 | H | $CO_2CH_2CH_3$ |
| 3-$CH_3$ | F | F | H | 0 | H | $CO_2CH_2CH_3$ |
| 4-$CH_3$ | F | F | H | 0 | H | $CO_2CH_2CH_3$ |
| 3-$CH_3$ | H | Cl | H | 0 | H | $CO_2CH_2CH_3$ |
| 4-$CH_3$ | H | Cl | H | 0 | H | $CO_2CH_2CH_3$ |
| 3-$CH_3$ | F | Cl | H | 0 | H | $CO_2CH_2CH_3$ |
| 4-$CH_3$ | F | Cl | H | 0 | H | $CO_2CH_2CH_3$ |
| 3-$CH_3$ | H | F | $CH_3$ | 0 | H | $CO_2CH_2CH_3$ |
| 4-$CH_3$ | H | F | $CH_3$ | 0 | H | $CO_2CH_2CH_3$ |
| 3-$CH_3$ | F | F | $CH_3$ | 0 | H | $CO_2CH_2CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 4-$CH_3$ | F | F | $CH_3$ | 0 | H | $CO_2CH_2CH_3$ |
| 3-$CH_3$ | H | Cl | $CH_3$ | 0 | H | $CO_2CH_2CH_3$ |
| 4-$CH_3$ | H | Cl | $CH_3$ | 0 | H | $CO_2CH_2CH_3$ |
| 3-$CH_3$ | F | Cl | $CH_3$ | 0 | H | $CO_2CH_2CH_3$ |
| 4-$CH_3$ | F | Cl | $CH_3$ | 0 | H | $CO_2CH_2CH_3$ |
| 3-$CH_3$ | H | F | H | 0 | H | $CO_2(CH_2)_2CH_3$ |
| 4-$CH_3$ | H | F | H | 0 | H | $CO_2(CH_2)_2CH_3$ |
| 3-$CH_3$ | F | F | H | 0 | H | $CO_2(CH_2)_2CH_3$ |
| 4-$CH_3$ | F | F | H | 0 | H | $CO_2(CH_2)_2CH_3$ |
| 3-$CH_3$ | H | Cl | H | 0 | H | $CO_2(CH_2)_2CH_3$ |
| 4-$CH_3$ | H | Cl | H | 0 | H | $CO_2(CH_2)_2CH_3$ |
| 3-$CH_3$ | F | Cl | H | 0 | H | $CO_2(CH_2)_2CH_3$ |
| 4-$CH_3$ | F | Cl | H | 0 | H | $CO_2(CH_2)_2CH_3$ |
| 3-$CH_3$ | H | F | $CH_3$ | 0 | H | $CO_2(CH_2)_2CH_3$ |
| 4-$CH_3$ | H | F | $CH_3$ | 0 | H | $CO_2(CH_2)_2CH_3$ |
| 3-$CH_3$ | F | F | $CH_3$ | 0 | H | $CO_2(CH_2)_2CH_3$ |
| 4-$CH_3$ | F | F | $CH_3$ | 0 | H | $CO_2(CH_2)_2CH_3$ |
| 3-$CH_3$ | H | Cl | $CH_3$ | 0 | H | $CO_2(CH_2)_2CH_3$ |
| 4-$CH_3$ | H | Cl | $CH_3$ | 0 | H | $CO_2(CH_2)_2CH_3$ |
| 3-$CH_3$ | F | Cl | $CH_3$ | 0 | H | $CO_2(CH_2)_2CH_3$ |
| 4-$CH_3$ | F | Cl | $CH_3$ | 0 | H | $CO_2(CH_2)_2CH_3$ |
| 3-$CH_3$ | H | F | H | 0 | H | $CO_2CH(CH_3)_2$ |
| 4-$CH_3$ | H | F | H | 0 | H | $CO_2CH(CH_3)_2$ |
| 3-$CH_3$ | F | F | H | 0 | H | $CO_2CH(CH_3)_2$ |
| 4-$CH_3$ | F | F | H | 0 | H | $CO_2CH(CH_3)_2$ |
| 3-$CH_3$ | H | Cl | H | 0 | H | $CO_2CH(CH_3)_2$ |
| 4-$CH_3$ | H | Cl | H | 0 | H | $CO_2CH(CH_3)_2$ |
| 3-$CH_3$ | F | Cl | H | 0 | H | $CO_2CH(CH_3)_2$ |
| 4-$CH_3$ | F | Cl | H | 0 | H | $CO_2CH(CH_3)_2$ |
| 3-$CH_3$ | H | F | $CH_3$ | 0 | H | $CO_2CH(CH_3)_2$ |
| 4-$CH_3$ | H | F | $CH_3$ | 0 | H | $CO_2CH(CH_3)_2$ |
| 3-$CH_3$ | F | F | $CH_3$ | 0 | H | $CO_2CH(CH_3)_2$ |
| 4-$CH_3$ | F | F | $CH_3$ | 0 | H | $CO_2CH(CH_3)_2$ |

Tabelle A (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | Cl | $CH_3$ | 0 | H | $CO_2CH(CH_3)_2$ |
| 4-$CH_3$ | H | Cl | $CH_3$ | 0 | H | $CO_2CH(CH_3)_2$ |
| 3-$CH_3$ | F | Cl | $CH_3$ | 0 | H | $CO_2CH(CH_3)_2$ |
| 4-$CH_3$ | F | Cl | $CH_3$ | 0 | H | $CO_2CH(CH_3)_2$ |
| 3-$CH_3$ | H | F | H | 0 | H | $CO_2(CH_2)_3CH_3$ |
| 4-$CH_3$ | H | F | H | 0 | H | $CO_2(CH_2)_3CH_3$ |
| 3-$CH_3$ | F | F | H | 0 | H | $CO_2(CH_2)_3CH_3$ |
| 4-$CH_3$ | F | F | H | 0 | H | $CO_2(CH_2)_3CH_3$ |
| 3-$CH_3$ | H | Cl | H | 0 | H | $CO_2(CH_2)_3CH_3$ |
| 4-$CH_3$ | H | Cl | H | 0 | H | $CO_2(CH_2)_3CH_3$ |
| 3-$CH_3$ | F | Cl | H | 0 | H | $CO_2(CH_2)_3CH_3$ |
| 4-$CH_3$ | F | Cl | H | 0 | H | $CO_2(CH_2)_3CH_3$ |
| 3-$CH_3$ | H | F | $CH_3$ | 0 | H | $CO_2(CH_2)_3CH_3$ |
| 4-$CH_3$ | H | F | $CH_3$ | 0 | H | $CO_2(CH_2)_3CH_3$ |
| 3-$CH_3$ | F | F | $CH_3$ | 0 | H | $CO_2(CH_2)_3CH_3$ |
| 4-$CH_3$ | F | F | $CH_3$ | 0 | H | $CO_2(CH_2)_3CH_3$ |
| 3-$CH_3$ | H | Cl | $CH_3$ | 0 | H | $CO_2(CH_2)_3CH_3$ |
| 4-$CH_3$ | H | Cl | $CH_3$ | 0 | H | $CO_2(CH_2)_3CH_3$ |
| 3-$CH_3$ | F | Cl | $CH_3$ | 0 | H | $CO_2(CH_2)_3CH_3$ |
| 4-$CH_3$ | F | Cl | $CH_3$ | 0 | H | $CO_2(CH_2)_3CH_3$ |
| 3-$CH_3$ | H | F | H | 0 | H | $CO_2(CH_2)_2OCH_3$ |
| 4-$CH_3$ | H | F | H | 0 | H | $CO_2(CH_2)_2OCH_3$ |
| 3-$CH_3$ | F | F | H | 0 | H | $CO_2(CH_2)_2OCH_3$ |
| 4-$CH_3$ | F | F | H | 0 | H | $CO_2(CH_2)_2OCH_3$ |
| 3-$CH_3$ | H | Cl | H | 0 | H | $CO_2(CH_2)_2OCH_3$ |
| 4-$CH_3$ | H | Cl | H | 0 | H | $CO_2(CH_2)_2OCH_3$ |
| 3-$CH_3$ | F | Cl | H | 0 | H | $CO_2(CH_2)_2OCH_3$ |
| 4-$CH_3$ | F | Cl | H | 0 | H | $CO_2(CH_2)_2OCH_3$ |
| 3-$CH_3$ | H | F | $CH_3$ | 0 | H | $CO_2(CH_2)_2OCH_3$ |
| 4-$CH_3$ | H | F | $CH_3$ | 0 | H | $CO_2(CH_2)_2OCH_3$ |
| 3-$CH_3$ | F | F | $CH_3$ | 0 | H | $CO_2(CH_2)_2OCH_3$ |
| 4-$CH_3$ | F | F | $CH_3$ | 0 | H | $CO_2(CH_2)_2OCH_3$ |
| 3-$CH_3$ | H | Cl | $CH_3$ | 0 | H | $CO_2(CH_2)_2OCH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 4-$CH_3$ | H | Cl | $CH_3$ | 0 | H | $CO_2(CH_2)_2OCH_3$ |
| 3-$CH_3$ | F | Cl | $CH_3$ | 0 | H | $CO_2(CH_2)_2OCH_3$ |
| 4-$CH_3$ | F | Cl | $CH_3$ | 0 | H | $CO_2(CH_2)_2OCH_3$ |
| 3-$CH_3$ | H | F | H | 0 | H | $CO_2(CH_2)_2OCH_2CH_3$ |
| 4-$CH_3$ | H | F | H | 0 | H | $CO_2(CH_2)_2OCH_2CH_3$ |
| 3-$CH_3$ | F | F | H | 0 | H | $CO_2(CH_2)_2OCH_2CH_3$ |
| 4-$CH_3$ | F | F | H | 0 | H | $CO_2(CH_2)_2OCH_2CH_3$ |
| 3-$CH_3$ | H | Cl | H | 0 | H | $CO_2(CH_2)_2OCH_2CH_3$ |
| 4-$CH_3$ | H | Cl | H | 0 | H | $CO_2(CH_2)_2OCH_2CH_3$ |
| 3-$CH_3$ | F | Cl | H | 0 | H | $CO_2(CH_2)_2OCH_2CH_3$ |
| 4-$CH_3$ | F | Cl | H | 0 | H | $CO_2(CH_2)_2OCH_2CH_3$ |
| 3-$CH_3$ | H | F | $CH_3$ | 0 | H | $CO_2(CH_2)_2OCH_2CH_3$ |
| 4-$CH_3$ | H | F | $CH_3$ | 0 | H | $CO_2(CH_2)_2OCH_2CH_3$ |
| 3-$CH_3$ | F | F | $CH_3$ | 0 | H | $CO_2(CH_2)_2OCH_2CH_3$ |
| 4-$CH_3$ | F | F | $CH_3$ | 0 | H | $CO_2(CH_2)_2OCH_2CH_3$ |
| 3-$CH_3$ | H | Cl | $CH_3$ | 0 | H | $CO_2(CH_2)_2OCH_2CH_3$ |
| 4-$CH_3$ | H | Cl | $CH_3$ | 0 | H | $CO_2(CH_2)_2OCH_2CH_3$ |
| 3-$CH_3$ | F | Cl | $CH_3$ | 0 | H | $CO_2(CH_2)_2OCH_2CH_3$ |
| 4-$CH_3$ | F | Cl | $CH_3$ | 0 | H | $CO_2(CH_2)_2OCH_2CH_3$ |
| 3-$CH_3$ | H | F | H | 0 | 4-$CH_3$ | $CO_2CH_3$ |
| 4-$CH_3$ | H | F | H | 0 | 4-$CH_3$ | $CO_2CH_3$ |
| 3-$CH_3$ | F | F | H | 0 | 4-$CH_3$ | $CO_2CH_3$ |
| 4-$CH_3$ | F | F | H | 0 | 4-$CH_3$ | $CO_2CH_3$ |
| 3-$CH_3$ | H | Cl | H | 0 | 4-$CH_3$ | $CO_2CH_3$ |
| 4-$CH_3$ | H | Cl | H | 0 | 4-$CH_3$ | $CO_2CH_3$ |
| 3-$CH_3$ | F | Cl | H | 0 | 4-$CH_3$ | $CO_2CH_3$ |
| 4-$CH_3$ | F | Cl | H | 0 | 4-$CH_3$ | $CO_2CH_3$ |
| 3-$CH_3$ | H | F | $CH_3$ | 0 | 4-$CH_3$ | $CO_2CH_3$ |
| 4-$CH_3$ | H | F | $CH_3$ | 0 | 4-$CH_3$ | $CO_2CH_3$ |
| 3-$CH_3$ | F | F | $CH_3$ | 0 | 4-$CH_3$ | $CO_2CH_3$ |
| 4-$CH_3$ | F | F | $CH_3$ | 0 | 4-$CH_3$ | $CO_2CH_3$ |
| 3-$CH_3$ | H | Cl | $CH_3$ | 0 | 4-$CH_3$ | $CO_2CH_3$ |
| 4-$CH_3$ | H | Cl | $CH_3$ | 0 | 4-$CH_3$ | $CO_2CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 3-$CH_3$ | F | Cl | $CH_3$ | 0 | 4-$CH_3$ | $CO_2CH_3$ |
| 4-$CH_3$ | F | Cl | $CH_3$ | 0 | 4-$CH_3$ | $CO_2CH_3$ |
| 3-$CH_3$ | H | F | H | 0 | 4-$CH_3$ | $CO_2CH_2CH_3$ |
| 4-$CH_3$ | H | F | H | 0 | 4-$CH_3$ | $CO_2CH_2CH_3$ |
| 3-$CH_3$ | F | F | H | 0 | 4-$CH_3$ | $CO_2CH_2CH_3$ |
| 4-$CH_3$ | F | F | H | 0 | 4-$CH_3$ | $CO_2CH_2CH_3$ |
| 3-$CH_3$ | H | Cl | H | 0 | 4-$CH_3$ | $CO_2CH_2CH_3$ |
| 4-$CH_3$ | H | Cl | H | 0 | 4-$CH_3$ | $CO_2CH_2CH_3$ |
| 3-$CH_3$ | F | Cl | H | 0 | 4-$CH_3$ | $CO_2CH_2CH_3$ |
| 4-$CH_3$ | F | Cl | H | 0 | 4-$CH_3$ | $CO_2CH_2CH_3$ |
| 3-$CH_3$ | H | F | $CH_3$ | 0 | 4-$CH_3$ | $CO_2CH_2CH_3$ |
| 4-$CH_3$ | H | F | $CH_3$ | 0 | 4-$CH_3$ | $CO_2CH_2CH_3$ |
| 3-$CH_3$ | F | F | $CH_3$ | 0 | 4-$CH_3$ | $CO_2CH_2CH_3$ |
| 4-$CH_3$ | F | F | $CH_3$ | 0 | 4-$CH_3$ | $CO_2CH_2CH_3$ |
| 3-$CH_3$ | H | Cl | $CH_3$ | 0 | 4-$CH_3$ | $CO_2CH_2CH_3$ |
| 4-$CH_3$ | H | Cl | $CH_3$ | 0 | 4-$CH_3$ | $CO_2CH_2CH_3$ |
| 3-$CH_3$ | F | Cl | $CH_3$ | 0 | 4-$CH_3$ | $CO_2CH_2CH_3$ |
| 4-$CH_3$ | F | Cl | $CH_3$ | 0 | 4-$CH_3$ | $CO_2CH_2CH_3$ |
| 3-$CH_3$ | H | F | H | 0 | 4-$CH_3$ | $CO_2(CH_2)_2CH_3$ |
| 4-$CH_3$ | H | F | H | 0 | 4-$CH_3$ | $CO_2(CH_2)_2CH_3$ |
| 3-$CH_3$ | F | F | H | 0 | 4-$CH_3$ | $CO_2(CH_2)_2CH_3$ |
| 4-$CH_3$ | F | F | H | 0 | 4-$CH_3$ | $CO_2(CH_2)_2CH_3$ |
| 3-$CH_3$ | H | Cl | H | 0 | 4-$CH_3$ | $CO_2(CH_2)_2CH_3$ |
| 4-$CH_3$ | H | Cl | H | 0 | 4-$CH_3$ | $CO_2(CH_2)_2CH_3$ |
| 3-$CH_3$ | F | Cl | H | 0 | 4-$CH_3$ | $CO_2(CH_2)_2CH_3$ |
| 4-$CH_3$ | F | Cl | H | 0 | 4-$CH_3$ | $CO_2(CH_2)_2CH_3$ |
| 3-$CH_3$ | H | F | $CH_3$ | 0 | 4-$CH_3$ | $CO_2(CH_2)_2CH_3$ |
| 4-$CH_3$ | H | F | $CH_3$ | 0 | 4-$CH_3$ | $CO_2(CH_2)_2CH_3$ |
| 3-$CH_3$ | F | F | $CH_3$ | 0 | 4-$CH_3$ | $CO_2(CH_2)_2CH_3$ |
| 4-$CH_3$ | F | F | $CH_3$ | 0 | 4-$CH_3$ | $CO_2(CH_2)_2CH_3$ |
| 3-$CH_3$ | H | Cl | $CH_3$ | 0 | 4-$CH_3$ | $CO_2(CH_2)_2CH_3$ |
| 4-$CH_3$ | H | Cl | $CH_3$ | 0 | 4-$CH_3$ | $CO_2(CH_2)_2CH_3$ |
| 3-$CH_3$ | F | Cl | $CH_3$ | 0 | 4-$CH_3$ | $CO_2(CH_2)_2CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 4-$CH_3$ | F | Cl | $CH_3$ | 0 | 4-$CH_3$ | $CO_2(CH_2)_2CH_3$ |
| 3-$CH_3$ | H | F | H | 0 | 4-$CH_3$ | $CO_2CH(CH_3)_2$ |
| 4-$CH_3$ | H | F | H | 0 | 4-$CH_3$ | $CO_2CH(CH_3)_2$ |
| 3-$CH_3$ | F | F | H | 0 | 4-$CH_3$ | $CO_2CH(CH_3)_2$ |
| 4-$CH_3$ | F | F | H | 0 | 4-$CH_3$ | $CO_2CH(CH_3)_2$ |
| 3-$CH_3$ | H | Cl | H | 0 | 4-$CH_3$ | $CO_2CH(CH_3)_2$ |
| 4-$CH_3$ | H | Cl | H | 0 | 4-$CH_3$ | $CO_2CH(CH_3)_2$ |
| 3-$CH_3$ | F | Cl | H | 0 | 4-$CH_3$ | $CO_2CH(CH_3)_2$ |
| 4-$CH_3$ | F | Cl | H | 0 | 4-$CH_3$ | $CO_2CH(CH_3)_2$ |
| 3-$CH_3$ | H | F | $CH_3$ | 0 | 4-$CH_3$ | $CO_2CH(CH_3)_2$ |
| 4-$CH_3$ | H | F | $CH_3$ | 0 | 4-$CH_3$ | $CO_2CH(CH_3)_2$ |
| 3-$CH_3$ | F | F | $CH_3$ | 0 | 4-$CH_3$ | $CO_2CH(CH_3)_2$ |
| 4-$CH_3$ | F | F | $CH_3$ | 0 | 4-$CH_3$ | $CO_2CH(CH_3)_2$ |
| 3-$CH_3$ | H | Cl | $CH_3$ | 0 | 4-$CH_3$ | $CO_2CH(CH_3)_2$ |
| 4-$CH_3$ | H | Cl | $CH_3$ | 0 | 4-$CH_3$ | $CO_2CH(CH_3)_2$ |
| 3-$CH_3$ | F | Cl | $CH_3$ | 0 | 4-$CH_3$ | $CO_2CH(CH_3)_2$ |
| 4-$CH_3$ | F | Cl | $CH_3$ | 0 | 4-$CH_3$ | $CO_2CH(CH_3)_2$ |
| 3-$CH_3$ | H | F | H | 0 | 4-$CH_3$ | $CO_2(CH_2)_3CH_3$ |
| 4-$CH_3$ | H | F | H | 0 | 4-$CH_3$ | $CO_2(CH_2)_3CH_3$ |
| 3-$CH_3$ | F | F | H | 0 | 4-$CH_3$ | $CO_2(CH_2)_3CH_3$ |
| 4-$CH_3$ | F | F | H | 0 | 4-$CH_3$ | $CO_2(CH_2)_3CH_3$ |
| 3-$CH_3$ | H | Cl | H | 0 | 4-$CH_3$ | $CO_2(CH_2)_3CH_3$ |
| 4-$CH_3$ | H | Cl | H | 0 | 4-$CH_3$ | $CO_2(CH_2)_3CH_3$ |
| 3-$CH_3$ | F | Cl | H | 0 | 4-$CH_3$ | $CO_2(CH_2)_3CH_3$ |
| 4-$CH_3$ | F | Cl | H | 0 | 4-$CH_3$ | $CO_2(CH_2)_3CH_3$ |
| 3-$CH_3$ | H | F | $CH_3$ | 0 | 4-$CH_3$ | $CO_2(CH_2)_3CH_3$ |
| 4-$CH_3$ | H | F | $CH_3$ | 0 | 4-$CH_3$ | $CO_2(CH_2)_3CH_3$ |
| 3-$CH_3$ | F | F | $CH_3$ | 0 | 4-$CH_3$ | $CO_2(CH_2)_3CH_3$ |
| 4-$CH_3$ | F | F | $CH_3$ | 0 | 4-$CH_3$ | $CO_2(CH_2)_3CH_3$ |
| 3-$CH_3$ | H | Cl | $CH_3$ | 0 | 4-$CH_3$ | $CO_2(CH_2)_3CH_3$ |
| 4-$CH_3$ | H | Cl | $CH_3$ | 0 | 4-$CH_3$ | $CO_2(CH_2)_3CH_3$ |
| 3-$CH_3$ | F | Cl | $CH_3$ | 0 | 4-$CH_3$ | $CO_2(CH_2)_3CH_3$ |
| 4-$CH_3$ | F | Cl | $CH_3$ | 0 | 4-$CH_3$ | $CO_2(CH_2)_3CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 4-$CH_3$ | $CO_2(CH_2)_2OCH_3$ |
| 4-$CH_3$ | H | F | H | 0 | 4-$CH_3$ | $CO_2(CH_2)_2OCH_3$ |
| 3-$CH_3$ | F | F | H | 0 | 4-$CH_3$ | $CO_2(CH_2)_2OCH_3$ |
| 4-$CH_3$ | F | F | H | 0 | 4-$CH_3$ | $CO_2(CH_2)_2OCH_3$ |
| 3-$CH_3$ | H | Cl | H | 0 | 4-$CH_3$ | $CO_2(CH_2)_2OCH_3$ |
| 4-$CH_3$ | H | Cl | H | 0 | 4-$CH_3$ | $CO_2(CH_2)_2OCH_3$ |
| 3-$CH_3$ | F | Cl | H | 0 | 4-$CH_3$ | $CO_2(CH_2)_2OCH_3$ |
| 4-$CH_3$ | F | Cl | H | 0 | 4-$CH_3$ | $CO_2(CH_2)_2OCH_3$ |
| 3-$CH_3$ | H | F | $CH_3$ | 0 | 4-$CH_3$ | $CO_2(CH_2)_2OCH_3$ |
| 4-$CH_3$ | H | F | $CH_3$ | 0 | 4-$CH_3$ | $CO_2(CH_2)_2OCH_3$ |
| 3-$CH_3$ | F | F | $CH_3$ | 0 | 4-$CH_3$ | $CO_2(CH_2)_2OCH_3$ |
| 4-$CH_3$ | F | F | $CH_3$ | 0 | 4-$CH_3$ | $CO_2(CH_2)_2OCH_3$ |
| 3-$CH_3$ | H | Cl | $CH_3$ | 0 | 4-$CH_3$ | $CO_2(CH_2)_2OCH_3$ |
| 4-$CH_3$ | H | Cl | $CH_3$ | 0 | 4-$CH_3$ | $CO_2(CH_2)_2OCH_3$ |
| 3-$CH_3$ | F | Cl | $CH_3$ | 0 | 4-$CH_3$ | $CO_2(CH_2)_2OCH_3$ |
| 4-$CH_3$ | F | Cl | $CH_3$ | 0 | 4-$CH_3$ | $CO_2(CH_2)_2OCH_3$ |
| 3-$CH_3$ | H | F | H | 0 | 4-$CH_3$ | $CO_2(CH_2)_2OCH_2CH_3$ |
| 4-$CH_3$ | H | F | H | 0 | 4-$CH_3$ | $CO_2(CH_2)_2OCH_2CH_3$ |
| 3-$CH_3$ | F | F | H | 0 | 4-$CH_3$ | $CO_2(CH_2)_2OCH_2CH_3$ |
| 4-$CH_3$ | F | F | H | 0 | 4-$CH_3$ | $CO_2(CH_2)_2OCH_2CH_3$ |
| 3-$CH_3$ | H | Cl | H | 0 | 4-$CH_3$ | $CO_2(CH_2)_2OCH_2CH_3$ |
| 4-$CH_3$ | H | Cl | H | 0 | 4-$CH_3$ | $CO_2(CH_2)_2OCH_2CH_3$ |
| 3-$CH_3$ | F | Cl | H | 0 | 4-$CH_3$ | $CO_2(CH_2)_2OCH_2CH_3$ |
| 4-$CH_3$ | F | Cl | H | 0 | 4-$CH_3$ | $CO_2(CH_2)_2OCH_2CH_3$ |
| 3-$CH_3$ | H | F | $CH_3$ | 0 | 4-$CH_3$ | $CO_2(CH_2)_2OCH_2CH_3$ |
| 4-$CH_3$ | H | F | $CH_3$ | 0 | 4-$CH_3$ | $CO_2(CH_2)_2OCH_2CH_3$ |
| 3-$CH_3$ | F | F | $CH_3$ | 0 | 4-$CH_3$ | $CO_2(CH_2)_2OCH_2CH_3$ |
| 4-$CH_3$ | F | F | $CH_3$ | 0 | 4-$CH_3$ | $CO_2(CH_2)_2OCH_2CH_3$ |
| 3-$CH_3$ | H | Cl | $CH_3$ | 0 | 4-$CH_3$ | $CO_2(CH_2)_2OCH_2CH_3$ |
| 4-$CH_3$ | H | Cl | $CH_3$ | 0 | 4-$CH_3$ | $CO_2(CH_2)_2OCH_2CH_3$ |
| 3-$CH_3$ | F | Cl | $CH_3$ | 0 | 4-$CH_3$ | $CO_2(CH_2)_2OCH_2CH_3$ |
| 4-$CH_3$ | F | Cl | $CH_3$ | 0 | 4-$CH_3$ | $CO_2(CH_2)_2OCH_2CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 5-$CH_3$ | $CO_2CH_3$ |
| 4-$CH_3$ | H | F | H | 0 | 5-$CH_3$ | $CO_2CH_3$ |
| 3-$CH_3$ | F | F | H | 0 | 5-$CH_3$ | $CO_2CH_3$ |
| 4-$CH_3$ | F | F | H | 0 | 5-$CH_3$ | $CO_2CH_3$ |
| 3-$CH_3$ | H | Cl | H | 0 | 5-$CH_3$ | $CO_2CH_3$ |
| 4-$CH_3$ | H | Cl | H | 0 | 5-$CH_3$ | $CO_2CH_3$ |
| 3-$CH_3$ | F | Cl | H | 0 | 5-$CH_3$ | $CO_2CH_3$ |
| 4-$CH_3$ | F | Cl | H | 0 | 5-$CH_3$ | $CO_2CH_3$ |
| 3-$CH_3$ | H | F | $CH_3$ | 0 | 5-$CH_3$ | $CO_2CH_3$ |
| 4-$CH_3$ | H | F | $CH_3$ | 0 | 5-$CH_3$ | $CO_2CH_3$ |
| 3-$CH_3$ | F | F | $CH_3$ | 0 | 5-$CH_3$ | $CO_2CH_3$ |
| 4-$CH_3$ | F | F | $CH_3$ | 0 | 5-$CH_3$ | $CO_2CH_3$ |
| 3-$CH_3$ | H | Cl | $CH_3$ | 0 | 5-$CH_3$ | $CO_2CH_3$ |
| 4-$CH_3$ | H | Cl | $CH_3$ | 0 | 5-$CH_3$ | $CO_2CH_3$ |
| 3-$CH_3$ | F | Cl | $CH_3$ | 0 | 5-$CH_3$ | $CO_2CH_3$ |
| 4-$CH_3$ | F | Cl | $CH_3$ | 0 | 5-$CH_3$ | $CO_2CH_3$ |
| 3-$CH_3$ | H | F | H | 0 | 5-$CH_3$ | $CO_2CH_2CH_3$ |
| 4-$CH_3$ | H | F | H | 0 | 5-$CH_3$ | $CO_2CH_2CH_3$ |
| 3-$CH_3$ | F | F | H | 0 | 5-$CH_3$ | $CO_2CH_2CH_3$ |
| 4-$CH_3$ | F | F | H | 0 | 5-$CH_3$ | $CO_2CH_2CH_3$ |
| 3-$CH_3$ | H | Cl | H | 0 | 5-$CH_3$ | $CO_2CH_2CH_3$ |
| 4-$CH_3$ | H | Cl | H | 0 | 5-$CH_3$ | $CO_2CH_2CH_3$ |
| 3-$CH_3$ | F | Cl | H | 0 | 5-$CH_3$ | $CO_2CH_2CH_3$ |
| 4-$CH_3$ | F | Cl | H | 0 | 5-$CH_3$ | $CO_2CH_2CH_3$ |
| 3-$CH_3$ | H | F | $CH_3$ | 0 | 5-$CH_3$ | $CO_2CH_2CH_3$ |
| 4-$CH_3$ | H | F | $CH_3$ | 0 | 5-$CH_3$ | $CO_2CH_2CH_3$ |
| 3-$CH_3$ | F | F | $CH_3$ | 0 | 5-$CH_3$ | $CO_2CH_2CH_3$ |
| 4-$CH_3$ | F | F | $CH_3$ | 0 | 5-$CH_3$ | $CO_2CH_2CH_3$ |
| 3-$CH_3$ | H | Cl | $CH_3$ | 0 | 5-$CH_3$ | $CO_2CH_2CH_3$ |
| 4-$CH_3$ | H | Cl | $CH_3$ | 0 | 5-$CH_3$ | $CO_2CH_2CH_3$ |
| 3-$CH_3$ | F | Cl | $CH_3$ | 0 | 5-$CH_3$ | $CO_2CH_2CH_3$ |
| 4-$CH_3$ | F | Cl | $CH_3$ | 0 | 5-$CH_3$ | $CO_2CH_2CH_3$ |
| 3-$CH_3$ | H | F | H | 0 | 5-$CH_3$ | $CO_2(CH_2)_2CH_3$ |
| 4-$CH_3$ | H | F | H | 0 | 5-$CH_3$ | $CO_2(CH_2)_2CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| $3-CH_3$ | F | F | H | 0 | $5-CH_3$ | $CO_2(CH_2)_2CH_3$ |
| $4-CH_3$ | F | F | H | 0 | $5-CH_3$ | $CO_2(CH_2)_2CH_3$ |
| $3-CH_3$ | H | Cl | H | 0 | $5-CH_3$ | $CO_2(CH_2)_2CH_3$ |
| $4-CH_3$ | H | Cl | H | 0 | $5-CH_3$ | $CO_2(CH_2)_2CH_3$ |
| $3-CH_3$ | F | Cl | H | 0 | $5-CH_3$ | $CO_2(CH_2)_2CH_3$ |
| $4-CH_3$ | F | Cl | H | 0 | $5-CH_3$ | $CO_2(CH_2)_2CH_3$ |
| $3-CH_3$ | H | F | $CH_3$ | 0 | $5-CH_3$ | $CO_2(CH_2)_2CH_3$ |
| $4-CH_3$ | H | F | $CH_3$ | 0 | $5-CH_3$ | $CO_2(CH_2)_2CH_3$ |
| $3-CH_3$ | F | F | $CH_3$ | 0 | $5-CH_3$ | $CO_2(CH_2)_2CH_3$ |
| $4-CH_3$ | F | F | $CH_3$ | 0 | $5-CH_3$ | $CO_2(CH_2)_2CH_3$ |
| $3-CH_3$ | H | Cl | $CH_3$ | 0 | $5-CH_3$ | $CO_2(CH_2)_2CH_3$ |
| $4-CH_3$ | H | Cl | $CH_3$ | 0 | $5-CH_3$ | $CO_2(CH_2)_2CH_3$ |
| $3-CH_3$ | F | Cl | $CH_3$ | 0 | $5-CH_3$ | $CO_2(CH_2)_2CH_3$ |
| $4-CH_3$ | F | Cl | $CH_3$ | 0 | $5-CH_3$ | $CO_2(CH_2)_2CH_3$ |
| $3-CH_3$ | H | F | H | 0 | $5-CH_3$ | $CO_2CH(CH_3)_2$ |
| $4-CH_3$ | H | F | H | 0 | $5-CH_3$ | $CO_2CH(CH_3)_2$ |
| $3-CH_3$ | F | F | H | 0 | $5-CH_3$ | $CO_2CH(CH_3)_2$ |
| $4-CH_3$ | F | F | H | 0 | $5-CH_3$ | $CO_2CH(CH_3)_2$ |
| $3-CH_3$ | H | Cl | H | 0 | $5-CH_3$ | $CO_2CH(CH_3)_2$ |
| $4-CH_3$ | H | Cl | H | 0 | $5-CH_3$ | $CO_2CH(CH_3)_2$ |
| $3-CH_3$ | F | Cl | H | 0 | $5-CH_3$ | $CO_2CH(CH_3)_2$ |
| $4-CH_3$ | F | Cl | H | 0 | $5-CH_3$ | $CO_2CH(CH_3)_2$ |
| $3-CH_3$ | H | F | $CH_3$ | 0 | $5-CH_3$ | $CO_2CH(CH_3)_2$ |
| $4-CH_3$ | H | F | $CH_3$ | 0 | $5-CH_3$ | $CO_2CH(CH_3)_2$ |
| $3-CH_3$ | F | F | $CH_3$ | 0 | $5-CH_3$ | $CO_2CH(CH_3)_2$ |
| $4-CH_3$ | F | F | $CH_3$ | 0 | $5-CH_3$ | $CO_2CH(CH_3)_2$ |
| $3-CH_3$ | H | Cl | $CH_3$ | 0 | $5-CH_3$ | $CO_2CH(CH_3)_2$ |
| $4-CH_3$ | H | Cl | $CH_3$ | 0 | $5-CH_3$ | $CO_2CH(CH_3)_2$ |
| $3-CH_3$ | F | Cl | $CH_3$ | 0 | $5-CH_3$ | $CO_2CH(CH_3)_2$ |
| $4-CH_3$ | F | Cl | $CH_3$ | 0 | $5-CH_3$ | $CO_2CH(CH_3)_2$ |
| $3-CH_3$ | H | F | H | 0 | $5-CH_3$ | $CO_2(CH_2)_3CH_3$ |
| $4-CH_3$ | H | F | H | 0 | $5-CH_3$ | $CO_2(CH_2)_3CH_3$ |
| $3-CH_3$ | F | F | H | 0 | $5-CH_3$ | $CO_2(CH_2)_3CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 4-$CH_3$ | F | F | H | 0 | 5-$CH_3$ | $CO_2(CH_2)_3CH_3$ |
| 3-$CH_3$ | H | Cl | H | 0 | 5-$CH_3$ | $CO_2(CH_2)_3CH_3$ |
| 4-$CH_3$ | H | Cl | H | 0 | 5-$CH_3$ | $CO_2(CH_2)_3CH_3$ |
| 3-$CH_3$ | F | Cl | H | 0 | 5-$CH_3$ | $CO_2(CH_2)_3CH_3$ |
| 4-$CH_3$ | F | Cl | H | 0 | 5-$CH_3$ | $CO_2(CH_2)_3CH_3$ |
| 3-$CH_3$ | H | F | $CH_3$ | 0 | 5-$CH_3$ | $CO_2(CH_2)_3CH_3$ |
| 4-$CH_3$ | H | F | $CH_3$ | 0 | 5-$CH_3$ | $CO_2(CH_2)_3CH_3$ |
| 3-$CH_3$ | F | F | $CH_3$ | 0 | 5-$CH_3$ | $CO_2(CH_2)_3CH_3$ |
| 4-$CH_3$ | F | F | $CH_3$ | 0 | 5-$CH_3$ | $CO_2(CH_2)_3CH_3$ |
| 3-$CH_3$ | H | Cl | $CH_3$ | 0 | 5-$CH_3$ | $CO_2(CH_2)_3CH_3$ |
| 4-$CH_3$ | H | Cl | $CH_3$ | 0 | 5-$CH_3$ | $CO_2(CH_2)_3CH_3$ |
| 3-$CH_3$ | F | Cl | $CH_3$ | 0 | 5-$CH_3$ | $CO_2(CH_2)_3CH_3$ |
| 4-$CH_3$ | F | Cl | $CH_3$ | 0 | 5-$CH_3$ | $CO_2(CH_2)_3CH_3$ |
| 3-$CH_3$ | H | F | H | 0 | 5-$CH_3$ | $CO_2(CH_2)_2OCH_3$ |
| 4-$CH_3$ | H | F | H | 0 | 5-$CH_3$ | $CO_2(CH_2)_2OCH_3$ |
| 3-$CH_3$ | F | F | H | 0 | 5-$CH_3$ | $CO_2(CH_2)_2OCH_3$ |
| 4-$CH_3$ | F | F | H | 0 | 5-$CH_3$ | $CO_2(CH_2)_2OCH_3$ |
| 3-$CH_3$ | H | Cl | H | 0 | 5-$CH_3$ | $CO_2(CH_2)_2OCH_3$ |
| 4-$CH_3$ | H | Cl | H | 0 | 5-$CH_3$ | $CO_2(CH_2)_2OCH_3$ |
| 3-$CH_3$ | F | Cl | H | 0 | 5-$CH_3$ | $CO_2(CH_2)_2OCH_3$ |
| 4-$CH_3$ | F | Cl | H | 0 | 5-$CH_3$ | $CO_2(CH_2)_2OCH_3$ |
| 3-$CH_3$ | H | F | $CH_3$ | 0 | 5-$CH_3$ | $CO_2(CH_2)_2OCH_3$ |
| 4-$CH_3$ | H | F | $CH_3$ | 0 | 5-$CH_3$ | $CO_2(CH_2)_2OCH_3$ |
| 3-$CH_3$ | F | F | $CH_3$ | 0 | 5-$CH_3$ | $CO_2(CH_2)_2OCH_3$ |
| 4-$CH_3$ | F | F | $CH_3$ | 0 | 5-$CH_3$ | $CO_2(CH_2)_2OCH_3$ |
| 3-$CH_3$ | H | Cl | $CH_3$ | 0 | 5-$CH_3$ | $CO_2(CH_2)_2OCH_3$ |
| 4-$CH_3$ | H | Cl | $CH_3$ | 0 | 5-$CH_3$ | $CO_2(CH_2)_2OCH_3$ |
| 3-$CH_3$ | F | Cl | $CH_3$ | 0 | 5-$CH_3$ | $CO_2(CH_2)_2OCH_3$ |
| 4-$CH_3$ | F | Cl | $CH_3$ | 0 | 5-$CH_3$ | $CO_2(CH_2)_2OCH_3$ |
| 3-$CH_3$ | H | F | H | 0 | 5-$CH_3$ | $CO_2(CH_2)_2OCH_2CH_3$ |
| 4-$CH_3$ | H | F | H | 0 | 5-$CH_3$ | $CO_2(CH_2)_2OCH_2CH_3$ |
| 3-$CH_3$ | F | F | H | 0 | 5-$CH_3$ | $CO_2(CH_2)_2OCH_2CH_3$ |
| 4-$CH_3$ | F | F | H | 0 | 5-$CH_3$ | $CO_2(CH_2)_2OCH_2CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | Cl | H | 0 | 5-$CH_3$ | $CO_2(CH_2)_2OCH_2CH_3$ |
| 4-$CH_3$ | H | Cl | H | 0 | 5-$CH_3$ | $CO_2(CH_2)_2OCH_2CH_3$ |
| 3-$CH_3$ | F | Cl | H | 0 | 5-$CH_3$ | $CO_2(CH_2)_2OCH_2CH_3$ |
| 4-$CH_3$ | F | Cl | H | 0 | 5-$CH_3$ | $CO_2(CH_2)_2OCH_2CH_3$ |
| 3-$CH_3$ | H | F | $CH_3$ | 0 | 5-$CH_3$ | $CO_2(CH_2)_2OCH_2CH_3$ |
| 4-$CH_3$ | H | F | $CH_3$ | 0 | 5-$CH_3$ | $CO_2(CH_2)_2OCH_2CH_3$ |
| 3-$CH_3$ | F | F | $CH_3$ | 0 | 5-$CH_3$ | $CO_2(CH_2)_2OCH_2CH_3$ |
| 4-$CH_3$ | F | F | $CH_3$ | 0 | 5-$CH_3$ | $CO_2(CH_2)_2OCH_2CH_3$ |
| 3-$CH_3$ | H | Cl | $CH_3$ | 0 | 5-$CH_3$ | $CO_2(CH_2)_2OCH_2CH_3$ |
| 4-$CH_3$ | H | Cl | $CH_3$ | 0 | 5-$CH_3$ | $CO_2(CH_2)_2OCH_2CH_3$ |
| 3-$CH_3$ | F | Cl | $CH_3$ | 0 | 5-$CH_3$ | $CO_2(CH_2)_2OCH_2CH_3$ |
| 4-$CH_3$ | F | Cl | $CH_3$ | 0 | 5-$CH_3$ | $CO_2(CH_2)_2OCH_2CH_3$ |
| 3-$CH_3$ | H | F | H | 0 | 4,5-Di-$CH_3$ | $CO_2CH_3$ |
| 4-$CH_3$ | H | F | H | 0 | 4,5-Di-$CH_3$ | $CO_2CH_3$ |
| 3-$CH_3$ | F | F | H | 0 | 4,5-Di-$CH_3$ | $CO_2CH_3$ |
| 4-$CH_3$ | F | F | H | 0 | 4,5-Di-$CH_3$ | $CO_2CH_3$ |
| 3-$CH_3$ | H | Cl | H | 0 | 4,5-Di-$CH_3$ | $CO_2CH_3$ |
| 4-$CH_3$ | H | Cl | H | 0 | 4,5-Di-$CH_3$ | $CO_2CH_3$ |
| 3-$CH_3$ | F | Cl | H | 0 | 4,5-Di-$CH_3$ | $CO_2CH_3$ |
| 4-$CH_3$ | F | Cl | H | 0 | 4,5-Di-$CH_3$ | $CO_2CH_3$ |
| 3-$CH_3$ | H | F | $CH_3$ | 0 | 4,5-Di-$CH_3$ | $CO_2CH_3$ |
| 4-$CH_3$ | H | F | $CH_3$ | 0 | 4,5-Di-$CH_3$ | $CO_2CH_3$ |
| 3-$CH_3$ | F | F | $CH_3$ | 0 | 4,5-Di-$CH_3$ | $CO_2CH_3$ |
| 4-$CH_3$ | F | F | $CH_3$ | 0 | 4,5-Di-$CH_3$ | $CO_2CH_3$ |
| 3-$CH_3$ | H | Cl | $CH_3$ | 0 | 4,5-Di-$CH_3$ | $CO_2CH_3$ |
| 4-$CH_3$ | H | Cl | $CH_3$ | 0 | 4,5-Di-$CH_3$ | $CO_2CH_3$ |
| 3-$CH_3$ | F | Cl | $CH_3$ | 0 | 4,5-Di-$CH_3$ | $CO_2CH_3$ |
| 4-$CH_3$ | F | Cl | $CH_3$ | 0 | 4,5-Di-$CH_3$ | $CO_2CH_3$ |
| 3-$CH_3$ | H | F | H | 0 | 4,5-Di-$CH_3$ | $CO_2CH_2CH_3$ |
| 4-$CH_3$ | H | F | H | 0 | 4,5-Di-$CH_3$ | $CO_2CH_2CH_3$ |
| 3-$CH_3$ | F | F | H | 0 | 4,5-Di-$CH_3$ | $CO_2CH_2CH_3$ |
| 4-$CH_3$ | F | F | H | 0 | 4,5-Di-$CH_3$ | $CO_2CH_2CH_3$ |
| 3-$CH_3$ | H | Cl | H | 0 | 4,5-Di-$CH_3$ | $CO_2CH_2CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 4-$CH_3$ | H | Cl | H | 0 | 4,5-Di-$CH_3$ | $CO_2CH_2CH_3$ |
| 3-$CH_3$ | F | Cl | H | 0 | 4,5-Di-$CH_3$ | $CO_2CH_2CH_3$ |
| 4-$CH_3$ | F | Cl | H | 0 | 4,5-Di-$CH_3$ | $CO_2CH_2CH_3$ |
| 3-$CH_3$ | H | F | $CH_3$ | 0 | 4,5-Di-$CH_3$ | $CO_2CH_2CH_3$ |
| 4-$CH_3$ | H | F | $CH_3$ | 0 | 4,5-Di-$CH_3$ | $CO_2CH_2CH_3$ |
| 3-$CH_3$ | F | F | $CH_3$ | 0 | 4,5-Di-$CH_3$ | $CO_2CH_2CH_3$ |
| 4-$CH_3$ | F | F | $CH_3$ | 0 | 4,5-Di-$CH_3$ | $CO_2CH_2CH_3$ |
| 3-$CH_3$ | H | Cl | $CH_3$ | 0 | 4,5-Di-$CH_3$ | $CO_2CH_2CH_3$ |
| 4-$CH_3$ | H | Cl | $CH_3$ | 0 | 4,5-Di-$CH_3$ | $CO_2CH_2CH_3$ |
| 3-$CH_3$ | F | Cl | $CH_3$ | 0 | 4,5-Di-$CH_3$ | $CO_2CH_2CH_3$ |
| 4-$CH_3$ | F | Cl | $CH_3$ | 0 | 4,5-Di-$CH_3$ | $CO_2CH_2CH_3$ |
| 3-$CH_3$ | H | F | H | 0 | 4,5-Di-$CH_3$ | $CO_2(CH_2)_2CH_3$ |
| 4-$CH_3$ | H | F | H | 0 | 4,5-Di-$CH_3$ | $CO_2(CH_2)_2CH_3$ |
| 3-$CH_3$ | F | F | H | 0 | 4,5-Di-$CH_3$ | $CO_2(CH_2)_2CH_3$ |
| 4-$CH_3$ | F | F | H | 0 | 4,5-Di-$CH_3$ | $CO_2(CH_2)_2CH_3$ |
| 3-$CH_3$ | H | Cl | H | 0 | 4,5-Di-$CH_3$ | $CO_2(CH_2)_2CH_3$ |
| 4-$CH_3$ | H | Cl | H | 0 | 4,5-Di-$CH_3$ | $CO_2(CH_2)_2CH_3$ |
| 3-$CH_3$ | F | Cl | H | 0 | 4,5-Di-$CH_3$ | $CO_2(CH_2)_2CH_3$ |
| 4-$CH_3$ | F | Cl | H | 0 | 4,5-Di-$CH_3$ | $CO_2(CH_2)_2CH_3$ |
| 3-$CH_3$ | H | F | $CH_3$ | 0 | 4,5-Di-$CH_3$ | $CO_2(CH_2)_2CH_3$ |
| 4-$CH_3$ | H | F | $CH_3$ | 0 | 4,5-Di-$CH_3$ | $CO_2(CH_2)_2CH_3$ |
| 3-$CH_3$ | F | F | $CH_3$ | 0 | 4,5-Di-$CH_3$ | $CO_2(CH_2)_2CH_3$ |
| 4-$CH_3$ | F | F | $CH_3$ | 0 | 4,5-Di-$CH_3$ | $CO_2(CH_2)_2CH_3$ |
| 3-$CH_3$ | H | Cl | $CH_3$ | 0 | 4,5-Di-$CH_3$ | $CO_2(CH_2)_2CH_3$ |
| 4-$CH_3$ | H | Cl | $CH_3$ | 0 | 4,5-Di-$CH_3$ | $CO_2(CH_2)_2CH_3$ |
| 3-$CH_3$ | F | Cl | $CH_3$ | 0 | 4,5-Di-$CH_3$ | $CO_2(CH_2)_2CH_3$ |
| 4-$CH_3$ | F | Cl | $CH_3$ | 0 | 4,5-Di-$CH_3$ | $CO_2(CH_2)_2CH_3$ |
| 3-$CH_3$ | H | F | H | 0 | 4,5-Di-$CH_3$ | $CO_2CH(CH_3)_2$ |
| 4-$CH_3$ | H | F | H | 0 | 4,5-Di-$CH_3$ | $CO_2CH(CH_3)_2$ |
| 3-$CH_3$ | F | F | H | 0 | 4,5-Di-$CH_3$ | $CO_2CH(CH_3)_2$ |
| 4-$CH_3$ | F | F | H | 0 | 4,5-Di-$CH_3$ | $CO_2CH(CH_3)_2$ |
| 3-$CH_3$ | H | Cl | H | 0 | 4,5-Di-$CH_3$ | $CO_2CH(CH_3)_2$ |
| 4-$CH_3$ | H | Cl | H | 0 | 4,5-Di-$CH_3$ | $CO_2CH(CH_3)_2$ |

Tabelle A (Fortsetzung)

| $R_n$ | $R^1$ | $R^2$ | $R^3$ | m | $(R^a)_q$ | $R^4$ |
|---|---|---|---|---|---|---|
| $3-CH_3$ | F | Cl | H | 0 | $4,5-Di-CH_3$ | $CO_2CH(CH_3)_2$ |
| $4-CH_3$ | F | Cl | H | 0 | $4,5-Di-CH_3$ | $CO_2CH(CH_3)_2$ |
| $3-CH_3$ | H | F | $CH_3$ | 0 | $4,5-Di-CH_3$ | $CO_2CH(CH_3)_2$ |
| $4-CH_3$ | H | F | $CH_3$ | 0 | $4,5-Di-CH_3$ | $CO_2CH(CH_3)_2$ |
| $3-CH_3$ | F | F | $CH_3$ | 0 | $4,5-Di-CH_3$ | $CO_2CH(CH_3)_2$ |
| $4-CH_3$ | F | F | $CH_3$ | 0 | $4,5-Di-CH_3$ | $CO_2CH(CH_3)_2$ |
| $3-CH_3$ | H | Cl | $CH_3$ | 0 | $4,5-Di-CH_3$ | $CO_2CH(CH_3)_2$ |
| $4-CH_3$ | H | Cl | $CH_3$ | 0 | $4,5-Di-CH_3$ | $CO_2CH(CH_3)_2$ |
| $3-CH_3$ | F | Cl | $CH_3$ | 0 | $4,5-Di-CH_3$ | $CO_2CH(CH_3)_2$ |
| $4-CH_3$ | F | Cl | $CH_3$ | 0 | $4,5-Di-CH_3$ | $CO_2CH(CH_3)_2$ |
| $3-CH_3$ | H | F | H | 0 | $4,5-Di-CH_3$ | $CO_2(CH_2)_3CH_3$ |
| $4-CH_3$ | H | F | H | 0 | $4,5-Di-CH_3$ | $CO_2(CH_2)_3CH_3$ |
| $3-CH_3$ | F | F | H | 0 | $4,5-Di-CH_3$ | $CO_2(CH_2)_3CH_3$ |
| $4-CH_3$ | F | F | H | 0 | $4,5-Di-CH_3$ | $CO_2(CH_2)_3CH_3$ |
| $3-CH_3$ | H | Cl | H | 0 | $4,5-Di-CH_3$ | $CO_2(CH_2)_3CH_3$ |
| $4-CH_3$ | H | Cl | H | 0 | $4,5-Di-CH_3$ | $CO_2(CH_2)_3CH_3$ |
| $3-CH_3$ | F | Cl | H | 0 | $4,5-Di-CH_3$ | $CO_2(CH_2)_3CH_3$ |
| $4-CH_3$ | F | Cl | H | 0 | $4,5-Di-CH_3$ | $CO_2(CH_2)_3CH_3$ |
| $3-CH_3$ | H | F | $CH_3$ | 0 | $4,5-Di-CH_3$ | $CO_2(CH_2)_3CH_3$ |
| $4-CH_3$ | H | F | $CH_3$ | 0 | $4,5-Di-CH_3$ | $CO_2(CH_2)_3CH_3$ |
| $3-CH_3$ | F | F | $CH_3$ | 0 | $4,5-Di-CH_3$ | $CO_2(CH_2)_3CH_3$ |
| $4-CH_3$ | F | F | $CH_3$ | 0 | $4,5-Di-CH_3$ | $CO_2(CH_2)_3CH_3$ |
| $3-CH_3$ | H | Cl | $CH_3$ | 0 | $4,5-Di-CH_3$ | $CO_2(CH_2)_3CH_3$ |
| $4-CH_3$ | H | Cl | $CH_3$ | 0 | $4,5-Di-CH_3$ | $CO_2(CH_2)_3CH_3$ |
| $3-CH_3$ | F | Cl | $CH_3$ | 0 | $4,5-Di-CH_3$ | $CO_2(CH_2)_3CH_3$ |
| $4-CH_3$ | F | Cl | $CH_3$ | 0 | $4,5-Di-CH_3$ | $CO_2(CH_2)_3CH_3$ |
| $3-CH_3$ | H | F | H | 0 | $4,5-Di-CH_3$ | $CO_2(CH_2)_2OCH_3$ |
| $4-CH_3$ | H | F | H | 0 | $4,5-Di-CH_3$ | $CO_2(CH_2)_2OCH_3$ |
| $3-CH_3$ | F | F | H | 0 | $4,5-Di-CH_3$ | $CO_2(CH_2)_2OCH_3$ |
| $4-CH_3$ | F | F | H | 0 | $4,5-Di-CH_3$ | $CO_2(CH_2)_2OCH_3$ |
| $3-CH_3$ | H | Cl | H | 0 | $4,5-Di-CH_3$ | $CO_2(CH_2)_2OCH_3$ |
| $4-CH_3$ | H | Cl | H | 0 | $4,5-Di-CH_3$ | $CO_2(CH_2)_2OCH_3$ |
| $3-CH_3$ | F | Cl | H | 0 | $4,5-Di-CH_3$ | $CO_2(CH_2)_2OCH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 4-$CH_3$ | F | Cl | H | 0 | 4,5-Di-$CH_3$ | $CO_2(CH_2)_2OCH_3$ |
| 3-$CH_3$ | H | F | $CH_3$ | 0 | 4,5-Di-$CH_3$ | $CO_2(CH_2)_2OCH_3$ |
| 4-$CH_3$ | H | F | $CH_3$ | 0 | 4,5-Di-$CH_3$ | $CO_2(CH_2)_2OCH_3$ |
| 3-$CH_3$ | F | F | $CH_3$ | 0 | 4,5-Di-$CH_3$ | $CO_2(CH_2)_2OCH_3$ |
| 4-$CH_3$ | F | F | $CH_3$ | 0 | 4,5-Di-$CH_3$ | $CO_2(CH_2)_2OCH_3$ |
| 3-$CH_3$ | H | Cl | $CH_3$ | 0 | 4,5-Di-$CH_3$ | $CO_2(CH_2)_2OCH_3$ |
| 4-$CH_3$ | H | Cl | $CH_3$ | 0 | 4,5-Di-$CH_3$ | $CO_2(CH_2)_2OCH_3$ |
| 3-$CH_3$ | F | Cl | $CH_3$ | 0 | 4,5-Di-$CH_3$ | $CO_2(CH_2)_2OCH_3$ |
| 4-$CH_3$ | F | Cl | $CH_3$ | 0 | 4,5-Di-$CH_3$ | $CO_2(CH_2)_2OCH_3$ |
| 3-$CH_3$ | H | F | H | 0 | 4,5-Di-$CH_3$ | $CO_2(CH_2)_2OCH_2CH_3$ |
| 4-$CH_3$ | H | F | H | 0 | 4,5-Di-$CH_3$ | $CO_2(CH_2)_2OCH_2CH_3$ |
| 3-$CH_3$ | F | F | H | 0 | 4,5-Di-$CH_3$ | $CO_2(CH_2)_2OCH_2CH_3$ |
| 4-$CH_3$ | F | F | H | 0 | 4,5-Di-$CH_3$ | $CO_2(CH_2)_2OCH_2CH_3$ |
| 3-$CH_3$ | H | Cl | H | 0 | 4,5-Di-$CH_3$ | $CO_2(CH_2)_2OCH_2CH_3$ |
| 4-$CH_3$ | H | Cl | H | 0 | 4,5-Di-$CH_3$ | $CO_2(CH_2)_2OCH_2CH_3$ |
| 3-$CH_3$ | F | Cl | H | 0 | 4,5-Di-$CH_3$ | $CO_2(CH_2)_2OCH_2CH_3$ |
| 4-$CH_3$ | F | Cl | H | 0 | 4,5-Di-$CH_3$ | $CO_2(CH_2)_2OCH_2CH_3$ |
| 3-$CH_3$ | H | F | $CH_3$ | 0 | 4,5-Di-$CH_3$ | $CO_2(CH_2)_2OCH_2CH_3$ |
| 4-$CH_3$ | H | F | $CH_3$ | 0 | 4,5-Di-$CH_3$ | $CO_2(CH_2)_2OCH_2CH_3$ |
| 3-$CH_3$ | F | F | $CH_3$ | 0 | 4,5-Di-$CH_3$ | $CO_2(CH_2)_2OCH_2CH_3$ |
| 4-$CH_3$ | F | F | $CH_3$ | 0 | 4,5-Di-$CH_3$ | $CO_2(CH_2)_2OCH_2CH_3$ |
| 3-$CH_3$ | H | Cl | $CH_3$ | 0 | 4,5-Di-$CH_3$ | $CO_2(CH_2)_2OCH_2CH_3$ |
| 4-$CH_3$ | H | Cl | $CH_3$ | 0 | 4,5-Di-$CH_3$ | $CO_2(CH_2)_2OCH_2CH_3$ |
| 3-$CH_3$ | F | Cl | $CH_3$ | 0 | 4,5-Di-$CH_3$ | $CO_2(CH_2)_2OCH_2CH_3$ |
| 4-$CH_3$ | F | Cl | $CH_3$ | 0 | 4,5-Di-$CH_3$ | $CO_2(CH_2)_2OCH_2CH_3$ |
| 3-$CH_3$ | H | F | H | 1 | H | $CO_2CH_3$ |
| 4-$CH_3$ | H | F | H | 1 | H | $CO_2CH_3$ |
| 3-$CH_3$ | F | F | H | 1 | H | $CO_2CH_3$ |
| 4-$CH_3$ | F | F | H | 1 | H | $CO_2CH_3$ |
| 3-$CH_3$ | H | Cl | H | 1 | H | $CO_2CH_3$ |
| 4-$CH_3$ | H | Cl | H | 1 | H | $CO_2CH_3$ |
| 3-$CH_3$ | F | Cl | H | 1 | H | $CO_2CH_3$ |
| 4-$CH_3$ | F | Cl | H | 1 | H | $CO_2CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | $CH_3$ | 1 | H | $CO_2CH_3$ |
| 4-$CH_3$ | H | F | $CH_3$ | 1 | H | $CO_2CH_3$ |
| 3-$CH_3$ | F | F | $CH_3$ | 1 | H | $CO_2CH_3$ |
| 4-$CH_3$ | F | F | $CH_3$ | 1 | H | $CO_2CH_3$ |
| 3-$CH_3$ | H | Cl | $CH_3$ | 1 | H | $CO_2CH_3$ |
| 4-$CH_3$ | H | Cl | $CH_3$ | 1 | H | $CO_2CH_3$ |
| 3-$CH_3$ | F | Cl | $CH_3$ | 1 | H | $CO_2CH_3$ |
| 4-$CH_3$ | F | Cl | $CH_3$ | 1 | H | $CO_2CH_3$ |
| 3-$CH_3$ | H | F | H | 1 | H | $CO_2CH_2CH_3$ |
| 4-$CH_3$ | H | F | H | 1 | H | $CO_2CH_2CH_3$ |
| 3-$CH_3$ | F | F | H | 1 | H | $CO_2CH_2CH_3$ |
| 4-$CH_3$ | F | F | H | 1 | H | $CO_2CH_2CH_3$ |
| 3-$CH_3$ | H | Cl | H | 1 | H | $CO_2CH_2CH_3$ |
| 4-$CH_3$ | H | Cl | H | 1 | H | $CO_2CH_2CH_3$ |
| 3-$CH_3$ | F | Cl | H | 1 | H | $CO_2CH_2CH_3$ |
| 4-$CH_3$ | F | Cl | H | 1 | H | $CO_2CH_2CH_3$ |
| 3-$CH_3$ | H | F | $CH_3$ | 1 | H | $CO_2CH_2CH_3$ |
| 4-$CH_3$ | H | F | $CH_3$ | 1 | H | $CO_2CH_2CH_3$ |
| 3-$CH_3$ | F | F | $CH_3$ | 1 | H | $CO_2CH_2CH_3$ |
| 4-$CH_3$ | F | F | $CH_3$ | 1 | H | $CO_2CH_2CH_3$ |
| 3-$CH_3$ | H | Cl | $CH_3$ | 1 | H | $CO_2CH_2CH_3$ |
| 4-$CH_3$ | H | Cl | $CH_3$ | 1 | H | $CO_2CH_2CH_3$ |
| 3-$CH_3$ | F | Cl | $CH_3$ | 1 | H | $CO_2CH_2CH_3$ |
| 4-$CH_3$ | F | Cl | $CH_3$ | 1 | H | $CO_2CH_2CH_3$ |
| 3-$CH_3$ | H | F | H | 1 | H | $CO_2(CH_2)_2CH_3$ |
| 4-$CH_3$ | H | F | H | 1 | H | $CO_2(CH_2)_2CH_3$ |
| 3-$CH_3$ | F | F | H | 1 | H | $CO_2(CH_2)_2CH_3$ |
| 4-$CH_3$ | F | F | H | 1 | H | $CO_2(CH_2)_2CH_3$ |
| 3-$CH_3$ | H | Cl | H | 1 | H | $CO_2(CH_2)_2CH_3$ |
| 4-$CH_3$ | H | Cl | H | 1 | H | $CO_2(CH_2)_2CH_3$ |
| 3-$CH_3$ | F | Cl | H | 1 | H | $CO_2(CH_2)_2CH_3$ |
| 4-$CH_3$ | F | Cl | H | 1 | H | $CO_2(CH_2)_2CH_3$ |
| 3-$CH_3$ | H | F | $CH_3$ | 1 | H | $CO_2(CH_2)_2CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 4-$CH_3$ | H | F | $CH_3$ | 1 | H | $CO_2(CH_2)_2CH_3$ |
| 3-$CH_3$ | F | F | $CH_3$ | 1 | H | $CO_2(CH_2)_2CH_3$ |
| 4-$CH_3$ | F | F | $CH_3$ | 1 | H | $CO_2(CH_2)_2CH_3$ |
| 3-$CH_3$ | H | Cl | $CH_3$ | 1 | H | $CO_2(CH_2)_2CH_3$ |
| 4-$CH_3$ | H | Cl | $CH_3$ | 1 | H | $CO_2(CH_2)_2CH_3$ |
| 3-$CH_3$ | F | Cl | $CH_3$ | 1 | H | $CO_2(CH_2)_2CH_3$ |
| 4-$CH_3$ | F | Cl | $CH_3$ | 1 | H | $CO_2(CH_2)_2CH_3$ |
| 3-$CH_3$ | H | F | H | 1 | H | $CO_2CH(CH_3)_2$ |
| 4-$CH_3$ | H | F | H | 1 | H | $CO_2CH(CH_3)_2$ |
| 3-$CH_3$ | F | F | H | 1 | H | $CO_2CH(CH_3)_2$ |
| 4-$CH_3$ | F | F | H | 1 | H | $CO_2CH(CH_3)_2$ |
| 3-$CH_3$ | H | Cl | H | 1 | H | $CO_2CH(CH_3)_2$ |
| 4-$CH_3$ | H | Cl | H | 1 | H | $CO_2CH(CH_3)_2$ |
| 3-$CH_3$ | F | Cl | H | 1 | H | $CO_2CH(CH_3)_2$ |
| 4-$CH_3$ | F | Cl | H | 1 | H | $CO_2CH(CH_3)_2$ |
| 3-$CH_3$ | H | F | $CH_3$ | 1 | H | $CO_2CH(CH_3)_2$ |
| 4-$CH_3$ | H | F | $CH_3$ | 1 | H | $CO_2CH(CH_3)_2$ |
| 3-$CH_3$ | F | F | $CH_3$ | 1 | H | $CO_2CH(CH_3)_2$ |
| 4-$CH_3$ | F | F | $CH_3$ | 1 | H | $CO_2CH(CH_3)_2$ |
| 3-$CH_3$ | H | Cl | $CH_3$ | 1 | H | $CO_2CH(CH_3)_2$ |
| 4-$CH_3$ | H | Cl | $CH_3$ | 1 | H | $CO_2CH(CH_3)_2$ |
| 3-$CH_3$ | F | Cl | $CH_3$ | 1 | H | $CO_2CH(CH_3)_2$ |
| 4-$CH_3$ | F | Cl | $CH_3$ | 1 | H | $CO_2CH(CH_3)_2$ |
| 3-$CH_3$ | H | F | H | 1 | H | $CO_2(CH_2)_3CH_3$ |
| 4-$CH_3$ | H | F | H | 1 | H | $CO_2(CH_2)_3CH_3$ |
| 3-$CH_3$ | F | F | H | 1 | H | $CO_2(CH_2)_3CH_3$ |
| 4-$CH_3$ | F | F | H | 1 | H | $CO_2(CH_2)_3CH_3$ |
| 3-$CH_3$ | H | Cl | H | 1 | H | $CO_2(CH_2)_3CH_3$ |
| 4-$CH_3$ | H | Cl | H | 1 | H | $CO_2(CH_2)_3CH_3$ |
| 3-$CH_3$ | F | Cl | H | 1 | H | $CO_2(CH_2)_3CH_3$ |
| 4-$CH_3$ | F | Cl | H | 1 | H | $CO_2(CH_2)_3CH_3$ |
| 3-$CH_3$ | H | F | $CH_3$ | 1 | H | $CO_2(CH_2)_3CH_3$ |
| 4-$CH_3$ | H | F | $CH_3$ | 1 | H | $CO_2(CH_2)_3CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 3-$CH_3$ | F | F | $CH_3$ | 1 | H | $CO_2(CH_2)_3CH_3$ |
| 4-$CH_3$ | F | F | $CH_3$ | 1 | H | $CO_2(CH_2)_3CH_3$ |
| 3-$CH_3$ | H | Cl | $CH_3$ | 1 | H | $CO_2(CH_2)_3CH_3$ |
| 4-$CH_3$ | H | Cl | $CH_3$ | 1 | H | $CO_2(CH_2)_3CH_3$ |
| 3-$CH_3$ | F | Cl | $CH_3$ | 1 | H | $CO_2(CH_2)_3CH_3$ |
| 4-$CH_3$ | F | Cl | $CH_3$ | 1 | H | $CO_2(CH_2)_3CH_3$ |
| 3-$CH_3$ | H | F | H | 1 | H | $CO_2(CH_2)_2OCH_3$ |
| 4-$CH_3$ | H | F | H | 1 | H | $CO_2(CH_2)_2OCH_3$ |
| 3-$CH_3$ | F | F | H | 1 | H | $CO_2(CH_2)_2OCH_3$ |
| 4-$CH_3$ | F | F | H | 1 | H | $CO_2(CH_2)_2OCH_3$ |
| 3-$CH_3$ | H | Cl | H | 1 | H | $CO_2(CH_2)_2OCH_3$ |
| 4-$CH_3$ | H | Cl | H | 1 | H | $CO_2(CH_2)_2OCH_3$ |
| 3-$CH_3$ | F | Cl | H | 1 | H | $CO_2(CH_2)_2OCH_3$ |
| 4-$CH_3$ | F | Cl | H | 1 | H | $CO_2(CH_2)_2OCH_3$ |
| 3-$CH_3$ | H | F | $CH_3$ | 1 | H | $CO_2(CH_2)_2OCH_3$ |
| 4-$CH_3$ | H | F | $CH_3$ | 1 | H | $CO_2(CH_2)_2OCH_3$ |
| 3-$CH_3$ | F | F | $CH_3$ | 1 | H | $CO_2(CH_2)_2OCH_3$ |
| 4-$CH_3$ | F | F | $CH_3$ | 1 | H | $CO_2(CH_2)_2OCH_3$ |
| 3-$CH_3$ | H | Cl | $CH_3$ | 1 | H | $CO_2(CH_2)_2OCH_3$ |
| 4-$CH_3$ | H | Cl | $CH_3$ | 1 | H | $CO_2(CH_2)_2OCH_3$ |
| 3-$CH_3$ | F | Cl | $CH_3$ | 1 | H | $CO_2(CH_2)_2OCH_3$ |
| 4-$CH_3$ | F | Cl | $CH_3$ | 1 | H | $CO_2(CH_2)_2OCH_3$ |
| 3-$CH_3$ | H | F | H | 1 | H | $CO_2(CH_2)_2OCH_2CH_3$ |
| 4-$CH_3$ | H | F | H | 1 | H | $CO_2(CH_2)_2OCH_2CH_3$ |
| 3-$CH_3$ | F | F | H | 1 | H | $CO_2(CH_2)_2OCH_2CH_3$ |
| 4-$CH_3$ | F | F | H | 1 | H | $CO_2(CH_2)_2OCH_2CH_3$ |
| 3-$CH_3$ | H | Cl | H | 1 | H | $CO_2(CH_2)_2OCH_2CH_3$ |
| 4-$CH_3$ | H | Cl | H | 1 | H | $CO_2(CH_2)_2OCH_2CH_3$ |
| 3-$CH_3$ | F | Cl | H | 1 | H | $CO_2(CH_2)_2OCH_2CH_3$ |
| 4-$CH_3$ | F | Cl | H | 1 | H | $CO_2(CH_2)_2OCH_2CH_3$ |
| 3-$CH_3$ | H | F | $CH_3$ | 1 | H | $CO_2(CH_2)_2OCH_2CH_3$ |
| 4-$CH_3$ | H | F | $CH_3$ | 1 | H | $CO_2(CH_2)_2OCH_2CH_3$ |
| 3-$CH_3$ | F | F | $CH_3$ | 1 | H | $CO_2(CH_2)_2OCH_2CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 4-$CH_3$ | F | F | $CH_3$ | 1 | H | $CO_2(CH_2)_2OCH_2CH_3$ |
| 3-$CH_3$ | H | Cl | $CH_3$ | 1 | H | $CO_2(CH_2)_2OCH_2CH_3$ |
| 4-$CH_3$ | H | Cl | $CH_3$ | 1 | H | $CO_2(CH_2)_2OCH_2CH_3$ |
| 3-$CH_3$ | F | Cl | $CH_3$ | 1 | H | $CO_2(CH_2)_2OCH_2CH_3$ |
| 4-$CH_3$ | F | Cl | $CH_3$ | 1 | H | $CO_2(CH_2)_2OCH_2CH_3$ |
| 3-$CH_3$ | H | F | H | 1 | 4-$CH_3$ | $CO_2CH_3$ |
| 4-$CH_3$ | H | F | H | 1 | 4-$CH_3$ | $CO_2CH_3$ |
| 3-$CH_3$ | F | F | H | 1 | 4-$CH_3$ | $CO_2CH_3$ |
| 4-$CH_3$ | F | F | H | 1 | 4-$CH_3$ | $CO_2CH_3$ |
| 3-$CH_3$ | H | Cl | H | 1 | 4-$CH_3$ | $CO_2CH_3$ |
| 4-$CH_3$ | H | Cl | H | 1 | 4-$CH_3$ | $CO_2CH_3$ |
| 3-$CH_3$ | F | Cl | H | 1 | 4-$CH_3$ | $CO_2CH_3$ |
| 4-$CH_3$ | F | Cl | H | 1 | 4-$CH_3$ | $CO_2CH_3$ |
| 3-$CH_3$ | H | F | $CH_3$ | 1 | 4-$CH_3$ | $CO_2CH_3$ |
| 4-$CH_3$ | H | F | $CH_3$ | 1 | 4-$CH_3$ | $CO_2CH_3$ |
| 3-$CH_3$ | F | F | $CH_3$ | 1 | 4-$CH_3$ | $CO_2CH_3$ |
| 4-$CH_3$ | F | F | $CH_3$ | 1 | 4-$CH_3$ | $CO_2CH_3$ |
| 3-$CH_3$ | H | Cl | $CH_3$ | 1 | 4-$CH_3$ | $CO_2CH_3$ |
| 4-$CH_3$ | H | Cl | $CH_3$ | 1 | 4-$CH_3$ | $CO_2CH_3$ |
| 3-$CH_3$ | F | Cl | $CH_3$ | 1 | 4-$CH_3$ | $CO_2CH_3$ |
| 4-$CH_3$ | F | Cl | $CH_3$ | 1 | 4-$CH_3$ | $CO_2CH_3$ |
| 3-$CH_3$ | H | F | H | 1 | 4-$CH_3$ | $CO_2CH_2CH_3$ |
| 4-$CH_3$ | H | F | H | 1 | 4-$CH_3$ | $CO_2CH_2CH_3$ |
| 3-$CH_3$ | F | F | H | 1 | 4-$CH_3$ | $CO_2CH_2CH_3$ |
| 4-$CH_3$ | F | F | H | 1 | 4-$CH_3$ | $CO_2CH_2CH_3$ |
| 3-$CH_3$ | H | Cl | H | 1 | 4-$CH_3$ | $CO_2CH_2CH_3$ |
| 4-$CH_3$ | H | Cl | H | 1 | 4-$CH_3$ | $CO_2CH_2CH_3$ |
| 3-$CH_3$ | F | Cl | H | 1 | 4-$CH_3$ | $CO_2CH_2CH_3$ |
| 4-$CH_3$ | F | Cl | H | 1 | 4-$CH_3$ | $CO_2CH_2CH_3$ |
| 3-$CH_3$ | H | F | $CH_3$ | 1 | 4-$CH_3$ | $CO_2CH_2CH_3$ |
| 4-$CH_3$ | H | F | $CH_3$ | 1 | 4-$CH_3$ | $CO_2CH_2CH_3$ |
| 3-$CH_3$ | F | F | $CH_3$ | 1 | 4-$CH_3$ | $CO_2CH_2CH_3$ |
| 4-$CH_3$ | F | F | $CH_3$ | 1 | 4-$CH_3$ | $CO_2CH_2CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | Cl | $CH_3$ | 1 | 4-$CH_3$ | $CO_2CH_2CH_3$ |
| 4-$CH_3$ | H | Cl | $CH_3$ | 1 | 4-$CH_3$ | $CO_2CH_2CH_3$ |
| 3-$CH_3$ | F | Cl | $CH_3$ | 1 | 4-$CH_3$ | $CO_2CH_2CH_3$ |
| 4-$CH_3$ | F | Cl | $CH_3$ | 1 | 4-$CH_3$ | $CO_2CH_2CH_3$ |
| 3-$CH_3$ | H | F | H | 1 | 4-$CH_3$ | $CO_2(CH_2)_2CH_3$ |
| 4-$CH_3$ | H | F | H | 1 | 4-$CH_3$ | $CO_2(CH_2)_2CH_3$ |
| 3-$CH_3$ | F | F | H | 1 | 4-$CH_3$ | $CO_2(CH_2)_2CH_3$ |
| 4-$CH_3$ | F | F | H | 1 | 4-$CH_3$ | $CO_2(CH_2)_2CH_3$ |
| 3-$CH_3$ | H | Cl | H | 1 | 4-$CH_3$ | $CO_2(CH_2)_2CH_3$ |
| 4-$CH_3$ | H | Cl | H | 1 | 4-$CH_3$ | $CO_2(CH_2)_2CH_3$ |
| 3-$CH_3$ | F | Cl | H | 1 | 4-$CH_3$ | $CO_2(CH_2)_2CH_3$ |
| 4-$CH_3$ | F | Cl | H | 1 | 4-$CH_3$ | $CO_2(CH_2)_2CH_3$ |
| 3-$CH_3$ | H | F | $CH_3$ | 1 | 4-$CH_3$ | $CO_2(CH_2)_2CH_3$ |
| 4-$CH_3$ | H | F | $CH_3$ | 1 | 4-$CH_3$ | $CO_2(CH_2)_2CH_3$ |
| 3-$CH_3$ | F | F | $CH_3$ | 1 | 4-$CH_3$ | $CO_2(CH_2)_2CH_3$ |
| 4-$CH_3$ | F | F | $CH_3$ | 1 | 4-$CH_3$ | $CO_2(CH_2)_2CH_3$ |
| 3-$CH_3$ | H | Cl | $CH_3$ | 1 | 4-$CH_3$ | $CO_2(CH_2)_2CH_3$ |
| 4-$CH_3$ | H | Cl | $CH_3$ | 1 | 4-$CH_3$ | $CO_2(CH_2)_2CH_3$ |
| 3-$CH_3$ | F | Cl | $CH_3$ | 1 | 4-$CH_3$ | $CO_2(CH_2)_2CH_3$ |
| 4-$CH_3$ | F | Cl | $CH_3$ | 1 | 4-$CH_3$ | $CO_2(CH_2)_2CH_3$ |
| 3-$CH_3$ | H | F | H | 1 | 4-$CH_3$ | $CO_2CH(CH_3)_2$ |
| 4-$CH_3$ | H | F | H | 1 | 4-$CH_3$ | $CO_2CH(CH_3)_2$ |
| 3-$CH_3$ | F | F | H | 1 | 4-$CH_3$ | $CO_2CH(CH_3)_2$ |
| 4-$CH_3$ | F | F | H | 1 | 4-$CH_3$ | $CO_2CH(CH_3)_2$ |
| 3-$CH_3$ | H | Cl | H | 1 | 4-$CH_3$ | $CO_2CH(CH_3)_2$ |
| 4-$CH_3$ | H | Cl | H | 1 | 4-$CH_3$ | $CO_2CH(CH_3)_2$ |
| 3-$CH_3$ | F | Cl | H | 1 | 4-$CH_3$ | $CO_2CH(CH_3)_2$ |
| 4-$CH_3$ | F | Cl | H | 1 | 4-$CH_3$ | $CO_2CH(CH_3)_2$ |
| 3-$CH_3$ | H | F | $CH_3$ | 1 | 4-$CH_3$ | $CO_2CH(CH_3)_2$ |
| 4-$CH_3$ | H | F | $CH_3$ | 1 | 4-$CH_3$ | $CO_2CH(CH_3)_2$ |
| 3-$CH_3$ | F | F | $CH_3$ | 1 | 4-$CH_3$ | $CO_2CH(CH_3)_2$ |
| 4-$CH_3$ | F | F | $CH_3$ | 1 | 4-$CH_3$ | $CO_2CH(CH_3)_2$ |
| 3-$CH_3$ | H | Cl | $CH_3$ | 1 | 4-$CH_3$ | $CO_2CH(CH_3)_2$ |

Tabelle A (Fortsetzung)

| $R_n$ | $R^1$ | $R^2$ | $R^3$ | m | $(R^a)_q$ | $R^4$ |
|---|---|---|---|---|---|---|
| 4-$CH_3$ | H | Cl | $CH_3$ | 1 | 4-$CH_3$ | $CO_2CH(CH_3)_2$ |
| 3-$CH_3$ | F | Cl | $CH_3$ | 1 | 4-$CH_3$ | $CO_2CH(CH_3)_2$ |
| 4-$CH_3$ | F | Cl | $CH_3$ | 1 | 4-$CH_3$ | $CO_2CH(CH_3)_2$ |
| 3-$CH_3$ | H | F | H | 1 | 4-$CH_3$ | $CO_2(CH_2)_3CH_3$ |
| 4-$CH_3$ | H | F | H | 1 | 4-$CH_3$ | $CO_2(CH_2)_3CH_3$ |
| 3-$CH_3$ | F | F | H | 1 | 4-$CH_3$ | $CO_2(CH_2)_3CH_3$ |
| 4-$CH_3$ | F | F | H | 1 | 4-$CH_3$ | $CO_2(CH_2)_3CH_3$ |
| 3-$CH_3$ | H | Cl | H | 1 | 4-$CH_3$ | $CO_2(CH_2)_3CH_3$ |
| 4-$CH_3$ | H | Cl | H | 1 | 4-$CH_3$ | $CO_2(CH_2)_3CH_3$ |
| 3-$CH_3$ | F | Cl | H | 1 | 4-$CH_3$ | $CO_2(CH_2)_3CH_3$ |
| 4-$CH_3$ | F | Cl | H | 1 | 4-$CH_3$ | $CO_2(CH_2)_3CH_3$ |
| 3-$CH_3$ | H | F | $CH_3$ | 1 | 4-$CH_3$ | $CO_2(CH_2)_3CH_3$ |
| 4-$CH_3$ | H | F | $CH_3$ | 1 | 4-$CH_3$ | $CO_2(CH_2)_3CH_3$ |
| 3-$CH_3$ | F | F | $CH_3$ | 1 | 4-$CH_3$ | $CO_2(CH_2)_3CH_3$ |
| 4-$CH_3$ | F | F | $CH_3$ | 1 | 4-$CH_3$ | $CO_2(CH_2)_3CH_3$ |
| 3-$CH_3$ | H | Cl | $CH_3$ | 1 | 4-$CH_3$ | $CO_2(CH_2)_3CH_3$ |
| 4-$CH_3$ | H | Cl | $CH_3$ | 1 | 4-$CH_3$ | $CO_2(CH_2)_3CH_3$ |
| 3-$CH_3$ | F | Cl | $CH_3$ | 1 | 4-$CH_3$ | $CO_2(CH_2)_3CH_3$ |
| 4-$CH_3$ | F | Cl | $CH_3$ | 1 | 4-$CH_3$ | $CO_2(CH_2)_3CH_3$ |
| 3-$CH_3$ | H | F | H | 1 | 4-$CH_3$ | $CO_2(CH_2)_2OCH_3$ |
| 4-$CH_3$ | H | F | H | 1 | 4-$CH_3$ | $CO_2(CH_2)_2OCH_3$ |
| 3-$CH_3$ | F | F | H | 1 | 4-$CH_3$ | $CO_2(CH_2)_2OCH_3$ |
| 4-$CH_3$ | F | F | H | 1 | 4-$CH_3$ | $CO_2(CH_2)_2OCH_3$ |
| 3-$CH_3$ | H | Cl | H | 1 | 4-$CH_3$ | $CO_2(CH_2)_2OCH_3$ |
| 4-$CH_3$ | H | Cl | H | 1 | 4-$CH_3$ | $CO_2(CH_2)_2OCH_3$ |
| 3-$CH_3$ | F | Cl | H | 1 | 4-$CH_3$ | $CO_2(CH_2)_2OCH_3$ |
| 4-$CH_3$ | F | Cl | H | 1 | 4-$CH_3$ | $CO_2(CH_2)_2OCH_3$ |
| 3-$CH_3$ | H | F | $CH_3$ | 1 | 4-$CH_3$ | $CO_2(CH_2)_2OCH_3$ |
| 4-$CH_3$ | H | F | $CH_3$ | 1 | 4-$CH_3$ | $CO_2(CH_2)_2OCH_3$ |
| 3-$CH_3$ | F | F | $CH_3$ | 1 | 4-$CH_3$ | $CO_2(CH_2)_2OCH_3$ |
| 4-$CH_3$ | F | F | $CH_3$ | 1 | 4-$CH_3$ | $CO_2(CH_2)_2OCH_3$ |
| 3-$CH_3$ | H | Cl | $CH_3$ | 1 | 4-$CH_3$ | $CO_2(CH_2)_2OCH_3$ |
| 4-$CH_3$ | H | Cl | $CH_3$ | 1 | 4-$CH_3$ | $CO_2(CH_2)_2OCH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 3-$CH_3$ | F | Cl | $CH_3$ | 1 | 4-$CH_3$ | $CO_2(CH_2)_2OCH_3$ |
| 4-$CH_3$ | F | Cl | $CH_3$ | 1 | 4-$CH_3$ | $CO_2(CH_2)_2OCH_3$ |
| 3-$CH_3$ | H | F | H | 1 | 4-$CH_3$ | $CO_2(CH_2)_2OCH_2CH_3$ |
| 4-$CH_3$ | H | F | H | 1 | 4-$CH_3$ | $CO_2(CH_2)_2OCH_2CH_3$ |
| 3-$CH_3$ | F | F | H | 1 | 4-$CH_3$ | $CO_2(CH_2)_2OCH_2CH_3$ |
| 4-$CH_3$ | F | F | H | 1 | 4-$CH_3$ | $CO_2(CH_2)_2OCH_2CH_3$ |
| 3-$CH_3$ | H | Cl | H | 1 | 4-$CH_3$ | $CO_2(CH_2)_2OCH_2CH_3$ |
| 4-$CH_3$ | H | Cl | H | 1 | 4-$CH_3$ | $CO_2(CH_2)_2OCH_2CH_3$ |
| 3-$CH_3$ | F | Cl | H | 1 | 4-$CH_3$ | $CO_2(CH_2)_2OCH_2CH_3$ |
| 4-$CH_3$ | F | Cl | H | 1 | 4-$CH_3$ | $CO_2(CH_2)_2OCH_2CH_3$ |
| 3-$CH_3$ | H | F | $CH_3$ | 1 | 4-$CH_3$ | $CO_2(CH_2)_2OCH_2CH_3$ |
| 4-$CH_3$ | H | F | $CH_3$ | 1 | 4-$CH_3$ | $CO_2(CH_2)_2OCH_2CH_3$ |
| 3-$CH_3$ | F | F | $CH_3$ | 1 | 4-$CH_3$ | $CO_2(CH_2)_2OCH_2CH_3$ |
| 4-$CH_3$ | F | F | $CH_3$ | 1 | 4-$CH_3$ | $CO_2(CH_2)_2OCH_2CH_3$ |
| 3-$CH_3$ | H | Cl | $CH_3$ | 1 | 4-$CH_3$ | $CO_2(CH_2)_2OCH_2CH_3$ |
| 4-$CH_3$ | H | Cl | $CH_3$ | 1 | 4-$CH_3$ | $CO_2(CH_2)_2OCH_2CH_3$ |
| 3-$CH_3$ | F | Cl | $CH_3$ | 1 | 4-$CH_3$ | $CO_2(CH_2)_2OCH_2CH_3$ |
| 4-$CH_3$ | F | Cl | $CH_3$ | 1 | 4-$CH_3$ | $CO_2(CH_2)_2OCH_2CH_3$ |
| 3-$CH_3$ | H | F | H | 1 | 6-$CH_3$ | $CO_2CH_3$ |
| 4-$CH_3$ | H | F | H | 1 | 6-$CH_3$ | $CO_2CH_3$ |
| 3-$CH_3$ | F | F | H | 1 | 6-$CH_3$ | $CO_2CH_3$ |
| 4-$CH_3$ | F | F | H | 1 | 6-$CH_3$ | $CO_2CH_3$ |
| 3-$CH_3$ | H | Cl | H | 1 | 6-$CH_3$ | $CO_2CH_3$ |
| 4-$CH_3$ | H | Cl | H | 1 | 6-$CH_3$ | $CO_2CH_3$ |
| 3-$CH_3$ | F | Cl | H | 1 | 6-$CH_3$ | $CO_2CH_3$ |
| 4-$CH_3$ | F | Cl | H | 1 | 6-$CH_3$ | $CO_2CH_3$ |
| 3-$CH_3$ | H | F | $CH_3$ | 1 | 6-$CH_3$ | $CO_2CH_3$ |
| 4-$CH_3$ | H | F | $CH_3$ | 1 | 6-$CH_3$ | $CO_2CH_3$ |
| 3-$CH_3$ | F | F | $CH_3$ | 1 | 6-$CH_3$ | $CO_2CH_3$ |
| 4-$CH_3$ | F | F | $CH_3$ | 1 | 6-$CH_3$ | $CO_2CH_3$ |
| 3-$CH_3$ | H | Cl | $CH_3$ | 1 | 6-$CH_3$ | $CO_2CH_3$ |
| 4-$CH_3$ | H | Cl | $CH_3$ | 1 | 6-$CH_3$ | $CO_2CH_3$ |
| 3-$CH_3$ | F | Cl | $CH_3$ | 1 | 6-$CH_3$ | $CO_2CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 4-$CH_3$ | F | Cl | $CH_3$ | 1 | 6-$CH_3$ | $CO_2CH_3$ |
| 3-$CH_3$ | H | F | H | 1 | 6-$CH_3$ | $CO_2CH_2CH_3$ |
| 4-$CH_3$ | H | F | H | 1 | 6-$CH_3$ | $CO_2CH_2CH_3$ |
| 3-$CH_3$ | F | F | H | 1 | 6-$CH_3$ | $CO_2CH_2CH_3$ |
| 4-$CH_3$ | F | F | H | 1 | 6-$CH_3$ | $CO_2CH_2CH_3$ |
| 3-$CH_3$ | H | Cl | H | 1 | 6-$CH_3$ | $CO_2CH_2CH_3$ |
| 4-$CH_3$ | H | Cl | H | 1 | 6-$CH_3$ | $CO_2CH_2CH_3$ |
| 3-$CH_3$ | F | Cl | H | 1 | 6-$CH_3$ | $CO_2CH_2CH_3$ |
| 4-$CH_3$ | F | Cl | H | 1 | 6-$CH_3$ | $CO_2CH_2CH_3$ |
| 3-$CH_3$ | H | F | $CH_3$ | 1 | 6-$CH_3$ | $CO_2CH_2CH_3$ |
| 4-$CH_3$ | H | F | $CH_3$ | 1 | 6-$CH_3$ | $CO_2CH_2CH_3$ |
| 3-$CH_3$ | F | F | $CH_3$ | 1 | 6-$CH_3$ | $CO_2CH_2CH_3$ |
| 4-$CH_3$ | F | F | $CH_3$ | 1 | 6-$CH_3$ | $CO_2CH_2CH_3$ |
| 3-$CH_3$ | H | Cl | $CH_3$ | 1 | 6-$CH_3$ | $CO_2CH_2CH_3$ |
| 4-$CH_3$ | H | Cl | $CH_3$ | 1 | 6-$CH_3$ | $CO_2CH_2CH_3$ |
| 3-$CH_3$ | F | Cl | $CH_3$ | 1 | 6-$CH_3$ | $CO_2CH_2CH_3$ |
| 4-$CH_3$ | F | Cl | $CH_3$ | 1 | 6-$CH_3$ | $CO_2CH_2CH_3$ |
| 3-$CH_3$ | H | F | H | 1 | 6-$CH_3$ | $CO_2(CH_2)_2CH_3$ |
| 4-$CH_3$ | H | F | H | 1 | 6-$CH_3$ | $CO_2(CH_2)_2CH_3$ |
| 3-$CH_3$ | F | F | H | 1 | 6-$CH_3$ | $CO_2(CH_2)_2CH_3$ |
| 4-$CH_3$ | F | F | H | 1 | 6-$CH_3$ | $CO_2(CH_2)_2CH_3$ |
| 3-$CH_3$ | H | Cl | H | 1 | 6-$CH_3$ | $CO_2(CH_2)_2CH_3$ |
| 4-$CH_3$ | H | Cl | H | 1 | 6-$CH_3$ | $CO_2(CH_2)_2CH_3$ |
| 3-$CH_3$ | F | Cl | H | 1 | 6-$CH_3$ | $CO_2(CH_2)_2CH_3$ |
| 4-$CH_3$ | F | Cl | H | 1 | 6-$CH_3$ | $CO_2(CH_2)_2CH_3$ |
| 3-$CH_3$ | H | F | $CH_3$ | 1 | 6-$CH_3$ | $CO_2(CH_2)_2CH_3$ |
| 4-$CH_3$ | H | F | $CH_3$ | 1 | 6-$CH_3$ | $CO_2(CH_2)_2CH_3$ |
| 3-$CH_3$ | F | F | $CH_3$ | 1 | 6-$CH_3$ | $CO_2(CH_2)_2CH_3$ |
| 4-$CH_3$ | F | F | $CH_3$ | 1 | 6-$CH_3$ | $CO_2(CH_2)_2CH_3$ |
| 3-$CH_3$ | H | Cl | $CH_3$ | 1 | 6-$CH_3$ | $CO_2(CH_2)_2CH_3$ |
| 4-$CH_3$ | H | Cl | $CH_3$ | 1 | 6-$CH_3$ | $CO_2(CH_2)_2CH_3$ |
| 3-$CH_3$ | F | Cl | $CH_3$ | 1 | 6-$CH_3$ | $CO_2(CH_2)_2CH_3$ |
| 4-$CH_3$ | F | Cl | $CH_3$ | 1 | 6-$CH_3$ | $CO_2(CH_2)_2CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 6-$CH_3$ | $CO_2CH(CH_3)_2$ |
| 4-$CH_3$ | H | F | H | 1 | 6-$CH_3$ | $CO_2CH(CH_3)_2$ |
| 3-$CH_3$ | F | F | H | 1 | 6-$CH_3$ | $CO_2CH(CH_3)_2$ |
| 4-$CH_3$ | F | F | H | 1 | 6-$CH_3$ | $CO_2CH(CH_3)_2$ |
| 3-$CH_3$ | H | Cl | H | 1 | 6-$CH_3$ | $CO_2CH(CH_3)_2$ |
| 4-$CH_3$ | H | Cl | H | 1 | 6-$CH_3$ | $CO_2CH(CH_3)_2$ |
| 3-$CH_3$ | F | Cl | H | 1 | 6-$CH_3$ | $CO_2CH(CH_3)_2$ |
| 4-$CH_3$ | F | Cl | H | 1 | 6-$CH_3$ | $CO_2CH(CH_3)_2$ |
| 3-$CH_3$ | H | F | $CH_3$ | 1 | 6-$CH_3$ | $CO_2CH(CH_3)_2$ |
| 4-$CH_3$ | H | F | $CH_3$ | 1 | 6-$CH_3$ | $CO_2CH(CH_3)_2$ |
| 3-$CH_3$ | F | F | $CH_3$ | 1 | 6-$CH_3$ | $CO_2CH(CH_3)_2$ |
| 4-$CH_3$ | F | F | $CH_3$ | 1 | 6-$CH_3$ | $CO_2CH(CH_3)_2$ |
| 3-$CH_3$ | H | Cl | $CH_3$ | 1 | 6-$CH_3$ | $CO_2CH(CH_3)_2$ |
| 4-$CH_3$ | H | Cl | $CH_3$ | 1 | 6-$CH_3$ | $CO_2CH(CH_3)_2$ |
| 3-$CH_3$ | F | Cl | $CH_3$ | 1 | 6-$CH_3$ | $CO_2CH(CH_3)_2$ |
| 4-$CH_3$ | F | Cl | $CH_3$ | 1 | 6-$CH_3$ | $CO_2CH(CH_3)_2$ |
| 3-$CH_3$ | H | F | H | 1 | 6-$CH_3$ | $CO_2(CH_2)_3CH_3$ |
| 4-$CH_3$ | H | F | H | 1 | 6-$CH_3$ | $CO_2(CH_2)_3CH_3$ |
| 3-$CH_3$ | F | F | H | 1 | 6-$CH_3$ | $CO_2(CH_2)_3CH_3$ |
| 4-$CH_3$ | F | F | H | 1 | 6-$CH_3$ | $CO_2(CH_2)_3CH_3$ |
| 3-$CH_3$ | H | Cl | H | 1 | 6-$CH_3$ | $CO_2(CH_2)_3CH_3$ |
| 4-$CH_3$ | H | Cl | H | 1 | 6-$CH_3$ | $CO_2(CH_2)_3CH_3$ |
| 3-$CH_3$ | F | Cl | H | 1 | 6-$CH_3$ | $CO_2(CH_2)_3CH_3$ |
| 4-$CH_3$ | F | Cl | H | 1 | 6-$CH_3$ | $CO_2(CH_2)_3CH_3$ |
| 3-$CH_3$ | H | F | $CH_3$ | 1 | 6-$CH_3$ | $CO_2(CH_2)_3CH_3$ |
| 4-$CH_3$ | H | F | $CH_3$ | 1 | 6-$CH_3$ | $CO_2(CH_2)_3CH_3$ |
| 3-$CH_3$ | F | F | $CH_3$ | 1 | 6-$CH_3$ | $CO_2(CH_2)_3CH_3$ |
| 4-$CH_3$ | F | F | $CH_3$ | 1 | 6-$CH_3$ | $CO_2(CH_2)_3CH_3$ |
| 3-$CH_3$ | H | Cl | $CH_3$ | 1 | 6-$CH_3$ | $CO_2(CH_2)_3CH_3$ |
| 4-$CH_3$ | H | Cl | $CH_3$ | 1 | 6-$CH_3$ | $CO_2(CH_2)_3CH_3$ |
| 3-$CH_3$ | F | Cl | $CH_3$ | 1 | 6-$CH_3$ | $CO_2(CH_2)_3CH_3$ |
| 4-$CH_3$ | F | Cl | $CH_3$ | 1 | 6-$CH_3$ | $CO_2(CH_2)_3CH_3$ |
| 3-$CH_3$ | H | F | H | 1 | 6-$CH_3$ | $CO_2(CH_2)_2OCH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 4-$CH_3$ | H | F | H | 1 | 6-$CH_3$ | $CO_2(CH_2)_2OCH_3$ |
| 3-$CH_3$ | F | F | H | 1 | 6-$CH_3$ | $CO_2(CH_2)_2OCH_3$ |
| 4-$CH_3$ | F | F | H | 1 | 6-$CH_3$ | $CO_2(CH_2)_2OCH_3$ |
| 3-$CH_3$ | H | Cl | H | 1 | 6-$CH_3$ | $CO_2(CH_2)_2OCH_3$ |
| 4-$CH_3$ | H | Cl | H | 1 | 6-$CH_3$ | $CO_2(CH_2)_2OCH_3$ |
| 3-$CH_3$ | F | Cl | H | 1 | 6-$CH_3$ | $CO_2(CH_2)_2OCH_3$ |
| 4-$CH_3$ | F | Cl | H | 1 | 6-$CH_3$ | $CO_2(CH_2)_2OCH_3$ |
| 3-$CH_3$ | H | F | $CH_3$ | 1 | 6-$CH_3$ | $CO_2(CH_2)_2OCH_3$ |
| 4-$CH_3$ | H | F | $CH_3$ | 1 | 6-$CH_3$ | $CO_2(CH_2)_2OCH_3$ |
| 3-$CH_3$ | F | F | $CH_3$ | 1 | 6-$CH_3$ | $CO_2(CH_2)_2OCH_3$ |
| 4-$CH_3$ | F | F | $CH_3$ | 1 | 6-$CH_3$ | $CO_2(CH_2)_2OCH_3$ |
| 3-$CH_3$ | H | Cl | $CH_3$ | 1 | 6-$CH_3$ | $CO_2(CH_2)_2OCH_3$ |
| 4-$CH_3$ | H | Cl | $CH_3$ | 1 | 6-$CH_3$ | $CO_2(CH_2)_2OCH_3$ |
| 3-$CH_3$ | F | Cl | $CH_3$ | 1 | 6-$CH_3$ | $CO_2(CH_2)_2OCH_3$ |
| 4-$CH_3$ | F | Cl | $CH_3$ | 1 | 6-$CH_3$ | $CO_2(CH_2)_2OCH_3$ |
| 3-$CH_3$ | H | F | H | 1 | 6-$CH_3$ | $CO_2(CH_2)_2OCH_2CH_3$ |
| 4-$CH_3$ | H | F | H | 1 | 6-$CH_3$ | $CO_2(CH_2)_2OCH_2CH_3$ |
| 3-$CH_3$ | F | F | H | 1 | 6-$CH_3$ | $CO_2(CH_2)_2OCH_2CH_3$ |
| 4-$CH_3$ | F | F | H | 1 | 6-$CH_3$ | $CO_2(CH_2)_2OCH_2CH_3$ |
| 3-$CH_3$ | H | Cl | H | 1 | 6-$CH_3$ | $CO_2(CH_2)_2OCH_2CH_3$ |
| 4-$CH_3$ | H | Cl | H | 1 | 6-$CH_3$ | $CO_2(CH_2)_2OCH_2CH_3$ |
| 3-$CH_3$ | F | Cl | H | 1 | 6-$CH_3$ | $CO_2(CH_2)_2OCH_2CH_3$ |
| 4-$CH_3$ | F | Cl | H | 1 | 6-$CH_3$ | $CO_2(CH_2)_2OCH_2CH_3$ |
| 3-$CH_3$ | H | F | $CH_3$ | 1 | 6-$CH_3$ | $CO_2(CH_2)_2OCH_2CH_3$ |
| 4-$CH_3$ | H | F | $CH_3$ | 1 | 6-$CH_3$ | $CO_2(CH_2)_2OCH_2CH_3$ |
| 3-$CH_3$ | F | F | $CH_3$ | 1 | 6-$CH_3$ | $CO_2(CH_2)_2OCH_2CH_3$ |
| 4-$CH_3$ | F | F | $CH_3$ | 1 | 6-$CH_3$ | $CO_2(CH_2)_2OCH_2CH_3$ |
| 3-$CH_3$ | H | Cl | $CH_3$ | 1 | 6-$CH_3$ | $CO_2(CH_2)_2OCH_2CH_3$ |
| 4-$CH_3$ | H | Cl | $CH_3$ | 1 | 6-$CH_3$ | $CO_2(CH_2)_2OCH_2CH_3$ |
| 3-$CH_3$ | F | Cl | $CH_3$ | 1 | 6-$CH_3$ | $CO_2(CH_2)_2OCH_2CH_3$ |
| 4-$CH_3$ | F | Cl | $CH_3$ | 1 | 6-$CH_3$ | $CO_2(CH_2)_2OCH_2CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4,6-Di-$CH_3$ | $CO_2CH_3$ |
| 4-$CH_3$ | H | F | H | 1 | 4,6-Di-$CH_3$ | $CO_2CH_3$ |
| 3-$CH_3$ | F | F | H | 1 | 4,6-Di-$CH_3$ | $CO_2CH_3$ |
| 4-$CH_3$ | F | F | H | 1 | 4,6-Di-$CH_3$ | $CO_2CH_3$ |
| 3-$CH_3$ | H | Cl | H | 1 | 4,6-Di-$CH_3$ | $CO_2CH_3$ |
| 4-$CH_3$ | H | Cl | H | 1 | 4,6-Di-$CH_3$ | $CO_2CH_3$ |
| 3-$CH_3$ | F | Cl | H | 1 | 4,6-Di-$CH_3$ | $CO_2CH_3$ |
| 4-$CH_3$ | F | Cl | H | 1 | 4,6-Di-$CH_3$ | $CO_2CH_3$ |
| 3-$CH_3$ | H | F | $CH_3$ | 1 | 4,6-Di-$CH_3$ | $CO_2CH_3$ |
| 4-$CH_3$ | H | F | $CH_3$ | 1 | 4,6-Di-$CH_3$ | $CO_2CH_3$ |
| 3-$CH_3$ | F | F | $CH_3$ | 1 | 4,6-Di-$CH_3$ | $CO_2CH_3$ |
| 4-$CH_3$ | F | F | $CH_3$ | 1 | 4,6-Di-$CH_3$ | $CO_2CH_3$ |
| 3-$CH_3$ | H | Cl | $CH_3$ | 1 | 4,6-Di-$CH_3$ | $CO_2CH_3$ |
| 4-$CH_3$ | H | Cl | $CH_3$ | 1 | 4,6-Di-$CH_3$ | $CO_2CH_3$ |
| 3-$CH_3$ | F | Cl | $CH_3$ | 1 | 4,6-Di-$CH_3$ | $CO_2CH_3$ |
| 4-$CH_3$ | F | Cl | $CH_3$ | 1 | 4,6-Di-$CH_3$ | $CO_2CH_3$ |
| 3-$CH_3$ | H | F | H | 1 | 4,6-Di-$CH_3$ | $CO_2CH_2CH_3$ |
| 4-$CH_3$ | H | F | H | 1 | 4,6-Di-$CH_3$ | $CO_2CH_2CH_3$ |
| 3-$CH_3$ | F | F | H | 1 | 4,6-Di-$CH_3$ | $CO_2CH_2CH_3$ |
| 4-$CH_3$ | F | F | H | 1 | 4,6-Di-$CH_3$ | $CO_2CH_2CH_3$ |
| 3-$CH_3$ | H | Cl | H | 1 | 4,6-Di-$CH_3$ | $CO_2CH_2CH_3$ |
| 4-$CH_3$ | H | Cl | H | 1 | 4,6-Di-$CH_3$ | $CO_2CH_2CH_3$ |
| 3-$CH_3$ | F | Cl | H | 1 | 4,6-Di-$CH_3$ | $CO_2CH_2CH_3$ |
| 4-$CH_3$ | F | Cl | H | 1 | 4,6-Di-$CH_3$ | $CO_2CH_2CH_3$ |
| 3-$CH_3$ | H | F | $CH_3$ | 1 | 4,6-Di-$CH_3$ | $CO_2CH_2CH_3$ |
| 4-$CH_3$ | H | F | $CH_3$ | 1 | 4,6-Di-$CH_3$ | $CO_2CH_2CH_3$ |
| 3-$CH_3$ | F | F | $CH_3$ | 1 | 4,6-Di-$CH_3$ | $CO_2CH_2CH_3$ |
| 4-$CH_3$ | F | F | $CH_3$ | 1 | 4,6-Di-$CH_3$ | $CO_2CH_2CH_3$ |
| 3-$CH_3$ | H | Cl | $CH_3$ | 1 | 4,6-Di-$CH_3$ | $CO_2CH_2CH_3$ |
| 4-$CH_3$ | H | Cl | $CH_3$ | 1 | 4,6-Di-$CH_3$ | $CO_2CH_2CH_3$ |
| 3-$CH_3$ | F | Cl | $CH_3$ | 1 | 4,6-Di-$CH_3$ | $CO_2CH_2CH_3$ |
| 4-$CH_3$ | F | Cl | $CH_3$ | 1 | 4,6-Di-$CH_3$ | $CO_2CH_2CH_3$ |
| 3-$CH_3$ | H | F | H | 1 | 4,6-Di-$CH_3$ | $CO_2(CH_2)_2CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 4-$CH_3$ | H | F | H | 1 | 4,6-Di-$CH_3$ | $CO_2(CH_2)_2CH_3$ |
| 3-$CH_3$ | F | F | H | 1 | 4,6-Di-$CH_3$ | $CO_2(CH_2)_2CH_3$ |
| 4-$CH_3$ | F | F | H | 1 | 4,6-Di-$CH_3$ | $CO_2(CH_2)_2CH_3$ |
| 3-$CH_3$ | H | Cl | H | 1 | 4,6-Di-$CH_3$ | $CO_2(CH_2)_2CH_3$ |
| 4-$CH_3$ | H | Cl | H | 1 | 4,6-Di-$CH_3$ | $CO_2(CH_2)_2CH_3$ |
| 3-$CH_3$ | F | Cl | H | 1 | 4,6-Di-$CH_3$ | $CO_2(CH_2)_2CH_3$ |
| 4-$CH_3$ | F | Cl | H | 1 | 4,6-Di-$CH_3$ | $CO_2(CH_2)_2CH_3$ |
| 3-$CH_3$ | H | F | $CH_3$ | 1 | 4,6-Di-$CH_3$ | $CO_2(CH_2)_2CH_3$ |
| 4-$CH_3$ | H | F | $CH_3$ | 1 | 4,6-Di-$CH_3$ | $CO_2(CH_2)_2CH_3$ |
| 3-$CH_3$ | F | F | $CH_3$ | 1 | 4,6-Di-$CH_3$ | $CO_2(CH_2)_2CH_3$ |
| 4-$CH_3$ | F | F | $CH_3$ | 1 | 4,6-Di-$CH_3$ | $CO_2(CH_2)_2CH_3$ |
| 3-$CH_3$ | H | Cl | $CH_3$ | 1 | 4,6-Di-$CH_3$ | $CO_2(CH_2)_2CH_3$ |
| 4-$CH_3$ | H | Cl | $CH_3$ | 1 | 4,6-Di-$CH_3$ | $CO_2(CH_2)_2CH_3$ |
| 3-$CH_3$ | F | Cl | $CH_3$ | 1 | 4,6-Di-$CH_3$ | $CO_2(CH_2)_2CH_3$ |
| 4-$CH_3$ | F | Cl | $CH_3$ | 1 | 4,6-Di-$CH_3$ | $CO_2(CH_2)_2CH_3$ |
| 3-$CH_3$ | H | F | H | 1 | 4,6-Di-$CH_3$ | $CO_2CH(CH_3)_2$ |
| 4-$CH_3$ | H | F | H | 1 | 4,6-Di-$CH_3$ | $CO_2CH(CH_3)_2$ |
| 3-$CH_3$ | F | F | H | 1 | 4,6-Di-$CH_3$ | $CO_2CH(CH_3)_2$ |
| 4-$CH_3$ | F | F | H | 1 | 4,6-Di-$CH_3$ | $CO_2CH(CH_3)_2$ |
| 3-$CH_3$ | H | Cl | H | 1 | 4,6-Di-$CH_3$ | $CO_2CH(CH_3)_2$ |
| 4-$CH_3$ | H | Cl | H | 1 | 4,6-Di-$CH_3$ | $CO_2CH(CH_3)_2$ |
| 3-$CH_3$ | F | Cl | H | 1 | 4,6-Di-$CH_3$ | $CO_2CH(CH_3)_2$ |
| 4-$CH_3$ | F | Cl | H | 1 | 4,6-Di-$CH_3$ | $CO_2CH(CH_3)_2$ |
| 3-$CH_3$ | H | F | $CH_3$ | 1 | 4,6-Di-$CH_3$ | $CO_2CH(CH_3)_2$ |
| 4-$CH_3$ | H | F | $CH_3$ | 1 | 4,6-Di-$CH_3$ | $CO_2CH(CH_3)_2$ |
| 3-$CH_3$ | F | F | $CH_3$ | 1 | 4,6-Di-$CH_3$ | $CO_2CH(CH_3)_2$ |
| 4-$CH_3$ | F | F | $CH_3$ | 1 | 4,6-Di-$CH_3$ | $CO_2CH(CH_3)_2$ |
| 3-$CH_3$ | H | Cl | $CH_3$ | 1 | 4,6-Di-$CH_3$ | $CO_2CH(CH_3)_2$ |
| 4-$CH_3$ | H | Cl | $CH_3$ | 1 | 4,6-Di-$CH_3$ | $CO_2CH(CH_3)_2$ |
| 3-$CH_3$ | F | Cl | $CH_3$ | 1 | 4,6-Di-$CH_3$ | $CO_2CH(CH_3)_2$ |
| 4-$CH_3$ | F | Cl | $CH_3$ | 1 | 4,6-Di-$CH_3$ | $CO_2CH(CH_3)_2$ |
| 3-$CH_3$ | H | F | H | 1 | 4,6-Di-$CH_3$ | $CO_2(CH_2)_3CH_3$ |
| 4-$CH_3$ | H | F | H | 1 | 4,6-Di-$CH_3$ | $CO_2(CH_2)_3CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 3-$CH_3$ | F | F | H | 1 | 4,6-Di-$CH_3$ | $CO_2(CH_2)_3CH_3$ |
| 4-$CH_3$ | F | F | H | 1 | 4,6-Di-$CH_3$ | $CO_2(CH_2)_3CH_3$ |
| 3-$CH_3$ | H | Cl | H | 1 | 4,6-Di-$CH_3$ | $CO_2(CH_2)_3CH_3$ |
| 4-$CH_3$ | H | Cl | H | 1 | 4,6-Di-$CH_3$ | $CO_2(CH_2)_3CH_3$ |
| 3-$CH_3$ | F | Cl | H | 1 | 4,6-Di-$CH_3$ | $CO_2(CH_2)_3CH_3$ |
| 4-$CH_3$ | F | Cl | H | 1 | 4,6-Di-$CH_3$ | $CO_2(CH_2)_3CH_3$ |
| 3-$CH_3$ | H | F | $CH_3$ | 1 | 4,6-Di-$CH_3$ | $CO_2(CH_2)_3CH_3$ |
| 4-$CH_3$ | H | F | $CH_3$ | 1 | 4,6-Di-$CH_3$ | $CO_2(CH_2)_3CH_3$ |
| 3-$CH_3$ | F | F | $CH_3$ | 1 | 4,6-Di-$CH_3$ | $CO_2(CH_2)_3CH_3$ |
| 4-$CH_3$ | F | F | $CH_3$ | 1 | 4,6-Di-$CH_3$ | $CO_2(CH_2)_3CH_3$ |
| 3-$CH_3$ | H | Cl | $CH_3$ | 1 | 4,6-Di-$CH_3$ | $CO_2(CH_2)_3CH_3$ |
| 4-$CH_3$ | H | Cl | $CH_3$ | 1 | 4,6-Di-$CH_3$ | $CO_2(CH_2)_3CH_3$ |
| 3-$CH_3$ | F | Cl | $CH_3$ | 1 | 4,6-Di-$CH_3$ | $CO_2(CH_2)_3CH_3$ |
| 4-$CH_3$ | F | Cl | $CH_3$ | 1 | 4,6-Di-$CH_3$ | $CO_2(CH_2)_3CH_3$ |
| 3-$CH_3$ | H | F | H | 1 | 4,6-Di-$CH_3$ | $CO_2(CH_2)_2OCH_3$ |
| 4-$CH_3$ | H | F | H | 1 | 4,6-Di-$CH_3$ | $CO_2(CH_2)_2OCH_3$ |
| 3-$CH_3$ | F | F | H | 1 | 4,6-Di-$CH_3$ | $CO_2(CH_2)_2OCH_3$ |
| 4-$CH_3$ | F | F | H | 1 | 4,6-Di-$CH_3$ | $CO_2(CH_2)_2OCH_3$ |
| 3-$CH_3$ | H | Cl | H | 1 | 4,6-Di-$CH_3$ | $CO_2(CH_2)_2OCH_3$ |
| 4-$CH_3$ | H | Cl | H | 1 | 4,6-Di-$CH_3$ | $CO_2(CH_2)_2OCH_3$ |
| 3-$CH_3$ | F | Cl | H | 1 | 4,6-Di-$CH_3$ | $CO_2(CH_2)_2OCH_3$ |
| 4-$CH_3$ | F | Cl | H | 1 | 4,6-Di-$CH_3$ | $CO_2(CH_2)_2OCH_3$ |
| 3-$CH_3$ | H | F | $CH_3$ | 1 | 4,6-Di-$CH_3$ | $CO_2(CH_2)_2OCH_3$ |
| 4-$CH_3$ | H | F | $CH_3$ | 1 | 4,6-Di-$CH_3$ | $CO_2(CH_2)_2OCH_3$ |
| 3-$CH_3$ | F | F | $CH_3$ | 1 | 4,6-Di-$CH_3$ | $CO_2(CH_2)_2OCH_3$ |
| 4-$CH_3$ | F | F | $CH_3$ | 1 | 4,6-Di-$CH_3$ | $CO_2(CH_2)_2OCH_3$ |
| 3-$CH_3$ | H | Cl | $CH_3$ | 1 | 4,6-Di-$CH_3$ | $CO_2(CH_2)_2OCH_3$ |
| 4-$CH_3$ | H | Cl | $CH_3$ | 1 | 4,6-Di-$CH_3$ | $CO_2(CH_2)_2OCH_3$ |
| 3-$CH_3$ | F | Cl | $CH_3$ | 1 | 4,6-Di-$CH_3$ | $CO_2(CH_2)_2OCH_3$ |
| 4-$CH_3$ | F | Cl | $CH_3$ | 1 | 4,6-Di-$CH_3$ | $CO_2(CH_2)_2OCH_3$ |
| 3-$CH_3$ | H | F | H | 1 | 4,6-Di-$CH_3$ | $CO_2(CH_2)_2OCH_2CH_3$ |
| 4-$CH_3$ | H | F | H | 1 | 4,6-Di-$CH_3$ | $CO_2(CH_2)_2OCH_2CH_3$ |
| 3-$CH_3$ | F | F | H | 1 | 4,6-Di-$CH_3$ | $CO_2(CH_2)_2OCH_2CH_3$ |

Tabelle A (Fortsetzung)

| $R_n$ | $R^1$ | $R^2$ | $R^3$ | m | $(R^a)_q$ | $R^4$ |
|---|---|---|---|---|---|---|
| 4-$CH_3$ | F | F | H | 1 | 4,6-Di-$CH_3$ | $CO_2(CH_2)_2OCH_2CH_3$ |
| 3-$CH_3$ | H | Cl | H | 1 | 4,6-Di-$CH_3$ | $CO_2(CH_2)_2OCH_2CH_3$ |
| 4-$CH_3$ | H | Cl | H | 1 | 4,6-Di-$CH_3$ | $CO_2(CH_2)_2OCH_2CH_3$ |
| 3-$CH_3$ | F | Cl | H | 1 | 4,6-Di-$CH_3$ | $CO_2(CH_2)_2OCH_2CH_3$ |
| 4-$CH_3$ | F | Cl | H | 1 | 4,6-Di-$CH_3$ | $CO_2(CH_2)_2OCH_2CH_3$ |
| 3-$CH_3$ | H | F | $CH_3$ | 1 | 4,6-Di-$CH_3$ | $CO_2(CH_2)_2OCH_2CH_3$ |
| 4-$CH_3$ | H | F | $CH_3$ | 1 | 4,6-Di-$CH_3$ | $CO_2(CH_2)_2OCH_2CH_3$ |
| 3-$CH_3$ | F | F | $CH_3$ | 1 | 4,6-Di-$CH_3$ | $CO_2(CH_2)_2OCH_2CH_3$ |
| 4-$CH_3$ | F | F | $CH_3$ | 1 | 4,6-Di-$CH_3$ | $CO_2(CH_2)_2OCH_2CH_3$ |
| 3-$CH_3$ | H | Cl | $CH_3$ | 1 | 4,6-Di-$CH_3$ | $CO_2(CH_2)_2OCH_2CH_3$ |
| 4-$CH_3$ | H | Cl | $CH_3$ | 1 | 4,6-Di-$CH_3$ | $CO_2(CH_2)_2OCH_2CH_3$ |
| 3-$CH_3$ | F | Cl | $CH_3$ | 1 | 4,6-Di-$CH_3$ | $CO_2(CH_2)_2OCH_2CH_3$ |
| 4-$CH_3$ | F | Cl | $CH_3$ | 1 | 4,6-Di-$CH_3$ | $CO_2(CH_2)_2OCH_2CH_3$ |

Tabelle B

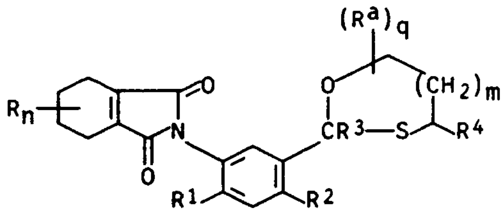

| $R_n$ | $R^1$ | $R^2$ | $R^3$ | m | $(R^a)_q$ | $R^4$ |
|---|---|---|---|---|---|---|
| 3-$CH_3$ | H | F | H | 0 | H | H |
| 4-$CH_3$ | H | F | H | 0 | H | H |
| 3-$CH_3$ | F | F | H | 0 | H | H |
| 4-$CH_3$ | F | F | H | 0 | H | H |
| 3-$CH_3$ | H | Cl | H | 0 | H | H |
| 4-$CH_3$ | H | Cl | H | 0 | H | H |
| 3-$CH_3$ | F | Cl | H | 0 | H | H |
| 4-$CH_3$ | F | Cl | H | 0 | H | H |
| 3-$CH_3$ | H | F | $CH_3$ | 0 | H | H |
| 4-$CH_3$ | H | F | $CH_3$ | 0 | H | H |
| 3-$CH_3$ | F | F | $CH_3$ | 0 | H | H |
| 4-$CH_3$ | F | F | $CH_3$ | 0 | H | H |
| 3-$CH_3$ | H | Cl | $CH_3$ | 0 | H | H |
| 4-$CH_3$ | H | Cl | $CH_3$ | 0 | H | H |
| 3-$CH_3$ | F | Cl | $CH_3$ | 0 | H | H |
| 4-$CH_3$ | F | Cl | $CH_3$ | 0 | H | H |
| 3-$CH_3$ | H | F | H | 0 | 4-$CH_3$ | H |
| 4-$CH_3$ | H | F | H | 0 | 4-$CH_3$ | H |
| 3-$CH_3$ | F | F | H | 0 | 4-$CH_3$ | H |
| 4-$CH_3$ | F | F | H | 0 | 4-$CH_3$ | H |
| 3-$CH_3$ | H | Cl | H | 0 | 4-$CH_3$ | H |
| 4-$CH_3$ | H | Cl | H | 0 | 4-$CH_3$ | H |
| 3-$CH_3$ | F | Cl | H | 0 | 4-$CH_3$ | H |
| 4-$CH_3$ | F | Cl | H | 0 | 4-$CH_3$ | H |
| 3-$CH_3$ | H | F | $CH_3$ | 0 | 4-$CH_3$ | H |
| 4-$CH_3$ | H | F | $CH_3$ | 0 | 4-$CH_3$ | H |
| 3-$CH_3$ | F | F | $CH_3$ | 0 | 4-$CH_3$ | H |

Tabelle B (Fortsetzung)

| $R_n$ | $R^1$ | $R^2$ | $R^3$ | m | $(R^a)_q$ | $R^4$ |
|---|---|---|---|---|---|---|
| $4-CH_3$ | F | F | $CH_3$ | 0 | $4-CH_3$ | H |
| $3-CH_3$ | H | Cl | $CH_3$ | 0 | $4-CH_3$ | H |
| $4-CH_3$ | H | Cl | $CH_3$ | 0 | $4-CH_3$ | H |
| $3-CH_3$ | F | Cl | $CH_3$ | 0 | $4-CH_3$ | H |
| $4-CH_3$ | F | Cl | $CH_3$ | 0 | $4-CH_3$ | H |
| $3-CH_3$ | H | F | H | 0 | $4,5-Di-CH_3$ | H |
| $4-CH_3$ | H | F | H | 0 | $4,5-Di-CH_3$ | H |
| $3-CH_3$ | F | F | H | 0 | $4,5-Di-CH_3$ | H |
| $4-CH_3$ | F | F | H | 0 | $4,5-Di-CH_3$ | H |
| $3-CH_3$ | H | Cl | H | 0 | $4,5-Di-CH_3$ | H |
| $4-CH_3$ | H | Cl | H | 0 | $4,5-Di-CH_3$ | H |
| $3-CH_3$ | F | Cl | H | 0 | $4,5-Di-CH_3$ | H |
| $4-CH_3$ | F | Cl | H | 0 | $4,5-Di-CH_3$ | H |
| $3-CH_3$ | H | F | $CH_3$ | 0 | $4,5-Di-CH_3$ | H |
| $4-CH_3$ | H | F | $CH_3$ | 0 | $4,5-Di-CH_3$ | H |
| $3-CH_3$ | F | F | $CH_3$ | 0 | $4,5-Di-CH_3$ | H |
| $4-CH_3$ | F | F | $CH_3$ | 0 | $4,5-Di-CH_3$ | H |
| $3-CH_3$ | H | Cl | $CH_3$ | 0 | $4,5-Di-CH_3$ | H |
| $4-CH_3$ | H | Cl | $CH_3$ | 0 | $4,5-Di-CH_3$ | H |
| $3-CH_3$ | F | Cl | $CH_3$ | 0 | $4,5-Di-CH_3$ | H |
| $4-CH_3$ | F | Cl | $CH_3$ | 0 | $4,5-Di-CH_3$ | H |
| $3-CH_3$ | H | F | H | 0 | $4-CH_2CH_3$ | H |
| $4-CH_3$ | H | F | H | 0 | $4-CH_2CH_3$ | H |
| $3-CH_3$ | F | F | H | 0 | $4-CH_2CH_3$ | H |
| $4-CH_3$ | F | F | H | 0 | $4-CH_2CH_3$ | H |
| $3-CH_3$ | H | Cl | H | 0 | $4-CH_2CH_3$ | H |
| $4-CH_3$ | H | Cl | H | 0 | $4-CH_2CH_3$ | H |
| $3-CH_3$ | F | Cl | H | 0 | $4-CH_2CH_3$ | H |
| $4-CH_3$ | F | Cl | H | 0 | $4-CH_2CH_3$ | H |
| $3-CH_3$ | H | F | $CH_3$ | 0 | $4-CH_2CH_3$ | H |
| $4-CH_3$ | H | F | $CH_3$ | 0 | $4-CH_2CH_3$ | H |
| $3-CH_3$ | F | F | $CH_3$ | 0 | $4-CH_2CH_3$ | H |

Tabelle B (Fortsetzung)

| $R_n$ | $R^1$ | $R^2$ | $R^3$ | m | $(R^a)_q$ | $R^4$ |
|---|---|---|---|---|---|---|
| 4-$CH_3$ | F | F | $CH_3$ | 0 | 4-$CH_2CH_3$ | H |
| 3-$CH_3$ | H | Cl | $CH_3$ | 0 | 4-$CH_2CH_3$ | H |
| 4-$CH_3$ | H | Cl | $CH_3$ | 0 | 4-$CH_2CH_3$ | H |
| 3-$CH_3$ | F | Cl | $CH_3$ | 0 | 4-$CH_2CH_3$ | H |
| 4-$CH_3$ | F | Cl | $CH_3$ | 0 | 4-$CH_2CH_3$ | H |
| 3-$CH_3$ | H | F | H | 0 | 4-$CH_2Cl$ | H |
| 4-$CH_3$ | H | F | H | 0 | 4-$CH_2Cl$ | H |
| 3-$CH_3$ | F | F | H | 0 | 4-$CH_2Cl$ | H |
| 4-$CH_3$ | F | F | H | 0 | 4-$CH_2Cl$ | H |
| 3-$CH_3$ | H | Cl | H | 0 | 4-$CH_2Cl$ | H |
| 4-$CH_3$ | H | Cl | H | 0 | 4-$CH_2Cl$ | H |
| 3-$CH_3$ | F | Cl | H | 0 | 4-$CH_2Cl$ | H |
| 4-$CH_3$ | F | Cl | H | 0 | 4-$CH_2Cl$ | H |
| 3-$CH_3$ | H | F | $CH_3$ | 0 | 4-$CH_2Cl$ | H |
| 4-$CH_3$ | H | F | $CH_3$ | 0 | 4-$CH_2Cl$ | H |
| 3-$CH_3$ | F | F | $CH_3$ | 0 | 4-$CH_2Cl$ | H |
| 4-$CH_3$ | F | F | $CH_3$ | 0 | 4-$CH_2Cl$ | H |
| 3-$CH_3$ | H | Cl | $CH_3$ | 0 | 4-$CH_2Cl$ | H |
| 4-$CH_3$ | H | Cl | $CH_3$ | 0 | 4-$CH_2Cl$ | H |
| 3-$CH_3$ | F | Cl | $CH_3$ | 0 | 4-$CH_2Cl$ | H |
| 4-$CH_3$ | F | Cl | $CH_3$ | 0 | 4-$CH_2Cl$ | H |
| 3-$CH_3$ | H | F | H | 0 | 4-$CH_2OH$ | H |
| 4-$CH_3$ | H | F | H | 0 | 4-$CH_2OH$ | H |
| 3-$CH_3$ | F | F | H | 0 | 4-$CH_2OH$ | H |
| 4-$CH_3$ | F | F | H | 0 | 4-$CH_2OH$ | H |
| 3-$CH_3$ | H | Cl | H | 0 | 4-$CH_2OH$ | H |
| 4-$CH_3$ | H | Cl | H | 0 | 4-$CH_2OH$ | H |
| 3-$CH_3$ | F | Cl | H | 0 | 4-$CH_2OH$ | H |
| 4-$CH_3$ | F | Cl | H | 0 | 4-$CH_2OH$ | H |
| 3-$CH_3$ | H | F | $CH_3$ | 0 | 4-$CH_2OH$ | H |
| 4-$CH_3$ | H | F | $CH_3$ | 0 | 4-$CH_2OH$ | H |
| 3-$CH_3$ | F | F | $CH_3$ | 0 | 4-$CH_2OH$ | H |
| 4-$CH_3$ | F | F | $CH_3$ | 0 | 4-$CH_2OH$ | H |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | Cl | $CH_3$ | 0 | 4-$CH_2OH$ | H |
| 4-$CH_3$ | H | Cl | $CH_3$ | 0 | 4-$CH_2OH$ | H |
| 3-$CH_3$ | F | Cl | $CH_3$ | 0 | 4-$CH_2OH$ | H |
| 4-$CH_3$ | F | Cl | $CH_3$ | 0 | 4-$CH_2OH$ | H |
| 3-$CH_3$ | H | F | H | 0 | 4-$CH_2OCH_3$ | H |
| 4-$CH_3$ | H | F | H | 0 | 4-$CH_2OCH_3$ | H |
| 3-$CH_3$ | F | F | H | 0 | 4-$CH_2OCH_3$ | H |
| 4-$CH_3$ | F | F | H | 0 | 4-$CH_2OCH_3$ | H |
| 3-$CH_3$ | H | Cl | H | 0 | 4-$CH_2OCH_3$ | H |
| 4-$CH_3$ | H | Cl | H | 0 | 4-$CH_2OCH_3$ | H |
| 3-$CH_3$ | F | Cl | H | 0 | 4-$CH_2OCH_3$ | H |
| 4-$CH_3$ | F | Cl | H | 0 | 4-$CH_2OCH_3$ | H |
| 3-$CH_3$ | H | F | $CH_3$ | 0 | 4-$CH_2OCH_3$ | H |
| 4-$CH_3$ | H | F | $CH_3$ | 0 | 4-$CH_2OCH_3$ | H |
| 3-$CH_3$ | F | F | $CH_3$ | 0 | 4-$CH_2OCH_3$ | H |
| 4-$CH_3$ | F | F | $CH_3$ | 0 | 4-$CH_2OCH_3$ | H |
| 3-$CH_3$ | H | Cl | $CH_3$ | 0 | 4-$CH_2OCH_3$ | H |
| 4-$CH_3$ | H | Cl | $CH_3$ | 0 | 4-$CH_2OCH_3$ | H |
| 3-$CH_3$ | F | Cl | $CH_3$ | 0 | 4-$CH_2OCH_3$ | H |
| 4-$CH_3$ | F | Cl | $CH_3$ | 0 | 4-$CH_2OCH_3$ | H |
| 3-$CH_3$ | H | F | H | 0 | 4-$CH_2OCH_2CH=CH_2$ | H |
| 4-$CH_3$ | H | F | H | 0 | 4-$CH_2OCH_2CH=CH_2$ | H |
| 3-$CH_3$ | F | F | H | 0 | 4-$CH_2OCH_2CH=CH_2$ | H |
| 4-$CH_3$ | F | F | H | 0 | 4-$CH_2OCH_2CH=CH_2$ | H |
| 3-$CH_3$ | H | Cl | H | 0 | 4-$CH_2OCH_2CH=CH_2$ | H |
| 4-$CH_3$ | H | Cl | H | 0 | 4-$CH_2OCH_2CH=CH_2$ | H |
| 3-$CH_3$ | F | Cl | H | 0 | 4-$CH_2OCH_2CH=CH_2$ | H |
| 4-$CH_3$ | F | Cl | H | 0 | 4-$CH_2OCH_2CH=CH_2$ | H |
| 3-$CH_3$ | H | F | $CH_3$ | 0 | 4-$CH_2OCH_2CH=CH_2$ | H |
| 4-$CH_3$ | H | F | $CH_3$ | 0 | 4-$CH_2OCH_2CH=CH_2$ | H |
| 3-$CH_3$ | F | F | $CH_3$ | 0 | 4-$CH_2OCH_2CH=CH_2$ | H |
| 4-$CH_3$ | F | F | $CH_3$ | 0 | 4-$CH_2OCH_2CH=CH_2$ | H |
| 3-$CH_3$ | H | Cl | $CH_3$ | 0 | 4-$CH_2OCH_2CH=CH_2$ | H |

Tabelle B (Fortsetzung)

| $R_n$ | $R^1$ | $R^2$ | $R^3$ | m | $(R^a)_q$ | $R^4$ |
|---|---|---|---|---|---|---|
| 4-$CH_3$ | H | Cl | $CH_3$ | 0 | 4-$CH_2OCH_2CH{=}CH_2$ | H |
| 3-$CH_3$ | F | Cl | $CH_3$ | 0 | 4-$CH_2OCH_2CH{=}CH_2$ | H |
| 4-$CH_3$ | F | Cl | $CH_3$ | 0 | 4-$CH_2OCH_2CH{=}CH_2$ | H |
| 3-$CH_3$ | H | F | H | 0 | 4-$CH_2OCH_2C{\equiv}CH$ | H |
| 4-$CH_3$ | H | F | H | 0 | 4-$CH_2OCH_2C{\equiv}CH$ | H |
| 3-$CH_3$ | F | F | H | 0 | 4-$CH_2OCH_2C{\equiv}CH$ | H |
| 4-$CH_3$ | F | F | H | 0 | 4-$CH_2OCH_2C{\equiv}CH$ | H |
| 3-$CH_3$ | H | Cl | H | 0 | 4-$CH_2OCH_2C{\equiv}CH$ | H |
| 4-$CH_3$ | H | Cl | H | 0 | 4-$CH_2OCH_2C{\equiv}CH$ | H |
| 3-$CH_3$ | F | Cl | H | 0 | 4-$CH_2OCH_2C{\equiv}CH$ | H |
| 4-$CH_3$ | F | Cl | H | 0 | 4-$CH_2OCH_2C{\equiv}CH$ | H |
| 3-$CH_3$ | H | F | $CH_3$ | 0 | 4-$CH_2OCH_2C{\equiv}CH$ | H |
| 4-$CH_3$ | H | F | $CH_3$ | 0 | 4-$CH_2OCH_2C{\equiv}CH$ | H |
| 3-$CH_3$ | F | F | $CH_3$ | 0 | 4-$CH_2OCH_2C{\equiv}CH$ | H |
| 4-$CH_3$ | F | F | $CH_3$ | 0 | 4-$CH_2OCH_2C{\equiv}CH$ | H |
| 3-$CH_3$ | H | Cl | $CH_3$ | 0 | 4-$CH_2OCH_2C{\equiv}CH$ | H |
| 4-$CH_3$ | H | Cl | $CH_3$ | 0 | 4-$CH_2OCH_2C{\equiv}CH$ | H |
| 3-$CH_3$ | F | Cl | $CH_3$ | 0 | 4-$CH_2OCH_2C{\equiv}CH$ | H |
| 4-$CH_3$ | F | Cl | $CH_3$ | 0 | 4-$CH_2OCH_2C{\equiv}CH$ | H |
| 3-$CH_3$ | H | F | H | 0 | 4-$CH_2OCOCH_3$ | H |
| 4-$CH_3$ | H | F | H | 0 | 4-$CH_2OCOCH_3$ | H |
| 3-$CH_3$ | F | F | H | 0 | 4-$CH_2OCOCH_3$ | H |
| 4-$CH_3$ | F | F | H | 0 | 4-$CH_2OCOCH_3$ | H |
| 3-$CH_3$ | H | Cl | H | 0 | 4-$CH_2OCOCH_3$ | H |
| 4-$CH_3$ | H | Cl | H | 0 | 4-$CH_2OCOCH_3$ | H |
| 3-$CH_3$ | F | Cl | H | 0 | 4-$CH_2OCOCH_3$ | H |
| 4-$CH_3$ | F | Cl | H | 0 | 4-$CH_2OCOCH_3$ | H |
| 3-$CH_3$ | H | F | $CH_3$ | 0 | 4-$CH_2OCOCH_3$ | H |
| 4-$CH_3$ | H | F | $CH_3$ | 0 | 4-$CH_2OCOCH_3$ | H |
| 3-$CH_3$ | F | F | $CH_3$ | 0 | 4-$CH_2OCOCH_3$ | H |
| 4-$CH_3$ | F | F | $CH_3$ | 0 | 4-$CH_2OCOCH_3$ | H |
| 3-$CH_3$ | H | Cl | $CH_3$ | 0 | 4-$CH_2OCOCH_3$ | H |
| 4-$CH_3$ | H | Cl | $CH_3$ | 0 | 4-$CH_2OCOCH_3$ | H |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | F | Cl | $CH_3$ | 0 | 4-$CH_2OCOCH_3$ | H |
| 4-$CH_3$ | F | Cl | $CH_3$ | 0 | 4-$CH_2OCOCH_3$ | H |
| 3-$CH_3$ | H | F | H | 0 | 4-$CH_2OCH_2CO_2CH_3$ | H |
| 4-$CH_3$ | H | F | H | 0 | 4-$CH_2OCH_2CO_2CH_3$ | H |
| 3-$CH_3$ | F | F | H | 0 | 4-$CH_2OCH_2CO_2CH_3$ | H |
| 4-$CH_3$ | F | F | H | 0 | 4-$CH_2OCH_2CO_2CH_3$ | H |
| 3-$CH_3$ | H | Cl | H | 0 | 4-$CH_2OCH_2CO_2CH_3$ | H |
| 4-$CH_3$ | H | Cl | H | 0 | 4-$CH_2OCH_2CO_2CH_3$ | H |
| 3-$CH_3$ | F | Cl | H | 0 | 4-$CH_2OCH_2CO_2CH_3$ | H |
| 4-$CH_3$ | F | Cl | H | 0 | 4-$CH_2OCH_2CO_2CH_3$ | H |
| 3-$CH_3$ | H | F | $CH_3$ | 0 | 4-$CH_2OCH_2CO_2CH_3$ | H |
| 4-$CH_3$ | H | F | $CH_3$ | 0 | 4-$CH_2OCH_2CO_2CH_3$ | H |
| 3-$CH_3$ | F | F | $CH_3$ | 0 | 4-$CH_2OCH_2CO_2CH_3$ | H |
| 4-$CH_3$ | F | F | $CH_3$ | 0 | 4-$CH_2OCH_2CO_2CH_3$ | H |
| 3-$CH_3$ | H | Cl | $CH_3$ | 0 | 4-$CH_2OCH_2CO_2CH_3$ | H |
| 4-$CH_3$ | H | Cl | $CH_3$ | 0 | 4-$CH_2OCH_2CO_2CH_3$ | H |
| 3-$CH_3$ | F | Cl | $CH_3$ | 0 | 4-$CH_2OCH_2CO_2CH_3$ | H |
| 4-$CH_3$ | F | Cl | $CH_3$ | 0 | 4-$CH_2OCH_2CO_2CH_3$ | H |
| 3-$CH_3$ | H | F | H | 0 | 4-$CH_2CO_2CH_3$ | H |
| 4-$CH_3$ | H | F | H | 0 | 4-$CH_2CO_2CH_3$ | H |
| 3-$CH_3$ | F | F | H | 0 | 4-$CH_2CO_2CH_3$ | H |
| 4-$CH_3$ | F | F | H | 0 | 4-$CH_2CO_2CH_3$ | H |
| 3-$CH_3$ | H | Cl | H | 0 | 4-$CH_2CO_2CH_3$ | H |
| 4-$CH_3$ | H | Cl | H | 0 | 4-$CH_2CO_2CH_3$ | H |
| 3-$CH_3$ | F | Cl | H | 0 | 4-$CH_2CO_2CH_3$ | H |
| 4-$CH_3$ | F | Cl | H | 0 | 4-$CH_2CO_2CH_3$ | H |
| 3-$CH_3$ | H | F | $CH_3$ | 0 | 4-$CH_2CO_2CH_3$ | H |
| 4-$CH_3$ | H | F | $CH_3$ | 0 | 4-$CH_2CO_2CH_3$ | H |
| 3-$CH_3$ | F | F | $CH_3$ | 0 | 4-$CH_2CO_2CH_3$ | H |
| 4-$CH_3$ | F | F | $CH_3$ | 0 | 4-$CH_2CO_2CH_3$ | H |
| 3-$CH_3$ | H | Cl | $CH_3$ | 0 | 4-$CH_2CO_2CH_3$ | H |
| 4-$CH_3$ | H | Cl | $CH_3$ | 0 | 4-$CH_2CO_2CH_3$ | H |
| 3-$CH_3$ | F | Cl | $CH_3$ | 0 | 4-$CH_2CO_2CH_3$ | H |

Tabelle B (Fortsetzung)

| $R_n$ | $R^1$ | $R^2$ | $R^3$ | m | $(R^a)_q$ | $R^4$ |
|---|---|---|---|---|---|---|
| 4-$CH_3$ | F | Cl | $CH_3$ | 0 | 4-$CH_2CO_2CH_3$ | H |
| 3-$CH_3$ | H | F | H | 0 | 4-$CH_2SH$ | H |
| 4-$CH_3$ | H | F | H | 0 | 4-$CH_2SH$ | H |
| 3-$CH_3$ | F | F | H | 0 | 4-$CH_2SH$ | H |
| 4-$CH_3$ | F | F | H | 0 | 4-$CH_2SH$ | H |
| 3-$CH_3$ | H | Cl | H | 0 | 4-$CH_2SH$ | H |
| 4-$CH_3$ | H | Cl | H | 0 | 4-$CH_2SH$ | H |
| 3-$CH_3$ | F | Cl | H | 0 | 4-$CH_2SH$ | H |
| 4-$CH_3$ | F | Cl | H | 0 | 4-$CH_2SH$ | H |
| 3-$CH_3$ | H | F | $CH_3$ | 0 | 4-$CH_2SH$ | H |
| 4-$CH_3$ | H | F | $CH_3$ | 0 | 4-$CH_2SH$ | H |
| 3-$CH_3$ | F | F | $CH_3$ | 0 | 4-$CH_2SH$ | H |
| 4-$CH_3$ | F | F | $CH_3$ | 0 | 4-$CH_2SH$ | H |
| 3-$CH_3$ | H | Cl | $CH_3$ | 0 | 4-$CH_2SH$ | H |
| 4-$CH_3$ | H | Cl | $CH_3$ | 0 | 4-$CH_2SH$ | H |
| 3-$CH_3$ | F | Cl | $CH_3$ | 0 | 4-$CH_2SH$ | H |
| 4-$CH_3$ | F | Cl | $CH_3$ | 0 | 4-$CH_2SH$ | H |
| 3-$CH_3$ | H | F | H | 0 | 4-$CH_2SCH_2CH_3$ | H |
| 4-$CH_3$ | H | F | H | 0 | 4-$CH_2SCH_2CH_3$ | H |
| 3-$CH_3$ | F | F | H | 0 | 4-$CH_2SCH_2CH_3$ | H |
| 4-$CH_3$ | F | F | H | 0 | 4-$CH_2SCH_2CH_3$ | H |
| 3-$CH_3$ | H | Cl | H | 0 | 4-$CH_2SCH_2CH_3$ | H |
| 4-$CH_3$ | H | Cl | H | 0 | 4-$CH_2SCH_2CH_3$ | H |
| 3-$CH_3$ | F | Cl | H | 0 | 4-$CH_2SCH_2CH_3$ | H |
| 4-$CH_3$ | F | Cl | H | 0 | 4-$CH_2SCH_2CH_3$ | H |
| 3-$CH_3$ | H | F | $CH_3$ | 0 | 4-$CH_2SCH_2CH_3$ | H |
| 4-$CH_3$ | H | F | $CH_3$ | 0 | 4-$CH_2SCH_2CH_3$ | H |
| 3-$CH_3$ | F | F | $CH_3$ | 0 | 4-$CH_2SCH_2CH_3$ | H |
| 4-$CH_3$ | F | F | $CH_3$ | 0 | 4-$CH_2SCH_2CH_3$ | H |
| 3-$CH_3$ | H | Cl | $CH_3$ | 0 | 4-$CH_2SCH_2CH_3$ | H |
| 4-$CH_3$ | H | Cl | $CH_3$ | 0 | 4-$CH_2SCH_2CH_3$ | H |
| 3-$CH_3$ | F | Cl | $CH_3$ | 0 | 4-$CH_2SCH_2CH_3$ | H |
| 4-$CH_3$ | F | Cl | $CH_3$ | 0 | 4-$CH_2SCH_2CH_3$ | H |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 4-$CH_2SCH_2CH=CH_2$ | H |
| 4-$CH_3$ | H | F | H | 0 | 4-$CH_2SCH_2CH=CH_2$ | H |
| 3-$CH_3$ | F | F | H | 0 | 4-$CH_2SCH_2CH=CH_2$ | H |
| 4-$CH_3$ | F | F | H | 0 | 4-$CH_2SCH_2CH=CH_2$ | H |
| 3-$CH_3$ | H | Cl | H | 0 | 4-$CH_2SCH_2CH=CH_2$ | H |
| 4-$CH_3$ | H | Cl | H | 0 | 4-$CH_2SCH_2CH=CH_2$ | H |
| 3-$CH_3$ | F | Cl | H | 0 | 4-$CH_2SCH_2CH=CH_2$ | H |
| 4-$CH_3$ | F | Cl | H | 0 | 4-$CH_2SCH_2CH=CH_2$ | H |
| 3-$CH_3$ | H | F | $CH_3$ | 0 | 4-$CH_2SCH_2CH=CH_2$ | H |
| 4-$CH_3$ | H | F | $CH_3$ | 0 | 4-$CH_2SCH_2CH=CH_2$ | H |
| 3-$CH_3$ | F | F | $CH_3$ | 0 | 4-$CH_2SCH_2CH=CH_2$ | H |
| 4-$CH_3$ | F | F | $CH_3$ | 0 | 4-$CH_2SCH_2CH=CH_2$ | H |
| 3-$CH_3$ | H | Cl | $CH_3$ | 0 | 4-$CH_2SCH_2CH=CH_2$ | H |
| 4-$CH_3$ | H | Cl | $CH_3$ | 0 | 4-$CH_2SCH_2CH=CH_2$ | H |
| 3-$CH_3$ | F | Cl | $CH_3$ | 0 | 4-$CH_2SCH_2CH=CH_2$ | H |
| 4-$CH_3$ | F | Cl | $CH_3$ | 0 | 4-$CH_2SCH_2CH=CH_2$ | H |
| 3-$CH_3$ | H | F | H | 0 | 4-$CO_2H$ | H |
| 4-$CH_3$ | H | F | H | 0 | 4-$CO_2H$ | H |
| 3-$CH_3$ | F | F | H | 0 | 4-$CO_2H$ | H |
| 4-$CH_3$ | F | F | H | 0 | 4-$CO_2H$ | H |
| 3-$CH_3$ | H | Cl | H | 0 | 4-$CO_2H$ | H |
| 4-$CH_3$ | H | Cl | H | 0 | 4-$CO_2H$ | H |
| 3-$CH_3$ | F | Cl | H | 0 | 4-$CO_2H$ | H |
| 4-$CH_3$ | F | Cl | H | 0 | 4-$CO_2H$ | H |
| 3-$CH_3$ | H | F | $CH_3$ | 0 | 4-$CO_2H$ | H |
| 4-$CH_3$ | H | F | $CH_3$ | 0 | 4-$CO_2H$ | H |
| 3-$CH_3$ | F | F | $CH_3$ | 0 | 4-$CO_2H$ | H |
| 4-$CH_3$ | F | F | $CH_3$ | 0 | 4-$CO_2H$ | H |
| 3-$CH_3$ | H | Cl | $CH_3$ | 0 | 4-$CO_2H$ | H |
| 4-$CH_3$ | H | Cl | $CH_3$ | 0 | 4-$CO_2H$ | H |
| 3-$CH_3$ | F | Cl | $CH_3$ | 0 | 4-$CO_2H$ | H |
| 4-$CH_3$ | F | Cl | $CH_3$ | 0 | 4-$CO_2H$ | H |
| 3-$CH_3$ | H | F | H | 1 | H | H |

Tabelle B (Fortsetzung)

| $R_n$ | $R^1$ | $R^2$ | $R^3$ | m | $(R^a)_q$ | $R^4$ |
|---|---|---|---|---|---|---|
| 4-$CH_3$ | H | F | H | 1 | H | H |
| 3-$CH_3$ | F | F | H | 1 | H | H |
| 4-$CH_3$ | F | F | H | 1 | H | H |
| 3-$CH_3$ | H | Cl | H | 1 | H | H |
| 4-$CH_3$ | H | Cl | H | 1 | H | H |
| 3-$CH_3$ | F | Cl | H | 1 | H | H |
| 4-$CH_3$ | F | Cl | H | 1 | H | H |
| 3-$CH_3$ | H | F | $CH_3$ | 1 | H | H |
| 4-$CH_3$ | H | F | $CH_3$ | 1 | H | H |
| 3-$CH_3$ | F | F | $CH_3$ | 1 | H | H |
| 4-$CH_3$ | F | F | $CH_3$ | 1 | H | H |
| 3-$CH_3$ | H | Cl | $CH_3$ | 1 | H | H |
| 4-$CH_3$ | H | Cl | $CH_3$ | 1 | H | H |
| 3-$CH_3$ | F | Cl | $CH_3$ | 1 | H | H |
| 4-$CH_3$ | F | Cl | $CH_3$ | 1 | H | H |
| 3-$CH_3$ | H | F | H | 1 | 4-$CH_3$ | H |
| 4-$CH_3$ | H | F | H | 1 | 4-$CH_3$ | H |
| 3-$CH_3$ | F | F | H | 1 | 4-$CH_3$ | H |
| 4-$CH_3$ | F | F | H | 1 | 4-$CH_3$ | H |
| 3-$CH_3$ | H | Cl | H | 1 | 4-$CH_3$ | H |
| 4-$CH_3$ | H | Cl | H | 1 | 4-$CH_3$ | H |
| 3-$CH_3$ | F | Cl | H | 1 | 4-$CH_3$ | H |
| 4-$CH_3$ | F | Cl | H | 1 | 4-$CH_3$ | H |
| 3-$CH_3$ | H | F | $CH_3$ | 1 | 4-$CH_3$ | H |
| 4-$CH_3$ | H | F | $CH_3$ | 1 | 4-$CH_3$ | H |
| 3-$CH_3$ | F | F | $CH_3$ | 1 | 4-$CH_3$ | H |
| 4-$CH_3$ | F | F | $CH_3$ | 1 | 4-$CH_3$ | H |
| 3-$CH_3$ | H | Cl | $CH_3$ | 1 | 4-$CH_3$ | H |
| 4-$CH_3$ | H | Cl | $CH_3$ | 1 | 4-$CH_3$ | H |
| 3-$CH_3$ | F | Cl | $CH_3$ | 1 | 4-$CH_3$ | H |
| 4-$CH_3$ | F | Cl | $CH_3$ | 1 | 4-$CH_3$ | H |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4,6-Di-$CH_3$ | H |
| 4-$CH_3$ | H | F | H | 1 | 4,6-Di-$CH_3$ | H |
| 3-$CH_3$ | F | F | H | 1 | 4,6-Di-$CH_3$ | H |
| 4-$CH_3$ | F | F | H | 1 | 4,6-Di-$CH_3$ | H |
| 3-$CH_3$ | H | Cl | H | 1 | 4,6-Di-$CH_3$ | H |
| 4-$CH_3$ | H | Cl | H | 1 | 4,6-Di-$CH_3$ | H |
| 3-$CH_3$ | F | Cl | H | 1 | 4,6-Di-$CH_3$ | H |
| 4-$CH_3$ | F | Cl | H | 1 | 4,6-Di-$CH_3$ | H |
| 3-$CH_3$ | H | F | $CH_3$ | 1 | 4,6-Di-$CH_3$ | H |
| 4-$CH_3$ | H | F | $CH_3$ | 1 | 4,6-Di-$CH_3$ | H |
| 3-$CH_3$ | F | F | $CH_3$ | 1 | 4,6-Di-$CH_3$ | H |
| 4-$CH_3$ | F | F | $CH_3$ | 1 | 4,6-Di-$CH_3$ | H |
| 3-$CH_3$ | H | Cl | $CH_3$ | 1 | 4,6-Di-$CH_3$ | H |
| 4-$CH_3$ | H | Cl | $CH_3$ | 1 | 4,6-Di-$CH_3$ | H |
| 3-$CH_3$ | F | Cl | $CH_3$ | 1 | 4,6-Di-$CH_3$ | H |
| 4-$CH_3$ | F | Cl | $CH_3$ | 1 | 4,6-Di-$CH_3$ | H |
| 3-$CH_3$ | H | F | H | 1 | 4-$CH_2CH_3$ | H |
| 4-$CH_3$ | H | F | H | 1 | 4-$CH_2CH_3$ | H |
| 3-$CH_3$ | F | F | H | 1 | 4-$CH_2CH_3$ | H |
| 4-$CH_3$ | F | F | H | 1 | 4-$CH_2CH_3$ | H |
| 3-$CH_3$ | H | Cl | H | 1 | 4-$CH_2CH_3$ | H |
| 4-$CH_3$ | H | Cl | H | 1 | 4-$CH_2CH_3$ | H |
| 3-$CH_3$ | F | Cl | H | 1 | 4-$CH_2CH_3$ | H |
| 4-$CH_3$ | F | Cl | H | 1 | 4-$CH_2CH_3$ | H |
| 3-$CH_3$ | H | F | $CH_3$ | 1 | 4-$CH_2CH_3$ | H |
| 4-$CH_3$ | H | F | $CH_3$ | 1 | 4-$CH_2CH_3$ | H |
| 3-$CH_3$ | F | F | $CH_3$ | 1 | 4-$CH_2CH_3$ | H |
| 4-$CH_3$ | F | F | $CH_3$ | 1 | 4-$CH_2CH_3$ | H |
| 3-$CH_3$ | H | Cl | $CH_3$ | 1 | 4-$CH_2CH_3$ | H |
| 4-$CH_3$ | H | Cl | $CH_3$ | 1 | 4-$CH_2CH_3$ | H |
| 3-$CH_3$ | F | Cl | $CH_3$ | 1 | 4-$CH_2CH_3$ | H |
| 4-$CH_3$ | F | Cl | $CH_3$ | 1 | 4-$CH_2CH_3$ | H |
| 3-$CH_3$ | H | F | H | 1 | 4-$CH_2Cl$ | H |

Tabelle B (Fortsetzung)

| $R_n$ | $R^1$ | $R^2$ | $R^3$ | m | $(R^a)_q$ | $R^4$ |
|---|---|---|---|---|---|---|
| 4-$CH_3$ | H | F | H | 1 | 4-$CH_2Cl$ | H |
| 3-$CH_3$ | F | F | H | 1 | 4-$CH_2Cl$ | H |
| 4-$CH_3$ | F | F | H | 1 | 4-$CH_2Cl$ | H |
| 3-$CH_3$ | H | Cl | H | 1 | 4-$CH_2Cl$ | H |
| 4-$CH_3$ | H | Cl | H | 1 | 4-$CH_2Cl$ | H |
| 3-$CH_3$ | F | Cl | H | 1 | 4-$CH_2Cl$ | H |
| 4-$CH_3$ | F | Cl | H | 1 | 4-$CH_2Cl$ | H |
| 3-$CH_3$ | H | F | $CH_3$ | 1 | 4-$CH_2Cl$ | H |
| 4-$CH_3$ | H | F | $CH_3$ | 1 | 4-$CH_2Cl$ | H |
| 3-$CH_3$ | F | F | $CH_3$ | 1 | 4-$CH_2Cl$ | H |
| 4-$CH_3$ | F | F | $CH_3$ | 1 | 4-$CH_2Cl$ | H |
| 3-$CH_3$ | H | Cl | $CH_3$ | 1 | 4-$CH_2Cl$ | H |
| 4-$CH_3$ | H | Cl | $CH_3$ | 1 | 4-$CH_2Cl$ | H |
| 3-$CH_3$ | F | Cl | $CH_3$ | 1 | 4-$CH_2Cl$ | H |
| 4-$CH_3$ | F | Cl | $CH_3$ | 1 | 4-$CH_2Cl$ | H |
| 3-$CH_3$ | H | F | H | 1 | 4-$CH_2OH$ | H |
| 4-$CH_3$ | H | F | H | 1 | 4-$CH_2OH$ | H |
| 3-$CH_3$ | F | F | H | 1 | 4-$CH_2OH$ | H |
| 4-$CH_3$ | F | F | H | 1 | 4-$CH_2OH$ | H |
| 3-$CH_3$ | H | Cl | H | 1 | 4-$CH_2OH$ | H |
| 4-$CH_3$ | H | Cl | H | 1 | 4-$CH_2OH$ | H |
| 3-$CH_3$ | F | Cl | H | 1 | 4-$CH_2OH$ | H |
| 4-$CH_3$ | F | Cl | H | 1 | 4-$CH_2OH$ | H |
| 3-$CH_3$ | H | F | $CH_3$ | 1 | 4-$CH_2OH$ | H |
| 4-$CH_3$ | H | F | $CH_3$ | 1 | 4-$CH_2OH$ | H |
| 3-$CH_3$ | F | F | $CH_3$ | 1 | 4-$CH_2OH$ | H |
| 4-$CH_3$ | F | F | $CH_3$ | 1 | 4-$CH_2OH$ | H |
| 3-$CH_3$ | H | Cl | $CH_3$ | 1 | 4-$CH_2OH$ | H |
| 4-$CH_3$ | H | Cl | $CH_3$ | 1 | 4-$CH_2OH$ | H |
| 3-$CH_3$ | F | Cl | $CH_3$ | 1 | 4-$CH_2OH$ | H |
| 4-$CH_3$ | F | Cl | $CH_3$ | 1 | 4-$CH_2OH$ | H |

Tabelle B (Fortsetzung)

| $R_n$ | $R^1$ | $R^2$ | $R^3$ | m | $(R^a)_q$ | $R^4$ |
|---|---|---|---|---|---|---|
| $3-CH_3$ | H | F | H | 1 | $4-CH_2OCH_3$ | H |
| $4-CH_3$ | H | F | H | 1 | $4-CH_2OCH_3$ | H |
| $3-CH_3$ | F | F | H | 1 | $4-CH_2OCH_3$ | H |
| $4-CH_3$ | F | F | H | 1 | $4-CH_2OCH_3$ | H |
| $3-CH_3$ | H | Cl | H | 1 | $4-CH_2OCH_3$ | H |
| $4-CH_3$ | H | Cl | H | 1 | $4-CH_2OCH_3$ | H |
| $3-CH_3$ | F | Cl | H | 1 | $4-CH_2OCH_3$ | H |
| $4-CH_3$ | F | Cl | H | 1 | $4-CH_2OCH_3$ | H |
| $3-CH_3$ | H | F | $CH_3$ | 1 | $4-CH_2OCH_3$ | H |
| $4-CH_3$ | H | F | $CH_3$ | 1 | $4-CH_2OCH_3$ | H |
| $3-CH_3$ | F | F | $CH_3$ | 1 | $4-CH_2OCH_3$ | H |
| $4-CH_3$ | F | F | $CH_3$ | 1 | $4-CH_2OCH_3$ | H |
| $3-CH_3$ | H | Cl | $CH_3$ | 1 | $4-CH_2OCH_3$ | H |
| $4-CH_3$ | H | Cl | $CH_3$ | 1 | $4-CH_2OCH_3$ | H |
| $3-CH_3$ | F | Cl | $CH_3$ | 1 | $4-CH_2OCH_3$ | H |
| $4-CH_3$ | F | Cl | $CH_3$ | 1 | $4-CH_2OCH_3$ | H |
| $3-CH_3$ | H | F | H | 1 | $4-CH_2OCH_2CH=CH_2$ | H |
| $4-CH_3$ | H | F | H | 1 | $4-CH_2OCH_2CH=CH_2$ | H |
| $3-CH_3$ | F | F | H | 1 | $4-CH_2OCH_2CH=CH_2$ | H |
| $4-CH_3$ | F | F | H | 1 | $4-CH_2OCH_2CH=CH_2$ | H |
| $3-CH_3$ | H | Cl | H | 1 | $4-CH_2OCH_2CH=CH_2$ | H |
| $4-CH_3$ | H | Cl | H | 1 | $4-CH_2OCH_2CH=CH_2$ | H |
| $3-CH_3$ | F | Cl | H | 1 | $4-CH_2OCH_2CH=CH_2$ | H |
| $4-CH_3$ | F | Cl | H | 1 | $4-CH_2OCH_2CH=CH_2$ | H |
| $3-CH_3$ | H | F | $CH_3$ | 1 | $4-CH_2OCH_2CH=CH_2$ | H |
| $4-CH_3$ | H | F | $CH_3$ | 1 | $4-CH_2OCH_2CH=CH_2$ | H |
| $3-CH_3$ | F | F | $CH_3$ | 1 | $4-CH_2OCH_2CH=CH_2$ | H |
| $4-CH_3$ | F | F | $CH_3$ | 1 | $4-CH_2OCH_2CH=CH_2$ | H |
| $3-CH_3$ | H | Cl | $CH_3$ | 1 | $4-CH_2OCH_2CH=CH_2$ | H |
| $4-CH_3$ | H | Cl | $CH_3$ | 1 | $4-CH_2OCH_2CH=CH_2$ | H |
| $3-CH_3$ | F | Cl | $CH_3$ | 1 | $4-CH_2OCH_2CH=CH_2$ | H |
| $4-CH_3$ | F | Cl | $CH_3$ | 1 | $4-CH_2OCH_2CH=CH_2$ | H |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4-$CH_2OCH_2C{\equiv}CH$ | H |
| 4-$CH_3$ | H | F | H | 1 | 4-$CH_2OCH_2C{\equiv}CH$ | H |
| 3-$CH_3$ | F | F | H | 1 | 4-$CH_2OCH_2C{\equiv}CH$ | H |
| 4-$CH_3$ | F | F | H | 1 | 4-$CH_2OCH_2C{\equiv}CH$ | H |
| 3-$CH_3$ | H | Cl | H | 1 | 4-$CH_2OCH_2C{\equiv}CH$ | H |
| 4-$CH_3$ | H | Cl | H | 1 | 4-$CH_2OCH_2C{\equiv}CH$ | H |
| 3-$CH_3$ | F | Cl | H | 1 | 4-$CH_2OCH_2C{\equiv}CH$ | H |
| 4-$CH_3$ | F | Cl | H | 1 | 4-$CH_2OCH_2C{\equiv}CH$ | H |
| 3-$CH_3$ | H | F | $CH_3$ | 1 | 4-$CH_2OCH_2C{\equiv}CH$ | H |
| 4-$CH_3$ | H | F | $CH_3$ | 1 | 4-$CH_2OCH_2C{\equiv}CH$ | H |
| 3-$CH_3$ | F | F | $CH_3$ | 1 | 4-$CH_2OCH_2C{\equiv}CH$ | H |
| 4-$CH_3$ | F | F | $CH_3$ | 1 | 4-$CH_2OCH_2C{\equiv}CH$ | H |
| 3-$CH_3$ | H | Cl | $CH_3$ | 1 | 4-$CH_2OCH_2C{\equiv}CH$ | H |
| 4-$CH_3$ | H | Cl | $CH_3$ | 1 | 4-$CH_2OCH_2C{\equiv}CH$ | H |
| 3-$CH_3$ | F | Cl | $CH_3$ | 1 | 4-$CH_2OCH_2C{\equiv}CH$ | H |
| 4-$CH_3$ | F | Cl | $CH_3$ | 1 | 4-$CH_2OCH_2C{\equiv}CH$ | H |
| 3-$CH_3$ | H | F | H | 1 | 4-$CH_2OCOCH_3$ | H |
| 4-$CH_3$ | H | F | H | 1 | 4-$CH_2OCOCH_3$ | H |
| 3-$CH_3$ | F | F | H | 1 | 4-$CH_2OCOCH_3$ | H |
| 4-$CH_3$ | F | F | H | 1 | 4-$CH_2OCOCH_3$ | H |
| 3-$CH_3$ | H | Cl | H | 1 | 4-$CH_2OCOCH_3$ | H |
| 4-$CH_3$ | H | Cl | H | 1 | 4-$CH_2OCOCH_3$ | H |
| 3-$CH_3$ | F | Cl | H | 1 | 4-$CH_2OCOCH_3$ | H |
| 4-$CH_3$ | F | Cl | H | 1 | 4-$CH_2OCOCH_3$ | H |
| 3-$CH_3$ | H | F | $CH_3$ | 1 | 4-$CH_2OCOCH_3$ | H |
| 4-$CH_3$ | H | F | $CH_3$ | 1 | 4-$CH_2OCOCH_3$ | H |
| 3-$CH_3$ | F | F | $CH_3$ | 1 | 4-$CH_2OCOCH_3$ | H |
| 4-$CH_3$ | F | F | $CH_3$ | 1 | 4-$CH_2OCOCH_3$ | H |
| 3-$CH_3$ | H | Cl | $CH_3$ | 1 | 4-$CH_2OCOCH_3$ | H |
| 4-$CH_3$ | H | Cl | $CH_3$ | 1 | 4-$CH_2OCOCH_3$ | H |
| 3-$CH_3$ | F | Cl | $CH_3$ | 1 | 4-$CH_2OCOCH_3$ | H |
| 4-$CH_3$ | F | Cl | $CH_3$ | 1 | 4-$CH_2OCOCH_3$ | H |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4-$CH_2OCH_2CO_2CH_3$ | H |
| 4-$CH_3$ | H | F | H | 1 | 4-$CH_2OCH_2CO_2CH_3$ | H |
| 3-$CH_3$ | F | F | H | 1 | 4-$CH_2OCH_2CO_2CH_3$ | H |
| 4-$CH_3$ | F | F | H | 1 | 4-$CH_2OCH_2CO_2CH_3$ | H |
| 3-$CH_3$ | H | Cl | H | 1 | 4-$CH_2OCH_2CO_2CH_3$ | H |
| 4-$CH_3$ | H | Cl | H | 1 | 4-$CH_2OCH_2CO_2CH_3$ | H |
| 3-$CH_3$ | F | Cl | H | 1 | 4-$CH_2OCH_2CO_2CH_3$ | H |
| 4-$CH_3$ | F | Cl | H | 1 | 4-$CH_2OCH_2CO_2CH_3$ | H |
| 3-$CH_3$ | H | F | $CH_3$ | 1 | 4-$CH_2OCH_2CO_2CH_3$ | H |
| 4-$CH_3$ | H | F | $CH_3$ | 1 | 4-$CH_2OCH_2CO_2CH_3$ | H |
| 3-$CH_3$ | F | F | $CH_3$ | 1 | 4-$CH_2OCH_2CO_2CH_3$ | H |
| 4-$CH_3$ | F | F | $CH_3$ | 1 | 4-$CH_2OCH_2CO_2CH_3$ | H |
| 3-$CH_3$ | H | Cl | $CH_3$ | 1 | 4-$CH_2OCH_2CO_2CH_3$ | H |
| 4-$CH_3$ | H | Cl | $CH_3$ | 1 | 4-$CH_2OCH_2CO_2CH_3$ | H |
| 3-$CH_3$ | F | Cl | $CH_3$ | 1 | 4-$CH_2OCH_2CO_2CH_3$ | H |
| 4-$CH_3$ | F | Cl | $CH_3$ | 1 | 4-$CH_2OCH_2CO_2CH_3$ | H |
| 3-$CH_3$ | H | F | H | 1 | 4-$CH_2CO_2CH_3$ | H |
| 4-$CH_3$ | H | F | H | 1 | 4-$CH_2CO_2CH_3$ | H |
| 3-$CH_3$ | F | F | H | 1 | 4-$CH_2CO_2CH_3$ | H |
| 4-$CH_3$ | F | F | H | 1 | 4-$CH_2CO_2CH_3$ | H |
| 3-$CH_3$ | H | Cl | H | 1 | 4-$CH_2CO_2CH_3$ | H |
| 4-$CH_3$ | H | Cl | H | 1 | 4-$CH_2CO_2CH_3$ | H |
| 3-$CH_3$ | F | Cl | H | 1 | 4-$CH_2CO_2CH_3$ | H |
| 4-$CH_3$ | F | Cl | H | 1 | 4-$CH_2CO_2CH_3$ | H |
| 3-$CH_3$ | H | F | $CH_3$ | 1 | 4-$CH_2CO_2CH_3$ | H |
| 4-$CH_3$ | H | F | $CH_3$ | 1 | 4-$CH_2CO_2CH_3$ | H |
| 3-$CH_3$ | F | F | $CH_3$ | 1 | 4-$CH_2CO_2CH_3$ | H |
| 4-$CH_3$ | F | F | $CH_3$ | 1 | 4-$CH_2CO_2CH_3$ | H |
| 3-$CH_3$ | H | Cl | $CH_3$ | 1 | 4-$CH_2CO_2CH_3$ | H |
| 4-$CH_3$ | H | Cl | $CH_3$ | 1 | 4-$CH_2CO_2CH_3$ | H |
| 3-$CH_3$ | F | Cl | $CH_3$ | 1 | 4-$CH_2CO_2CH_3$ | H |
| 4-$CH_3$ | F | Cl | $CH_3$ | 1 | 4-$CH_2CO_2CH_3$ | H |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4-$CH_2SH$ | H |
| 4-$CH_3$ | H | F | H | 1 | 4-$CH_2SH$ | H |
| 3-$CH_3$ | F | F | H | 1 | 4-$CH_2SH$ | H |
| 4-$CH_3$ | F | F | H | 1 | 4-$CH_2SH$ | H |
| 3-$CH_3$ | H | Cl | H | 1 | 4-$CH_2SH$ | H |
| 4-$CH_3$ | H | Cl | H | 1 | 4-$CH_2SH$ | H |
| 3-$CH_3$ | F | Cl | H | 1 | 4-$CH_2SH$ | H |
| 4-$CH_3$ | F | Cl | H | 1 | 4-$CH_2SH$ | H |
| 3-$CH_3$ | H | F | $CH_3$ | 1 | 4-$CH_2SH$ | H |
| 4-$CH_3$ | H | F | $CH_3$ | 1 | 4-$CH_2SH$ | H |
| 3-$CH_3$ | F | F | $CH_3$ | 1 | 4-$CH_2SH$ | H |
| 4-$CH_3$ | F | F | $CH_3$ | 1 | 4-$CH_2SH$ | H |
| 3-$CH_3$ | H | Cl | $CH_3$ | 1 | 4-$CH_2SH$ | H |
| 4-$CH_3$ | H | Cl | $CH_3$ | 1 | 4-$CH_2SH$ | H |
| 3-$CH_3$ | F | Cl | $CH_3$ | 1 | 4-$CH_2SH$ | H |
| 4-$CH_3$ | F | Cl | $CH_3$ | 1 | 4-$CH_2SH$ | H |
| 3-$CH_3$ | H | F | H | 1 | 4-$CH_2SCH_2CH_3$ | H |
| 4-$CH_3$ | H | F | H | 1 | 4-$CH_2SCH_2CH_3$ | H |
| 3-$CH_3$ | F | F | H | 1 | 4-$CH_2SCH_2CH_3$ | H |
| 4-$CH_3$ | F | F | H | 1 | 4-$CH_2SCH_2CH_3$ | H |
| 3-$CH_3$ | H | Cl | H | 1 | 4-$CH_2SCH_2CH_3$ | H |
| 4-$CH_3$ | H | Cl | H | 1 | 4-$CH_2SCH_2CH_3$ | H |
| 3-$CH_3$ | F | Cl | H | 1 | 4-$CH_2SCH_2CH_3$ | H |
| 4-$CH_3$ | F | Cl | H | 1 | 4-$CH_2SCH_2CH_3$ | H |
| 3-$CH_3$ | H | F | $CH_3$ | 1 | 4-$CH_2SCH_2CH_3$ | H |
| 4-$CH_3$ | H | F | $CH_3$ | 1 | 4-$CH_2SCH_2CH_3$ | H |
| 3-$CH_3$ | F | F | $CH_3$ | 1 | 4-$CH_2SCH_2CH_3$ | H |
| 4-$CH_3$ | F | F | $CH_3$ | 1 | 4-$CH_2SCH_2CH_3$ | H |
| 3-$CH_3$ | H | Cl | $CH_3$ | 1 | 4-$CH_2SCH_2CH_3$ | H |
| 4-$CH_3$ | H | Cl | $CH_3$ | 1 | 4-$CH_2SCH_2CH_3$ | H |
| 3-$CH_3$ | F | Cl | $CH_3$ | 1 | 4-$CH_2SCH_2CH_3$ | H |
| 4-$CH_3$ | F | Cl | $CH_3$ | 1 | 4-$CH_2SCH_2CH_3$ | H |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4-$CH_2SCH_2CH=CH_2$ | H |
| 4-$CH_3$ | H | F | H | 1 | 4-$CH_2SCH_2CH=CH_2$ | H |
| 3-$CH_3$ | F | F | H | 1 | 4-$CH_2SCH_2CH=CH_2$ | H |
| 4-$CH_3$ | F | F | H | 1 | 4-$CH_2SCH_2CH=CH_2$ | H |
| 3-$CH_3$ | H | Cl | H | 1 | 4-$CH_2SCH_2CH=CH_2$ | H |
| 4-$CH_3$ | H | Cl | H | 1 | 4-$CH_2SCH_2CH=CH_2$ | H |
| 3-$CH_3$ | F | Cl | H | 1 | 4-$CH_2SCH_2CH=CH_2$ | H |
| 4-$CH_3$ | F | Cl | H | 1 | 4-$CH_2SCH_2CH=CH_2$ | H |
| 3-$CH_3$ | H | F | $CH_3$ | 1 | 4-$CH_2SCH_2CH=CH_2$ | H |
| 4-$CH_3$ | H | F | $CH_3$ | 1 | 4-$CH_2SCH_2CH=CH_2$ | H |
| 3-$CH_3$ | F | F | $CH_3$ | 1 | 4-$CH_2SCH_2CH=CH_2$ | H |
| 4-$CH_3$ | F | F | $CH_3$ | 1 | 4-$CH_2SCH_2CH=CH_2$ | H |
| 3-$CH_3$ | H | Cl | $CH_3$ | 1 | 4-$CH_2SCH_2CH=CH_2$ | H |
| 4-$CH_3$ | H | Cl | $CH_3$ | 1 | 4-$CH_2SCH_2CH=CH_2$ | H |
| 3-$CH_3$ | F | Cl | $CH_3$ | 1 | 4-$CH_2SCH_2CH=CH_2$ | H |
| 4-$CH_3$ | F | Cl | $CH_3$ | 1 | 4-$CH_2SCH_2CH=CH_2$ | H |
| 3-$CH_3$ | H | F | H | 1 | 4-$CO_2H$ | H |
| 4-$CH_3$ | H | F | H | 1 | 4-$CO_2H$ | H |
| 3-$CH_3$ | F | F | H | 1 | 4-$CO_2H$ | H |
| 4-$CH_3$ | F | F | H | 1 | 4-$CO_2H$ | H |
| 3-$CH_3$ | H | Cl | H | 1 | 4-$CO_2H$ | H |
| 4-$CH_3$ | H | Cl | H | 1 | 4-$CO_2H$ | H |
| 3-$CH_3$ | F | Cl | H | 1 | 4-$CO_2H$ | H |
| 4-$CH_3$ | F | Cl | H | 1 | 4-$CO_2H$ | H |
| 3-$CH_3$ | H | F | $CH_3$ | 1 | 4-$CO_2H$ | H |
| 4-$CH_3$ | H | F | $CH_3$ | 1 | 4-$CO_2H$ | H |
| 3-$CH_3$ | F | F | $CH_3$ | 1 | 4-$CO_2H$ | H |
| 4-$CH_3$ | F | F | $CH_3$ | 1 | 4-$CO_2H$ | H |
| 3-$CH_3$ | H | Cl | $CH_3$ | 1 | 4-$CO_2H$ | H |
| 4-$CH_3$ | H | Cl | $CH_3$ | 1 | 4-$CO_2H$ | H |
| 3-$CH_3$ | F | Cl | $CH_3$ | 1 | 4-$CO_2H$ | H |
| 4-$CH_3$ | F | Cl | $CH_3$ | 1 | 4-$CO_2H$ | H |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | H | $CO_2CH_3$ |
| 4-$CH_3$ | H | F | H | 0 | H | $CO_2CH_3$ |
| 3-$CH_3$ | F | F | H | 0 | H | $CO_2CH_3$ |
| 4-$CH_3$ | F | F | H | 0 | H | $CO_2CH_3$ |
| 3-$CH_3$ | H | Cl | H | 0 | H | $CO_2CH_3$ |
| 4-$CH_3$ | H | Cl | H | 0 | H | $CO_2CH_3$ |
| 3-$CH_3$ | F | Cl | H | 0 | H | $CO_2CH_3$ |
| 4-$CH_3$ | F | Cl | H | 0 | H | $CO_2CH_3$ |
| 3-$CH_3$ | H | F | $CH_3$ | 0 | H | $CO_2CH_3$ |
| 4-$CH_3$ | H | F | $CH_3$ | 0 | H | $CO_2CH_3$ |
| 3-$CH_3$ | F | F | $CH_3$ | 0 | H | $CO_2CH_3$ |
| 4-$CH_3$ | F | F | $CH_3$ | 0 | H | $CO_2CH_3$ |
| 3-$CH_3$ | H | Cl | $CH_3$ | 0 | H | $CO_2CH_3$ |
| 4-$CH_3$ | H | Cl | $CH_3$ | 0 | H | $CO_2CH_3$ |
| 3-$CH_3$ | F | Cl | $CH_3$ | 0 | H | $CO_2CH_3$ |
| 4-$CH_3$ | F | Cl | $CH_3$ | 0 | H | $CO_2CH_3$ |
| 3-$CH_3$ | H | F | H | 0 | H | $CO_2CH_2CH_3$ |
| 4-$CH_3$ | H | F | H | 0 | H | $CO_2CH_2CH_3$ |
| 3-$CH_3$ | F | F | H | 0 | H | $CO_2CH_2CH_3$ |
| 4-$CH_3$ | F | F | H | 0 | H | $CO_2CH_2CH_3$ |
| 3-$CH_3$ | H | Cl | H | 0 | H | $CO_2CH_2CH_3$ |
| 4-$CH_3$ | H | Cl | H | 0 | H | $CO_2CH_2CH_3$ |
| 3-$CH_3$ | F | Cl | H | 0 | H | $CO_2CH_2CH_3$ |
| 4-$CH_3$ | F | Cl | H | 0 | H | $CO_2CH_2CH_3$ |
| 3-$CH_3$ | H | F | $CH_3$ | 0 | H | $CO_2CH_2CH_3$ |
| 4-$CH_3$ | H | F | $CH_3$ | 0 | H | $CO_2CH_2CH_3$ |
| 3-$CH_3$ | F | F | $CH_3$ | 0 | H | $CO_2CH_2CH_3$ |
| 4-$CH_3$ | F | F | $CH_3$ | 0 | H | $CO_2CH_2CH_3$ |
| 3-$CH_3$ | H | Cl | $CH_3$ | 0 | H | $CO_2CH_2CH_3$ |
| 4-$CH_3$ | H | Cl | $CH_3$ | 0 | H | $CO_2CH_2CH_3$ |
| 3-$CH_3$ | F | Cl | $CH_3$ | 0 | H | $CO_2CH_2CH_3$ |
| 4-$CH_3$ | F | Cl | $CH_3$ | 0 | H | $CO_2CH_2CH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | H | $CO_2(CH_2)_2CH_3$ |
| 4-$CH_3$ | H | F | H | 0 | H | $CO_2(CH_2)_2CH_3$ |
| 3-$CH_3$ | F | F | H | 0 | H | $CO_2(CH_2)_2CH_3$ |
| 4-$CH_3$ | F | F | H | 0 | H | $CO_2(CH_2)_2CH_3$ |
| 3-$CH_3$ | H | Cl | H | 0 | H | $CO_2(CH_2)_2CH_3$ |
| 4-$CH_3$ | H | Cl | H | 0 | H | $CO_2(CH_2)_2CH_3$ |
| 3-$CH_3$ | F | Cl | H | 0 | H | $CO_2(CH_2)_2CH_3$ |
| 4-$CH_3$ | F | Cl | H | 0 | H | $CO_2(CH_2)_2CH_3$ |
| 3-$CH_3$ | H | F | $CH_3$ | 0 | H | $CO_2(CH_2)_2CH_3$ |
| 4-$CH_3$ | H | F | $CH_3$ | 0 | H | $CO_2(CH_2)_2CH_3$ |
| 3-$CH_3$ | F | F | $CH_3$ | 0 | H | $CO_2(CH_2)_2CH_3$ |
| 4-$CH_3$ | F | F | $CH_3$ | 0 | H | $CO_2(CH_2)_2CH_3$ |
| 3-$CH_3$ | H | Cl | $CH_3$ | 0 | H | $CO_2(CH_2)_2CH_3$ |
| 4-$CH_3$ | H | Cl | $CH_3$ | 0 | H | $CO_2(CH_2)_2CH_3$ |
| 3-$CH_3$ | F | Cl | $CH_3$ | 0 | H | $CO_2(CH_2)_2CH_3$ |
| 4-$CH_3$ | F | Cl | $CH_3$ | 0 | H | $CO_2(CH_2)_2CH_3$ |
| 3-$CH_3$ | H | F | H | 0 | H | $CO_2CH(CH_3)_2$ |
| 4-$CH_3$ | H | F | H | 0 | H | $CO_2CH(CH_3)_2$ |
| 3-$CH_3$ | F | F | H | 0 | H | $CO_2CH(CH_3)_2$ |
| 4-$CH_3$ | F | F | H | 0 | H | $CO_2CH(CH_3)_2$ |
| 3-$CH_3$ | H | Cl | H | 0 | H | $CO_2CH(CH_3)_2$ |
| 4-$CH_3$ | H | Cl | H | 0 | H | $CO_2CH(CH_3)_2$ |
| 3-$CH_3$ | F | Cl | H | 0 | H | $CO_2CH(CH_3)_2$ |
| 4-$CH_3$ | F | Cl | H | 0 | H | $CO_2CH(CH_3)_2$ |
| 3-$CH_3$ | H | F | $CH_3$ | 0 | H | $CO_2CH(CH_3)_2$ |
| 4-$CH_3$ | H | F | $CH_3$ | 0 | H | $CO_2CH(CH_3)_2$ |
| 3-$CH_3$ | F | F | $CH_3$ | 0 | H | $CO_2CH(CH_3)_2$ |
| 4-$CH_3$ | F | F | $CH_3$ | 0 | H | $CO_2CH(CH_3)_2$ |
| 3-$CH_3$ | H | Cl | $CH_3$ | 0 | H | $CO_2CH(CH_3)_2$ |
| 4-$CH_3$ | H | Cl | $CH_3$ | 0 | H | $CO_2CH(CH_3)_2$ |
| 3-$CH_3$ | F | Cl | $CH_3$ | 0 | H | $CO_2CH(CH_3)_2$ |
| 4-$CH_3$ | F | Cl | $CH_3$ | 0 | H | $CO_2CH(CH_3)_2$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | H | $CO_2(CH_2)_3CH_3$ |
| 4-$CH_3$ | H | F | H | 0 | H | $CO_2(CH_2)_3CH_3$ |
| 3-$CH_3$ | F | F | H | 0 | H | $CO_2(CH_2)_3CH_3$ |
| 4-$CH_3$ | F | F | H | 0 | H | $CO_2(CH_2)_3CH_3$ |
| 3-$CH_3$ | H | Cl | H | 0 | H | $CO_2(CH_2)_3CH_3$ |
| 4-$CH_3$ | H | Cl | H | 0 | H | $CO_2(CH_2)_3CH_3$ |
| 3-$CH_3$ | F | Cl | H | 0 | H | $CO_2(CH_2)_3CH_3$ |
| 4-$CH_3$ | F | Cl | H | 0 | H | $CO_2(CH_2)_3CH_3$ |
| 3-$CH_3$ | H | F | $CH_3$ | 0 | H | $CO_2(CH_2)_3CH_3$ |
| 4-$CH_3$ | H | F | $CH_3$ | 0 | H | $CO_2(CH_2)_3CH_3$ |
| 3-$CH_3$ | F | F | $CH_3$ | 0 | H | $CO_2(CH_2)_3CH_3$ |
| 4-$CH_3$ | F | F | $CH_3$ | 0 | H | $CO_2(CH_2)_3CH_3$ |
| 3-$CH_3$ | H | Cl | $CH_3$ | 0 | H | $CO_2(CH_2)_3CH_3$ |
| 4-$CH_3$ | H | Cl | $CH_3$ | 0 | H | $CO_2(CH_2)_3CH_3$ |
| 3-$CH_3$ | F | Cl | $CH_3$ | 0 | H | $CO_2(CH_2)_3CH_3$ |
| 4-$CH_3$ | F | Cl | $CH_3$ | 0 | H | $CO_2(CH_2)_3CH_3$ |
| 3-$CH_3$ | H | F | H | 0 | H | $CO_2(CH_2)_2OCH_3$ |
| 4-$CH_3$ | H | F | H | 0 | H | $CO_2(CH_2)_2OCH_3$ |
| 3-$CH_3$ | F | F | H | 0 | H | $CO_2(CH_2)_2OCH_3$ |
| 4-$CH_3$ | F | F | H | 0 | H | $CO_2(CH_2)_2OCH_3$ |
| 3-$CH_3$ | H | Cl | H | 0 | H | $CO_2(CH_2)_2OCH_3$ |
| 4-$CH_3$ | H | Cl | H | 0 | H | $CO_2(CH_2)_2OCH_3$ |
| 3-$CH_3$ | F | Cl | H | 0 | H | $CO_2(CH_2)_2OCH_3$ |
| 4-$CH_3$ | F | Cl | H | 0 | H | $CO_2(CH_2)_2OCH_3$ |
| 3-$CH_3$ | H | F | $CH_3$ | 0 | H | $CO_2(CH_2)_2OCH_3$ |
| 4-$CH_3$ | H | F | $CH_3$ | 0 | H | $CO_2(CH_2)_2OCH_3$ |
| 3-$CH_3$ | F | F | $CH_3$ | 0 | H | $CO_2(CH_2)_2OCH_3$ |
| 4-$CH_3$ | F | F | $CH_3$ | 0 | H | $CO_2(CH_2)_2OCH_3$ |
| 3-$CH_3$ | H | Cl | $CH_3$ | 0 | H | $CO_2(CH_2)_2OCH_3$ |
| 4-$CH_3$ | H | Cl | $CH_3$ | 0 | H | $CO_2(CH_2)_2OCH_3$ |
| 3-$CH_3$ | F | Cl | $CH_3$ | 0 | H | $CO_2(CH_2)_2OCH_3$ |
| 4-$CH_3$ | F | Cl | $CH_3$ | 0 | H | $CO_2(CH_2)_2OCH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | H | $CO_2(CH_2)_2OCH_2CH_3$ |
| 4-$CH_3$ | H | F | H | 0 | H | $CO_2(CH_2)_2OCH_2CH_3$ |
| 3-$CH_3$ | F | F | H | 0 | H | $CO_2(CH_2)_2OCH_2CH_3$ |
| 4-$CH_3$ | F | F | H | 0 | H | $CO_2(CH_2)_2OCH_2CH_3$ |
| 3-$CH_3$ | H | Cl | H | 0 | H | $CO_2(CH_2)_2OCH_2CH_3$ |
| 4-$CH_3$ | H | Cl | H | 0 | H | $CO_2(CH_2)_2OCH_2CH_3$ |
| 3-$CH_3$ | F | Cl | H | 0 | H | $CO_2(CH_2)_2OCH_2CH_3$ |
| 4-$CH_3$ | F | Cl | H | 0 | H | $CO_2(CH_2)_2OCH_2CH_3$ |
| 3-$CH_3$ | H | F | $CH_3$ | 0 | H | $CO_2(CH_2)_2OCH_2CH_3$ |
| 4-$CH_3$ | H | F | $CH_3$ | 0 | H | $CO_2(CH_2)_2OCH_2CH_3$ |
| 3-$CH_3$ | F | F | $CH_3$ | 0 | H | $CO_2(CH_2)_2OCH_2CH_3$ |
| 4-$CH_3$ | F | F | $CH_3$ | 0 | H | $CO_2(CH_2)_2OCH_2CH_3$ |
| 3-$CH_3$ | H | Cl | $CH_3$ | 0 | H | $CO_2(CH_2)_2OCH_2CH_3$ |
| 4-$CH_3$ | H | Cl | $CH_3$ | 0 | H | $CO_2(CH_2)_2OCH_2CH_3$ |
| 3-$CH_3$ | F | Cl | $CH_3$ | 0 | H | $CO_2(CH_2)_2OCH_2CH_3$ |
| 4-$CH_3$ | F | Cl | $CH_3$ | 0 | H | $CO_2(CH_2)_2OCH_2CH_3$ |
| 3-$CH_3$ | H | F | H | 0 | 4-$CH_3$ | $CO_2CH_3$ |
| 4-$CH_3$ | H | F | H | 0 | 4-$CH_3$ | $CO_2CH_3$ |
| 3-$CH_3$ | F | F | H | 0 | 4-$CH_3$ | $CO_2CH_3$ |
| 4-$CH_3$ | F | F | H | 0 | 4-$CH_3$ | $CO_2CH_3$ |
| 3-$CH_3$ | H | Cl | H | 0 | 4-$CH_3$ | $CO_2CH_3$ |
| 4-$CH_3$ | H | Cl | H | 0 | 4-$CH_3$ | $CO_2CH_3$ |
| 3-$CH_3$ | F | Cl | H | 0 | 4-$CH_3$ | $CO_2CH_3$ |
| 4-$CH_3$ | F | Cl | H | 0 | 4-$CH_3$ | $CO_2CH_3$ |
| 3-$CH_3$ | H | F | $CH_3$ | 0 | 4-$CH_3$ | $CO_2CH_3$ |
| 4-$CH_3$ | H | F | $CH_3$ | 0 | 4-$CH_3$ | $CO_2CH_3$ |
| 3-$CH_3$ | F | F | $CH_3$ | 0 | 4-$CH_3$ | $CO_2CH_3$ |
| 4-$CH_3$ | F | F | $CH_3$ | 0 | 4-$CH_3$ | $CO_2CH_3$ |
| 3-$CH_3$ | H | Cl | $CH_3$ | 0 | 4-$CH_3$ | $CO_2CH_3$ |
| 4-$CH_3$ | H | Cl | $CH_3$ | 0 | 4-$CH_3$ | $CO_2CH_3$ |
| 3-$CH_3$ | F | Cl | $CH_3$ | 0 | 4-$CH_3$ | $CO_2CH_3$ |
| 4-$CH_3$ | F | Cl | $CH_3$ | 0 | 4-$CH_3$ | $CO_2CH_3$ |

Tabelle 8 (Fortsetzung)

| $R_n$ | $R^1$ | $R^2$ | $R^3$ | m | $(R^a)_q$ | $R^4$ |
|---|---|---|---|---|---|---|
| $3-CH_3$ | H | F | H | 0 | $4-CH_3$ | $CO_2CH_2CH_3$ |
| $4-CH_3$ | H | F | H | 0 | $4-CH_3$ | $CO_2CH_2CH_3$ |
| $3-CH_3$ | F | F | H | 0 | $4-CH_3$ | $CO_2CH_2CH_3$ |
| $4-CH_3$ | F | F | H | 0 | $4-CH_3$ | $CO_2CH_2CH_3$ |
| $3-CH_3$ | H | Cl | H | 0 | $4-CH_3$ | $CO_2CH_2CH_3$ |
| $4-CH_3$ | H | Cl | H | 0 | $4-CH_3$ | $CO_2CH_2CH_3$ |
| $3-CH_3$ | F | Cl | H | 0 | $4-CH_3$ | $CO_2CH_2CH_3$ |
| $4-CH_3$ | F | Cl | H | 0 | $4-CH_3$ | $CO_2CH_2CH_3$ |
| $3-CH_3$ | H | F | $CH_3$ | 0 | $4-CH_3$ | $CO_2CH_2CH_3$ |
| $4-CH_3$ | H | F | $CH_3$ | 0 | $4-CH_3$ | $CO_2CH_2CH_3$ |
| $3-CH_3$ | F | F | $CH_3$ | 0 | $4-CH_3$ | $CO_2CH_2CH_3$ |
| $4-CH_3$ | F | F | $CH_3$ | 0 | $4-CH_3$ | $CO_2CH_2CH_3$ |
| $3-CH_3$ | H | Cl | $CH_3$ | 0 | $4-CH_3$ | $CO_2CH_2CH_3$ |
| $4-CH_3$ | H | Cl | $CH_3$ | 0 | $4-CH_3$ | $CO_2CH_2CH_3$ |
| $3-CH_3$ | F | Cl | $CH_3$ | 0 | $4-CH_3$ | $CO_2CH_2CH_3$ |
| $4-CH_3$ | F | Cl | $CH_3$ | 0 | $4-CH_3$ | $CO_2CH_2CH_3$ |
| $3-CH_3$ | H | F | H | 0 | $4-CH_3$ | $CO_2(CH_2)_2CH_3$ |
| $4-CH_3$ | H | F | H | 0 | $4-CH_3$ | $CO_2(CH_2)_2CH_3$ |
| $3-CH_3$ | F | F | H | 0 | $4-CH_3$ | $CO_2(CH_2)_2CH_3$ |
| $4-CH_3$ | F | F | H | 0 | $4-CH_3$ | $CO_2(CH_2)_2CH_3$ |
| $3-CH_3$ | H | Cl | H | 0 | $4-CH_3$ | $CO_2(CH_2)_2CH_3$ |
| $4-CH_3$ | H | Cl | H | 0 | $4-CH_3$ | $CO_2(CH_2)_2CH_3$ |
| $3-CH_3$ | F | Cl | H | 0 | $4-CH_3$ | $CO_2(CH_2)_2CH_3$ |
| $4-CH_3$ | F | Cl | H | 0 | $4-CH_3$ | $CO_2(CH_2)_2CH_3$ |
| $3-CH_3$ | H | F | $CH_3$ | 0 | $4-CH_3$ | $CO_2(CH_2)_2CH_3$ |
| $4-CH_3$ | H | F | $CH_3$ | 0 | $4-CH_3$ | $CO_2(CH_2)_2CH_3$ |
| $3-CH_3$ | F | F | $CH_3$ | 0 | $4-CH_3$ | $CO_2(CH_2)_2CH_3$ |
| $4-CH_3$ | F | F | $CH_3$ | 0 | $4-CH_3$ | $CO_2(CH_2)_2CH_3$ |
| $3-CH_3$ | H | Cl | $CH_3$ | 0 | $4-CH_3$ | $CO_2(CH_2)_2CH_3$ |
| $4-CH_3$ | H | Cl | $CH_3$ | 0 | $4-CH_3$ | $CO_2(CH_2)_2CH_3$ |
| $3-CH_3$ | F | Cl | $CH_3$ | 0 | $4-CH_3$ | $CO_2(CH_2)_2CH_3$ |
| $4-CH_3$ | F | Cl | $CH_3$ | 0 | $4-CH_3$ | $CO_2(CH_2)_2CH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 4-$CH_3$ | $CO_2CH(CH_3)_2$ |
| 4-$CH_3$ | H | F | H | 0 | 4-$CH_3$ | $CO_2CH(CH_3)_2$ |
| 3-$CH_3$ | F | F | H | 0 | 4-$CH_3$ | $CO_2CH(CH_3)_2$ |
| 4-$CH_3$ | F | F | H | 0 | 4-$CH_3$ | $CO_2CH(CH_3)_2$ |
| 3-$CH_3$ | H | Cl | H | 0 | 4-$CH_3$ | $CO_2CH(CH_3)_2$ |
| 4-$CH_3$ | H | Cl | H | 0 | 4-$CH_3$ | $CO_2CH(CH_3)_2$ |
| 3-$CH_3$ | F | Cl | H | 0 | 4-$CH_3$ | $CO_2CH(CH_3)_2$ |
| 4-$CH_3$ | F | Cl | H | 0 | 4-$CH_3$ | $CO_2CH(CH_3)_2$ |
| 3-$CH_3$ | H | F | $CH_3$ | 0 | 4-$CH_3$ | $CO_2CH(CH_3)_2$ |
| 4-$CH_3$ | H | F | $CH_3$ | 0 | 4-$CH_3$ | $CO_2CH(CH_3)_2$ |
| 3-$CH_3$ | F | F | $CH_3$ | 0 | 4-$CH_3$ | $CO_2CH(CH_3)_2$ |
| 4-$CH_3$ | F | F | $CH_3$ | 0 | 4-$CH_3$ | $CO_2CH(CH_3)_2$ |
| 3-$CH_3$ | H | Cl | $CH_3$ | 0 | 4-$CH_3$ | $CO_2CH(CH_3)_2$ |
| 4-$CH_3$ | H | Cl | $CH_3$ | 0 | 4-$CH_3$ | $CO_2CH(CH_3)_2$ |
| 3-$CH_3$ | F | Cl | $CH_3$ | 0 | 4-$CH_3$ | $CO_2CH(CH_3)_2$ |
| 4-$CH_3$ | F | Cl | $CH_3$ | 0 | 4-$CH_3$ | $CO_2CH(CH_3)_2$ |
| 3-$CH_3$ | H | F | H | 0 | 4-$CH_3$ | $CO_2(CH_2)_3CH_3$ |
| 4-$CH_3$ | H | F | H | 0 | 4-$CH_3$ | $CO_2(CH_2)_3CH_3$ |
| 3-$CH_3$ | F | F | H | 0 | 4-$CH_3$ | $CO_2(CH_2)_3CH_3$ |
| 4-$CH_3$ | F | F | H | 0 | 4-$CH_3$ | $CO_2(CH_2)_3CH_3$ |
| 3-$CH_3$ | H | Cl | H | 0 | 4-$CH_3$ | $CO_2(CH_2)_3CH_3$ |
| 4-$CH_3$ | H | Cl | H | 0 | 4-$CH_3$ | $CO_2(CH_2)_3CH_3$ |
| 3-$CH_3$ | F | Cl | H | 0 | 4-$CH_3$ | $CO_2(CH_2)_3CH_3$ |
| 4-$CH_3$ | F | Cl | H | 0 | 4-$CH_3$ | $CO_2(CH_2)_3CH_3$ |
| 3-$CH_3$ | H | F | $CH_3$ | 0 | 4-$CH_3$ | $CO_2(CH_2)_3CH_3$ |
| 4-$CH_3$ | H | F | $CH_3$ | 0 | 4-$CH_3$ | $CO_2(CH_2)_3CH_3$ |
| 3-$CH_3$ | F | F | $CH_3$ | 0 | 4-$CH_3$ | $CO_2(CH_2)_3CH_3$ |
| 4-$CH_3$ | F | F | $CH_3$ | 0 | 4-$CH_3$ | $CO_2(CH_2)_3CH_3$ |
| 3-$CH_3$ | H | Cl | $CH_3$ | 0 | 4-$CH_3$ | $CO_2(CH_2)_3CH_3$ |
| 4-$CH_3$ | H | Cl | $CH_3$ | 0 | 4-$CH_3$ | $CO_2(CH_2)_3CH_3$ |
| 3-$CH_3$ | F | Cl | $CH_3$ | 0 | 4-$CH_3$ | $CO_2(CH_2)_3CH_3$ |
| 4-$CH_3$ | F | Cl | $CH_3$ | 0 | 4-$CH_3$ | $CO_2(CH_2)_3CH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 4-$CH_3$ | $CO_2(CH_2)_2OCH_3$ |
| 4-$CH_3$ | H | F | H | 0 | 4-$CH_3$ | $CO_2(CH_2)_2OCH_3$ |
| 3-$CH_3$ | F | F | H | 0 | 4-$CH_3$ | $CO_2(CH_2)_2OCH_3$ |
| 4-$CH_3$ | F | F | H | 0 | 4-$CH_3$ | $CO_2(CH_2)_2OCH_3$ |
| 3-$CH_3$ | H | Cl | H | 0 | 4-$CH_3$ | $CO_2(CH_2)_2OCH_3$ |
| 4-$CH_3$ | H | Cl | H | 0 | 4-$CH_3$ | $CO_2(CH_2)_2OCH_3$ |
| 3-$CH_3$ | F | Cl | H | 0 | 4-$CH_3$ | $CO_2(CH_2)_2OCH_3$ |
| 4-$CH_3$ | F | Cl | H | 0 | 4-$CH_3$ | $CO_2(CH_2)_2OCH_3$ |
| 3-$CH_3$ | H | F | $CH_3$ | 0 | 4-$CH_3$ | $CO_2(CH_2)_2OCH_3$ |
| 4-$CH_3$ | H | F | $CH_3$ | 0 | 4-$CH_3$ | $CO_2(CH_2)_2OCH_3$ |
| 3-$CH_3$ | F | F | $CH_3$ | 0 | 4-$CH_3$ | $CO_2(CH_2)_2OCH_3$ |
| 4-$CH_3$ | F | F | $CH_3$ | 0 | 4-$CH_3$ | $CO_2(CH_2)_2OCH_3$ |
| 3-$CH_3$ | H | Cl | $CH_3$ | 0 | 4-$CH_3$ | $CO_2(CH_2)_2OCH_3$ |
| 4-$CH_3$ | H | Cl | $CH_3$ | 0 | 4-$CH_3$ | $CO_2(CH_2)_2OCH_3$ |
| 3-$CH_3$ | F | Cl | $CH_3$ | 0 | 4-$CH_3$ | $CO_2(CH_2)_2OCH_3$ |
| 4-$CH_3$ | F | Cl | $CH_3$ | 0 | 4-$CH_3$ | $CO_2(CH_2)_2OCH_3$ |
| 3-$CH_3$ | H | F | H | 0 | 4-$CH_3$ | $CO_2(CH_2)_2OCH_2CH_3$ |
| 4-$CH_3$ | H | F | H | 0 | 4-$CH_3$ | $CO_2(CH_2)_2OCH_2CH_3$ |
| 3-$CH_3$ | F | F | H | 0 | 4-$CH_3$ | $CO_2(CH_2)_2OCH_2CH_3$ |
| 4-$CH_3$ | F | F | H | 0 | 4-$CH_3$ | $CO_2(CH_2)_2OCH_2CH_3$ |
| 3-$CH_3$ | H | Cl | H | 0 | 4-$CH_3$ | $CO_2(CH_2)_2OCH_2CH_3$ |
| 4-$CH_3$ | H | Cl | H | 0 | 4-$CH_3$ | $CO_2(CH_2)_2OCH_2CH_3$ |
| 3-$CH_3$ | F | Cl | H | 0 | 4-$CH_3$ | $CO_2(CH_2)_2OCH_2CH_3$ |
| 4-$CH_3$ | F | Cl | H | 0 | 4-$CH_3$ | $CO_2(CH_2)_2OCH_2CH_3$ |
| 3-$CH_3$ | H | F | $CH_3$ | 0 | 4-$CH_3$ | $CO_2(CH_2)_2OCH_2CH_3$ |
| 4-$CH_3$ | H | F | $CH_3$ | 0 | 4-$CH_3$ | $CO_2(CH_2)_2OCH_2CH_3$ |
| 3-$CH_3$ | F | F | $CH_3$ | 0 | 4-$CH_3$ | $CO_2(CH_2)_2OCH_2CH_3$ |
| 4-$CH_3$ | F | F | $CH_3$ | 0 | 4-$CH_3$ | $CO_2(CH_2)_2OCH_2CH_3$ |
| 3-$CH_3$ | H | Cl | $CH_3$ | 0 | 4-$CH_3$ | $CO_2(CH_2)_2OCH_2CH_3$ |
| 4-$CH_3$ | H | Cl | $CH_3$ | 0 | 4-$CH_3$ | $CO_2(CH_2)_2OCH_2CH_3$ |
| 3-$CH_3$ | F | Cl | $CH_3$ | 0 | 4-$CH_3$ | $CO_2(CH_2)_2OCH_2CH_3$ |
| 4-$CH_3$ | F | Cl | $CH_3$ | 0 | 4-$CH_3$ | $CO_2(CH_2)_2OCH_2CH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 5-$CH_3$ | $CO_2CH_3$ |
| 4-$CH_3$ | H | F | H | 0 | 5-$CH_3$ | $CO_2CH_3$ |
| 3-$CH_3$ | F | F | H | 0 | 5-$CH_3$ | $CO_2CH_3$ |
| 4-$CH_3$ | F | F | H | 0 | 5-$CH_3$ | $CO_2CH_3$ |
| 3-$CH_3$ | H | Cl | H | 0 | 5-$CH_3$ | $CO_2CH_3$ |
| 4-$CH_3$ | H | Cl | H | 0 | 5-$CH_3$ | $CO_2CH_3$ |
| 3-$CH_3$ | F | Cl | H | 0 | 5-$CH_3$ | $CO_2CH_3$ |
| 4-$CH_3$ | F | Cl | H | 0 | 5-$CH_3$ | $CO_2CH_3$ |
| 3-$CH_3$ | H | F | $CH_3$ | 0 | 5-$CH_3$ | $CO_2CH_3$ |
| 4-$CH_3$ | H | F | $CH_3$ | 0 | 5-$CH_3$ | $CO_2CH_3$ |
| 3-$CH_3$ | F | F | $CH_3$ | 0 | 5-$CH_3$ | $CO_2CH_3$ |
| 4-$CH_3$ | F | F | $CH_3$ | 0 | 5-$CH_3$ | $CO_2CH_3$ |
| 3-$CH_3$ | H | Cl | $CH_3$ | 0 | 5-$CH_3$ | $CO_2CH_3$ |
| 4-$CH_3$ | H | Cl | $CH_3$ | 0 | 5-$CH_3$ | $CO_2CH_3$ |
| 3-$CH_3$ | F | Cl | $CH_3$ | 0 | 5-$CH_3$ | $CO_2CH_3$ |
| 4-$CH_3$ | F | Cl | $CH_3$ | 0 | 5-$CH_3$ | $CO_2CH_3$ |
| 3-$CH_3$ | H | F | H | 0 | 5-$CH_3$ | $CO_2CH_2CH_3$ |
| 4-$CH_3$ | H | F | H | 0 | 5-$CH_3$ | $CO_2CH_2CH_3$ |
| 3-$CH_3$ | F | F | H | 0 | 5-$CH_3$ | $CO_2CH_2CH_3$ |
| 4-$CH_3$ | F | F | H | 0 | 5-$CH_3$ | $CO_2CH_2CH_3$ |
| 3-$CH_3$ | H | Cl | H | 0 | 5-$CH_3$ | $CO_2CH_2CH_3$ |
| 4-$CH_3$ | H | Cl | H | 0 | 5-$CH_3$ | $CO_2CH_2CH_3$ |
| 3-$CH_3$ | F | Cl | H | 0 | 5-$CH_3$ | $CO_2CH_2CH_3$ |
| 4-$CH_3$ | F | Cl | H | 0 | 5-$CH_3$ | $CO_2CH_2CH_3$ |
| 3-$CH_3$ | H | F | $CH_3$ | 0 | 5-$CH_3$ | $CO_2CH_2CH_3$ |
| 4-$CH_3$ | H | F | $CH_3$ | 0 | 5-$CH_3$ | $CO_2CH_2CH_3$ |
| 3-$CH_3$ | F | F | $CH_3$ | 0 | 5-$CH_3$ | $CO_2CH_2CH_3$ |
| 4-$CH_3$ | F | F | $CH_3$ | 0 | 5-$CH_3$ | $CO_2CH_2CH_3$ |
| 3-$CH_3$ | H | Cl | $CH_3$ | 0 | 5-$CH_3$ | $CO_2CH_2CH_3$ |
| 4-$CH_3$ | H | Cl | $CH_3$ | 0 | 5-$CH_3$ | $CO_2CH_2CH_3$ |
| 3-$CH_3$ | F | Cl | $CH_3$ | 0 | 5-$CH_3$ | $CO_2CH_2CH_3$ |
| 4-$CH_3$ | F | Cl | $CH_3$ | 0 | 5-$CH_3$ | $CO_2CH_2CH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 5-$CH_3$ | $CO_2(CH_2)_2CH_3$ |
| 4-$CH_3$ | H | F | H | 0 | 5-$CH_3$ | $CO_2(CH_2)_2CH_3$ |
| 3-$CH_3$ | F | F | H | 0 | 5-$CH_3$ | $CO_2(CH_2)_2CH_3$ |
| 4-$CH_3$ | F | F | H | 0 | 5-$CH_3$ | $CO_2(CH_2)_2CH_3$ |
| 3-$CH_3$ | H | Cl | H | 0 | 5-$CH_3$ | $CO_2(CH_2)_2CH_3$ |
| 4-$CH_3$ | H | Cl | H | 0 | 5-$CH_3$ | $CO_2(CH_2)_2CH_3$ |
| 3-$CH_3$ | F | Cl | H | 0 | 5-$CH_3$ | $CO_2(CH_2)_2CH_3$ |
| 4-$CH_3$ | F | Cl | H | 0 | 5-$CH_3$ | $CO_2(CH_2)_2CH_3$ |
| 3-$CH_3$ | H | F | $CH_3$ | 0 | 5-$CH_3$ | $CO_2(CH_2)_2CH_3$ |
| 4-$CH_3$ | H | F | $CH_3$ | 0 | 5-$CH_3$ | $CO_2(CH_2)_2CH_3$ |
| 3-$CH_3$ | F | F | $CH_3$ | 0 | 5-$CH_3$ | $CO_2(CH_2)_2CH_3$ |
| 4-$CH_3$ | F | F | $CH_3$ | 0 | 5-$CH_3$ | $CO_2(CH_2)_2CH_3$ |
| 3-$CH_3$ | H | Cl | $CH_3$ | 0 | 5-$CH_3$ | $CO_2(CH_2)_2CH_3$ |
| 4-$CH_3$ | H | Cl | $CH_3$ | 0 | 5-$CH_3$ | $CO_2(CH_2)_2CH_3$ |
| 3-$CH_3$ | F | Cl | $CH_3$ | 0 | 5-$CH_3$ | $CO_2(CH_2)_2CH_3$ |
| 4-$CH_3$ | F | Cl | $CH_3$ | 0 | 5-$CH_3$ | $CO_2(CH_2)_2CH_3$ |
| 3-$CH_3$ | H | F | H | 0 | 5-$CH_3$ | $CO_2CH(CH_3)_2$ |
| 4-$CH_3$ | H | F | H | 0 | 5-$CH_3$ | $CO_2CH(CH_3)_2$ |
| 3-$CH_3$ | F | F | H | 0 | 5-$CH_3$ | $CO_2CH(CH_3)_2$ |
| 4-$CH_3$ | F | F | H | 0 | 5-$CH_3$ | $CO_2CH(CH_3)_2$ |
| 3-$CH_3$ | H | Cl | H | 0 | 5-$CH_3$ | $CO_2CH(CH_3)_2$ |
| 4-$CH_3$ | H | Cl | H | 0 | 5-$CH_3$ | $CO_2CH(CH_3)_2$ |
| 3-$CH_3$ | F | Cl | H | 0 | 5-$CH_3$ | $CO_2CH(CH_3)_2$ |
| 4-$CH_3$ | F | Cl | H | 0 | 5-$CH_3$ | $CO_2CH(CH_3)_2$ |
| 3-$CH_3$ | H | F | $CH_3$ | 0 | 5-$CH_3$ | $CO_2CH(CH_3)_2$ |
| 4-$CH_3$ | H | F | $CH_3$ | 0 | 5-$CH_3$ | $CO_2CH(CH_3)_2$ |
| 3-$CH_3$ | F | F | $CH_3$ | 0 | 5-$CH_3$ | $CO_2CH(CH_3)_2$ |
| 4-$CH_3$ | F | F | $CH_3$ | 0 | 5-$CH_3$ | $CO_2CH(CH_3)_2$ |
| 3-$CH_3$ | H | Cl | $CH_3$ | 0 | 5-$CH_3$ | $CO_2CH(CH_3)_2$ |
| 4-$CH_3$ | H | Cl | $CH_3$ | 0 | 5-$CH_3$ | $CO_2CH(CH_3)_2$ |
| 3-$CH_3$ | F | Cl | $CH_3$ | 0 | 5-$CH_3$ | $CO_2CH(CH_3)_2$ |
| 4-$CH_3$ | F | Cl | $CH_3$ | 0 | 5-$CH_3$ | $CO_2CH(CH_3)_2$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 5-$CH_3$ | $CO_2(CH_2)_3CH_3$ |
| 4-$CH_3$ | H | F | H | 0 | 5-$CH_3$ | $CO_2(CH_2)_3CH_3$ |
| 3-$CH_3$ | F | F | H | 0 | 5-$CH_3$ | $CO_2(CH_2)_3CH_3$ |
| 4-$CH_3$ | F | F | H | 0 | 5-$CH_3$ | $CO_2(CH_2)_3CH_3$ |
| 3-$CH_3$ | H | Cl | H | 0 | 5-$CH_3$ | $CO_2(CH_2)_3CH_3$ |
| 4-$CH_3$ | H | Cl | H | 0 | 5-$CH_3$ | $CO_2(CH_2)_3CH_3$ |
| 3-$CH_3$ | F | Cl | H | 0 | 5-$CH_3$ | $CO_2(CH_2)_3CH_3$ |
| 4-$CH_3$ | F | Cl | H | 0 | 5-$CH_3$ | $CO_2(CH_2)_3CH_3$ |
| 3-$CH_3$ | H | F | $CH_3$ | 0 | 5-$CH_3$ | $CO_2(CH_2)_3CH_3$ |
| 4-$CH_3$ | H | F | $CH_3$ | 0 | 5-$CH_3$ | $CO_2(CH_2)_3CH_3$ |
| 3-$CH_3$ | F | F | $CH_3$ | 0 | 5-$CH_3$ | $CO_2(CH_2)_3CH_3$ |
| 4-$CH_3$ | F | F | $CH_3$ | 0 | 5-$CH_3$ | $CO_2(CH_2)_3CH_3$ |
| 3-$CH_3$ | H | Cl | $CH_3$ | 0 | 5-$CH_3$ | $CO_2(CH_2)_3CH_3$ |
| 4-$CH_3$ | H | Cl | $CH_3$ | 0 | 5-$CH_3$ | $CO_2(CH_2)_3CH_3$ |
| 3-$CH_3$ | F | Cl | $CH_3$ | 0 | 5-$CH_3$ | $CO_2(CH_2)_3CH_3$ |
| 4-$CH_3$ | F | Cl | $CH_3$ | 0 | 5-$CH_3$ | $CO_2(CH_2)_3CH_3$ |
| 3-$CH_3$ | H | F | H | 0 | 5-$CH_3$ | $CO_2(CH_2)_2OCH_3$ |
| 4-$CH_3$ | H | F | H | 0 | 5-$CH_3$ | $CO_2(CH_2)_2OCH_3$ |
| 3-$CH_3$ | F | F | H | 0 | 5-$CH_3$ | $CO_2(CH_2)_2OCH_3$ |
| 4-$CH_3$ | F | F | H | 0 | 5-$CH_3$ | $CO_2(CH_2)_2OCH_3$ |
| 3-$CH_3$ | H | Cl | H | 0 | 5-$CH_3$ | $CO_2(CH_2)_2OCH_3$ |
| 4-$CH_3$ | H | Cl | H | 0 | 5-$CH_3$ | $CO_2(CH_2)_2OCH_3$ |
| 3-$CH_3$ | F | Cl | H | 0 | 5-$CH_3$ | $CO_2(CH_2)_2OCH_3$ |
| 4-$CH_3$ | F | Cl | H | 0 | 5-$CH_3$ | $CO_2(CH_2)_2OCH_3$ |
| 3-$CH_3$ | H | F | $CH_3$ | 0 | 5-$CH_3$ | $CO_2(CH_2)_2OCH_3$ |
| 4-$CH_3$ | H | F | $CH_3$ | 0 | 5-$CH_3$ | $CO_2(CH_2)_2OCH_3$ |
| 3-$CH_3$ | F | F | $CH_3$ | 0 | 5-$CH_3$ | $CO_2(CH_2)_2OCH_3$ |
| 4-$CH_3$ | F | F | $CH_3$ | 0 | 5-$CH_3$ | $CO_2(CH_2)_2OCH_3$ |
| 3-$CH_3$ | H | Cl | $CH_3$ | 0 | 5-$CH_3$ | $CO_2(CH_2)_2OCH_3$ |
| 4-$CH_3$ | H | Cl | $CH_3$ | 0 | 5-$CH_3$ | $CO_2(CH_2)_2OCH_3$ |
| 3-$CH_3$ | F | Cl | $CH_3$ | 0 | 5-$CH_3$ | $CO_2(CH_2)_2OCH_3$ |
| 4-$CH_3$ | F | Cl | $CH_3$ | 0 | 5-$CH_3$ | $CO_2(CH_2)_2OCH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 5-$CH_3$ | $CO_2(CH_2)_2OCH_2CH_3$ |
| 4-$CH_3$ | H | F | H | 0 | 5-$CH_3$ | $CO_2(CH_2)_2OCH_2CH_3$ |
| 3-$CH_3$ | F | F | H | 0 | 5-$CH_3$ | $CO_2(CH_2)_2OCH_2CH_3$ |
| 4-$CH_3$ | F | F | H | 0 | 5-$CH_3$ | $CO_2(CH_2)_2OCH_2CH_3$ |
| 3-$CH_3$ | H | Cl | H | 0 | 5-$CH_3$ | $CO_2(CH_2)_2OCH_2CH_3$ |
| 4-$CH_3$ | H | Cl | H | 0 | 5-$CH_3$ | $CO_2(CH_2)_2OCH_2CH_3$ |
| 3-$CH_3$ | F | Cl | H | 0 | 5-$CH_3$ | $CO_2(CH_2)_2OCH_2CH_3$ |
| 4-$CH_3$ | F | Cl | H | 0 | 5-$CH_3$ | $CO_2(CH_2)_2OCH_2CH_3$ |
| 3-$CH_3$ | H | F | $CH_3$ | 0 | 5-$CH_3$ | $CO_2(CH_2)_2OCH_2CH_3$ |
| 4-$CH_3$ | H | F | $CH_3$ | 0 | 5-$CH_3$ | $CO_2(CH_2)_2OCH_2CH_3$ |
| 3-$CH_3$ | F | F | $CH_3$ | 0 | 5-$CH_3$ | $CO_2(CH_2)_2OCH_2CH_3$ |
| 4-$CH_3$ | F | F | $CH_3$ | 0 | 5-$CH_3$ | $CO_2(CH_2)_2OCH_2CH_3$ |
| 3-$CH_3$ | H | Cl | $CH_3$ | 0 | 5-$CH_3$ | $CO_2(CH_2)_2OCH_2CH_3$ |
| 4-$CH_3$ | H | Cl | $CH_3$ | 0 | 5-$CH_3$ | $CO_2(CH_2)_2OCH_2CH_3$ |
| 3-$CH_3$ | F | Cl | $CH_3$ | 0 | 5-$CH_3$ | $CO_2(CH_2)_2OCH_2CH_3$ |
| 4-$CH_3$ | F | Cl | $CH_3$ | 0 | 5-$CH_3$ | $CO_2(CH_2)_2OCH_2CH_3$ |
| 3-$CH_3$ | H | F | H | 0 | 4,5-Di-$CH_3$ | $CO_2CH_3$ |
| 4-$CH_3$ | H | F | H | 0 | 4,5-Di-$CH_3$ | $CO_2CH_3$ |
| 3-$CH_3$ | F | F | H | 0 | 4,5-Di-$CH_3$ | $CO_2CH_3$ |
| 4-$CH_3$ | F | F | H | 0 | 4,5-Di-$CH_3$ | $CO_2CH_3$ |
| 3-$CH_3$ | H | Cl | H | 0 | 4,5-Di-$CH_3$ | $CO_2CH_3$ |
| 4-$CH_3$ | H | Cl | H | 0 | 4,5-Di-$CH_3$ | $CO_2CH_3$ |
| 3-$CH_3$ | F | Cl | H | 0 | 4,5-Di-$CH_3$ | $CO_2CH_3$ |
| 4-$CH_3$ | F | Cl | H | 0 | 4,5-Di-$CH_3$ | $CO_2CH_3$ |
| 3-$CH_3$ | H | F | $CH_3$ | 0 | 4,5-Di-$CH_3$ | $CO_2CH_3$ |
| 4-$CH_3$ | H | F | $CH_3$ | 0 | 4,5-Di-$CH_3$ | $CO_2CH_3$ |
| 3-$CH_3$ | F | F | $CH_3$ | 0 | 4,5-Di-$CH_3$ | $CO_2CH_3$ |
| 4-$CH_3$ | F | F | $CH_3$ | 0 | 4,5-Di-$CH_3$ | $CO_2CH_3$ |
| 3-$CH_3$ | H | Cl | $CH_3$ | 0 | 4,5-Di-$CH_3$ | $CO_2CH_3$ |
| 4-$CH_3$ | H | Cl | $CH_3$ | 0 | 4,5-Di-$CH_3$ | $CO_2CH_3$ |
| 3-$CH_3$ | F | Cl | $CH_3$ | 0 | 4,5-Di-$CH_3$ | $CO_2CH_3$ |
| 4-$CH_3$ | F | Cl | $CH_3$ | 0 | 4,5-Di-$CH_3$ | $CO_2CH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 4,5-Di-$CH_3$ | $CO_2CH_2CH_3$ |
| 4-$CH_3$ | H | F | H | 0 | 4,5-Di-$CH_3$ | $CO_2CH_2CH_3$ |
| 3-$CH_3$ | F | F | H | 0 | 4,5-Di-$CH_3$ | $CO_2CH_2CH_3$ |
| 4-$CH_3$ | F | F | H | 0 | 4,5-Di-$CH_3$ | $CO_2CH_2CH_3$ |
| 3-$CH_3$ | H | Cl | H | 0 | 4,5-Di-$CH_3$ | $CO_2CH_2CH_3$ |
| 4-$CH_3$ | H | Cl | H | 0 | 4,5-Di-$CH_3$ | $CO_2CH_2CH_3$ |
| 3-$CH_3$ | F | Cl | H | 0 | 4,5-Di-$CH_3$ | $CO_2CH_2CH_3$ |
| 4-$CH_3$ | F | Cl | H | 0 | 4,5-Di-$CH_3$ | $CO_2CH_2CH_3$ |
| 3-$CH_3$ | H | F | $CH_3$ | 0 | 4,5-Di-$CH_3$ | $CO_2CH_2CH_3$ |
| 4-$CH_3$ | H | F | $CH_3$ | 0 | 4,5-Di-$CH_3$ | $CO_2CH_2CH_3$ |
| 3-$CH_3$ | F | F | $CH_3$ | 0 | 4,5-Di-$CH_3$ | $CO_2CH_2CH_3$ |
| 4-$CH_3$ | F | F | $CH_3$ | 0 | 4,5-Di-$CH_3$ | $CO_2CH_2CH_3$ |
| 3-$CH_3$ | H | Cl | $CH_3$ | 0 | 4,5-Di-$CH_3$ | $CO_2CH_2CH_3$ |
| 4-$CH_3$ | H | Cl | $CH_3$ | 0 | 4,5-Di-$CH_3$ | $CO_2CH_2CH_3$ |
| 3-$CH_3$ | F | Cl | $CH_3$ | 0 | 4,5-Di-$CH_3$ | $CO_2CH_2CH_3$ |
| 4-$CH_3$ | F | Cl | $CH_3$ | 0 | 4,5-Di-$CH_3$ | $CO_2CH_2CH_3$ |
| 3-$CH_3$ | H | F | H | 0 | 4,5-Di-$CH_3$ | $CO_2(CH_2)_2CH_3$ |
| 4-$CH_3$ | H | F | H | 0 | 4,5-Di-$CH_3$ | $CO_2(CH_2)_2CH_3$ |
| 3-$CH_3$ | F | F | H | 0 | 4,5-Di-$CH_3$ | $CO_2(CH_2)_2CH_3$ |
| 4-$CH_3$ | F | F | H | 0 | 4,5-Di-$CH_3$ | $CO_2(CH_2)_2CH_3$ |
| 3-$CH_3$ | H | Cl | H | 0 | 4,5-Di-$CH_3$ | $CO_2(CH_2)_2CH_3$ |
| 4-$CH_3$ | H | Cl | H | 0 | 4,5-Di-$CH_3$ | $CO_2(CH_2)_2CH_3$ |
| 3-$CH_3$ | F | Cl | H | 0 | 4,5-Di-$CH_3$ | $CO_2(CH_2)_2CH_3$ |
| 4-$CH_3$ | F | Cl | H | 0 | 4,5-Di-$CH_3$ | $CO_2(CH_2)_2CH_3$ |
| 3-$CH_3$ | H | F | $CH_3$ | 0 | 4,5-Di-$CH_3$ | $CO_2(CH_2)_2CH_3$ |
| 4-$CH_3$ | H | F | $CH_3$ | 0 | 4,5-Di-$CH_3$ | $CO_2(CH_2)_2CH_3$ |
| 3-$CH_3$ | F | F | $CH_3$ | 0 | 4,5-Di-$CH_3$ | $CO_2(CH_2)_2CH_3$ |
| 4-$CH_3$ | F | F | $CH_3$ | 0 | 4,5-Di-$CH_3$ | $CO_2(CH_2)_2CH_3$ |
| 3-$CH_3$ | H | Cl | $CH_3$ | 0 | 4,5-Di-$CH_3$ | $CO_2(CH_2)_2CH_3$ |
| 4-$CH_3$ | H | Cl | $CH_3$ | 0 | 4,5-Di-$CH_3$ | $CO_2(CH_2)_2CH_3$ |
| 3-$CH_3$ | F | Cl | $CH_3$ | 0 | 4,5-Di-$CH_3$ | $CO_2(CH_2)_2CH_3$ |
| 4-$CH_3$ | F | Cl | $CH_3$ | 0 | 4,5-Di-$CH_3$ | $CO_2(CH_2)_2CH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 4,5-Di-$CH_3$ | $CO_2CH(CH_3)_2$ |
| 4-$CH_3$ | H | F | H | 0 | 4,5-Di-$CH_3$ | $CO_2CH(CH_3)_2$ |
| 3-$CH_3$ | F | F | H | 0 | 4,5-Di-$CH_3$ | $CO_2CH(CH_3)_2$ |
| 4-$CH_3$ | F | F | H | 0 | 4,5-Di-$CH_3$ | $CO_2CH(CH_3)_2$ |
| 3-$CH_3$ | H | Cl | H | 0 | 4,5-Di-$CH_3$ | $CO_2CH(CH_3)_2$ |
| 4-$CH_3$ | H | Cl | H | 0 | 4,5-Di-$CH_3$ | $CO_2CH(CH_3)_2$ |
| 3-$CH_3$ | F | Cl | H | 0 | 4,5-Di-$CH_3$ | $CO_2CH(CH_3)_2$ |
| 4-$CH_3$ | F | Cl | H | 0 | 4,5-Di-$CH_3$ | $CO_2CH(CH_3)_2$ |
| 3-$CH_3$ | H | F | $CH_3$ | 0 | 4,5-Di-$CH_3$ | $CO_2CH(CH_3)_2$ |
| 4-$CH_3$ | H | F | $CH_3$ | 0 | 4,5-Di-$CH_3$ | $CO_2CH(CH_3)_2$ |
| 3-$CH_3$ | F | F | $CH_3$ | 0 | 4,5-Di-$CH_3$ | $CO_2CH(CH_3)_2$ |
| 4-$CH_3$ | F | F | $CH_3$ | 0 | 4,5-Di-$CH_3$ | $CO_2CH(CH_3)_2$ |
| 3-$CH_3$ | H | Cl | $CH_3$ | 0 | 4,5-Di-$CH_3$ | $CO_2CH(CH_3)_2$ |
| 4-$CH_3$ | H | Cl | $CH_3$ | 0 | 4,5-Di-$CH_3$ | $CO_2CH(CH_3)_2$ |
| 3-$CH_3$ | F | Cl | $CH_3$ | 0 | 4,5-Di-$CH_3$ | $CO_2CH(CH_3)_2$ |
| 4-$CH_3$ | F | Cl | $CH_3$ | 0 | 4,5-Di-$CH_3$ | $CO_2CH(CH_3)_2$ |
| 3-$CH_3$ | H | F | H | 0 | 4,5-Di-$CH_3$ | $CO_2(CH_2)_3CH_3$ |
| 4-$CH_3$ | H | F | H | 0 | 4,5-Di-$CH_3$ | $CO_2(CH_2)_3CH_3$ |
| 3-$CH_3$ | F | F | H | 0 | 4,5-Di-$CH_3$ | $CO_2(CH_2)_3CH_3$ |
| 4-$CH_3$ | F | F | H | 0 | 4,5-Di-$CH_3$ | $CO_2(CH_2)_3CH_3$ |
| 3-$CH_3$ | H | Cl | H | 0 | 4,5-Di-$CH_3$ | $CO_2(CH_2)_3CH_3$ |
| 4-$CH_3$ | H | Cl | H | 0 | 4,5-Di-$CH_3$ | $CO_2(CH_2)_3CH_3$ |
| 3-$CH_3$ | F | Cl | H | 0 | 4,5-Di-$CH_3$ | $CO_2(CH_2)_3CH_3$ |
| 4-$CH_3$ | F | Cl | H | 0 | 4,5-Di-$CH_3$ | $CO_2(CH_2)_3CH_3$ |
| 3-$CH_3$ | H | F | $CH_3$ | 0 | 4,5-Di-$CH_3$ | $CO_2(CH_2)_3CH_3$ |
| 4-$CH_3$ | H | F | $CH_3$ | 0 | 4,5-Di-$CH_3$ | $CO_2(CH_2)_3CH_3$ |
| 3-$CH_3$ | F | F | $CH_3$ | 0 | 4,5-Di-$CH_3$ | $CO_2(CH_2)_3CH_3$ |
| 4-$CH_3$ | F | F | $CH_3$ | 0 | 4,5-Di-$CH_3$ | $CO_2(CH_2)_3CH_3$ |
| 3-$CH_3$ | H | Cl | $CH_3$ | 0 | 4,5-Di-$CH_3$ | $CO_2(CH_2)_3CH_3$ |
| 4-$CH_3$ | H | Cl | $CH_3$ | 0 | 4,5-Di-$CH_3$ | $CO_2(CH_2)_3CH_3$ |
| 3-$CH_3$ | F | Cl | $CH_3$ | 0 | 4,5-Di-$CH_3$ | $CO_2(CH_2)_3CH_3$ |
| 4-$CH_3$ | F | Cl | $CH_3$ | 0 | 4,5-Di-$CH_3$ | $CO_2(CH_2)_3CH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| $3\text{-}CH_3$ | H | F | H | 0 | $4,5\text{-Di-}CH_3$ | $CO_2(CH_2)_2OCH_3$ |
| $4\text{-}CH_3$ | H | F | H | 0 | $4,5\text{-Di-}CH_3$ | $CO_2(CH_2)_2OCH_3$ |
| $3\text{-}CH_3$ | F | F | H | 0 | $4,5\text{-Di-}CH_3$ | $CO_2(CH_2)_2OCH_3$ |
| $4\text{-}CH_3$ | F | F | H | 0 | $4,5\text{-Di-}CH_3$ | $CO_2(CH_2)_2OCH_3$ |
| $3\text{-}CH_3$ | H | Cl | H | 0 | $4,5\text{-Di-}CH_3$ | $CO_2(CH_2)_2OCH_3$ |
| $4\text{-}CH_3$ | H | Cl | H | 0 | $4,5\text{-Di-}CH_3$ | $CO_2(CH_2)_2OCH_3$ |
| $3\text{-}CH_3$ | F | Cl | H | 0 | $4,5\text{-Di-}CH_3$ | $CO_2(CH_2)_2OCH_3$ |
| $4\text{-}CH_3$ | F | Cl | H | 0 | $4,5\text{-Di-}CH_3$ | $CO_2(CH_2)_2OCH_3$ |
| $3\text{-}CH_3$ | H | F | $CH_3$ | 0 | $4,5\text{-Di-}CH_3$ | $CO_2(CH_2)_2OCH_3$ |
| $4\text{-}CH_3$ | H | F | $CH_3$ | 0 | $4,5\text{-Di-}CH_3$ | $CO_2(CH_2)_2OCH_3$ |
| $3\text{-}CH_3$ | F | F | $CH_3$ | 0 | $4,5\text{-Di-}CH_3$ | $CO_2(CH_2)_2OCH_3$ |
| $4\text{-}CH_3$ | F | F | $CH_3$ | 0 | $4,5\text{-Di-}CH_3$ | $CO_2(CH_2)_2OCH_3$ |
| $3\text{-}CH_3$ | H | Cl | $CH_3$ | 0 | $4,5\text{-Di-}CH_3$ | $CO_2(CH_2)_2OCH_3$ |
| $4\text{-}CH_3$ | H | Cl | $CH_3$ | 0 | $4,5\text{-Di-}CH_3$ | $CO_2(CH_2)_2OCH_3$ |
| $3\text{-}CH_3$ | F | Cl | $CH_3$ | 0 | $4,5\text{-Di-}CH_3$ | $CO_2(CH_2)_2OCH_3$ |
| $4\text{-}CH_3$ | F | Cl | $CH_3$ | 0 | $4,5\text{-Di-}CH_3$ | $CO_2(CH_2)_2OCH_3$ |
| $3\text{-}CH_3$ | H | F | H | 0 | $4,5\text{-Di-}CH_3$ | $CO_2(CH_2)_2OCH_2CH_3$ |
| $4\text{-}CH_3$ | H | F | H | 0 | $4,5\text{-Di-}CH_3$ | $CO_2(CH_2)_2OCH_2CH_3$ |
| $3\text{-}CH_3$ | F | F | H | 0 | $4,5\text{-Di-}CH_3$ | $CO_2(CH_2)_2OCH_2CH_3$ |
| $4\text{-}CH_3$ | F | F | H | 0 | $4,5\text{-Di-}CH_3$ | $CO_2(CH_2)_2OCH_2CH_3$ |
| $3\text{-}CH_3$ | H | Cl | H | 0 | $4,5\text{-Di-}CH_3$ | $CO_2(CH_2)_2OCH_2CH_3$ |
| $4\text{-}CH_3$ | H | Cl | H | 0 | $4,5\text{-Di-}CH_3$ | $CO_2(CH_2)_2OCH_2CH_3$ |
| $3\text{-}CH_3$ | F | Cl | H | 0 | $4,5\text{-Di-}CH_3$ | $CO_2(CH_2)_2OCH_2CH_3$ |
| $4\text{-}CH_3$ | F | Cl | H | 0 | $4,5\text{-Di-}CH_3$ | $CO_2(CH_2)_2OCH_2CH_3$ |
| $3\text{-}CH_3$ | H | F | $CH_3$ | 0 | $4,5\text{-Di-}CH_3$ | $CO_2(CH_2)_2OCH_2CH_3$ |
| $4\text{-}CH_3$ | H | F | $CH_3$ | 0 | $4,5\text{-Di-}CH_3$ | $CO_2(CH_2)_2OCH_2CH_3$ |
| $3\text{-}CH_3$ | F | F | $CH_3$ | 0 | $4,5\text{-Di-}CH_3$ | $CO_2(CH_2)_2OCH_2CH_3$ |
| $4\text{-}CH_3$ | F | F | $CH_3$ | 0 | $4,5\text{-Di-}CH_3$ | $CO_2(CH_2)_2OCH_2CH_3$ |
| $3\text{-}CH_3$ | H | Cl | $CH_3$ | 0 | $4,5\text{-Di-}CH_3$ | $CO_2(CH_2)_2OCH_2CH_3$ |
| $4\text{-}CH_3$ | H | Cl | $CH_3$ | 0 | $4,5\text{-Di-}CH_3$ | $CO_2(CH_2)_2OCH_2CH_3$ |
| $3\text{-}CH_3$ | F | Cl | $CH_3$ | 0 | $4,5\text{-Di-}CH_3$ | $CO_2(CH_2)_2OCH_2CH_3$ |
| $4\text{-}CH_3$ | F | Cl | $CH_3$ | 0 | $4,5\text{-Di-}CH_3$ | $CO_2(CH_2)_2OCH_2CH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | H | $CO_2CH_3$ |
| 4-$CH_3$ | H | F | H | 1 | H | $CO_2CH_3$ |
| 3-$CH_3$ | F | F | H | 1 | H | $CO_2CH_3$ |
| 4-$CH_3$ | F | F | H | 1 | H | $CO_2CH_3$ |
| 3-$CH_3$ | H | Cl | H | 1 | H | $CO_2CH_3$ |
| 4-$CH_3$ | H | Cl | H | 1 | H | $CO_2CH_3$ |
| 3-$CH_3$ | F | Cl | H | 1 | H | $CO_2CH_3$ |
| 4-$CH_3$ | F | Cl | H | 1 | H | $CO_2CH_3$ |
| 3-$CH_3$ | H | F | $CH_3$ | 1 | H | $CO_2CH_3$ |
| 4-$CH_3$ | H | F | $CH_3$ | 1 | H | $CO_2CH_3$ |
| 3-$CH_3$ | F | F | $CH_3$ | 1 | H | $CO_2CH_3$ |
| 4-$CH_3$ | F | F | $CH_3$ | 1 | H | $CO_2CH_3$ |
| 3-$CH_3$ | H | Cl | $CH_3$ | 1 | H | $CO_2CH_3$ |
| 4-$CH_3$ | H | Cl | $CH_3$ | 1 | H | $CO_2CH_3$ |
| 3-$CH_3$ | F | Cl | $CH_3$ | 1 | H | $CO_2CH_3$ |
| 4-$CH_3$ | F | Cl | $CH_3$ | 1 | H | $CO_2CH_3$ |
| 3-$CH_3$ | H | F | H | 1 | H | $CO_2CH_2CH_3$ |
| 4-$CH_3$ | H | F | H | 1 | H | $CO_2CH_2CH_3$ |
| 3-$CH_3$ | F | F | H | 1 | H | $CO_2CH_2CH_3$ |
| 4-$CH_3$ | F | F | H | 1 | H | $CO_2CH_2CH_3$ |
| 3-$CH_3$ | H | Cl | H | 1 | H | $CO_2CH_2CH_3$ |
| 4-$CH_3$ | H | Cl | H | 1 | H | $CO_2CH_2CH_3$ |
| 3-$CH_3$ | F | Cl | H | 1 | H | $CO_2CH_2CH_3$ |
| 4-$CH_3$ | F | Cl | H | 1 | H | $CO_2CH_2CH_3$ |
| 3-$CH_3$ | H | F | $CH_3$ | 1 | H | $CO_2CH_2CH_3$ |
| 4-$CH_3$ | H | F | $CH_3$ | 1 | H | $CO_2CH_2CH_3$ |
| 3-$CH_3$ | F | F | $CH_3$ | 1 | H | $CO_2CH_2CH_3$ |
| 4-$CH_3$ | F | F | $CH_3$ | 1 | H | $CO_2CH_2CH_3$ |
| 3-$CH_3$ | H | Cl | $CH_3$ | 1 | H | $CO_2CH_2CH_3$ |
| 4-$CH_3$ | H | Cl | $CH_3$ | 1 | H | $CO_2CH_2CH_3$ |
| 3-$CH_3$ | F | Cl | $CH_3$ | 1 | H | $CO_2CH_2CH_3$ |
| 4-$CH_3$ | F | Cl | $CH_3$ | 1 | H | $CO_2CH_2CH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | H | $CO_2(CH_2)_2CH_3$ |
| 4-$CH_3$ | H | F | H | 1 | H | $CO_2(CH_2)_2CH_3$ |
| 3-$CH_3$ | F | F | H | 1 | H | $CO_2(CH_2)_2CH_3$ |
| 4-$CH_3$ | F | F | H | 1 | H | $CO_2(CH_2)_2CH_3$ |
| 3-$CH_3$ | H | Cl | H | 1 | H | $CO_2(CH_2)_2CH_3$ |
| 4-$CH_3$ | H | Cl | H | 1 | H | $CO_2(CH_2)_2CH_3$ |
| 3-$CH_3$ | F | Cl | H | 1 | H | $CO_2(CH_2)_2CH_3$ |
| 4-$CH_3$ | F | Cl | H | 1 | H | $CO_2(CH_2)_2CH_3$ |
| 3-$CH_3$ | H | F | $CH_3$ | 1 | H | $CO_2(CH_2)_2CH_3$ |
| 4-$CH_3$ | H | F | $CH_3$ | 1 | H | $CO_2(CH_2)_2CH_3$ |
| 3-$CH_3$ | F | F | $CH_3$ | 1 | H | $CO_2(CH_2)_2CH_3$ |
| 4-$CH_3$ | F | F | $CH_3$ | 1 | H | $CO_2(CH_2)_2CH_3$ |
| 3-$CH_3$ | H | Cl | $CH_3$ | 1 | H | $CO_2(CH_2)_2CH_3$ |
| 4-$CH_3$ | H | Cl | $CH_3$ | 1 | H | $CO_2(CH_2)_2CH_3$ |
| 3-$CH_3$ | F | Cl | $CH_3$ | 1 | H | $CO_2(CH_2)_2CH_3$ |
| 4-$CH_3$ | F | Cl | $CH_3$ | 1 | H | $CO_2(CH_2)_2CH_3$ |
| 3-$CH_3$ | H | F | H | 1 | H | $CO_2CH(CH_3)_2$ |
| 4-$CH_3$ | H | F | H | 1 | H | $CO_2CH(CH_3)_2$ |
| 3-$CH_3$ | F | F | H | 1 | H | $CO_2CH(CH_3)_2$ |
| 4-$CH_3$ | F | F | H | 1 | H | $CO_2CH(CH_3)_2$ |
| 3-$CH_3$ | H | Cl | H | 1 | H | $CO_2CH(CH_3)_2$ |
| 4-$CH_3$ | H | Cl | H | 1 | H | $CO_2CH(CH_3)_2$ |
| 3-$CH_3$ | F | Cl | H | 1 | H | $CO_2CH(CH_3)_2$ |
| 4-$CH_3$ | F | Cl | H | 1 | H | $CO_2CH(CH_3)_2$ |
| 3-$CH_3$ | H | F | $CH_3$ | 1 | H | $CO_2CH(CH_3)_2$ |
| 4-$CH_3$ | H | F | $CH_3$ | 1 | H | $CO_2CH(CH_3)_2$ |
| 3-$CH_3$ | F | F | $CH_3$ | 1 | H | $CO_2CH(CH_3)_2$ |
| 4-$CH_3$ | F | F | $CH_3$ | 1 | H | $CO_2CH(CH_3)_2$ |
| 3-$CH_3$ | H | Cl | $CH_3$ | 1 | H | $CO_2CH(CH_3)_2$ |
| 4-$CH_3$ | H | Cl | $CH_3$ | 1 | H | $CO_2CH(CH_3)_2$ |
| 3-$CH_3$ | F | Cl | $CH_3$ | 1 | H | $CO_2CH(CH_3)_2$ |
| 4-$CH_3$ | F | Cl | $CH_3$ | 1 | H | $CO_2CH(CH_3)_2$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | H | $CO_2(CH_2)_3CH_3$ |
| 4-$CH_3$ | H | F | H | 1 | H | $CO_2(CH_2)_3CH_3$ |
| 3-$CH_3$ | F | F | H | 1 | H | $CO_2(CH_2)_3CH_3$ |
| 4-$CH_3$ | F | F | H | 1 | H | $CO_2(CH_2)_3CH_3$ |
| 3-$CH_3$ | H | Cl | H | 1 | H | $CO_2(CH_2)_3CH_3$ |
| 4-$CH_3$ | H | Cl | H | 1 | H | $CO_2(CH_2)_3CH_3$ |
| 3-$CH_3$ | F | Cl | H | 1 | H | $CO_2(CH_2)_3CH_3$ |
| 4-$CH_3$ | F | Cl | H | 1 | H | $CO_2(CH_2)_3CH_3$ |
| 3-$CH_3$ | H | F | $CH_3$ | 1 | H | $CO_2(CH_2)_3CH_3$ |
| 4-$CH_3$ | H | F | $CH_3$ | 1 | H | $CO_2(CH_2)_3CH_3$ |
| 3-$CH_3$ | F | F | $CH_3$ | 1 | H | $CO_2(CH_2)_3CH_3$ |
| 4-$CH_3$ | F | F | $CH_3$ | 1 | H | $CO_2(CH_2)_3CH_3$ |
| 3-$CH_3$ | H | Cl | $CH_3$ | 1 | H | $CO_2(CH_2)_3CH_3$ |
| 4-$CH_3$ | H | Cl | $CH_3$ | 1 | H | $CO_2(CH_2)_3CH_3$ |
| 3-$CH_3$ | F | Cl | $CH_3$ | 1 | H | $CO_2(CH_2)_3CH_3$ |
| 4-$CH_3$ | F | Cl | $CH_3$ | 1 | H | $CO_2(CH_2)_3CH_3$ |
| 3-$CH_3$ | H | F | H | 1 | H | $CO_2(CH_2)_2OCH_3$ |
| 4-$CH_3$ | H | F | H | 1 | H | $CO_2(CH_2)_2OCH_3$ |
| 3-$CH_3$ | F | F | H | 1 | H | $CO_2(CH_2)_2OCH_3$ |
| 4-$CH_3$ | F | F | H | 1 | H | $CO_2(CH_2)_2OCH_3$ |
| 3-$CH_3$ | H | Cl | H | 1 | H | $CO_2(CH_2)_2OCH_3$ |
| 4-$CH_3$ | H | Cl | H | 1 | H | $CO_2(CH_2)_2OCH_3$ |
| 3-$CH_3$ | F | Cl | H | 1 | H | $CO_2(CH_2)_2OCH_3$ |
| 4-$CH_3$ | F | Cl | H | 1 | H | $CO_2(CH_2)_2OCH_3$ |
| 3-$CH_3$ | H | F | $CH_3$ | 1 | H | $CO_2(CH_2)_2OCH_3$ |
| 4-$CH_3$ | H | F | $CH_3$ | 1 | H | $CO_2(CH_2)_2OCH_3$ |
| 3-$CH_3$ | F | F | $CH_3$ | 1 | H | $CO_2(CH_2)_2OCH_3$ |
| 4-$CH_3$ | F | F | $CH_3$ | 1 | H | $CO_2(CH_2)_2OCH_3$ |
| 3-$CH_3$ | H | Cl | $CH_3$ | 1 | H | $CO_2(CH_2)_2OCH_3$ |
| 4-$CH_3$ | H | Cl | $CH_3$ | 1 | H | $CO_2(CH_2)_2OCH_3$ |
| 3-$CH_3$ | F | Cl | $CH_3$ | 1 | H | $CO_2(CH_2)_2OCH_3$ |
| 4-$CH_3$ | F | Cl | $CH_3$ | 1 | H | $CO_2(CH_2)_2OCH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | H | $CO_2(CH_2)_2OCH_2CH_3$ |
| 4-$CH_3$ | H | F | H | 1 | H | $CO_2(CH_2)_2OCH_2CH_3$ |
| 3-$CH_3$ | F | F | H | 1 | H | $CO_2(CH_2)_2OCH_2CH_3$ |
| 4-$CH_3$ | F | F | H | 1 | H | $CO_2(CH_2)_2OCH_2CH_3$ |
| 3-$CH_3$ | H | Cl | H | 1 | H | $CO_2(CH_2)_2OCH_2CH_3$ |
| 4-$CH_3$ | H | Cl | H | 1 | H | $CO_2(CH_2)_2OCH_2CH_3$ |
| 3-$CH_3$ | F | Cl | H | 1 | H | $CO_2(CH_2)_2OCH_2CH_3$ |
| 4-$CH_3$ | F | Cl | H | 1 | H | $CO_2(CH_2)_2OCH_2CH_3$ |
| 3-$CH_3$ | H | F | $CH_3$ | 1 | H | $CO_2(CH_2)_2OCH_2CH_3$ |
| 4-$CH_3$ | H | F | $CH_3$ | 1 | H | $CO_2(CH_2)_2OCH_2CH_3$ |
| 3-$CH_3$ | F | F | $CH_3$ | 1 | H | $CO_2(CH_2)_2OCH_2CH_3$ |
| 4-$CH_3$ | F | F | $CH_3$ | 1 | H | $CO_2(CH_2)_2OCH_2CH_3$ |
| 3-$CH_3$ | H | Cl | $CH_3$ | 1 | H | $CO_2(CH_2)_2OCH_2CH_3$ |
| 4-$CH_3$ | H | Cl | $CH_3$ | 1 | H | $CO_2(CH_2)_2OCH_2CH_3$ |
| 3-$CH_3$ | F | Cl | $CH_3$ | 1 | H | $CO_2(CH_2)_2OCH_2CH_3$ |
| 4-$CH_3$ | F | Cl | $CH_3$ | 1 | H | $CO_2(CH_2)_2OCH_2CH_3$ |
| 3-$CH_3$ | H | F | H | 1 | 4-$CH_3$ | $CO_2CH_3$ |
| 4-$CH_3$ | H | F | H | 1 | 4-$CH_3$ | $CO_2CH_3$ |
| 3-$CH_3$ | F | F | H | 1 | 4-$CH_3$ | $CO_2CH_3$ |
| 4-$CH_3$ | F | F | H | 1 | 4-$CH_3$ | $CO_2CH_3$ |
| 3-$CH_3$ | H | Cl | H | 1 | 4-$CH_3$ | $CO_2CH_3$ |
| 4-$CH_3$ | H | Cl | H | 1 | 4-$CH_3$ | $CO_2CH_3$ |
| 3-$CH_3$ | F | Cl | H | 1 | 4-$CH_3$ | $CO_2CH_3$ |
| 4-$CH_3$ | F | Cl | H | 1 | 4-$CH_3$ | $CO_2CH_3$ |
| 3-$CH_3$ | H | F | $CH_3$ | 1 | 4-$CH_3$ | $CO_2CH_3$ |
| 4-$CH_3$ | H | F | $CH_3$ | 1 | 4-$CH_3$ | $CO_2CH_3$ |
| 3-$CH_3$ | F | F | $CH_3$ | 1 | 4-$CH_3$ | $CO_2CH_3$ |
| 4-$CH_3$ | F | F | $CH_3$ | 1 | 4-$CH_3$ | $CO_2CH_3$ |
| 3-$CH_3$ | H | Cl | $CH_3$ | 1 | 4-$CH_3$ | $CO_2CH_3$ |
| 4-$CH_3$ | H | Cl | $CH_3$ | 1 | 4-$CH_3$ | $CO_2CH_3$ |
| 3-$CH_3$ | F | Cl | $CH_3$ | 1 | 4-$CH_3$ | $CO_2CH_3$ |
| 4-$CH_3$ | F | Cl | $CH_3$ | 1 | 4-$CH_3$ | $CO_2CH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4-$CH_3$ | $CO_2CH_2CH_3$ |
| 4-$CH_3$ | H | F | H | 1 | 4-$CH_3$ | $CO_2CH_2CH_3$ |
| 3-$CH_3$ | F | F | H | 1 | 4-$CH_3$ | $CO_2CH_2CH_3$ |
| 4-$CH_3$ | F | F | H | 1 | 4-$CH_3$ | $CO_2CH_2CH_3$ |
| 3-$CH_3$ | H | Cl | H | 1 | 4-$CH_3$ | $CO_2CH_2CH_3$ |
| 4-$CH_3$ | H | Cl | H | 1 | 4-$CH_3$ | $CO_2CH_2CH_3$ |
| 3-$CH_3$ | F | Cl | H | 1 | 4-$CH_3$ | $CO_2CH_2CH_3$ |
| 4-$CH_3$ | F | Cl | H | 1 | 4-$CH_3$ | $CO_2CH_2CH_3$ |
| 3-$CH_3$ | H | F | $CH_3$ | 1 | 4-$CH_3$ | $CO_2CH_2CH_3$ |
| 4-$CH_3$ | H | F | $CH_3$ | 1 | 4-$CH_3$ | $CO_2CH_2CH_3$ |
| 3-$CH_3$ | F | F | $CH_3$ | 1 | 4-$CH_3$ | $CO_2CH_2CH_3$ |
| 4-$CH_3$ | F | F | $CH_3$ | 1 | 4-$CH_3$ | $CO_2CH_2CH_3$ |
| 3-$CH_3$ | H | Cl | $CH_3$ | 1 | 4-$CH_3$ | $CO_2CH_2CH_3$ |
| 4-$CH_3$ | H | Cl | $CH_3$ | 1 | 4-$CH_3$ | $CO_2CH_2CH_3$ |
| 3-$CH_3$ | F | Cl | $CH_3$ | 1 | 4-$CH_3$ | $CO_2CH_2CH_3$ |
| 4-$CH_3$ | F | Cl | $CH_3$ | 1 | 4-$CH_3$ | $CO_2CH_2CH_3$ |
| 3-$CH_3$ | H | F | H | 1 | 4-$CH_3$ | $CO_2(CH_2)_2CH_3$ |
| 4-$CH_3$ | H | F | H | 1 | 4-$CH_3$ | $CO_2(CH_2)_2CH_3$ |
| 3-$CH_3$ | F | F | H | 1 | 4-$CH_3$ | $CO_2(CH_2)_2CH_3$ |
| 4-$CH_3$ | F | F | H | 1 | 4-$CH_3$ | $CO_2(CH_2)_2CH_3$ |
| 3-$CH_3$ | H | Cl | H | 1 | 4-$CH_3$ | $CO_2(CH_2)_2CH_3$ |
| 4-$CH_3$ | H | Cl | H | 1 | 4-$CH_3$ | $CO_2(CH_2)_2CH_3$ |
| 3-$CH_3$ | F | Cl | H | 1 | 4-$CH_3$ | $CO_2(CH_2)_2CH_3$ |
| 4-$CH_3$ | F | Cl | H | 1 | 4-$CH_3$ | $CO_2(CH_2)_2CH_3$ |
| 3-$CH_3$ | H | F | $CH_3$ | 1 | 4-$CH_3$ | $CO_2(CH_2)_2CH_3$ |
| 4-$CH_3$ | H | F | $CH_3$ | 1 | 4-$CH_3$ | $CO_2(CH_2)_2CH_3$ |
| 3-$CH_3$ | F | F | $CH_3$ | 1 | 4-$CH_3$ | $CO_2(CH_2)_2CH_3$ |
| 4-$CH_3$ | F | F | $CH_3$ | 1 | 4-$CH_3$ | $CO_2(CH_2)_2CH_3$ |
| 3-$CH_3$ | H | Cl | $CH_3$ | 1 | 4-$CH_3$ | $CO_2(CH_2)_2CH_3$ |
| 4-$CH_3$ | H | Cl | $CH_3$ | 1 | 4-$CH_3$ | $CO_2(CH_2)_2CH_3$ |
| 3-$CH_3$ | F | Cl | $CH_3$ | 1 | 4-$CH_3$ | $CO_2(CH_2)_2CH_3$ |
| 4-$CH_3$ | F | Cl | $CH_3$ | 1 | 4-$CH_3$ | $CO_2(CH_2)_2CH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4-$CH_3$ | $CO_2CH(CH_3)_2$ |
| 4-$CH_3$ | H | F | H | 1 | 4-$CH_3$ | $CO_2CH(CH_3)_2$ |
| 3-$CH_3$ | F | F | H | 1 | 4-$CH_3$ | $CO_2CH(CH_3)_2$ |
| 4-$CH_3$ | F | F | H | 1 | 4-$CH_3$ | $CO_2CH(CH_3)_2$ |
| 3-$CH_3$ | H | Cl | H | 1 | 4-$CH_3$ | $CO_2CH(CH_3)_2$ |
| 4-$CH_3$ | H | Cl | H | 1 | 4-$CH_3$ | $CO_2CH(CH_3)_2$ |
| 3-$CH_3$ | F | Cl | H | 1 | 4-$CH_3$ | $CO_2CH(CH_3)_2$ |
| 4-$CH_3$ | F | Cl | H | 1 | 4-$CH_3$ | $CO_2CH(CH_3)_2$ |
| 3-$CH_3$ | H | F | $CH_3$ | 1 | 4-$CH_3$ | $CO_2CH(CH_3)_2$ |
| 4-$CH_3$ | H | F | $CH_3$ | 1 | 4-$CH_3$ | $CO_2CH(CH_3)_2$ |
| 3-$CH_3$ | F | F | $CH_3$ | 1 | 4-$CH_3$ | $CO_2CH(CH_3)_2$ |
| 4-$CH_3$ | F | F | $CH_3$ | 1 | 4-$CH_3$ | $CO_2CH(CH_3)_2$ |
| 3-$CH_3$ | H | Cl | $CH_3$ | 1 | 4-$CH_3$ | $CO_2CH(CH_3)_2$ |
| 4-$CH_3$ | H | Cl | $CH_3$ | 1 | 4-$CH_3$ | $CO_2CH(CH_3)_2$ |
| 3-$CH_3$ | F | Cl | $CH_3$ | 1 | 4-$CH_3$ | $CO_2CH(CH_3)_2$ |
| 4-$CH_3$ | F | Cl | $CH_3$ | 1 | 4-$CH_3$ | $CO_2CH(CH_3)_2$ |
| 3-$CH_3$ | H | F | H | 1 | 4-$CH_3$ | $CO_2(CH_2)_3CH_3$ |
| 4-$CH_3$ | H | F | H | 1 | 4-$CH_3$ | $CO_2(CH_2)_3CH_3$ |
| 3-$CH_3$ | F | F | H | 1 | 4-$CH_3$ | $CO_2(CH_2)_3CH_3$ |
| 4-$CH_3$ | F | F | H | 1 | 4-$CH_3$ | $CO_2(CH_2)_3CH_3$ |
| 3-$CH_3$ | H | Cl | H | 1 | 4-$CH_3$ | $CO_2(CH_2)_3CH_3$ |
| 4-$CH_3$ | H | Cl | H | 1 | 4-$CH_3$ | $CO_2(CH_2)_3CH_3$ |
| 3-$CH_3$ | F | Cl | H | 1 | 4-$CH_3$ | $CO_2(CH_2)_3CH_3$ |
| 4-$CH_3$ | F | Cl | H | 1 | 4-$CH_3$ | $CO_2(CH_2)_3CH_3$ |
| 3-$CH_3$ | H | F | $CH_3$ | 1 | 4-$CH_3$ | $CO_2(CH_2)_3CH_3$ |
| 4-$CH_3$ | H | F | $CH_3$ | 1 | 4-$CH_3$ | $CO_2(CH_2)_3CH_3$ |
| 3-$CH_3$ | F | F | $CH_3$ | 1 | 4-$CH_3$ | $CO_2(CH_2)_3CH_3$ |
| 4-$CH_3$ | F | F | $CH_3$ | 1 | 4-$CH_3$ | $CO_2(CH_2)_3CH_3$ |
| 3-$CH_3$ | H | Cl | $CH_3$ | 1 | 4-$CH_3$ | $CO_2(CH_2)_3CH_3$ |
| 4-$CH_3$ | H | Cl | $CH_3$ | 1 | 4-$CH_3$ | $CO_2(CH_2)_3CH_3$ |
| 3-$CH_3$ | F | Cl | $CH_3$ | 1 | 4-$CH_3$ | $CO_2(CH_2)_3CH_3$ |
| 4-$CH_3$ | F | Cl | $CH_3$ | 1 | 4-$CH_3$ | $CO_2(CH_2)_3CH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4-$CH_3$ | $CO_2(CH_2)_2OCH_3$ |
| 4-$CH_3$ | H | F | H | 1 | 4-$CH_3$ | $CO_2(CH_2)_2OCH_3$ |
| 3-$CH_3$ | F | F | H | 1 | 4-$CH_3$ | $CO_2(CH_2)_2OCH_3$ |
| 4-$CH_3$ | F | F | H | 1 | 4-$CH_3$ | $CO_2(CH_2)_2OCH_3$ |
| 3-$CH_3$ | H | Cl | H | 1 | 4-$CH_3$ | $CO_2(CH_2)_2OCH_3$ |
| 4-$CH_3$ | H | Cl | H | 1 | 4-$CH_3$ | $CO_2(CH_2)_2OCH_3$ |
| 3-$CH_3$ | F | Cl | H | 1 | 4-$CH_3$ | $CO_2(CH_2)_2OCH_3$ |
| 4-$CH_3$ | F | Cl | H | 1 | 4-$CH_3$ | $CO_2(CH_2)_2OCH_3$ |
| 3-$CH_3$ | H | F | $CH_3$ | 1 | 4-$CH_3$ | $CO_2(CH_2)_2OCH_3$ |
| 4-$CH_3$ | H | F | $CH_3$ | 1 | 4-$CH_3$ | $CO_2(CH_2)_2OCH_3$ |
| 3-$CH_3$ | F | F | $CH_3$ | 1 | 4-$CH_3$ | $CO_2(CH_2)_2OCH_3$ |
| 4-$CH_3$ | F | F | $CH_3$ | 1 | 4-$CH_3$ | $CO_2(CH_2)_2OCH_3$ |
| 3-$CH_3$ | H | Cl | $CH_3$ | 1 | 4-$CH_3$ | $CO_2(CH_2)_2OCH_3$ |
| 4-$CH_3$ | H | Cl | $CH_3$ | 1 | 4-$CH_3$ | $CO_2(CH_2)_2OCH_3$ |
| 3-$CH_3$ | F | Cl | $CH_3$ | 1 | 4-$CH_3$ | $CO_2(CH_2)_2OCH_3$ |
| 4-$CH_3$ | F | Cl | $CH_3$ | 1 | 4-$CH_3$ | $CO_2(CH_2)_2OCH_3$ |
| 3-$CH_3$ | H | F | H | 1 | 4-$CH_3$ | $CO_2(CH_2)_2OCH_2CH_3$ |
| 4-$CH_3$ | H | F | H | 1 | 4-$CH_3$ | $CO_2(CH_2)_2OCH_2CH_3$ |
| 3-$CH_3$ | F | F | H | 1 | 4-$CH_3$ | $CO_2(CH_2)_2OCH_2CH_3$ |
| 4-$CH_3$ | F | F | H | 1 | 4-$CH_3$ | $CO_2(CH_2)_2OCH_2CH_3$ |
| 3-$CH_3$ | H | Cl | H | 1 | 4-$CH_3$ | $CO_2(CH_2)_2OCH_2CH_3$ |
| 4-$CH_3$ | H | Cl | H | 1 | 4-$CH_3$ | $CO_2(CH_2)_2OCH_2CH_3$ |
| 3-$CH_3$ | F | Cl | H | 1 | 4-$CH_3$ | $CO_2(CH_2)_2OCH_2CH_3$ |
| 4-$CH_3$ | F | Cl | H | 1 | 4-$CH_3$ | $CO_2(CH_2)_2OCH_2CH_3$ |
| 3-$CH_3$ | H | F | $CH_3$ | 1 | 4-$CH_3$ | $CO_2(CH_2)_2OCH_2CH_3$ |
| 4-$CH_3$ | H | F | $CH_3$ | 1 | 4-$CH_3$ | $CO_2(CH_2)_2OCH_2CH_3$ |
| 3-$CH_3$ | F | F | $CH_3$ | 1 | 4-$CH_3$ | $CO_2(CH_2)_2OCH_2CH_3$ |
| 4-$CH_3$ | F | F | $CH_3$ | 1 | 4-$CH_3$ | $CO_2(CH_2)_2OCH_2CH_3$ |
| 3-$CH_3$ | H | Cl | $CH_3$ | 1 | 4-$CH_3$ | $CO_2(CH_2)_2OCH_2CH_3$ |
| 4-$CH_3$ | H | Cl | $CH_3$ | 1 | 4-$CH_3$ | $CO_2(CH_2)_2OCH_2CH_3$ |
| 3-$CH_3$ | F | Cl | $CH_3$ | 1 | 4-$CH_3$ | $CO_2(CH_2)_2OCH_2CH_3$ |
| 4-$CH_3$ | F | Cl | $CH_3$ | 1 | 4-$CH_3$ | $CO_2(CH_2)_2OCH_2CH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 6-$CH_3$ | $CO_2CH_3$ |
| 4-$CH_3$ | H | F | H | 1 | 6-$CH_3$ | $CO_2CH_3$ |
| 3-$CH_3$ | F | F | H | 1 | 6-$CH_3$ | $CO_2CH_3$ |
| 4-$CH_3$ | F | F | H | 1 | 6-$CH_3$ | $CO_2CH_3$ |
| 3-$CH_3$ | H | Cl | H | 1 | 6-$CH_3$ | $CO_2CH_3$ |
| 4-$CH_3$ | H | Cl | H | 1 | 6-$CH_3$ | $CO_2CH_3$ |
| 3-$CH_3$ | F | Cl | H | 1 | 6-$CH_3$ | $CO_2CH_3$ |
| 4-$CH_3$ | F | Cl | H | 1 | 6-$CH_3$ | $CO_2CH_3$ |
| 3-$CH_3$ | H | F | $CH_3$ | 1 | 6-$CH_3$ | $CO_2CH_3$ |
| 4-$CH_3$ | H | F | $CH_3$ | 1 | 6-$CH_3$ | $CO_2CH_3$ |
| 3-$CH_3$ | F | F | $CH_3$ | 1 | 6-$CH_3$ | $CO_2CH_3$ |
| 4-$CH_3$ | F | F | $CH_3$ | 1 | 6-$CH_3$ | $CO_2CH_3$ |
| 3-$CH_3$ | H | Cl | $CH_3$ | 1 | 6-$CH_3$ | $CO_2CH_3$ |
| 4-$CH_3$ | H | Cl | $CH_3$ | 1 | 6-$CH_3$ | $CO_2CH_3$ |
| 3-$CH_3$ | F | Cl | $CH_3$ | 1 | 6-$CH_3$ | $CO_2CH_3$ |
| 4-$CH_3$ | F | Cl | $CH_3$ | 1 | 6-$CH_3$ | $CO_2CH_3$ |
| 3-$CH_3$ | H | F | H | 1 | 6-$CH_3$ | $CO_2CH_2CH_3$ |
| 4-$CH_3$ | H | F | H | 1 | 6-$CH_3$ | $CO_2CH_2CH_3$ |
| 3-$CH_3$ | F | F | H | 1 | 6-$CH_3$ | $CO_2CH_2CH_3$ |
| 4-$CH_3$ | F | F | H | 1 | 6-$CH_3$ | $CO_2CH_2CH_3$ |
| 3-$CH_3$ | H | Cl | H | 1 | 6-$CH_3$ | $CO_2CH_2CH_3$ |
| 4-$CH_3$ | H | Cl | H | 1 | 6-$CH_3$ | $CO_2CH_2CH_3$ |
| 3-$CH_3$ | F | Cl | H | 1 | 6-$CH_3$ | $CO_2CH_2CH_3$ |
| 4-$CH_3$ | F | Cl | H | 1 | 6-$CH_3$ | $CO_2CH_2CH_3$ |
| 3-$CH_3$ | H | F | $CH_3$ | 1 | 6-$CH_3$ | $CO_2CH_2CH_3$ |
| 4-$CH_3$ | H | F | $CH_3$ | 1 | 6-$CH_3$ | $CO_2CH_2CH_3$ |
| 3-$CH_3$ | F | F | $CH_3$ | 1 | 6-$CH_3$ | $CO_2CH_2CH_3$ |
| 4-$CH_3$ | F | F | $CH_3$ | 1 | 6-$CH_3$ | $CO_2CH_2CH_3$ |
| 3-$CH_3$ | H | Cl | $CH_3$ | 1 | 6-$CH_3$ | $CO_2CH_2CH_3$ |
| 4-$CH_3$ | H | Cl | $CH_3$ | 1 | 6-$CH_3$ | $CO_2CH_2CH_3$ |
| 3-$CH_3$ | F | Cl | $CH_3$ | 1 | 6-$CH_3$ | $CO_2CH_2CH_3$ |
| 4-$CH_3$ | F | Cl | $CH_3$ | 1 | 6-$CH_3$ | $CO_2CH_2CH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 6-$CH_3$ | $CO_2(CH_2)_2CH_3$ |
| 4-$CH_3$ | H | F | H | 1 | 6-$CH_3$ | $CO_2(CH_2)_2CH_3$ |
| 3-$CH_3$ | F | F | H | 1 | 6-$CH_3$ | $CO_2(CH_2)_2CH_3$ |
| 4-$CH_3$ | F | F | H | 1 | 6-$CH_3$ | $CO_2(CH_2)_2CH_3$ |
| 3-$CH_3$ | H | Cl | H | 1 | 6-$CH_3$ | $CO_2(CH_2)_2CH_3$ |
| 4-$CH_3$ | H | Cl | H | 1 | 6-$CH_3$ | $CO_2(CH_2)_2CH_3$ |
| 3-$CH_3$ | F | Cl | H | 1 | 6-$CH_3$ | $CO_2(CH_2)_2CH_3$ |
| 4-$CH_3$ | F | Cl | H | 1 | 6-$CH_3$ | $CO_2(CH_2)_2CH_3$ |
| 3-$CH_3$ | H | F | $CH_3$ | 1 | 6-$CH_3$ | $CO_2(CH_2)_2CH_3$ |
| 4-$CH_3$ | H | F | $CH_3$ | 1 | 6-$CH_3$ | $CO_2(CH_2)_2CH_3$ |
| 3-$CH_3$ | F | F | $CH_3$ | 1 | 6-$CH_3$ | $CO_2(CH_2)_2CH_3$ |
| 4-$CH_3$ | F | F | $CH_3$ | 1 | 6-$CH_3$ | $CO_2(CH_2)_2CH_3$ |
| 3-$CH_3$ | H | Cl | $CH_3$ | 1 | 6-$CH_3$ | $CO_2(CH_2)_2CH_3$ |
| 4-$CH_3$ | H | Cl | $CH_3$ | 1 | 6-$CH_3$ | $CO_2(CH_2)_2CH_3$ |
| 3-$CH_3$ | F | Cl | $CH_3$ | 1 | 6-$CH_3$ | $CO_2(CH_2)_2CH_3$ |
| 4-$CH_3$ | F | Cl | $CH_3$ | 1 | 6-$CH_3$ | $CO_2(CH_2)_2CH_3$ |
| 3-$CH_3$ | H | F | H | 1 | 6-$CH_3$ | $CO_2CH(CH_3)_2$ |
| 4-$CH_3$ | H | F | H | 1 | 6-$CH_3$ | $CO_2CH(CH_3)_2$ |
| 3-$CH_3$ | F | F | H | 1 | 6-$CH_3$ | $CO_2CH(CH_3)_2$ |
| 4-$CH_3$ | F | F | H | 1 | 6-$CH_3$ | $CO_2CH(CH_3)_2$ |
| 3-$CH_3$ | H | Cl | H | 1 | 6-$CH_3$ | $CO_2CH(CH_3)_2$ |
| 4-$CH_3$ | H | Cl | H | 1 | 6-$CH_3$ | $CO_2CH(CH_3)_2$ |
| 3-$CH_3$ | F | Cl | H | 1 | 6-$CH_3$ | $CO_2CH(CH_3)_2$ |
| 4-$CH_3$ | F | Cl | H | 1 | 6-$CH_3$ | $CO_2CH(CH_3)_2$ |
| 3-$CH_3$ | H | F | $CH_3$ | 1 | 6-$CH_3$ | $CO_2CH(CH_3)_2$ |
| 4-$CH_3$ | H | F | $CH_3$ | 1 | 6-$CH_3$ | $CO_2CH(CH_3)_2$ |
| 3-$CH_3$ | F | F | $CH_3$ | 1 | 6-$CH_3$ | $CO_2CH(CH_3)_2$ |
| 4-$CH_3$ | F | F | $CH_3$ | 1 | 6-$CH_3$ | $CO_2CH(CH_3)_2$ |
| 3-$CH_3$ | H | Cl | $CH_3$ | 1 | 6-$CH_3$ | $CO_2CH(CH_3)_2$ |
| 4-$CH_3$ | H | Cl | $CH_3$ | 1 | 6-$CH_3$ | $CO_2CH(CH_3)_2$ |
| 3-$CH_3$ | F | Cl | $CH_3$ | 1 | 6-$CH_3$ | $CO_2CH(CH_3)_2$ |
| 4-$CH_3$ | F | Cl | $CH_3$ | 1 | 6-$CH_3$ | $CO_2CH(CH_3)_2$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 6-$CH_3$ | $CO_2(CH_2)_3CH_3$ |
| 4-$CH_3$ | H | F | H | 1 | 6-$CH_3$ | $CO_2(CH_2)_3CH_3$ |
| 3-$CH_3$ | F | F | H | 1 | 6-$CH_3$ | $CO_2(CH_2)_3CH_3$ |
| 4-$CH_3$ | F | F | H | 1 | 6-$CH_3$ | $CO_2(CH_2)_3CH_3$ |
| 3-$CH_3$ | H | Cl | H | 1 | 6-$CH_3$ | $CO_2(CH_2)_3CH_3$ |
| 4-$CH_3$ | H | Cl | H | 1 | 6-$CH_3$ | $CO_2(CH_2)_3CH_3$ |
| 3-$CH_3$ | F | Cl | H | 1 | 6-$CH_3$ | $CO_2(CH_2)_3CH_3$ |
| 4-$CH_3$ | F | Cl | H | 1 | 6-$CH_3$ | $CO_2(CH_2)_3CH_3$ |
| 3-$CH_3$ | H | F | $CH_3$ | 1 | 6-$CH_3$ | $CO_2(CH_2)_3CH_3$ |
| 4-$CH_3$ | H | F | $CH_3$ | 1 | 6-$CH_3$ | $CO_2(CH_2)_3CH_3$ |
| 3-$CH_3$ | F | F | $CH_3$ | 1 | 6-$CH_3$ | $CO_2(CH_2)_3CH_3$ |
| 4-$CH_3$ | F | F | $CH_3$ | 1 | 6-$CH_3$ | $CO_2(CH_2)_3CH_3$ |
| 3-$CH_3$ | H | Cl | $CH_3$ | 1 | 6-$CH_3$ | $CO_2(CH_2)_3CH_3$ |
| 4-$CH_3$ | H | Cl | $CH_3$ | 1 | 6-$CH_3$ | $CO_2(CH_2)_3CH_3$ |
| 3-$CH_3$ | F | Cl | $CH_3$ | 1 | 6-$CH_3$ | $CO_2(CH_2)_3CH_3$ |
| 4-$CH_3$ | F | Cl | $CH_3$ | 1 | 6-$CH_3$ | $CO_2(CH_2)_3CH_3$ |
| 3-$CH_3$ | H | F | H | 1 | 6-$CH_3$ | $CO_2(CH_2)_2OCH_3$ |
| 4-$CH_3$ | H | F | H | 1 | 6-$CH_3$ | $CO_2(CH_2)_2OCH_3$ |
| 3-$CH_3$ | F | F | H | 1 | 6-$CH_3$ | $CO_2(CH_2)_2OCH_3$ |
| 4-$CH_3$ | F | F | H | 1 | 6-$CH_3$ | $CO_2(CH_2)_2OCH_3$ |
| 3-$CH_3$ | H | Cl | H | 1 | 6-$CH_3$ | $CO_2(CH_2)_2OCH_3$ |
| 4-$CH_3$ | H | Cl | H | 1 | 6-$CH_3$ | $CO_2(CH_2)_2OCH_3$ |
| 3-$CH_3$ | F | Cl | H | 1 | 6-$CH_3$ | $CO_2(CH_2)_2OCH_3$ |
| 4-$CH_3$ | F | Cl | H | 1 | 6-$CH_3$ | $CO_2(CH_2)_2OCH_3$ |
| 3-$CH_3$ | H | F | $CH_3$ | 1 | 6-$CH_3$ | $CO_2(CH_2)_2OCH_3$ |
| 4-$CH_3$ | H | F | $CH_3$ | 1 | 6-$CH_3$ | $CO_2(CH_2)_2OCH_3$ |
| 3-$CH_3$ | F | F | $CH_3$ | 1 | 6-$CH_3$ | $CO_2(CH_2)_2OCH_3$ |
| 4-$CH_3$ | F | F | $CH_3$ | 1 | 6-$CH_3$ | $CO_2(CH_2)_2OCH_3$ |
| 3-$CH_3$ | H | Cl | $CH_3$ | 1 | 6-$CH_3$ | $CO_2(CH_2)_2OCH_3$ |
| 4-$CH_3$ | H | Cl | $CH_3$ | 1 | 6-$CH_3$ | $CO_2(CH_2)_2OCH_3$ |
| 3-$CH_3$ | F | Cl | $CH_3$ | 1 | 6-$CH_3$ | $CO_2(CH_2)_2OCH_3$ |
| 4-$CH_3$ | F | Cl | $CH_3$ | 1 | 6-$CH_3$ | $CO_2(CH_2)_2OCH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 6-$CH_3$ | $CO_2(CH_2)_2OCH_2CH_3$ |
| 4-$CH_3$ | H | F | H | 1 | 6-$CH_3$ | $CO_2(CH_2)_2OCH_2CH_3$ |
| 3-$CH_3$ | F | F | H | 1 | 6-$CH_3$ | $CO_2(CH_2)_2OCH_2CH_3$ |
| 4-$CH_3$ | F | F | H | 1 | 6-$CH_3$ | $CO_2(CH_2)_2OCH_2CH_3$ |
| 3-$CH_3$ | H | Cl | H | 1 | 6-$CH_3$ | $CO_2(CH_2)_2OCH_2CH_3$ |
| 4-$CH_3$ | H | Cl | H | 1 | 6-$CH_3$ | $CO_2(CH_2)_2OCH_2CH_3$ |
| 3-$CH_3$ | F | Cl | H | 1 | 6-$CH_3$ | $CO_2(CH_2)_2OCH_2CH_3$ |
| 4-$CH_3$ | F | Cl | H | 1 | 6-$CH_3$ | $CO_2(CH_2)_2OCH_2CH_3$ |
| 3-$CH_3$ | H | F | $CH_3$ | 1 | 6-$CH_3$ | $CO_2(CH_2)_2OCH_2CH_3$ |
| 4-$CH_3$ | H | F | $CH_3$ | 1 | 6-$CH_3$ | $CO_2(CH_2)_2OCH_2CH_3$ |
| 3-$CH_3$ | F | F | $CH_3$ | 1 | 6-$CH_3$ | $CO_2(CH_2)_2OCH_2CH_3$ |
| 4-$CH_3$ | F | F | $CH_3$ | 1 | 6-$CH_3$ | $CO_2(CH_2)_2OCH_2CH_3$ |
| 3-$CH_3$ | H | Cl | $CH_3$ | 1 | 6-$CH_3$ | $CO_2(CH_2)_2OCH_2CH_3$ |
| 4-$CH_3$ | H | Cl | $CH_3$ | 1 | 6-$CH_3$ | $CO_2(CH_2)_2OCH_2CH_3$ |
| 3-$CH_3$ | F | Cl | $CH_3$ | 1 | 6-$CH_3$ | $CO_2(CH_2)_2OCH_2CH_3$ |
| 4-$CH_3$ | F | Cl | $CH_3$ | 1 | 6-$CH_3$ | $CO_2(CH_2)_2OCH_2CH_3$ |
| 3-$CH_3$ | H | F | H | 1 | 4,6-Di-$CH_3$ | $CO_2CH_3$ |
| 4-$CH_3$ | H | F | H | 1 | 4,6-Di-$CH_3$ | $CO_2CH_3$ |
| 3-$CH_3$ | F | F | H | 1 | 4,6-Di-$CH_3$ | $CO_2CH_3$ |
| 4-$CH_3$ | F | F | H | 1 | 4,6-Di-$CH_3$ | $CO_2CH_3$ |
| 3-$CH_3$ | H | Cl | H | 1 | 4,6-Di-$CH_3$ | $CO_2CH_3$ |
| 4-$CH_3$ | H | Cl | H | 1 | 4,6-Di-$CH_3$ | $CO_2CH_3$ |
| 3-$CH_3$ | F | Cl | H | 1 | 4,6-Di-$CH_3$ | $CO_2CH_3$ |
| 4-$CH_3$ | F | Cl | H | 1 | 4,6-Di-$CH_3$ | $CO_2CH_3$ |
| 3-$CH_3$ | H | F | $CH_3$ | 1 | 4,6-Di-$CH_3$ | $CO_2CH_3$ |
| 4-$CH_3$ | H | F | $CH_3$ | 1 | 4,6-Di-$CH_3$ | $CO_2CH_3$ |
| 3-$CH_3$ | F | F | $CH_3$ | 1 | 4,6-Di-$CH_3$ | $CO_2CH_3$ |
| 4-$CH_3$ | F | F | $CH_3$ | 1 | 4,6-Di-$CH_3$ | $CO_2CH_3$ |
| 3-$CH_3$ | H | Cl | $CH_3$ | 1 | 4,6-Di-$CH_3$ | $CO_2CH_3$ |
| 4-$CH_3$ | H | Cl | $CH_3$ | 1 | 4,6-Di-$CH_3$ | $CO_2CH_3$ |
| 3-$CH_3$ | F | Cl | $CH_3$ | 1 | 4,6-Di-$CH_3$ | $CO_2CH_3$ |
| 4-$CH_3$ | F | Cl | $CH_3$ | 1 | 4,6-Di-$CH_3$ | $CO_2CH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4,6-Di-$CH_3$ | $CO_2CH_2CH_3$ |
| 4-$CH_3$ | H | F | H | 1 | 4,6-Di-$CH_3$ | $CO_2CH_2CH_3$ |
| 3-$CH_3$ | F | F | H | 1 | 4,6-Di-$CH_3$ | $CO_2CH_2CH_3$ |
| 4-$CH_3$ | F | F | H | 1 | 4,6-Di-$CH_3$ | $CO_2CH_2CH_3$ |
| 3-$CH_3$ | H | Cl | H | 1 | 4,6-Di-$CH_3$ | $CO_2CH_2CH_3$ |
| 4-$CH_3$ | H | Cl | H | 1 | 4,6-Di-$CH_3$ | $CO_2CH_2CH_3$ |
| 3-$CH_3$ | F | Cl | H | 1 | 4,6-Di-$CH_3$ | $CO_2CH_2CH_3$ |
| 4-$CH_3$ | F | Cl | H | 1 | 4,6-Di-$CH_3$ | $CO_2CH_2CH_3$ |
| 3-$CH_3$ | H | F | $CH_3$ | 1 | 4,6-Di-$CH_3$ | $CO_2CH_2CH_3$ |
| 4-$CH_3$ | H | F | $CH_3$ | 1 | 4,6-Di-$CH_3$ | $CO_2CH_2CH_3$ |
| 3-$CH_3$ | F | F | $CH_3$ | 1 | 4,6-Di-$CH_3$ | $CO_2CH_2CH_3$ |
| 4-$CH_3$ | F | F | $CH_3$ | 1 | 4,6-Di-$CH_3$ | $CO_2CH_2CH_3$ |
| 3-$CH_3$ | H | Cl | $CH_3$ | 1 | 4,6-Di-$CH_3$ | $CO_2CH_2CH_3$ |
| 4-$CH_3$ | H | Cl | $CH_3$ | 1 | 4,6-Di-$CH_3$ | $CO_2CH_2CH_3$ |
| 3-$CH_3$ | F | Cl | $CH_3$ | 1 | 4,6-Di-$CH_3$ | $CO_2CH_2CH_3$ |
| 4-$CH_3$ | F | Cl | $CH_3$ | 1 | 4,6-Di-$CH_3$ | $CO_2CH_2CH_3$ |
| 3-$CH_3$ | H | F | H | 1 | 4,6-Di-$CH_3$ | $CO_2(CH_2)_2CH_3$ |
| 4-$CH_3$ | H | F | H | 1 | 4,6-Di-$CH_3$ | $CO_2(CH_2)_2CH_3$ |
| 3-$CH_3$ | F | F | H | 1 | 4,6-Di-$CH_3$ | $CO_2(CH_2)_2CH_3$ |
| 4-$CH_3$ | F | F | H | 1 | 4,6-Di-$CH_3$ | $CO_2(CH_2)_2CH_3$ |
| 3-$CH_3$ | H | Cl | H | 1 | 4,6-Di-$CH_3$ | $CO_2(CH_2)_2CH_3$ |
| 4-$CH_3$ | H | Cl | H | 1 | 4,6-Di-$CH_3$ | $CO_2(CH_2)_2CH_3$ |
| 3-$CH_3$ | F | Cl | H | 1 | 4,6-Di-$CH_3$ | $CO_2(CH_2)_2CH_3$ |
| 4-$CH_3$ | F | Cl | H | 1 | 4,6-Di-$CH_3$ | $CO_2(CH_2)_2CH_3$ |
| 3-$CH_3$ | H | F | $CH_3$ | 1 | 4,6-Di-$CH_3$ | $CO_2(CH_2)_2CH_3$ |
| 4-$CH_3$ | H | F | $CH_3$ | 1 | 4,6-Di-$CH_3$ | $CO_2(CH_2)_2CH_3$ |
| 3-$CH_3$ | F | F | $CH_3$ | 1 | 4,6-Di-$CH_3$ | $CO_2(CH_2)_2CH_3$ |
| 4-$CH_3$ | F | F | $CH_3$ | 1 | 4,6-Di-$CH_3$ | $CO_2(CH_2)_2CH_3$ |
| 3-$CH_3$ | H | Cl | $CH_3$ | 1 | 4,6-Di-$CH_3$ | $CO_2(CH_2)_2CH_3$ |
| 4-$CH_3$ | H | Cl | $CH_3$ | 1 | 4,6-Di-$CH_3$ | $CO_2(CH_2)_2CH_3$ |
| 3-$CH_3$ | F | Cl | $CH_3$ | 1 | 4,6-Di-$CH_3$ | $CO_2(CH_2)_2CH_3$ |
| 4-$CH_3$ | F | Cl | $CH_3$ | 1 | 4,6-Di-$CH_3$ | $CO_2(CH_2)_2CH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4,6-Di-$CH_3$ | $CO_2CH(CH_3)_2$ |
| 4-$CH_3$ | H | F | H | 1 | 4,6-Di-$CH_3$ | $CO_2CH(CH_3)_2$ |
| 3-$CH_3$ | F | F | H | 1 | 4,6-Di-$CH_3$ | $CO_2CH(CH_3)_2$ |
| 4-$CH_3$ | F | F | H | 1 | 4,6-Di-$CH_3$ | $CO_2CH(CH_3)_2$ |
| 3-$CH_3$ | H | Cl | H | 1 | 4,6-Di-$CH_3$ | $CO_2CH(CH_3)_2$ |
| 4-$CH_3$ | H | Cl | H | 1 | 4,6-Di-$CH_3$ | $CO_2CH(CH_3)_2$ |
| 3-$CH_3$ | F | Cl | H | 1 | 4,6-Di-$CH_3$ | $CO_2CH(CH_3)_2$ |
| 4-$CH_3$ | F | Cl | H | 1 | 4,6-Di-$CH_3$ | $CO_2CH(CH_3)_2$ |
| 3-$CH_3$ | H | F | $CH_3$ | 1 | 4,6-Di-$CH_3$ | $CO_2CH(CH_3)_2$ |
| 4-$CH_3$ | H | F | $CH_3$ | 1 | 4,6-Di-$CH_3$ | $CO_2CH(CH_3)_2$ |
| 3-$CH_3$ | F | F | $CH_3$ | 1 | 4,6-Di-$CH_3$ | $CO_2CH(CH_3)_2$ |
| 4-$CH_3$ | F | F | $CH_3$ | 1 | 4,6-Di-$CH_3$ | $CO_2CH(CH_3)_2$ |
| 3-$CH_3$ | H | Cl | $CH_3$ | 1 | 4,6-Di-$CH_3$ | $CO_2CH(CH_3)_2$ |
| 4-$CH_3$ | H | Cl | $CH_3$ | 1 | 4,6-Di-$CH_3$ | $CO_2CH(CH_3)_2$ |
| 3-$CH_3$ | F | Cl | $CH_3$ | 1 | 4,6-Di-$CH_3$ | $CO_2CH(CH_3)_2$ |
| 4-$CH_3$ | F | Cl | $CH_3$ | 1 | 4,6-Di-$CH_3$ | $CO_2CH(CH_3)_2$ |
| 3-$CH_3$ | H | F | H | 1 | 4,6-Di-$CH_3$ | $CO_2(CH_2)_3CH_3$ |
| 4-$CH_3$ | H | F | H | 1 | 4,6-Di-$CH_3$ | $CO_2(CH_2)_3CH_3$ |
| 3-$CH_3$ | F | F | H | 1 | 4,6-Di-$CH_3$ | $CO_2(CH_2)_3CH_3$ |
| 4-$CH_3$ | F | F | H | 1 | 4,6-Di-$CH_3$ | $CO_2(CH_2)_3CH_3$ |
| 3-$CH_3$ | H | Cl | H | 1 | 4,6-Di-$CH_3$ | $CO_2(CH_2)_3CH_3$ |
| 4-$CH_3$ | H | Cl | H | 1 | 4,6-Di-$CH_3$ | $CO_2(CH_2)_3CH_3$ |
| 3-$CH_3$ | F | Cl | H | 1 | 4,6-Di-$CH_3$ | $CO_2(CH_2)_3CH_3$ |
| 4-$CH_3$ | F | Cl | H | 1 | 4,6-Di-$CH_3$ | $CO_2(CH_2)_3CH_3$ |
| 3-$CH_3$ | H | F | $CH_3$ | 1 | 4,6-Di-$CH_3$ | $CO_2(CH_2)_3CH_3$ |
| 4-$CH_3$ | H | F | $CH_3$ | 1 | 4,6-Di-$CH_3$ | $CO_2(CH_2)_3CH_3$ |
| 3-$CH_3$ | F | F | $CH_3$ | 1 | 4,6-Di-$CH_3$ | $CO_2(CH_2)_3CH_3$ |
| 4-$CH_3$ | F | F | $CH_3$ | 1 | 4,6-Di-$CH_3$ | $CO_2(CH_2)_3CH_3$ |
| 3-$CH_3$ | H | Cl | $CH_3$ | 1 | 4,6-Di-$CH_3$ | $CO_2(CH_2)_3CH_3$ |
| 4-$CH_3$ | H | Cl | $CH_3$ | 1 | 4,6-Di-$CH_3$ | $CO_2(CH_2)_3CH_3$ |
| 3-$CH_3$ | F | Cl | $CH_3$ | 1 | 4,6-Di-$CH_3$ | $CO_2(CH_2)_3CH_3$ |
| 4-$CH_3$ | F | Cl | $CH_3$ | 1 | 4,6-Di-$CH_3$ | $CO_2(CH_2)_3CH_3$ |
| 3-$CH_3$ | H | F | H | 1 | 4,6-Di-$CH_3$ | $CO_2(CH_2)_2OCH_3$ |

Tabelle B (Fortsetzung)

| $R_n$ | $R^1$ | $R^2$ | $R^3$ | m | $(R^a)_q$ | $R^4$ |
|---|---|---|---|---|---|---|
| 4-$CH_3$ | H | F | H | 1 | 4,6-Di-$CH_3$ | $CO_2(CH_2)_2OCH_3$ |
| 3-$CH_3$ | F | F | H | 1 | 4,6-Di-$CH_3$ | $CO_2(CH_2)_2OCH_3$ |
| 4-$CH_3$ | F | F | H | 1 | 4,6-Di-$CH_3$ | $CO_2(CH_2)_2OCH_3$ |
| 3-$CH_3$ | H | Cl | H | 1 | 4,6-Di-$CH_3$ | $CO_2(CH_2)_2OCH_3$ |
| 4-$CH_3$ | H | Cl | H | 1 | 4,6-Di-$CH_3$ | $CO_2(CH_2)_2OCH_3$ |
| 3-$CH_3$ | F | Cl | H | 1 | 4,6-Di-$CH_3$ | $CO_2(CH_2)_2OCH_3$ |
| 4-$CH_3$ | F | Cl | H | 1 | 4,6-Di-$CH_3$ | $CO_2(CH_2)_2OCH_3$ |
| 3-$CH_3$ | H | F | $CH_3$ | 1 | 4,6-Di-$CH_3$ | $CO_2(CH_2)_2OCH_3$ |
| 4-$CH_3$ | H | F | $CH_3$ | 1 | 4,6-Di-$CH_3$ | $CO_2(CH_2)_2OCH_3$ |
| 3-$CH_3$ | F | F | $CH_3$ | 1 | 4,6-Di-$CH_3$ | $CO_2(CH_2)_2OCH_3$ |
| 4-$CH_3$ | F | F | $CH_3$ | 1 | 4,6-Di-$CH_3$ | $CO_2(CH_2)_2OCH_3$ |
| 3-$CH_3$ | H | Cl | $CH_3$ | 1 | 4,6-Di-$CH_3$ | $CO_2(CH_2)_2OCH_3$ |
| 4-$CH_3$ | H | Cl | $CH_3$ | 1 | 4,6-Di-$CH_3$ | $CO_2(CH_2)_2OCH_3$ |
| 3-$CH_3$ | F | Cl | $CH_3$ | 1 | 4,6-Di-$CH_3$ | $CO_2(CH_2)_2OCH_3$ |
| 4-$CH_3$ | F | Cl | $CH_3$ | 1 | 4,6-Di-$CH_3$ | $CO_2(CH_2)_2OCH_3$ |
| 3-$CH_3$ | H | F | H | 1 | 4,6-Di-$CH_3$ | $CO_2(CH_2)_2OCH_2CH_3$ |
| 4-$CH_3$ | H | F | H | 1 | 4,6-Di-$CH_3$ | $CO_2(CH_2)_2OCH_2CH_3$ |
| 3-$CH_3$ | F | F | H | 1 | 4,6-Di-$CH_3$ | $CO_2(CH_2)_2OCH_2CH_3$ |
| 4-$CH_3$ | F | F | H | 1 | 6-$CH_3$ | $CO_2(CH_2)_2OCH_2CH_3$ |
| 3-$CH_3$ | H | Cl | H | 1 | 6-$CH_3$ | $CO_2(CH_2)_2OCH_2CH_3$ |
| 4-$CH_3$ | H | Cl | H | 1 | 6-$CH_3$ | $CO_2(CH_2)_2OCH_2CH_3$ |
| 3-$CH_3$ | F | Cl | H | 1 | 6-$CH_3$ | $CO_2(CH_2)_2OCH_2CH_3$ |
| 4-$CH_3$ | F | Cl | H | 1 | 6-$CH_3$ | $CO_2(CH_2)_2OCH_2CH_3$ |
| 3-$CH_3$ | H | F | $CH_3$ | 1 | 6-$CH_3$ | $CO_2(CH_2)_2OCH_2CH_3$ |
| 4-$CH_3$ | H | F | $CH_3$ | 1 | 6-$CH_3$ | $CO_2(CH_2)_2OCH_2CH_3$ |
| 3-$CH_3$ | F | F | $CH_3$ | 1 | 6-$CH_3$ | $CO_2(CH_2)_2OCH_2CH_3$ |
| 4-$CH_3$ | F | F | $CH_3$ | 1 | 6-$CH_3$ | $CO_2(CH_2)_2OCH_2CH_3$ |
| 3-$CH_3$ | H | Cl | $CH_3$ | 1 | 6-$CH_3$ | $CO_2(CH_2)_2OCH_2CH_3$ |
| 4-$CH_3$ | H | Cl | $CH_3$ | 1 | 6-$CH_3$ | $CO_2(CH_2)_2OCH_2CH_3$ |
| 3-$CH_3$ | F | Cl | $CH_3$ | 1 | 6-$CH_3$ | $CO_2(CH_2)_2OCH_2CH_3$ |
| 4-$CH_3$ | F | Cl | $CH_3$ | 1 | 6-$CH_3$ | $CO_2(CH_2)_2OCH_2CH_3$ |

Tabelle C

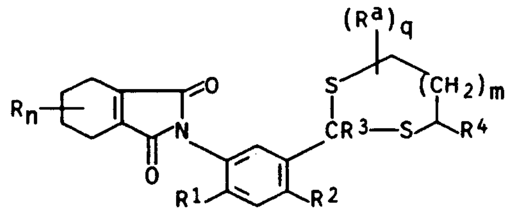

| $R_n$ | $R^1$ | $R^2$ | $R^3$ | m | $(R^a)_q$ | $R^4$ |
|---|---|---|---|---|---|---|
| 3-$CH_3$ | H | F | H | 0 | H | H |
| 4-$CH_3$ | H | F | H | 0 | H | H |
| 3-$CH_3$ | F | F | H | 0 | H | H |
| 4-$CH_3$ | F | F | H | 0 | H | H |
| 3-$CH_3$ | H | Cl | H | 0 | H | H |
| 4-$CH_3$ | H | Cl | H | 0 | H | H |
| 3-$CH_3$ | F | Cl | H | 0 | H | H |
| 4-$CH_3$ | F | Cl | H | 0 | H | H |
| 3-$CH_3$ | H | F | $CH_3$ | 0 | H | H |
| 4-$CH_3$ | H | F | $CH_3$ | 0 | H | H |
| 3-$CH_3$ | F | F | $CH_3$ | 0 | H | H |
| 4-$CH_3$ | F | F | $CH_3$ | 0 | H | H |
| 3-$CH_3$ | H | Cl | $CH_3$ | 0 | H | H |
| 4-$CH_3$ | H | Cl | $CH_3$ | 0 | H | H |
| 3-$CH_3$ | F | Cl | $CH_3$ | 0 | H | H |
| 4-$CH_3$ | F | Cl | $CH_3$ | 0 | H | H |
| 3-$CH_3$ | H | F | H | 0 | 4-$CH_3$ | H |
| 4-$CH_3$ | H | F | H | 0 | 4-$CH_3$ | H |
| 3-$CH_3$ | F | F | H | 0 | 4-$CH_3$ | H |
| 4-$CH_3$ | F | F | H | 0 | 4-$CH_3$ | H |
| 3-$CH_3$ | H | Cl | H | 0 | 4-$CH_3$ | H |
| 4-$CH_3$ | H | Cl | H | 0 | 4-$CH_3$ | H |
| 3-$CH_3$ | F | Cl | H | 0 | 4-$CH_3$ | H |
| 4-$CH_3$ | F | Cl | H | 0 | 4-$CH_3$ | H |
| 3-$CH_3$ | H | F | $CH_3$ | 0 | 4-$CH_3$ | H |
| 4-$CH_3$ | H | F | $CH_3$ | 0 | 4-$CH_3$ | H |
| 3-$CH_3$ | F | F | $CH_3$ | 0 | 4-$CH_3$ | H |

Tabelle C (Fortsetzung)

| $R_n$ | $R^1$ | $R^2$ | $R^3$ | m | $(R^a)_q$ | $R^4$ |
|---|---|---|---|---|---|---|
| 4-$CH_3$ | F | F | $CH_3$ | 0 | 4-$CH_3$ | H |
| 3-$CH_3$ | H | Cl | $CH_3$ | 0 | 4-$CH_3$ | H |
| 4-$CH_3$ | H | Cl | $CH_3$ | 0 | 4-$CH_3$ | H |
| 3-$CH_3$ | F | Cl | $CH_3$ | 0 | 4-$CH_3$ | H |
| 4-$CH_3$ | F | Cl | $CH_3$ | 0 | 4-$CH_3$ | H |
| 3-$CH_3$ | H | F | H | 0 | 4,5-Di-$CH_3$ | H |
| 4-$CH_3$ | H | F | H | 0 | 4,5-Di-$CH_3$ | H |
| 3-$CH_3$ | F | F | H | 0 | 4,5-Di-$CH_3$ | H |
| 4-$CH_3$ | F | F | H | 0 | 4,5-Di-$CH_3$ | H |
| 3-$CH_3$ | H | Cl | H | 0 | 4,5-Di-$CH_3$ | H |
| 4-$CH_3$ | H | Cl | H | 0 | 4,5-Di-$CH_3$ | H |
| 3-$CH_3$ | F | Cl | H | 0 | 4,5-Di-$CH_3$ | H |
| 4-$CH_3$ | F | Cl | H | 0 | 4,5-Di-$CH_3$ | H |
| 3-$CH_3$ | H | F | $CH_3$ | 0 | 4,5-Di-$CH_3$ | H |
| 4-$CH_3$ | H | F | $CH_3$ | 0 | 4,5-Di-$CH_3$ | H |
| 3-$CH_3$ | F | F | $CH_3$ | 0 | 4,5-Di-$CH_3$ | H |
| 4-$CH_3$ | F | F | $CH_3$ | 0 | 4,5-Di-$CH_3$ | H |
| 3-$CH_3$ | H | Cl | $CH_3$ | 0 | 4,5-Di-$CH_3$ | H |
| 4-$CH_3$ | H | Cl | $CH_3$ | 0 | 4,5-Di-$CH_3$ | H |
| 3-$CH_3$ | F | Cl | $CH_3$ | 0 | 4,5-Di-$CH_3$ | H |
| 4-$CH_3$ | F | Cl | $CH_3$ | 0 | 4,5-Di-$CH_3$ | H |
| 3-$CH_3$ | H | F | H | 0 | 4-$CH_2CH_3$ | H |
| 4-$CH_3$ | H | F | H | 0 | 4-$CH_2CH_3$ | H |
| 3-$CH_3$ | F | F | H | 0 | 4-$CH_2CH_3$ | H |
| 4-$CH_3$ | F | F | H | 0 | 4-$CH_2CH_3$ | H |
| 3-$CH_3$ | H | Cl | H | 0 | 4-$CH_2CH_3$ | H |
| 4-$CH_3$ | H | Cl | H | 0 | 4-$CH_2CH_3$ | H |
| 3-$CH_3$ | F | Cl | H | 0 | 4-$CH_2CH_3$ | H |
| 4-$CH_3$ | F | Cl | H | 0 | 4-$CH_2CH_3$ | H |
| 3-$CH_3$ | H | F | $CH_3$ | 0 | 4-$CH_2CH_3$ | H |
| 4-$CH_3$ | H | F | $CH_3$ | 0 | 4-$CH_2CH_3$ | H |
| 3-$CH_3$ | F | F | $CH_3$ | 0 | 4-$CH_2CH_3$ | H |

Tabelle C (Fortsetzung)

| $R_n$ | $R^1$ | $R^2$ | $R^3$ | m | $(R^a)_q$ | $R^4$ |
|---|---|---|---|---|---|---|
| 4-$CH_3$ | F | F | $CH_3$ | 0 | 4-$CH_2CH_3$ | H |
| 3-$CH_3$ | H | Cl | $CH_3$ | 0 | 4-$CH_2CH_3$ | H |
| 4-$CH_3$ | H | Cl | $CH_3$ | 0 | 4-$CH_2CH_3$ | H |
| 3-$CH_3$ | F | Cl | $CH_3$ | 0 | 4-$CH_2CH_3$ | H |
| 4-$CH_3$ | F | Cl | $CH_3$ | 0 | 4-$CH_2CH_3$ | H |
| 3-$CH_3$ | H | F | H | 0 | 4-$CH_2Cl$ | H |
| 4-$CH_3$ | H | F | H | 0 | 4-$CH_2Cl$ | H |
| 3-$CH_3$ | F | F | H | 0 | 4-$CH_2Cl$ | H |
| 4-$CH_3$ | F | F | H | 0 | 4-$CH_2Cl$ | H |
| 3-$CH_3$ | H | Cl | H | 0 | 4-$CH_2Cl$ | H |
| 4-$CH_3$ | H | Cl | H | 0 | 4-$CH_2Cl$ | H |
| 3-$CH_3$ | F | Cl | H | 0 | 4-$CH_2Cl$ | H |
| 4-$CH_3$ | F | Cl | H | 0 | 4-$CH_2Cl$ | H |
| 3-$CH_3$ | H | F | $CH_3$ | 0 | 4-$CH_2Cl$ | H |
| 4-$CH_3$ | H | F | $CH_3$ | 0 | 4-$CH_2Cl$ | H |
| 3-$CH_3$ | F | F | $CH_3$ | 0 | 4-$CH_2Cl$ | H |
| 4-$CH_3$ | F | F | $CH_3$ | 0 | 4-$CH_2Cl$ | H |
| 3-$CH_3$ | H | Cl | $CH_3$ | 0 | 4-$CH_2Cl$ | H |
| 4-$CH_3$ | H | Cl | $CH_3$ | 0 | 4-$CH_2Cl$ | H |
| 3-$CH_3$ | F | Cl | $CH_3$ | 0 | 4-$CH_2Cl$ | H |
| 4-$CH_3$ | F | Cl | $CH_3$ | 0 | 4-$CH_2Cl$ | H |
| 3-$CH_3$ | H | F | H | 0 | 4-$CH_2OH$ | H |
| 4-$CH_3$ | H | F | H | 0 | 4-$CH_2OH$ | H |
| 3-$CH_3$ | F | F | H | 0 | 4-$CH_2OH$ | H |
| 4-$CH_3$ | F | F | H | 0 | 4-$CH_2OH$ | H |
| 3-$CH_3$ | H | Cl | H | 0 | 4-$CH_2OH$ | H |
| 4-$CH_3$ | H | Cl | H | 0 | 4-$CH_2OH$ | H |
| 3-$CH_3$ | F | Cl | H | 0 | 4-$CH_2OH$ | H |
| 4-$CH_3$ | F | Cl | H | 0 | 4-$CH_2OH$ | H |
| 3-$CH_3$ | H | F | $CH_3$ | 0 | 4-$CH_2OH$ | H |
| 4-$CH_3$ | H | F | $CH_3$ | 0 | 4-$CH_2OH$ | H |
| 3-$CH_3$ | F | F | $CH_3$ | 0 | 4-$CH_2OH$ | H |
| 4-$CH_3$ | F | F | $CH_3$ | 0 | 4-$CH_2OH$ | H |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | Cl | $CH_3$ | 0 | 4-$CH_2OH$ | H |
| 4-$CH_3$ | H | Cl | $CH_3$ | 0 | 4-$CH_2OH$ | H |
| 3-$CH_3$ | F | Cl | $CH_3$ | 0 | 4-$CH_2OH$ | H |
| 4-$CH_3$ | F | Cl | $CH_3$ | 0 | 4-$CH_2OH$ | H |
| 3-$CH_3$ | H | F | H | 0 | 4-$CH_2OCH_3$ | H |
| 4-$CH_3$ | H | F | H | 0 | 4-$CH_2OCH_3$ | H |
| 3-$CH_3$ | F | F | H | 0 | 4-$CH_2OCH_3$ | H |
| 4-$CH_3$ | F | F | H | 0 | 4-$CH_2OCH_3$ | H |
| 3-$CH_3$ | H | Cl | H | 0 | 4-$CH_2OCH_3$ | H |
| 4-$CH_3$ | H | Cl | H | 0 | 4-$CH_2OCH_3$ | H |
| 3-$CH_3$ | F | Cl | H | 0 | 4-$CH_2OCH_3$ | H |
| 4-$CH_3$ | F | Cl | H | 0 | 4-$CH_2OCH_3$ | H |
| 3-$CH_3$ | H | F | $CH_3$ | 0 | 4-$CH_2OCH_3$ | H |
| 4-$CH_3$ | H | F | $CH_3$ | 0 | 4-$CH_2OCH_3$ | H |
| 3-$CH_3$ | F | F | $CH_3$ | 0 | 4-$CH_2OCH_3$ | H |
| 4-$CH_3$ | F | F | $CH_3$ | 0 | 4-$CH_2OCH_3$ | H |
| 3-$CH_3$ | H | Cl | $CH_3$ | 0 | 4-$CH_2OCH_3$ | H |
| 4-$CH_3$ | H | Cl | $CH_3$ | 0 | 4-$CH_2OCH_3$ | H |
| 3-$CH_3$ | F | Cl | $CH_3$ | 0 | 4-$CH_2OCH_3$ | H |
| 4-$CH_3$ | F | Cl | $CH_3$ | 0 | 4-$CH_2OCH_3$ | H |
| 3-$CH_3$ | H | F | H | 0 | 4-$CH_2OCH_2CH=CH_2$ | H |
| 4-$CH_3$ | H | F | H | 0 | 4-$CH_2OCH_2CH=CH_2$ | H |
| 3-$CH_3$ | F | F | H | 0 | 4-$CH_2OCH_2CH=CH_2$ | H |
| 4-$CH_3$ | F | F | H | 0 | 4-$CH_2OCH_2CH=CH_2$ | H |
| 3-$CH_3$ | H | Cl | H | 0 | 4-$CH_2OCH_2CH=CH_2$ | H |
| 4-$CH_3$ | H | Cl | H | 0 | 4-$CH_2OCH_2CH=CH_2$ | H |
| 3-$CH_3$ | F | Cl | H | 0 | 4-$CH_2OCH_2CH=CH_2$ | H |
| 4-$CH_3$ | F | Cl | H | 0 | 4-$CH_2OCH_2CH=CH_2$ | H |
| 3-$CH_3$ | H | F | $CH_3$ | 0 | 4-$CH_2OCH_2CH=CH_2$ | H |
| 4-$CH_3$ | H | F | $CH_3$ | 0 | 4-$CH_2OCH_2CH=CH_2$ | H |
| 3-$CH_3$ | F | F | $CH_3$ | 0 | 4-$CH_2OCH_2CH=CH_2$ | H |
| 4-$CH_3$ | F | F | $CH_3$ | 0 | 4-$CH_2OCH_2CH=CH_2$ | H |
| 3-$CH_3$ | H | Cl | $CH_3$ | 0 | 4-$CH_2OCH_2CH=CH_2$ | H |

Tabelle C (Fortsetzung)

| $R_n$ | $R^1$ | $R^2$ | $R^3$ | m | $(R^a)_q$ | $R^4$ |
|---|---|---|---|---|---|---|
| 4-$CH_3$ | H | Cl | $CH_3$ | 0 | 4-$CH_2OCH_2CH$=$CH_2$ | H |
| 3-$CH_3$ | F | Cl | $CH_3$ | 0 | 4-$CH_2OCH_2CH$=$CH_2$ | H |
| 4-$CH_3$ | F | Cl | $CH_3$ | 0 | 4-$CH_2OCH_2CH$=$CH_2$ | H |
| 3-$CH_3$ | H | F | H | 0 | 4-$CH_2OCH_2C≡CH$ | H |
| 4-$CH_3$ | H | F | H | 0 | 4-$CH_2OCH_2C≡CH$ | H |
| 3-$CH_3$ | F | F | H | 0 | 4-$CH_2OCH_2C≡CH$ | H |
| 4-$CH_3$ | F | F | H | 0 | 4-$CH_2OCH_2C≡CH$ | H |
| 3-$CH_3$ | H | Cl | H | 0 | 4-$CH_2OCH_2C≡CH$ | H |
| 4-$CH_3$ | H | Cl | H | 0 | 4-$CH_2OCH_2C≡CH$ | H |
| 3-$CH_3$ | F | Cl | H | 0 | 4-$CH_2OCH_2C≡CH$ | H |
| 4-$CH_3$ | F | Cl | H | 0 | 4-$CH_2OCH_2C≡CH$ | H |
| 3-$CH_3$ | H | F | $CH_3$ | 0 | 4-$CH_2OCH_2C≡CH$ | H |
| 4-$CH_3$ | H | F | $CH_3$ | 0 | 4-$CH_2OCH_2C≡CH$ | H |
| 3-$CH_3$ | F | F | $CH_3$ | 0 | 4-$CH_2OCH_2C≡CH$ | H |
| 4-$CH_3$ | F | F | $CH_3$ | 0 | 4-$CH_2OCH_2C≡CH$ | H |
| 3-$CH_3$ | H | Cl | $CH_3$ | 0 | 4-$CH_2OCH_2C≡CH$ | H |
| 4-$CH_3$ | H | Cl | $CH_3$ | 0 | 4-$CH_2OCH_2C≡CH$ | H |
| 3-$CH_3$ | F | Cl | $CH_3$ | 0 | 4-$CH_2OCH_2C≡CH$ | H |
| 4-$CH_3$ | F | Cl | $CH_3$ | 0 | 4-$CH_2OCH_2C≡CH$ | H |
| 3-$CH_3$ | H | F | H | 0 | 4-$CH_2OCOCH_3$ | H |
| 4-$CH_3$ | H | F | H | 0 | 4-$CH_2OCOCH_3$ | H |
| 3-$CH_3$ | F | F | H | 0 | 4-$CH_2OCOCH_3$ | H |
| 4-$CH_3$ | F | F | H | 0 | 4-$CH_2OCOCH_3$ | H |
| 3-$CH_3$ | H | Cl | H | 0 | 4-$CH_2OCOCH_3$ | H |
| 4-$CH_3$ | H | Cl | H | 0 | 4-$CH_2OCOCH_3$ | H |
| 3-$CH_3$ | F | Cl | H | 0 | 4-$CH_2OCOCH_3$ | H |
| 4-$CH_3$ | F | Cl | H | 0 | 4-$CH_2OCOCH_3$ | H |
| 3-$CH_3$ | H | F | $CH_3$ | 0 | 4-$CH_2OCOCH_3$ | H |
| 4-$CH_3$ | H | F | $CH_3$ | 0 | 4-$CH_2OCOCH_3$ | H |
| 3-$CH_3$ | F | F | $CH_3$ | 0 | 4-$CH_2OCOCH_3$ | H |
| 4-$CH_3$ | F | F | $CH_3$ | 0 | 4-$CH_2OCOCH_3$ | H |
| 3-$CH_3$ | H | Cl | $CH_3$ | 0 | 4-$CH_2OCOCH_3$ | H |
| 4-$CH_3$ | H | Cl | $CH_3$ | 0 | 4-$CH_2OCOCH_3$ | H |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | F | Cl | $CH_3$ | 0 | 4-$CH_2OCOCH_3$ | H |
| 4-$CH_3$ | F | Cl | $CH_3$ | 0 | 4-$CH_2OCOCH_3$ | H |
| 3-$CH_3$ | H | F | H | 0 | 4-$CH_2OCH_2CO_2CH_3$ | H |
| 4-$CH_3$ | H | F | H | 0 | 4-$CH_2OCH_2CO_2CH_3$ | H |
| 3-$CH_3$ | F | F | H | 0 | 4-$CH_2OCH_2CO_2CH_3$ | H |
| 4-$CH_3$ | F | F | H | 0 | 4-$CH_2OCH_2CO_2CH_3$ | H |
| 3-$CH_3$ | H | Cl | H | 0 | 4-$CH_2OCH_2CO_2CH_3$ | H |
| 4-$CH_3$ | H | Cl | H | 0 | 4-$CH_2OCH_2CO_2CH_3$ | H |
| 3-$CH_3$ | F | Cl | H | 0 | 4-$CH_2OCH_2CO_2CH_3$ | H |
| 4-$CH_3$ | F | Cl | H | 0 | 4-$CH_2OCH_2CO_2CH_3$ | H |
| 3-$CH_3$ | H | F | $CH_3$ | 0 | 4-$CH_2OCH_2CO_2CH_3$ | H |
| 4-$CH_3$ | H | F | $CH_3$ | 0 | 4-$CH_2OCH_2CO_2CH_3$ | H |
| 3-$CH_3$ | F | F | $CH_3$ | 0 | 4-$CH_2OCH_2CO_2CH_3$ | H |
| 4-$CH_3$ | F | F | $CH_3$ | 0 | 4-$CH_2OCH_2CO_2CH_3$ | H |
| 3-$CH_3$ | H | Cl | $CH_3$ | 0 | 4-$CH_2OCH_2CO_2CH_3$ | H |
| 4-$CH_3$ | H | Cl | $CH_3$ | 0 | 4-$CH_2OCH_2CO_2CH_3$ | H |
| 3-$CH_3$ | F | Cl | $CH_3$ | 0 | 4-$CH_2OCH_2CO_2CH_3$ | H |
| 4-$CH_3$ | F | Cl | $CH_3$ | 0 | 4-$CH_2OCH_2CO_2CH_3$ | H |
| 3-$CH_3$ | H | F | H | 0 | 4-$CH_2CO_2CH_3$ | H |
| 4-$CH_3$ | H | F | H | 0 | 4-$CH_2CO_2CH_3$ | H |
| 3-$CH_3$ | F | F | H | 0 | 4-$CH_2CO_2CH_3$ | H |
| 4-$CH_3$ | F | F | H | 0 | 4-$CH_2CO_2CH_3$ | H |
| 3-$CH_3$ | H | Cl | H | 0 | 4-$CH_2CO_2CH_3$ | H |
| 4-$CH_3$ | H | Cl | H | 0 | 4-$CH_2CO_2CH_3$ | H |
| 3-$CH_3$ | F | Cl | H | 0 | 4-$CH_2CO_2CH_3$ | H |
| 4-$CH_3$ | F | Cl | H | 0 | 4-$CH_2CO_2CH_3$ | H |
| 3-$CH_3$ | H | F | $CH_3$ | 0 | 4-$CH_2CO_2CH_3$ | H |
| 4-$CH_3$ | H | F | $CH_3$ | 0 | 4-$CH_2CO_2CH_3$ | H |
| 3-$CH_3$ | F | F | $CH_3$ | 0 | 4-$CH_2CO_2CH_3$ | H |
| 4-$CH_3$ | F | F | $CH_3$ | 0 | 4-$CH_2CO_2CH_3$ | H |
| 3-$CH_3$ | H | Cl | $CH_3$ | 0 | 4-$CH_2CO_2CH_3$ | H |
| 4-$CH_3$ | H | Cl | $CH_3$ | 0 | 4-$CH_2CO_2CH_3$ | H |
| 3-$CH_3$ | F | Cl | $CH_3$ | 0 | 4-$CH_2CO_2CH_3$ | H |

Tabelle C (Fortsetzung)

| $R_n$ | $R^1$ | $R^2$ | $R^3$ | m | $(R^a)_q$ | $R^4$ |
|---|---|---|---|---|---|---|
| 4-$CH_3$ | F | Cl | $CH_3$ | 0 | 4-$CH_2CO_2CH_3$ | H |
| 3-$CH_3$ | H | F | H | 0 | 4-$CH_2SH$ | H |
| 4-$CH_3$ | H | F | H | 0 | 4-$CH_2SH$ | H |
| 3-$CH_3$ | F | F | H | 0 | 4-$CH_2SH$ | H |
| 4-$CH_3$ | F | F | H | 0 | 4-$CH_2SH$ | H |
| 3-$CH_3$ | H | Cl | H | 0 | 4-$CH_2SH$ | H |
| 4-$CH_3$ | H | Cl | H | 0 | 4-$CH_2SH$ | H |
| 3-$CH_3$ | F | Cl | H | 0 | 4-$CH_2SH$ | H |
| 4-$CH_3$ | F | Cl | H | 0 | 4-$CH_2SH$ | H |
| 3-$CH_3$ | H | F | $CH_3$ | 0 | 4-$CH_2SH$ | H |
| 4-$CH_3$ | H | F | $CH_3$ | 0 | 4-$CH_2SH$ | H |
| 3-$CH_3$ | F | F | $CH_3$ | 0 | 4-$CH_2SH$ | H |
| 4-$CH_3$ | F | F | $CH_3$ | 0 | 4-$CH_2SH$ | H |
| 3-$CH_3$ | H | Cl | $CH_3$ | 0 | 4-$CH_2SH$ | H |
| 4-$CH_3$ | H | Cl | $CH_3$ | 0 | 4-$CH_2SH$ | H |
| 3-$CH_3$ | F | Cl | $CH_3$ | 0 | 4-$CH_2SH$ | H |
| 4-$CH_3$ | F | Cl | $CH_3$ | 0 | 4-$CH_2SH$ | H |
| 3-$CH_3$ | H | F | H | 0 | 4-$CH_2SCH_2CH_3$ | H |
| 4-$CH_3$ | H | F | H | 0 | 4-$CH_2SCH_2CH_3$ | H |
| 3-$CH_3$ | F | F | H | 0 | 4-$CH_2SCH_2CH_3$ | H |
| 4-$CH_3$ | F | F | H | 0 | 4-$CH_2SCH_2CH_3$ | H |
| 3-$CH_3$ | H | Cl | H | 0 | 4-$CH_2SCH_2CH_3$ | H |
| 4-$CH_3$ | H | Cl | H | 0 | 4-$CH_2SCH_2CH_3$ | H |
| 3-$CH_3$ | F | Cl | H | 0 | 4-$CH_2SCH_2CH_3$ | H |
| 4-$CH_3$ | F | Cl | H | 0 | 4-$CH_2SCH_2CH_3$ | H |
| 3-$CH_3$ | H | F | $CH_3$ | 0 | 4-$CH_2SCH_2CH_3$ | H |
| 4-$CH_3$ | H | F | $CH_3$ | 0 | 4-$CH_2SCH_2CH_3$ | H |
| 3-$CH_3$ | F | F | $CH_3$ | 0 | 4-$CH_2SCH_2CH_3$ | H |
| 4-$CH_3$ | F | F | $CH_3$ | 0 | 4-$CH_2SCH_2CH_3$ | H |
| 3-$CH_3$ | H | Cl | $CH_3$ | 0 | 4-$CH_2SCH_2CH_3$ | H |
| 4-$CH_3$ | H | Cl | $CH_3$ | 0 | 4-$CH_2SCH_2CH_3$ | H |
| 3-$CH_3$ | F | Cl | $CH_3$ | 0 | 4-$CH_2SCH_2CH_3$ | H |
| 4-$CH_3$ | F | Cl | $CH_3$ | 0 | 4-$CH_2SCH_2CH_3$ | H |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 4-$CH_2SCH_2CH{=}CH_2$ | H |
| 4-$CH_3$ | H | F | H | 0 | 4-$CH_2SCH_2CH{=}CH_2$ | H |
| 3-$CH_3$ | F | F | H | 0 | 4-$CH_2SCH_2CH{=}CH_2$ | H |
| 4-$CH_3$ | F | F | H | 0 | 4-$CH_2SCH_2CH{=}CH_2$ | H |
| 3-$CH_3$ | H | Cl | H | 0 | 4-$CH_2SCH_2CH{=}CH_2$ | H |
| 4-$CH_3$ | H | Cl | H | 0 | 4-$CH_2SCH_2CH{=}CH_2$ | H |
| 3-$CH_3$ | F | Cl | H | 0 | 4-$CH_2SCH_2CH{=}CH_2$ | H |
| 4-$CH_3$ | F | Cl | H | 0 | 4-$CH_2SCH_2CH{=}CH_2$ | H |
| 3-$CH_3$ | H | F | $CH_3$ | 0 | 4-$CH_2SCH_2CH{=}CH_2$ | H |
| 4-$CH_3$ | H | F | $CH_3$ | 0 | 4-$CH_2SCH_2CH{=}CH_2$ | H |
| 3-$CH_3$ | F | F | $CH_3$ | 0 | 4-$CH_2SCH_2CH{=}CH_2$ | H |
| 4-$CH_3$ | F | F | $CH_3$ | 0 | 4-$CH_2SCH_2CH{=}CH_2$ | H |
| 3-$CH_3$ | H | Cl | $CH_3$ | 0 | 4-$CH_2SCH_2CH{=}CH_2$ | H |
| 4-$CH_3$ | H | Cl | $CH_3$ | 0 | 4-$CH_2SCH_2CH{=}CH_2$ | H |
| 3-$CH_3$ | F | Cl | $CH_3$ | 0 | 4-$CH_2SCH_2CH{=}CH_2$ | H |
| 4-$CH_3$ | F | Cl | $CH_3$ | 0 | 4-$CH_2SCH_2CH{=}CH_2$ | H |
| 3-$CH_3$ | H | F | H | 0 | 4-$CO_2H$ | H |
| 4-$CH_3$ | H | F | H | 0 | 4-$CO_2H$ | H |
| 3-$CH_3$ | F | F | H | 0 | 4-$CO_2H$ | H |
| 4-$CH_3$ | F | F | H | 0 | 4-$CO_2H$ | H |
| 3-$CH_3$ | H | Cl | H | 0 | 4-$CO_2H$ | H |
| 4-$CH_3$ | H | Cl | H | 0 | 4-$CO_2H$ | H |
| 3-$CH_3$ | F | Cl | H | 0 | 4-$CO_2H$ | H |
| 4-$CH_3$ | F | Cl | H | 0 | 4-$CO_2H$ | H |
| 3-$CH_3$ | H | F | $CH_3$ | 0 | 4-$CO_2H$ | H |
| 4-$CH_3$ | H | F | $CH_3$ | 0 | 4-$CO_2H$ | H |
| 3-$CH_3$ | F | F | $CH_3$ | 0 | 4-$CO_2H$ | H |
| 4-$CH_3$ | F | F | $CH_3$ | 0 | 4-$CO_2H$ | H |
| 3-$CH_3$ | H | Cl | $CH_3$ | 0 | 4-$CO_2H$ | H |
| 4-$CH_3$ | H | Cl | $CH_3$ | 0 | 4-$CO_2H$ | H |
| 3-$CH_3$ | F | Cl | $CH_3$ | 0 | 4-$CO_2H$ | H |
| 4-$CH_3$ | F | Cl | $CH_3$ | 0 | 4-$CO_2H$ | H |
| 3-$CH_3$ | H | F | H | 1 | H | H |

Tabelle C (Fortsetzung)

| $R_n$ | $R^1$ | $R^2$ | $R^3$ | m | $(R^a)_q$ | $R^4$ |
|---|---|---|---|---|---|---|
| 4-$CH_3$ | H | F | H | 1 | H | H |
| 3-$CH_3$ | F | F | H | 1 | H | H |
| 4-$CH_3$ | F | F | H | 1 | H | H |
| 3-$CH_3$ | H | Cl | H | 1 | H | H |
| 4-$CH_3$ | H | Cl | H | 1 | H | H |
| 3-$CH_3$ | F | Cl | H | 1 | H | H |
| 4-$CH_3$ | F | Cl | H | 1 | H | H |
| 3-$CH_3$ | H | F | $CH_3$ | 1 | H | H |
| 4-$CH_3$ | H | F | $CH_3$ | 1 | H | H |
| 3-$CH_3$ | F | F | $CH_3$ | 1 | H | H |
| 4-$CH_3$ | F | F | $CH_3$ | 1 | H | H |
| 3-$CH_3$ | H | Cl | $CH_3$ | 1 | H | H |
| 4-$CH_3$ | H | Cl | $CH_3$ | 1 | H | H |
| 3-$CH_3$ | F | Cl | $CH_3$ | 1 | H | H |
| 4-$CH_3$ | F | Cl | $CH_3$ | 1 | H | H |
| 3-$CH_3$ | H | F | H | 1 | 4-$CH_3$ | H |
| 4-$CH_3$ | H | F | H | 1 | 4-$CH_3$ | H |
| 3-$CH_3$ | F | F | H | 1 | 4-$CH_3$ | H |
| 4-$CH_3$ | F | F | H | 1 | 4-$CH_3$ | H |
| 3-$CH_3$ | H | Cl | H | 1 | 4-$CH_3$ | H |
| 4-$CH_3$ | H | Cl | H | 1 | 4-$CH_3$ | H |
| 3-$CH_3$ | F | Cl | H | 1 | 4-$CH_3$ | H |
| 4-$CH_3$ | F | Cl | H | 1 | 4-$CH_3$ | H |
| 3-$CH_3$ | H | F | $CH_3$ | 1 | 4-$CH_3$ | H |
| 4-$CH_3$ | H | F | $CH_3$ | 1 | 4-$CH_3$ | H |
| 3-$CH_3$ | F | F | $CH_3$ | 1 | 4-$CH_3$ | H |
| 4-$CH_3$ | F | F | $CH_3$ | 1 | 4-$CH_3$ | H |
| 3-$CH_3$ | H | Cl | $CH_3$ | 1 | 4-$CH_3$ | H |
| 4-$CH_3$ | H | Cl | $CH_3$ | 1 | 4-$CH_3$ | H |
| 3-$CH_3$ | F | Cl | $CH_3$ | 1 | 4-$CH_3$ | H |
| 4-$CH_3$ | F | Cl | $CH_3$ | 1 | 4-$CH_3$ | H |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4,6-Di-$CH_3$ | H |
| 4-$CH_3$ | H | F | H | 1 | 4,6-Di-$CH_3$ | H |
| 3-$CH_3$ | F | F | H | 1 | 4,6-Di-$CH_3$ | H |
| 4-$CH_3$ | F | F | H | 1 | 4,6-Di-$CH_3$ | H |
| 3-$CH_3$ | H | Cl | H | 1 | 4,6-Di-$CH_3$ | H |
| 4-$CH_3$ | H | Cl | H | 1 | 4,6-Di-$CH_3$ | H |
| 3-$CH_3$ | F | Cl | H | 1 | 4,6-Di-$CH_3$ | H |
| 4-$CH_3$ | F | Cl | H | 1 | 4,6-Di-$CH_3$ | H |
| 3-$CH_3$ | H | F | $CH_3$ | 1 | 4,6-Di-$CH_3$ | H |
| 4-$CH_3$ | H | F | $CH_3$ | 1 | 4,6-Di-$CH_3$ | H |
| 3-$CH_3$ | F | F | $CH_3$ | 1 | 4,6-Di-$CH_3$ | H |
| 4-$CH_3$ | F | F | $CH_3$ | 1 | 4,6-Di-$CH_3$ | H |
| 3-$CH_3$ | H | Cl | $CH_3$ | 1 | 4,6-Di-$CH_3$ | H |
| 4-$CH_3$ | H | Cl | $CH_3$ | 1 | 4,6-Di-$CH_3$ | H |
| 3-$CH_3$ | F | Cl | $CH_3$ | 1 | 4,6-Di-$CH_3$ | H |
| 4-$CH_3$ | F | Cl | $CH_3$ | 1 | 4,6-Di-$CH_3$ | H |
| 3-$CH_3$ | H | F | H | 1 | 4-$CH_2CH_3$ | H |
| 4-$CH_3$ | H | F | H | 1 | 4-$CH_2CH_3$ | H |
| 3-$CH_3$ | F | F | H | 1 | 4-$CH_2CH_3$ | H |
| 4-$CH_3$ | F | F | H | 1 | 4-$CH_2CH_3$ | H |
| 3-$CH_3$ | H | Cl | H | 1 | 4-$CH_2CH_3$ | H |
| 4-$CH_3$ | H | Cl | H | 1 | 4-$CH_2CH_3$ | H |
| 3-$CH_3$ | F | Cl | H | 1 | 4-$CH_2CH_3$ | H |
| 4-$CH_3$ | F | Cl | H | 1 | 4-$CH_2CH_3$ | H |
| 3-$CH_3$ | H | F | $CH_3$ | 1 | 4-$CH_2CH_3$ | H |
| 4-$CH_3$ | H | F | $CH_3$ | 1 | 4-$CH_2CH_3$ | H |
| 3-$CH_3$ | F | F | $CH_3$ | 1 | 4-$CH_2CH_3$ | H |
| 4-$CH_3$ | F | F | $CH_3$ | 1 | 4-$CH_2CH_3$ | H |
| 3-$CH_3$ | H | Cl | $CH_3$ | 1 | 4-$CH_2CH_3$ | H |
| 4-$CH_3$ | H | Cl | $CH_3$ | 1 | 4-$CH_2CH_3$ | H |
| 3-$CH_3$ | F | Cl | $CH_3$ | 1 | 4-$CH_2CH_3$ | H |
| 4-$CH_3$ | F | Cl | $CH_3$ | 1 | 4-$CH_2CH_3$ | H |
| 3-$CH_3$ | H | F | H | 1 | 4-$CH_2Cl$ | H |

Tabelle C (Fortsetzung)

| $R_n$ | $R^1$ | $R^2$ | $R^3$ | m | $(R^a)_q$ | $R^4$ |
|---|---|---|---|---|---|---|
| 4-$CH_3$ | H | F | H | 1 | 4-$CH_2Cl$ | H |
| 3-$CH_3$ | F | F | H | 1 | 4-$CH_2Cl$ | H |
| 4-$CH_3$ | F | F | H | 1 | 4-$CH_2Cl$ | H |
| 3-$CH_3$ | H | Cl | H | 1 | 4-$CH_2Cl$ | H |
| 4-$CH_3$ | H | Cl | H | 1 | 4-$CH_2Cl$ | H |
| 3-$CH_3$ | F | Cl | H | 1 | 4-$CH_2Cl$ | H |
| 4-$CH_3$ | F | Cl | H | 1 | 4-$CH_2Cl$ | H |
| 3-$CH_3$ | H | F | $CH_3$ | 1 | 4-$CH_2Cl$ | H |
| 4-$CH_3$ | H | F | $CH_3$ | 1 | 4-$CH_2Cl$ | H |
| 3-$CH_3$ | F | F | $CH_3$ | 1 | 4-$CH_2Cl$ | H |
| 4-$CH_3$ | F | F | $CH_3$ | 1 | 4-$CH_2Cl$ | H |
| 3-$CH_3$ | H | Cl | $CH_3$ | 1 | 4-$CH_2Cl$ | H |
| 4-$CH_3$ | H | Cl | $CH_3$ | 1 | 4-$CH_2Cl$ | H |
| 3-$CH_3$ | F | Cl | $CH_3$ | 1 | 4-$CH_2Cl$ | H |
| 4-$CH_3$ | F | Cl | $CH_3$ | 1 | 4-$CH_2Cl$ | H |
| 3-$CH_3$ | H | F | H | 1 | 4-$CH_2OH$ | H |
| 4-$CH_3$ | H | F | H | 1 | 4-$CH_2OH$ | H |
| 3-$CH_3$ | F | F | H | 1 | 4-$CH_2OH$ | H |
| 4-$CH_3$ | F | F | H | 1 | 4-$CH_2OH$ | H |
| 3-$CH_3$ | H | Cl | H | 1 | 4-$CH_2OH$ | H |
| 4-$CH_3$ | H | Cl | H | 1 | 4-$CH_2OH$ | H |
| 3-$CH_3$ | F | Cl | H | 1 | 4-$CH_2OH$ | H |
| 4-$CH_3$ | F | Cl | H | 1 | 4-$CH_2OH$ | H |
| 3-$CH_3$ | H | F | $CH_3$ | 1 | 4-$CH_2OH$ | H |
| 4-$CH_3$ | H | F | $CH_3$ | 1 | 4-$CH_2OH$ | H |
| 3-$CH_3$ | F | F | $CH_3$ | 1 | 4-$CH_2OH$ | H |
| 4-$CH_3$ | F | F | $CH_3$ | 1 | 4-$CH_2OH$ | H |
| 3-$CH_3$ | H | Cl | $CH_3$ | 1 | 4-$CH_2OH$ | H |
| 4-$CH_3$ | H | Cl | $CH_3$ | 1 | 4-$CH_2OH$ | H |
| 3-$CH_3$ | F | Cl | $CH_3$ | 1 | 4-$CH_2OH$ | H |
| 4-$CH_3$ | F | Cl | $CH_3$ | 1 | 4-$CH_2OH$ | H |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4-$CH_2OCH_3$ | H |
| 4-$CH_3$ | H | F | H | 1 | 4-$CH_2OCH_3$ | H |
| 3-$CH_3$ | F | F | H | 1 | 4-$CH_2OCH_3$ | H |
| 4-$CH_3$ | F | F | H | 1 | 4-$CH_2OCH_3$ | H |
| 3-$CH_3$ | H | Cl | H | 1 | 4-$CH_2OCH_3$ | H |
| 4-$CH_3$ | H | Cl | H | 1 | 4-$CH_2OCH_3$ | H |
| 3-$CH_3$ | F | Cl | H | 1 | 4-$CH_2OCH_3$ | H |
| 4-$CH_3$ | F | Cl | H | 1 | 4-$CH_2OCH_3$ | H |
| 3-$CH_3$ | H | F | $CH_3$ | 1 | 4-$CH_2OCH_3$ | H |
| 4-$CH_3$ | H | F | $CH_3$ | 1 | 4-$CH_2OCH_3$ | H |
| 3-$CH_3$ | F | F | $CH_3$ | 1 | 4-$CH_2OCH_3$ | H |
| 4-$CH_3$ | F | F | $CH_3$ | 1 | 4-$CH_2OCH_3$ | H |
| 3-$CH_3$ | H | Cl | $CH_3$ | 1 | 4-$CH_2OCH_3$ | H |
| 4-$CH_3$ | H | Cl | $CH_3$ | 1 | 4-$CH_2OCH_3$ | H |
| 3-$CH_3$ | F | Cl | $CH_3$ | 1 | 4-$CH_2OCH_3$ | H |
| 4-$CH_3$ | F | Cl | $CH_3$ | 1 | 4-$CH_2OCH_3$ | H |
| 3-$CH_3$ | H | F | H | 1 | 4-$CH_2OCH_2CH=CH_2$ | H |
| 4-$CH_3$ | H | F | H | 1 | 4-$CH_2OCH_2CH=CH_2$ | H |
| 3-$CH_3$ | F | F | H | 1 | 4-$CH_2OCH_2CH=CH_2$ | H |
| 4-$CH_3$ | F | F | H | 1 | 4-$CH_2OCH_2CH=CH_2$ | H |
| 3-$CH_3$ | H | Cl | H | 1 | 4-$CH_2OCH_2CH=CH_2$ | H |
| 4-$CH_3$ | H | Cl | H | 1 | 4-$CH_2OCH_2CH=CH_2$ | H |
| 3-$CH_3$ | F | Cl | H | 1 | 4-$CH_2OCH_2CH=CH_2$ | H |
| 4-$CH_3$ | F | Cl | H | 1 | 4-$CH_2OCH_2CH=CH_2$ | H |
| 3-$CH_3$ | H | F | $CH_3$ | 1 | 4-$CH_2OCH_2CH=CH_2$ | H |
| 4-$CH_3$ | H | F | $CH_3$ | 1 | 4-$CH_2OCH_2CH=CH_2$ | H |
| 3-$CH_3$ | F | F | $CH_3$ | 1 | 4-$CH_2OCH_2CH=CH_2$ | H |
| 4-$CH_3$ | F | F | $CH_3$ | 1 | 4-$CH_2OCH_2CH=CH_2$ | H |
| 3-$CH_3$ | H | Cl | $CH_3$ | 1 | 4-$CH_2OCH_2CH=CH_2$ | H |
| 4-$CH_3$ | H | Cl | $CH_3$ | 1 | 4-$CH_2OCH_2CH=CH_2$ | H |
| 3-$CH_3$ | F | Cl | $CH_3$ | 1 | 4-$CH_2OCH_2CH=CH_2$ | H |
| 4-$CH_3$ | F | Cl | $CH_3$ | 1 | 4-$CH_2OCH_2CH=CH_2$ | H |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4-$CH_2OCH_2C{\equiv}CH$ | H |
| 4-$CH_3$ | H | F | H | 1 | 4-$CH_2OCH_2C{\equiv}CH$ | H |
| 3-$CH_3$ | F | F | H | 1 | 4-$CH_2OCH_2C{\equiv}CH$ | H |
| 4-$CH_3$ | F | F | H | 1 | 4-$CH_2OCH_2C{\equiv}CH$ | H |
| 3-$CH_3$ | H | Cl | H | 1 | 4-$CH_2OCH_2C{\equiv}CH$ | H |
| 4-$CH_3$ | H | Cl | H | 1 | 4-$CH_2OCH_2C{\equiv}CH$ | H |
| 3-$CH_3$ | F | Cl | H | 1 | 4-$CH_2OCH_2C{\equiv}CH$ | H |
| 4-$CH_3$ | F | Cl | H | 1 | 4-$CH_2OCH_2C{\equiv}CH$ | H |
| 3-$CH_3$ | H | F | $CH_3$ | 1 | 4-$CH_2OCH_2C{\equiv}CH$ | H |
| 4-$CH_3$ | H | F | $CH_3$ | 1 | 4-$CH_2OCH_2C{\equiv}CH$ | H |
| 3-$CH_3$ | F | F | $CH_3$ | 1 | 4-$CH_2OCH_2C{\equiv}CH$ | H |
| 4-$CH_3$ | F | F | $CH_3$ | 1 | 4-$CH_2OCH_2C{\equiv}CH$ | H |
| 3-$CH_3$ | H | Cl | $CH_3$ | 1 | 4-$CH_2OCH_2C{\equiv}CH$ | H |
| 4-$CH_3$ | H | Cl | $CH_3$ | 1 | 4-$CH_2OCH_2C{\equiv}CH$ | H |
| 3-$CH_3$ | F | Cl | $CH_3$ | 1 | 4-$CH_2OCH_2C{\equiv}CH$ | H |
| 4-$CH_3$ | F | Cl | $CH_3$ | 1 | 4-$CH_2OCH_2C{\equiv}CH$ | H |
| 3-$CH_3$ | H | F | H | 1 | 4-$CH_2OCOCH_3$ | H |
| 4-$CH_3$ | H | F | H | 1 | 4-$CH_2OCOCH_3$ | H |
| 3-$CH_3$ | F | F | H | 1 | 4-$CH_2OCOCH_3$ | H |
| 4-$CH_3$ | F | F | H | 1 | 4-$CH_2OCOCH_3$ | H |
| 3-$CH_3$ | H | Cl | H | 1 | 4-$CH_2OCOCH_3$ | H |
| 4-$CH_3$ | H | Cl | H | 1 | 4-$CH_2OCOCH_3$ | H |
| 3-$CH_3$ | F | Cl | H | 1 | 4-$CH_2OCOCH_3$ | H |
| 4-$CH_3$ | F | Cl | H | 1 | 4-$CH_2OCOCH_3$ | H |
| 3-$CH_3$ | H | F | $CH_3$ | 1 | 4-$CH_2OCOCH_3$ | H |
| 4-$CH_3$ | H | F | $CH_3$ | 1 | 4-$CH_2OCOCH_3$ | H |
| 3-$CH_3$ | F | F | $CH_3$ | 1 | 4-$CH_2OCOCH_3$ | H |
| 4-$CH_3$ | F | F | $CH_3$ | 1 | 4-$CH_2OCOCH_3$ | H |
| 3-$CH_3$ | H | Cl | $CH_3$ | 1 | 4-$CH_2OCOCH_3$ | H |
| 4-$CH_3$ | H | Cl | $CH_3$ | 1 | 4-$CH_2OCOCH_3$ | H |
| 3-$CH_3$ | F | Cl | $CH_3$ | 1 | 4-$CH_2OCOCH_3$ | H |
| 4-$CH_3$ | F | Cl | $CH_3$ | 1 | 4-$CH_2OCOCH_3$ | H |

Tabelle C (Fortsetzung)

| $R_n$ | $R^1$ | $R^2$ | $R^3$ | m | $(R^a)_q$ | $R^4$ |
|---|---|---|---|---|---|---|
| $3-CH_3$ | H | F | H | 1 | $4-CH_2OCH_2CO_2CH_3$ | H |
| $4-CH_3$ | H | F | H | 1 | $4-CH_2OCH_2CO_2CH_3$ | H |
| $3-CH_3$ | F | F | H | 1 | $4-CH_2OCH_2CO_2CH_3$ | H |
| $4-CH_3$ | F | F | H | 1 | $4-CH_2OCH_2CO_2CH_3$ | H |
| $3-CH_3$ | H | Cl | H | 1 | $4-CH_2OCH_2CO_2CH_3$ | H |
| $4-CH_3$ | H | Cl | H | 1 | $4-CH_2OCH_2CO_2CH_3$ | H |
| $3-CH_3$ | F | Cl | H | 1 | $4-CH_2OCH_2CO_2CH_3$ | H |
| $4-CH_3$ | F | Cl | H | 1 | $4-CH_2OCH_2CO_2CH_3$ | H |
| $3-CH_3$ | H | F | $CH_3$ | 1 | $4-CH_2OCH_2CO_2CH_3$ | H |
| $4-CH_3$ | H | F | $CH_3$ | 1 | $4-CH_2OCH_2CO_2CH_3$ | H |
| $3-CH_3$ | F | F | $CH_3$ | 1 | $4-CH_2OCH_2CO_2CH_3$ | H |
| $4-CH_3$ | F | F | $CH_3$ | 1 | $4-CH_2OCH_2CO_2CH_3$ | H |
| $3-CH_3$ | H | Cl | $CH_3$ | 1 | $4-CH_2OCH_2CO_2CH_3$ | H |
| $4-CH_3$ | H | Cl | $CH_3$ | 1 | $4-CH_2OCH_2CO_2CH_3$ | H |
| $3-CH_3$ | F | Cl | $CH_3$ | 1 | $4-CH_2OCH_2CO_2CH_3$ | H |
| $4-CH_3$ | F | Cl | $CH_3$ | 1 | $4-CH_2OCH_2CO_2CH_3$ | H |
| $3-CH_3$ | H | F | H | 1 | $4-CH_2CO_2CH_3$ | H |
| $4-CH_3$ | H | F | H | 1 | $4-CH_2CO_2CH_3$ | H |
| $3-CH_3$ | F | F | H | 1 | $4-CH_2CO_2CH_3$ | H |
| $4-CH_3$ | F | F | H | 1 | $4-CH_2CO_2CH_3$ | H |
| $3-CH_3$ | H | Cl | H | 1 | $4-CH_2CO_2CH_3$ | H |
| $4-CH_3$ | H | Cl | H | 1 | $4-CH_2CO_2CH_3$ | H |
| $3-CH_3$ | F | Cl | H | 1 | $4-CH_2CO_2CH_3$ | H |
| $4-CH_3$ | F | Cl | H | 1 | $4-CH_2CO_2CH_3$ | H |
| $3-CH_3$ | H | F | $CH_3$ | 1 | $4-CH_2CO_2CH_3$ | H |
| $4-CH_3$ | H | F | $CH_3$ | 1 | $4-CH_2CO_2CH_3$ | H |
| $3-CH_3$ | F | F | $CH_3$ | 1 | $4-CH_2CO_2CH_3$ | H |
| $4-CH_3$ | F | F | $CH_3$ | 1 | $4-CH_2CO_2CH_3$ | H |
| $3-CH_3$ | H | Cl | $CH_3$ | 1 | $4-CH_2CO_2CH_3$ | H |
| $4-CH_3$ | H | Cl | $CH_3$ | 1 | $4-CH_2CO_2CH_3$ | H |
| $3-CH_3$ | F | Cl | $CH_3$ | 1 | $4-CH_2CO_2CH_3$ | H |
| $4-CH_3$ | F | Cl | $CH_3$ | 1 | $4-CH_2CO_2CH_3$ | H |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4-$CH_2SH$ | H |
| 4-$CH_3$ | H | F | H | 1 | 4-$CH_2SH$ | H |
| 3-$CH_3$ | F | F | H | 1 | 4-$CH_2SH$ | H |
| 4-$CH_3$ | F | F | H | 1 | 4-$CH_2SH$ | H |
| 3-$CH_3$ | H | Cl | H | 1 | 4-$CH_2SH$ | H |
| 4-$CH_3$ | H | Cl | H | 1 | 4-$CH_2SH$ | H |
| 3-$CH_3$ | F | Cl | H | 1 | 4-$CH_2SH$ | H |
| 4-$CH_3$ | F | Cl | H | 1 | 4-$CH_2SH$ | H |
| 3-$CH_3$ | H | F | $CH_3$ | 1 | 4-$CH_2SH$ | H |
| 4-$CH_3$ | H | F | $CH_3$ | 1 | 4-$CH_2SH$ | H |
| 3-$CH_3$ | F | F | $CH_3$ | 1 | 4-$CH_2SH$ | H |
| 4-$CH_3$ | F | F | $CH_3$ | 1 | 4-$CH_2SH$ | H |
| 3-$CH_3$ | H | Cl | $CH_3$ | 1 | 4-$CH_2SH$ | H |
| 4-$CH_3$ | H | Cl | $CH_3$ | 1 | 4-$CH_2SH$ | H |
| 3-$CH_3$ | F | Cl | $CH_3$ | 1 | 4-$CH_2SH$ | H |
| 4-$CH_3$ | F | Cl | $CH_3$ | 1 | 4-$CH_2SH$ | H |
| 3-$CH_3$ | H | F | H | 1 | 4-$CH_2SCH_2CH_3$ | H |
| 4-$CH_3$ | H | F | H | 1 | 4-$CH_2SCH_2CH_3$ | H |
| 3-$CH_3$ | F | F | H | 1 | 4-$CH_2SCH_2CH_3$ | H |
| 4-$CH_3$ | F | F | H | 1 | 4-$CH_2SCH_2CH_3$ | H |
| 3-$CH_3$ | H | Cl | H | 1 | 4-$CH_2SCH_2CH_3$ | H |
| 4-$CH_3$ | H | Cl | H | 1 | 4-$CH_2SCH_2CH_3$ | H |
| 3-$CH_3$ | F | Cl | H | 1 | 4-$CH_2SCH_2CH_3$ | H |
| 4-$CH_3$ | F | Cl | H | 1 | 4-$CH_2SCH_2CH_3$ | H |
| 3-$CH_3$ | H | F | $CH_3$ | 1 | 4-$CH_2SCH_2CH_3$ | H |
| 4-$CH_3$ | H | F | $CH_3$ | 1 | 4-$CH_2SCH_2CH_3$ | H |
| 3-$CH_3$ | F | F | $CH_3$ | 1 | 4-$CH_2SCH_2CH_3$ | H |
| 4-$CH_3$ | F | F | $CH_3$ | 1 | 4-$CH_2SCH_2CH_3$ | H |
| 3-$CH_3$ | H | Cl | $CH_3$ | 1 | 4-$CH_2SCH_2CH_3$ | H |
| 4-$CH_3$ | H | Cl | $CH_3$ | 1 | 4-$CH_2SCH_2CH_3$ | H |
| 3-$CH_3$ | F | Cl | $CH_3$ | 1 | 4-$CH_2SCH_2CH_3$ | H |
| 4-$CH_3$ | F | Cl | $CH_3$ | 1 | 4-$CH_2SCH_2CH_3$ | H |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4-$CH_2SCH_2CH=CH_2$ | H |
| 4-$CH_3$ | H | F | H | 1 | 4-$CH_2SCH_2CH=CH_2$ | H |
| 3-$CH_3$ | F | F | H | 1 | 4-$CH_2SCH_2CH=CH_2$ | H |
| 4-$CH_3$ | F | F | H | 1 | 4-$CH_2SCH_2CH=CH_2$ | H |
| 3-$CH_3$ | H | Cl | H | 1 | 4-$CH_2SCH_2CH=CH_2$ | H |
| 4-$CH_3$ | H | Cl | H | 1 | 4-$CH_2SCH_2CH=CH_2$ | H |
| 3-$CH_3$ | F | Cl | H | 1 | 4-$CH_2SCH_2CH=CH_2$ | H |
| 4-$CH_3$ | F | Cl | H | 1 | 4-$CH_2SCH_2CH=CH_2$ | H |
| 3-$CH_3$ | H | F | $CH_3$ | 1 | 4-$CH_2SCH_2CH=CH_2$ | H |
| 4-$CH_3$ | H | F | $CH_3$ | 1 | 4-$CH_2SCH_2CH=CH_2$ | H |
| 3-$CH_3$ | F | F | $CH_3$ | 1 | 4-$CH_2SCH_2CH=CH_2$ | H |
| 4-$CH_3$ | F | F | $CH_3$ | 1 | 4-$CH_2SCH_2CH=CH_2$ | H |
| 3-$CH_3$ | H | Cl | $CH_3$ | 1 | 4-$CH_2SCH_2CH=CH_2$ | H |
| 4-$CH_3$ | H | Cl | $CH_3$ | 1 | 4-$CH_2SCH_2CH=CH_2$ | H |
| 3-$CH_3$ | F | Cl | $CH_3$ | 1 | 4-$CH_2SCH_2CH=CH_2$ | H |
| 4-$CH_3$ | F | Cl | $CH_3$ | 1 | 4-$CH_2SCH_2CH=CH_2$ | H |
| 3-$CH_3$ | H | F | H | 1 | 4-$CO_2H$ | H |
| 4-$CH_3$ | H | F | H | 1 | 4-$CO_2H$ | H |
| 3-$CH_3$ | F | F | H | 1 | 4-$CO_2H$ | H |
| 4-$CH_3$ | F | F | H | 1 | 4-$CO_2H$ | H |
| 3-$CH_3$ | H | Cl | H | 1 | 4-$CO_2H$ | H |
| 4-$CH_3$ | H | Cl | H | 1 | 4-$CO_2H$ | H |
| 3-$CH_3$ | F | Cl | H | 1 | 4-$CO_2H$ | H |
| 4-$CH_3$ | F | Cl | H | 1 | 4-$CO_2H$ | H |
| 3-$CH_3$ | H | F | $CH_3$ | 1 | 4-$CO_2H$ | H |
| 4-$CH_3$ | H | F | $CH_3$ | 1 | 4-$CO_2H$ | H |
| 3-$CH_3$ | F | F | $CH_3$ | 1 | 4-$CO_2H$ | H |
| 4-$CH_3$ | F | F | $CH_3$ | 1 | 4-$CO_2H$ | H |
| 3-$CH_3$ | H | Cl | $CH_3$ | 1 | 4-$CO_2H$ | H |
| 4-$CH_3$ | H | Cl | $CH_3$ | 1 | 4-$CO_2H$ | H |
| 3-$CH_3$ | F | Cl | $CH_3$ | 1 | 4-$CO_2H$ | H |
| 4-$CH_3$ | F | Cl | $CH_3$ | 1 | 4-$CO_2H$ | H |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | H | $CO_2CH_3$ |
| 4-$CH_3$ | H | F | H | 0 | H | $CO_2CH_3$ |
| 3-$CH_3$ | F | F | H | 0 | H | $CO_2CH_3$ |
| 4-$CH_3$ | F | F | H | 0 | H | $CO_2CH_3$ |
| 3-$CH_3$ | H | Cl | H | 0 | H | $CO_2CH_3$ |
| 4-$CH_3$ | H | Cl | H | 0 | H | $CO_2CH_3$ |
| 3-$CH_3$ | F | Cl | H | 0 | H | $CO_2CH_3$ |
| 4-$CH_3$ | F | Cl | H | 0 | H | $CO_2CH_3$ |
| 3-$CH_3$ | H | F | $CH_3$ | 0 | H | $CO_2CH_3$ |
| 4-$CH_3$ | H | F | $CH_3$ | 0 | H | $CO_2CH_3$ |
| 3-$CH_3$ | F | F | $CH_3$ | 0 | H | $CO_2CH_3$ |
| 4-$CH_3$ | F | F | $CH_3$ | 0 | H | $CO_2CH_3$ |
| 3-$CH_3$ | H | Cl | $CH_3$ | 0 | H | $CO_2CH_3$ |
| 4-$CH_3$ | H | Cl | $CH_3$ | 0 | H | $CO_2CH_3$ |
| 3-$CH_3$ | F | Cl | $CH_3$ | 0 | H | $CO_2CH_3$ |
| 4-$CH_3$ | F | Cl | $CH_3$ | 0 | H | $CO_2CH_3$ |
| 3-$CH_3$ | H | F | H | 0 | H | $CO_2CH_2CH_3$ |
| 4-$CH_3$ | H | F | H | 0 | H | $CO_2CH_2CH_3$ |
| 3-$CH_3$ | F | F | H | 0 | H | $CO_2CH_2CH_3$ |
| 4-$CH_3$ | F | F | H | 0 | H | $CO_2CH_2CH_3$ |
| 3-$CH_3$ | H | Cl | H | 0 | H | $CO_2CH_2CH_3$ |
| 4-$CH_3$ | H | Cl | H | 0 | H | $CO_2CH_2CH_3$ |
| 3-$CH_3$ | F | Cl | H | 0 | H | $CO_2CH_2CH_3$ |
| 4-$CH_3$ | F | Cl | H | 0 | H | $CO_2CH_2CH_3$ |
| 3-$CH_3$ | H | F | $CH_3$ | 0 | H | $CO_2CH_2CH_3$ |
| 4-$CH_3$ | H | F | $CH_3$ | 0 | H | $CO_2CH_2CH_3$ |
| 3-$CH_3$ | F | F | $CH_3$ | 0 | H | $CO_2CH_2CH_3$ |
| 4-$CH_3$ | F | F | $CH_3$ | 0 | H | $CO_2CH_2CH_3$ |
| 3-$CH_3$ | H | Cl | $CH_3$ | 0 | H | $CO_2CH_2CH_3$ |
| 4-$CH_3$ | H | Cl | $CH_3$ | 0 | H | $CO_2CH_2CH_3$ |
| 3-$CH_3$ | F | Cl | $CH_3$ | 0 | H | $CO_2CH_2CH_3$ |
| 4-$CH_3$ | F | Cl | $CH_3$ | 0 | H | $CO_2CH_2CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | H | $CO_2(CH_2)_2CH_3$ |
| 4-$CH_3$ | H | F | H | 0 | H | $CO_2(CH_2)_2CH_3$ |
| 3-$CH_3$ | F | F | H | 0 | H | $CO_2(CH_2)_2CH_3$ |
| 4-$CH_3$ | F | F | H | 0 | H | $CO_2(CH_2)_2CH_3$ |
| 3-$CH_3$ | H | Cl | H | 0 | H | $CO_2(CH_2)_2CH_3$ |
| 4-$CH_3$ | H | Cl | H | 0 | H | $CO_2(CH_2)_2CH_3$ |
| 3-$CH_3$ | F | Cl | H | 0 | H | $CO_2(CH_2)_2CH_3$ |
| 4-$CH_3$ | F | Cl | H | 0 | H | $CO_2(CH_2)_2CH_3$ |
| 3-$CH_3$ | H | F | $CH_3$ | 0 | H | $CO_2(CH_2)_2CH_3$ |
| 4-$CH_3$ | H | F | $CH_3$ | 0 | H | $CO_2(CH_2)_2CH_3$ |
| 3-$CH_3$ | F | F | $CH_3$ | 0 | H | $CO_2(CH_2)_2CH_3$ |
| 4-$CH_3$ | F | F | $CH_3$ | 0 | H | $CO_2(CH_2)_2CH_3$ |
| 3-$CH_3$ | H | Cl | $CH_3$ | 0 | H | $CO_2(CH_2)_2CH_3$ |
| 4-$CH_3$ | H | Cl | $CH_3$ | 0 | H | $CO_2(CH_2)_2CH_3$ |
| 3-$CH_3$ | F | Cl | $CH_3$ | 0 | H | $CO_2(CH_2)_2CH_3$ |
| 4-$CH_3$ | F | Cl | $CH_3$ | 0 | H | $CO_2(CH_2)_2CH_3$ |
| 3-$CH_3$ | H | F | H | 0 | H | $CO_2CH(CH_3)_2$ |
| 4-$CH_3$ | H | F | H | 0 | H | $CO_2CH(CH_3)_2$ |
| 3-$CH_3$ | F | F | H | 0 | H | $CO_2CH(CH_3)_2$ |
| 4-$CH_3$ | F | F | H | 0 | H | $CO_2CH(CH_3)_2$ |
| 3-$CH_3$ | H | Cl | H | 0 | H | $CO_2CH(CH_3)_2$ |
| 4-$CH_3$ | H | Cl | H | 0 | H | $CO_2CH(CH_3)_2$ |
| 3-$CH_3$ | F | Cl | H | 0 | H | $CO_2CH(CH_3)_2$ |
| 4-$CH_3$ | F | Cl | H | 0 | H | $CO_2CH(CH_3)_2$ |
| 3-$CH_3$ | H | F | $CH_3$ | 0 | H | $CO_2CH(CH_3)_2$ |
| 4-$CH_3$ | H | F | $CH_3$ | 0 | H | $CO_2CH(CH_3)_2$ |
| 3-$CH_3$ | F | F | $CH_3$ | 0 | H | $CO_2CH(CH_3)_2$ |
| 4-$CH_3$ | F | F | $CH_3$ | 0 | H | $CO_2CH(CH_3)_2$ |
| 3-$CH_3$ | H | Cl | $CH_3$ | 0 | H | $CO_2CH(CH_3)_2$ |
| 4-$CH_3$ | H | Cl | $CH_3$ | 0 | H | $CO_2CH(CH_3)_2$ |
| 3-$CH_3$ | F | Cl | $CH_3$ | 0 | H | $CO_2CH(CH_3)_2$ |
| 4-$CH_3$ | F | Cl | $CH_3$ | 0 | H | $CO_2CH(CH_3)_2$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | H | $CO_2(CH_2)_3CH_3$ |
| 4-$CH_3$ | H | F | H | 0 | H | $CO_2(CH_2)_3CH_3$ |
| 3-$CH_3$ | F | F | H | 0 | H | $CO_2(CH_2)_3CH_3$ |
| 4-$CH_3$ | F | F | H | 0 | H | $CO_2(CH_2)_3CH_3$ |
| 3-$CH_3$ | H | Cl | H | 0 | H | $CO_2(CH_2)_3CH_3$ |
| 4-$CH_3$ | H | Cl | H | 0 | H | $CO_2(CH_2)_3CH_3$ |
| 3-$CH_3$ | F | Cl | H | 0 | H | $CO_2(CH_2)_3CH_3$ |
| 4-$CH_3$ | F | Cl | H | 0 | H | $CO_2(CH_2)_3CH_3$ |
| 3-$CH_3$ | H | F | $CH_3$ | 0 | H | $CO_2(CH_2)_3CH_3$ |
| 4-$CH_3$ | H | F | $CH_3$ | 0 | H | $CO_2(CH_2)_3CH_3$ |
| 3-$CH_3$ | F | F | $CH_3$ | 0 | H | $CO_2(CH_2)_3CH_3$ |
| 4-$CH_3$ | F | F | $CH_3$ | 0 | H | $CO_2(CH_2)_3CH_3$ |
| 3-$CH_3$ | H | Cl | $CH_3$ | 0 | H | $CO_2(CH_2)_3CH_3$ |
| 4-$CH_3$ | H | Cl | $CH_3$ | 0 | H | $CO_2(CH_2)_3CH_3$ |
| 3-$CH_3$ | F | Cl | $CH_3$ | 0 | H | $CO_2(CH_2)_3CH_3$ |
| 4-$CH_3$ | F | Cl | $CH_3$ | 0 | H | $CO_2(CH_2)_3CH_3$ |
| 3-$CH_3$ | H | F | H | 0 | H | $CO_2(CH_2)_2OCH_3$ |
| 4-$CH_3$ | H | F | H | 0 | H | $CO_2(CH_2)_2OCH_3$ |
| 3-$CH_3$ | F | F | H | 0 | H | $CO_2(CH_2)_2OCH_3$ |
| 4-$CH_3$ | F | F | H | 0 | H | $CO_2(CH_2)_2OCH_3$ |
| 3-$CH_3$ | H | Cl | H | 0 | H | $CO_2(CH_2)_2OCH_3$ |
| 4-$CH_3$ | H | Cl | H | 0 | H | $CO_2(CH_2)_2OCH_3$ |
| 3-$CH_3$ | F | Cl | H | 0 | H | $CO_2(CH_2)_2OCH_3$ |
| 4-$CH_3$ | F | Cl | H | 0 | H | $CO_2(CH_2)_2OCH_3$ |
| 3-$CH_3$ | H | F | $CH_3$ | 0 | H | $CO_2(CH_2)_2OCH_3$ |
| 4-$CH_3$ | H | F | $CH_3$ | 0 | H | $CO_2(CH_2)_2OCH_3$ |
| 3-$CH_3$ | F | F | $CH_3$ | 0 | H | $CO_2(CH_2)_2OCH_3$ |
| 4-$CH_3$ | F | F | $CH_3$ | 0 | H | $CO_2(CH_2)_2OCH_3$ |
| 3-$CH_3$ | H | Cl | $CH_3$ | 0 | H | $CO_2(CH_2)_2OCH_3$ |
| 4-$CH_3$ | H | Cl | $CH_3$ | 0 | H | $CO_2(CH_2)_2OCH_3$ |
| 3-$CH_3$ | F | Cl | $CH_3$ | 0 | H | $CO_2(CH_2)_2OCH_3$ |
| 4-$CH_3$ | F | Cl | $CH_3$ | 0 | H | $CO_2(CH_2)_2OCH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| $3-CH_3$ | H | F | H | 0 | H | $CO_2(CH_2)_2OCH_2CH_3$ |
| $4-CH_3$ | H | F | H | 0 | H | $CO_2(CH_2)_2OCH_2CH_3$ |
| $3-CH_3$ | F | F | H | 0 | H | $CO_2(CH_2)_2OCH_2CH_3$ |
| $4-CH_3$ | F | F | H | 0 | H | $CO_2(CH_2)_2OCH_2CH_3$ |
| $3-CH_3$ | H | Cl | H | 0 | H | $CO_2(CH_2)_2OCH_2CH_3$ |
| $4-CH_3$ | H | Cl | H | 0 | H | $CO_2(CH_2)_2OCH_2CH_3$ |
| $3-CH_3$ | F | Cl | H | 0 | H | $CO_2(CH_2)_2OCH_2CH_3$ |
| $4-CH_3$ | F | Cl | H | 0 | H | $CO_2(CH_2)_2OCH_2CH_3$ |
| $3-CH_3$ | H | F | $CH_3$ | 0 | H | $CO_2(CH_2)_2OCH_2CH_3$ |
| $4-CH_3$ | H | F | $CH_3$ | 0 | H | $CO_2(CH_2)_2OCH_2CH_3$ |
| $3-CH_3$ | F | F | $CH_3$ | 0 | H | $CO_2(CH_2)_2OCH_2CH_3$ |
| $4-CH_3$ | F | F | $CH_3$ | 0 | H | $CO_2(CH_2)_2OCH_2CH_3$ |
| $3-CH_3$ | H | Cl | $CH_3$ | 0 | H | $CO_2(CH_2)_2OCH_2CH_3$ |
| $4-CH_3$ | H | Cl | $CH_3$ | 0 | H | $CO_2(CH_2)_2OCH_2CH_3$ |
| $3-CH_3$ | F | Cl | $CH_3$ | 0 | H | $CO_2(CH_2)_2OCH_2CH_3$ |
| $4-CH_3$ | F | Cl | $CH_3$ | 0 | H | $CO_2(CH_2)_2OCH_2CH_3$ |
| $3-CH_3$ | H | F | H | 0 | $4-CH_3$ | $CO_2CH_3$ |
| $4-CH_3$ | H | F | H | 0 | $4-CH_3$ | $CO_2CH_3$ |
| $3-CH_3$ | F | F | H | 0 | $4-CH_3$ | $CO_2CH_3$ |
| $4-CH_3$ | F | F | H | 0 | $4-CH_3$ | $CO_2CH_3$ |
| $3-CH_3$ | H | Cl | H | 0 | $4-CH_3$ | $CO_2CH_3$ |
| $4-CH_3$ | H | Cl | H | 0 | $4-CH_3$ | $CO_2CH_3$ |
| $3-CH_3$ | F | Cl | H | 0 | $4-CH_3$ | $CO_2CH_3$ |
| $4-CH_3$ | F | Cl | H | 0 | $4-CH_3$ | $CO_2CH_3$ |
| $3-CH_3$ | H | F | $CH_3$ | 0 | $4-CH_3$ | $CO_2CH_3$ |
| $4-CH_3$ | H | F | $CH_3$ | 0 | $4-CH_3$ | $CO_2CH_3$ |
| $3-CH_3$ | F | F | $CH_3$ | 0 | $4-CH_3$ | $CO_2CH_3$ |
| $4-CH_3$ | F | F | $CH_3$ | 0 | $4-CH_3$ | $CO_2CH_3$ |
| $3-CH_3$ | H | Cl | $CH_3$ | 0 | $4-CH_3$ | $CO_2CH_3$ |
| $4-CH_3$ | H | Cl | $CH_3$ | 0 | $4-CH_3$ | $CO_2CH_3$ |
| $3-CH_3$ | F | Cl | $CH_3$ | 0 | $4-CH_3$ | $CO_2CH_3$ |
| $4-CH_3$ | F | Cl | $CH_3$ | 0 | $4-CH_3$ | $CO_2CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 4-$CH_3$ | $CO_2CH_2CH_3$ |
| 4-$CH_3$ | H | F | H | 0 | 4-$CH_3$ | $CO_2CH_2CH_3$ |
| 3-$CH_3$ | F | F | H | 0 | 4-$CH_3$ | $CO_2CH_2CH_3$ |
| 4-$CH_3$ | F | F | H | 0 | 4-$CH_3$ | $CO_2CH_2CH_3$ |
| 3-$CH_3$ | H | Cl | H | 0 | 4-$CH_3$ | $CO_2CH_2CH_3$ |
| 4-$CH_3$ | H | Cl | H | 0 | 4-$CH_3$ | $CO_2CH_2CH_3$ |
| 3-$CH_3$ | F | Cl | H | 0 | 4-$CH_3$ | $CO_2CH_2CH_3$ |
| 4-$CH_3$ | F | Cl | H | 0 | 4-$CH_3$ | $CO_2CH_2CH_3$ |
| 3-$CH_3$ | H | F | $CH_3$ | 0 | 4-$CH_3$ | $CO_2CH_2CH_3$ |
| 4-$CH_3$ | H | F | $CH_3$ | 0 | 4-$CH_3$ | $CO_2CH_2CH_3$ |
| 3-$CH_3$ | F | F | $CH_3$ | 0 | 4-$CH_3$ | $CO_2CH_2CH_3$ |
| 4-$CH_3$ | F | F | $CH_3$ | 0 | 4-$CH_3$ | $CO_2CH_2CH_3$ |
| 3-$CH_3$ | H | Cl | $CH_3$ | 0 | 4-$CH_3$ | $CO_2CH_2CH_3$ |
| 4-$CH_3$ | H | Cl | $CH_3$ | 0 | 4-$CH_3$ | $CO_2CH_2CH_3$ |
| 3-$CH_3$ | F | Cl | $CH_3$ | 0 | 4-$CH_3$ | $CO_2CH_2CH_3$ |
| 4-$CH_3$ | F | Cl | $CH_3$ | 0 | 4-$CH_3$ | $CO_2CH_2CH_3$ |
| 3-$CH_3$ | H | F | H | 0 | 4-$CH_3$ | $CO_2(CH_2)_2CH_3$ |
| 4-$CH_3$ | H | F | H | 0 | 4-$CH_3$ | $CO_2(CH_2)_2CH_3$ |
| 3-$CH_3$ | F | F | H | 0 | 4-$CH_3$ | $CO_2(CH_2)_2CH_3$ |
| 4-$CH_3$ | F | F | H | 0 | 4-$CH_3$ | $CO_2(CH_2)_2CH_3$ |
| 3-$CH_3$ | H | Cl | H | 0 | 4-$CH_3$ | $CO_2(CH_2)_2CH_3$ |
| 4-$CH_3$ | H | Cl | H | 0 | 4-$CH_3$ | $CO_2(CH_2)_2CH_3$ |
| 3-$CH_3$ | F | Cl | H | 0 | 4-$CH_3$ | $CO_2(CH_2)_2CH_3$ |
| 4-$CH_3$ | F | Cl | H | 0 | 4-$CH_3$ | $CO_2(CH_2)_2CH_3$ |
| 3-$CH_3$ | H | F | $CH_3$ | 0 | 4-$CH_3$ | $CO_2(CH_2)_2CH_3$ |
| 4-$CH_3$ | H | F | $CH_3$ | 0 | 4-$CH_3$ | $CO_2(CH_2)_2CH_3$ |
| 3-$CH_3$ | F | F | $CH_3$ | 0 | 4-$CH_3$ | $CO_2(CH_2)_2CH_3$ |
| 4-$CH_3$ | F | F | $CH_3$ | 0 | 4-$CH_3$ | $CO_2(CH_2)_2CH_3$ |
| 3-$CH_3$ | H | Cl | $CH_3$ | 0 | 4-$CH_3$ | $CO_2(CH_2)_2CH_3$ |
| 4-$CH_3$ | H | Cl | $CH_3$ | 0 | 4-$CH_3$ | $CO_2(CH_2)_2CH_3$ |
| 3-$CH_3$ | F | Cl | $CH_3$ | 0 | 4-$CH_3$ | $CO_2(CH_2)_2CH_3$ |
| 4-$CH_3$ | F | Cl | $CH_3$ | 0 | 4-$CH_3$ | $CO_2(CH_2)_2CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 4-$CH_3$ | $CO_2CH(CH_3)_2$ |
| 4-$CH_3$ | H | F | H | 0 | 4-$CH_3$ | $CO_2CH(CH_3)_2$ |
| 3-$CH_3$ | F | F | H | 0 | 4-$CH_3$ | $CO_2CH(CH_3)_2$ |
| 4-$CH_3$ | F | F | H | 0 | 4-$CH_3$ | $CO_2CH(CH_3)_2$ |
| 3-$CH_3$ | H | Cl | H | 0 | 4-$CH_3$ | $CO_2CH(CH_3)_2$ |
| 4-$CH_3$ | H | Cl | H | 0 | 4-$CH_3$ | $CO_2CH(CH_3)_2$ |
| 3-$CH_3$ | F | Cl | H | 0 | 4-$CH_3$ | $CO_2CH(CH_3)_2$ |
| 4-$CH_3$ | F | Cl | H | 0 | 4-$CH_3$ | $CO_2CH(CH_3)_2$ |
| 3-$CH_3$ | H | F | $CH_3$ | 0 | 4-$CH_3$ | $CO_2CH(CH_3)_2$ |
| 4-$CH_3$ | H | F | $CH_3$ | 0 | 4-$CH_3$ | $CO_2CH(CH_3)_2$ |
| 3-$CH_3$ | F | F | $CH_3$ | 0 | 4-$CH_3$ | $CO_2CH(CH_3)_2$ |
| 4-$CH_3$ | F | F | $CH_3$ | 0 | 4-$CH_3$ | $CO_2CH(CH_3)_2$ |
| 3-$CH_3$ | H | Cl | $CH_3$ | 0 | 4-$CH_3$ | $CO_2CH(CH_3)_2$ |
| 4-$CH_3$ | H | Cl | $CH_3$ | 0 | 4-$CH_3$ | $CO_2CH(CH_3)_2$ |
| 3-$CH_3$ | F | Cl | $CH_3$ | 0 | 4-$CH_3$ | $CO_2CH(CH_3)_2$ |
| 4-$CH_3$ | F | Cl | $CH_3$ | 0 | 4-$CH_3$ | $CO_2CH(CH_3)_2$ |
| 3-$CH_3$ | H | F | H | 0 | 4-$CH_3$ | $CO_2(CH_2)_3CH_3$ |
| 4-$CH_3$ | H | F | H | 0 | 4-$CH_3$ | $CO_2(CH_2)_3CH_3$ |
| 3-$CH_3$ | F | F | H | 0 | 4-$CH_3$ | $CO_2(CH_2)_3CH_3$ |
| 4-$CH_3$ | F | F | H | 0 | 4-$CH_3$ | $CO_2(CH_2)_3CH_3$ |
| 3-$CH_3$ | H | Cl | H | 0 | 4-$CH_3$ | $CO_2(CH_2)_3CH_3$ |
| 4-$CH_3$ | H | Cl | H | 0 | 4-$CH_3$ | $CO_2(CH_2)_3CH_3$ |
| 3-$CH_3$ | F | Cl | H | 0 | 4-$CH_3$ | $CO_2(CH_2)_3CH_3$ |
| 4-$CH_3$ | F | Cl | H | 0 | 4-$CH_3$ | $CO_2(CH_2)_3CH_3$ |
| 3-$CH_3$ | H | F | $CH_3$ | 0 | 4-$CH_3$ | $CO_2(CH_2)_3CH_3$ |
| 4-$CH_3$ | H | F | $CH_3$ | 0 | 4-$CH_3$ | $CO_2(CH_2)_3CH_3$ |
| 3-$CH_3$ | F | F | $CH_3$ | 0 | 4-$CH_3$ | $CO_2(CH_2)_3CH_3$ |
| 4-$CH_3$ | F | F | $CH_3$ | 0 | 4-$CH_3$ | $CO_2(CH_2)_3CH_3$ |
| 3-$CH_3$ | H | Cl | $CH_3$ | 0 | 4-$CH_3$ | $CO_2(CH_2)_3CH_3$ |
| 4-$CH_3$ | H | Cl | $CH_3$ | 0 | 4-$CH_3$ | $CO_2(CH_2)_3CH_3$ |
| 3-$CH_3$ | F | Cl | $CH_3$ | 0 | 4-$CH_3$ | $CO_2(CH_2)_3CH_3$ |
| 4-$CH_3$ | F | Cl | $CH_3$ | 0 | 4-$CH_3$ | $CO_2(CH_2)_3CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 4-$CH_3$ | $CO_2(CH_2)_2OCH_3$ |
| 4-$CH_3$ | H | F | H | 0 | 4-$CH_3$ | $CO_2(CH_2)_2OCH_3$ |
| 3-$CH_3$ | F | F | H | 0 | 4-$CH_3$ | $CO_2(CH_2)_2OCH_3$ |
| 4-$CH_3$ | F | F | H | 0 | 4-$CH_3$ | $CO_2(CH_2)_2OCH_3$ |
| 3-$CH_3$ | H | Cl | H | 0 | 4-$CH_3$ | $CO_2(CH_2)_2OCH_3$ |
| 4-$CH_3$ | H | Cl | H | 0 | 4-$CH_3$ | $CO_2(CH_2)_2OCH_3$ |
| 3-$CH_3$ | F | Cl | H | 0 | 4-$CH_3$ | $CO_2(CH_2)_2OCH_3$ |
| 4-$CH_3$ | F | Cl | H | 0 | 4-$CH_3$ | $CO_2(CH_2)_2OCH_3$ |
| 3-$CH_3$ | H | F | $CH_3$ | 0 | 4-$CH_3$ | $CO_2(CH_2)_2OCH_3$ |
| 4-$CH_3$ | H | F | $CH_3$ | 0 | 4-$CH_3$ | $CO_2(CH_2)_2OCH_3$ |
| 3-$CH_3$ | F | F | $CH_3$ | 0 | 4-$CH_3$ | $CO_2(CH_2)_2OCH_3$ |
| 4-$CH_3$ | F | F | $CH_3$ | 0 | 4-$CH_3$ | $CO_2(CH_2)_2OCH_3$ |
| 3-$CH_3$ | H | Cl | $CH_3$ | 0 | 4-$CH_3$ | $CO_2(CH_2)_2OCH_3$ |
| 4-$CH_3$ | H | Cl | $CH_3$ | 0 | 4-$CH_3$ | $CO_2(CH_2)_2OCH_3$ |
| 3-$CH_3$ | F | Cl | $CH_3$ | 0 | 4-$CH_3$ | $CO_2(CH_2)_2OCH_3$ |
| 4-$CH_3$ | F | Cl | $CH_3$ | 0 | 4-$CH_3$ | $CO_2(CH_2)_2OCH_3$ |
| 3-$CH_3$ | H | F | H | 0 | 4-$CH_3$ | $CO_2(CH_2)_2OCH_2CH_3$ |
| 4-$CH_3$ | H | F | H | 0 | 4-$CH_3$ | $CO_2(CH_2)_2OCH_2CH_3$ |
| 3-$CH_3$ | F | F | H | 0 | 4-$CH_3$ | $CO_2(CH_2)_2OCH_2CH_3$ |
| 4-$CH_3$ | F | F | H | 0 | 4-$CH_3$ | $CO_2(CH_2)_2OCH_2CH_3$ |
| 3-$CH_3$ | H | Cl | H | 0 | 4-$CH_3$ | $CO_2(CH_2)_2OCH_2CH_3$ |
| 4-$CH_3$ | H | Cl | H | 0 | 4-$CH_3$ | $CO_2(CH_2)_2OCH_2CH_3$ |
| 3-$CH_3$ | F | Cl | H | 0 | 4-$CH_3$ | $CO_2(CH_2)_2OCH_2CH_3$ |
| 4-$CH_3$ | F | Cl | H | 0 | 4-$CH_3$ | $CO_2(CH_2)_2OCH_2CH_3$ |
| 3-$CH_3$ | H | F | $CH_3$ | 0 | 4-$CH_3$ | $CO_2(CH_2)_2OCH_2CH_3$ |
| 4-$CH_3$ | H | F | $CH_3$ | 0 | 4-$CH_3$ | $CO_2(CH_2)_2OCH_2CH_3$ |
| 3-$CH_3$ | F | F | $CH_3$ | 0 | 4-$CH_3$ | $CO_2(CH_2)_2OCH_2CH_3$ |
| 4-$CH_3$ | F | F | $CH_3$ | 0 | 4-$CH_3$ | $CO_2(CH_2)_2OCH_2CH_3$ |
| 3-$CH_3$ | H | Cl | $CH_3$ | 0 | 4-$CH_3$ | $CO_2(CH_2)_2OCH_2CH_3$ |
| 4-$CH_3$ | H | Cl | $CH_3$ | 0 | 4-$CH_3$ | $CO_2(CH_2)_2OCH_2CH_3$ |
| 3-$CH_3$ | F | Cl | $CH_3$ | 0 | 4-$CH_3$ | $CO_2(CH_2)_2OCH_2CH_3$ |
| 4-$CH_3$ | F | Cl | $CH_3$ | 0 | 4-$CH_3$ | $CO_2(CH_2)_2OCH_2CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 5-$CH_3$ | $CO_2CH_3$ |
| 4-$CH_3$ | H | F | H | 0 | 5-$CH_3$ | $CO_2CH_3$ |
| 3-$CH_3$ | F | F | H | 0 | 5-$CH_3$ | $CO_2CH_3$ |
| 4-$CH_3$ | F | F | H | 0 | 5-$CH_3$ | $CO_2CH_3$ |
| 3-$CH_3$ | H | Cl | H | 0 | 5-$CH_3$ | $CO_2CH_3$ |
| 4-$CH_3$ | H | Cl | H | 0 | 5-$CH_3$ | $CO_2CH_3$ |
| 3-$CH_3$ | F | Cl | H | 0 | 5-$CH_3$ | $CO_2CH_3$ |
| 4-$CH_3$ | F | Cl | H | 0 | 5-$CH_3$ | $CO_2CH_3$ |
| 3-$CH_3$ | H | F | $CH_3$ | 0 | 5-$CH_3$ | $CO_2CH_3$ |
| 4-$CH_3$ | H | F | $CH_3$ | 0 | 5-$CH_3$ | $CO_2CH_3$ |
| 3-$CH_3$ | F | F | $CH_3$ | 0 | 5-$CH_3$ | $CO_2CH_3$ |
| 4-$CH_3$ | F | F | $CH_3$ | 0 | 5-$CH_3$ | $CO_2CH_3$ |
| 3-$CH_3$ | H | Cl | $CH_3$ | 0 | 5-$CH_3$ | $CO_2CH_3$ |
| 4-$CH_3$ | H | Cl | $CH_3$ | 0 | 5-$CH_3$ | $CO_2CH_3$ |
| 3-$CH_3$ | F | Cl | $CH_3$ | 0 | 5-$CH_3$ | $CO_2CH_3$ |
| 4-$CH_3$ | F | Cl | $CH_3$ | 0 | 5-$CH_3$ | $CO_2CH_3$ |
| 3-$CH_3$ | H | F | H | 0 | 5-$CH_3$ | $CO_2CH_2CH_3$ |
| 4-$CH_3$ | H | F | H | 0 | 5-$CH_3$ | $CO_2CH_2CH_3$ |
| 3-$CH_3$ | F | F | H | 0 | 5-$CH_3$ | $CO_2CH_2CH_3$ |
| 4-$CH_3$ | F | F | H | 0 | 5-$CH_3$ | $CO_2CH_2CH_3$ |
| 3-$CH_3$ | H | Cl | H | 0 | 5-$CH_3$ | $CO_2CH_2CH_3$ |
| 4-$CH_3$ | H | Cl | H | 0 | 5-$CH_3$ | $CO_2CH_2CH_3$ |
| 3-$CH_3$ | F | Cl | H | 0 | 5-$CH_3$ | $CO_2CH_2CH_3$ |
| 4-$CH_3$ | F | Cl | H | 0 | 5-$CH_3$ | $CO_2CH_2CH_3$ |
| 3-$CH_3$ | H | F | $CH_3$ | 0 | 5-$CH_3$ | $CO_2CH_2CH_3$ |
| 4-$CH_3$ | H | F | $CH_3$ | 0 | 5-$CH_3$ | $CO_2CH_2CH_3$ |
| 3-$CH_3$ | F | F | $CH_3$ | 0 | 5-$CH_3$ | $CO_2CH_2CH_3$ |
| 4-$CH_3$ | F | F | $CH_3$ | 0 | 5-$CH_3$ | $CO_2CH_2CH_3$ |
| 3-$CH_3$ | H | Cl | $CH_3$ | 0 | 5-$CH_3$ | $CO_2CH_2CH_3$ |
| 4-$CH_3$ | H | Cl | $CH_3$ | 0 | 5-$CH_3$ | $CO_2CH_2CH_3$ |
| 3-$CH_3$ | F | Cl | $CH_3$ | 0 | 5-$CH_3$ | $CO_2CH_2CH_3$ |
| 4-$CH_3$ | F | Cl | $CH_3$ | 0 | 5-$CH_3$ | $CO_2CH_2CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 5-$CH_3$ | $CO_2(CH_2)_2CH_3$ |
| 4-$CH_3$ | H | F | H | 0 | 5-$CH_3$ | $CO_2(CH_2)_2CH_3$ |
| 3-$CH_3$ | F | F | H | 0 | 5-$CH_3$ | $CO_2(CH_2)_2CH_3$ |
| 4-$CH_3$ | F | F | H | 0 | 5-$CH_3$ | $CO_2(CH_2)_2CH_3$ |
| 3-$CH_3$ | H | Cl | H | 0 | 5-$CH_3$ | $CO_2(CH_2)_2CH_3$ |
| 4-$CH_3$ | H | Cl | H | 0 | 5-$CH_3$ | $CO_2(CH_2)_2CH_3$ |
| 3-$CH_3$ | F | Cl | H | 0 | 5-$CH_3$ | $CO_2(CH_2)_2CH_3$ |
| 4-$CH_3$ | F | Cl | H | 0 | 5-$CH_3$ | $CO_2(CH_2)_2CH_3$ |
| 3-$CH_3$ | H | F | $CH_3$ | 0 | 5-$CH_3$ | $CO_2(CH_2)_2CH_3$ |
| 4-$CH_3$ | H | F | $CH_3$ | 0 | 5-$CH_3$ | $CO_2(CH_2)_2CH_3$ |
| 3-$CH_3$ | F | F | $CH_3$ | 0 | 5-$CH_3$ | $CO_2(CH_2)_2CH_3$ |
| 4-$CH_3$ | F | F | $CH_3$ | 0 | 5-$CH_3$ | $CO_2(CH_2)_2CH_3$ |
| 3-$CH_3$ | H | Cl | $CH_3$ | 0 | 5-$CH_3$ | $CO_2(CH_2)_2CH_3$ |
| 4-$CH_3$ | H | Cl | $CH_3$ | 0 | 5-$CH_3$ | $CO_2(CH_2)_2CH_3$ |
| 3-$CH_3$ | F | Cl | $CH_3$ | 0 | 5-$CH_3$ | $CO_2(CH_2)_2CH_3$ |
| 4-$CH_3$ | F | Cl | $CH_3$ | 0 | 5-$CH_3$ | $CO_2(CH_2)_2CH_3$ |
| 3-$CH_3$ | H | F | H | 0 | 5-$CH_3$ | $CO_2CH(CH_3)_2$ |
| 4-$CH_3$ | H | F | H | 0 | 5-$CH_3$ | $CO_2CH(CH_3)_2$ |
| 3-$CH_3$ | F | F | H | 0 | 5-$CH_3$ | $CO_2CH(CH_3)_2$ |
| 4-$CH_3$ | F | F | H | 0 | 5-$CH_3$ | $CO_2CH(CH_3)_2$ |
| 3-$CH_3$ | H | Cl | H | 0 | 5-$CH_3$ | $CO_2CH(CH_3)_2$ |
| 4-$CH_3$ | H | Cl | H | 0 | 5-$CH_3$ | $CO_2CH(CH_3)_2$ |
| 3-$CH_3$ | F | Cl | H | 0 | 5-$CH_3$ | $CO_2CH(CH_3)_2$ |
| 4-$CH_3$ | F | Cl | H | 0 | 5-$CH_3$ | $CO_2CH(CH_3)_2$ |
| 3-$CH_3$ | H | F | $CH_3$ | 0 | 5-$CH_3$ | $CO_2CH(CH_3)_2$ |
| 4-$CH_3$ | H | F | $CH_3$ | 0 | 5-$CH_3$ | $CO_2CH(CH_3)_2$ |
| 3-$CH_3$ | F | F | $CH_3$ | 0 | 5-$CH_3$ | $CO_2CH(CH_3)_2$ |
| 4-$CH_3$ | F | F | $CH_3$ | 0 | 5-$CH_3$ | $CO_2CH(CH_3)_2$ |
| 3-$CH_3$ | H | Cl | $CH_3$ | 0 | 5-$CH_3$ | $CO_2CH(CH_3)_2$ |
| 4-$CH_3$ | H | Cl | $CH_3$ | 0 | 5-$CH_3$ | $CO_2CH(CH_3)_2$ |
| 3-$CH_3$ | F | Cl | $CH_3$ | 0 | 5-$CH_3$ | $CO_2CH(CH_3)_2$ |
| 4-$CH_3$ | F | Cl | $CH_3$ | 0 | 5-$CH_3$ | $CO_2CH(CH_3)_2$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 5-$CH_3$ | $CO_2(CH_2)_3CH_3$ |
| 4-$CH_3$ | H | F | H | 0 | 5-$CH_3$ | $CO_2(CH_2)_3CH_3$ |
| 3-$CH_3$ | F | F | H | 0 | 5-$CH_3$ | $CO_2(CH_2)_3CH_3$ |
| 4-$CH_3$ | F | F | H | 0 | 5-$CH_3$ | $CO_2(CH_2)_3CH_3$ |
| 3-$CH_3$ | H | Cl | H | 0 | 5-$CH_3$ | $CO_2(CH_2)_3CH_3$ |
| 4-$CH_3$ | H | Cl | H | 0 | 5-$CH_3$ | $CO_2(CH_2)_3CH_3$ |
| 3-$CH_3$ | F | Cl | H | 0 | 5-$CH_3$ | $CO_2(CH_2)_3CH_3$ |
| 4-$CH_3$ | F | Cl | H | 0 | 5-$CH_3$ | $CO_2(CH_2)_3CH_3$ |
| 3-$CH_3$ | H | F | $CH_3$ | 0 | 5-$CH_3$ | $CO_2(CH_2)_3CH_3$ |
| 4-$CH_3$ | H | F | $CH_3$ | 0 | 5-$CH_3$ | $CO_2(CH_2)_3CH_3$ |
| 3-$CH_3$ | F | F | $CH_3$ | 0 | 5-$CH_3$ | $CO_2(CH_2)_3CH_3$ |
| 4-$CH_3$ | F | F | $CH_3$ | 0 | 5-$CH_3$ | $CO_2(CH_2)_3CH_3$ |
| 3-$CH_3$ | H | Cl | $CH_3$ | 0 | 5-$CH_3$ | $CO_2(CH_2)_3CH_3$ |
| 4-$CH_3$ | H | Cl | $CH_3$ | 0 | 5-$CH_3$ | $CO_2(CH_2)_3CH_3$ |
| 3-$CH_3$ | F | Cl | $CH_3$ | 0 | 5-$CH_3$ | $CO_2(CH_2)_3CH_3$ |
| 4-$CH_3$ | F | Cl | $CH_3$ | 0 | 5-$CH_3$ | $CO_2(CH_2)_3CH_3$ |
| 3-$CH_3$ | H | F | H | 0 | 5-$CH_3$ | $CO_2(CH_2)_2OCH_3$ |
| 4-$CH_3$ | H | F | H | 0 | 5-$CH_3$ | $CO_2(CH_2)_2OCH_3$ |
| 3-$CH_3$ | F | F | H | 0 | 5-$CH_3$ | $CO_2(CH_2)_2OCH_3$ |
| 4-$CH_3$ | F | F | H | 0 | 5-$CH_3$ | $CO_2(CH_2)_2OCH_3$ |
| 3-$CH_3$ | H | Cl | H | 0 | 5-$CH_3$ | $CO_2(CH_2)_2OCH_3$ |
| 4-$CH_3$ | H | Cl | H | 0 | 5-$CH_3$ | $CO_2(CH_2)_2OCH_3$ |
| 3-$CH_3$ | F | Cl | H | 0 | 5-$CH_3$ | $CO_2(CH_2)_2OCH_3$ |
| 4-$CH_3$ | F | Cl | H | 0 | 5-$CH_3$ | $CO_2(CH_2)_2OCH_3$ |
| 3-$CH_3$ | H | F | $CH_3$ | 0 | 5-$CH_3$ | $CO_2(CH_2)_2OCH_3$ |
| 4-$CH_3$ | H | F | $CH_3$ | 0 | 5-$CH_3$ | $CO_2(CH_2)_2OCH_3$ |
| 3-$CH_3$ | F | F | $CH_3$ | 0 | 5-$CH_3$ | $CO_2(CH_2)_2OCH_3$ |
| 4-$CH_3$ | F | F | $CH_3$ | 0 | 5-$CH_3$ | $CO_2(CH_2)_2OCH_3$ |
| 3-$CH_3$ | H | Cl | $CH_3$ | 0 | 5-$CH_3$ | $CO_2(CH_2)_2OCH_3$ |
| 4-$CH_3$ | H | Cl | $CH_3$ | 0 | 5-$CH_3$ | $CO_2(CH_2)_2OCH_3$ |
| 3-$CH_3$ | F | Cl | $CH_3$ | 0 | 5-$CH_3$ | $CO_2(CH_2)_2OCH_3$ |
| 4-$CH_3$ | F | Cl | $CH_3$ | 0 | 5-$CH_3$ | $CO_2(CH_2)_2OCH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 5-$CH_3$ | $CO_2(CH_2)_2OCH_2CH_3$ |
| 4-$CH_3$ | H | F | H | 0 | 5-$CH_3$ | $CO_2(CH_2)_2OCH_2CH_3$ |
| 3-$CH_3$ | F | F | H | 0 | 5-$CH_3$ | $CO_2(CH_2)_2OCH_2CH_3$ |
| 4-$CH_3$ | F | F | H | 0 | 5-$CH_3$ | $CO_2(CH_2)_2OCH_2CH_3$ |
| 3-$CH_3$ | H | Cl | H | 0 | 5-$CH_3$ | $CO_2(CH_2)_2OCH_2CH_3$ |
| 4-$CH_3$ | H | Cl | H | 0 | 5-$CH_3$ | $CO_2(CH_2)_2OCH_2CH_3$ |
| 3-$CH_3$ | F | Cl | H | 0 | 5-$CH_3$ | $CO_2(CH_2)_2OCH_2CH_3$ |
| 4-$CH_3$ | F | Cl | H | 0 | 5-$CH_3$ | $CO_2(CH_2)_2OCH_2CH_3$ |
| 3-$CH_3$ | H | F | $CH_3$ | 0 | 5-$CH_3$ | $CO_2(CH_2)_2OCH_2CH_3$ |
| 4-$CH_3$ | H | F | $CH_3$ | 0 | 5-$CH_3$ | $CO_2(CH_2)_2OCH_2CH_3$ |
| 3-$CH_3$ | F | F | $CH_3$ | 0 | 5-$CH_3$ | $CO_2(CH_2)_2OCH_2CH_3$ |
| 4-$CH_3$ | F | F | $CH_3$ | 0 | 5-$CH_3$ | $CO_2(CH_2)_2OCH_2CH_3$ |
| 3-$CH_3$ | H | Cl | $CH_3$ | 0 | 5-$CH_3$ | $CO_2(CH_2)_2OCH_2CH_3$ |
| 4-$CH_3$ | H | Cl | $CH_3$ | 0 | 5-$CH_3$ | $CO_2(CH_2)_2OCH_2CH_3$ |
| 3-$CH_3$ | F | Cl | $CH_3$ | 0 | 5-$CH_3$ | $CO_2(CH_2)_2OCH_2CH_3$ |
| 4-$CH_3$ | F | Cl | $CH_3$ | 0 | 5-$CH_3$ | $CO_2(CH_2)_2OCH_2CH_3$ |
| 3-$CH_3$ | H | F | H | 0 | 4,5-Di-$CH_3$ | $CO_2CH_3$ |
| 4-$CH_3$ | H | F | H | 0 | 4,5-Di-$CH_3$ | $CO_2CH_3$ |
| 3-$CH_3$ | F | F | H | 0 | 4,5-Di-$CH_3$ | $CO_2CH_3$ |
| 4-$CH_3$ | F | F | H | 0 | 4,5-Di-$CH_3$ | $CO_2CH_3$ |
| 3-$CH_3$ | H | Cl | H | 0 | 4,5-Di-$CH_3$ | $CO_2CH_3$ |
| 4-$CH_3$ | H | Cl | H | 0 | 4,5-Di-$CH_3$ | $CO_2CH_3$ |
| 3-$CH_3$ | F | Cl | H | 0 | 4,5-Di-$CH_3$ | $CO_2CH_3$ |
| 4-$CH_3$ | F | Cl | H | 0 | 4,5-Di-$CH_3$ | $CO_2CH_3$ |
| 3-$CH_3$ | H | F | $CH_3$ | 0 | 4,5-Di-$CH_3$ | $CO_2CH_3$ |
| 4-$CH_3$ | H | F | $CH_3$ | 0 | 4,5-Di-$CH_3$ | $CO_2CH_3$ |
| 3-$CH_3$ | F | F | $CH_3$ | 0 | 4,5-Di-$CH_3$ | $CO_2CH_3$ |
| 4-$CH_3$ | F | F | $CH_3$ | 0 | 4,5-Di-$CH_3$ | $CO_2CH_3$ |
| 3-$CH_3$ | H | Cl | $CH_3$ | 0 | 4,5-Di-$CH_3$ | $CO_2CH_3$ |
| 4-$CH_3$ | H | Cl | $CH_3$ | 0 | 4,5-Di-$CH_3$ | $CO_2CH_3$ |
| 3-$CH_3$ | F | Cl | $CH_3$ | 0 | 4,5-Di-$CH_3$ | $CO_2CH_3$ |
| 4-$CH_3$ | F | Cl | $CH_3$ | 0 | 4,5-Di-$CH_3$ | $CO_2CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 4,5-Di-$CH_3$ | $CO_2CH_2CH_3$ |
| 4-$CH_3$ | H | F | H | 0 | 4,5-Di-$CH_3$ | $CO_2CH_2CH_3$ |
| 3-$CH_3$ | F | F | H | 0 | 4,5-Di-$CH_3$ | $CO_2CH_2CH_3$ |
| 4-$CH_3$ | F | F | H | 0 | 4,5-Di-$CH_3$ | $CO_2CH_2CH_3$ |
| 3-$CH_3$ | H | Cl | H | 0 | 4,5-Di-$CH_3$ | $CO_2CH_2CH_3$ |
| 4-$CH_3$ | H | Cl | H | 0 | 4,5-Di-$CH_3$ | $CO_2CH_2CH_3$ |
| 3-$CH_3$ | F | Cl | H | 0 | 4,5-Di-$CH_3$ | $CO_2CH_2CH_3$ |
| 4-$CH_3$ | F | Cl | H | 0 | 4,5-Di-$CH_3$ | $CO_2CH_2CH_3$ |
| 3-$CH_3$ | H | F | $CH_3$ | 0 | 4,5-Di-$CH_3$ | $CO_2CH_2CH_3$ |
| 4-$CH_3$ | H | F | $CH_3$ | 0 | 4,5-Di-$CH_3$ | $CO_2CH_2CH_3$ |
| 3-$CH_3$ | F | F | $CH_3$ | 0 | 4,5-Di-$CH_3$ | $CO_2CH_2CH_3$ |
| 4-$CH_3$ | F | F | $CH_3$ | 0 | 4,5-Di-$CH_3$ | $CO_2CH_2CH_3$ |
| 3-$CH_3$ | H | Cl | $CH_3$ | 0 | 4,5-Di-$CH_3$ | $CO_2CH_2CH_3$ |
| 4-$CH_3$ | H | Cl | $CH_3$ | 0 | 4,5-Di-$CH_3$ | $CO_2CH_2CH_3$ |
| 3-$CH_3$ | F | Cl | $CH_3$ | 0 | 4,5-Di-$CH_3$ | $CO_2CH_2CH_3$ |
| 4-$CH_3$ | F | Cl | $CH_3$ | 0 | 4,5-Di-$CH_3$ | $CO_2CH_2CH_3$ |
| 3-$CH_3$ | H | F | H | 0 | 4,5-Di-$CH_3$ | $CO_2(CH_2)_2CH_3$ |
| 4-$CH_3$ | H | F | H | 0 | 4,5-Di-$CH_3$ | $CO_2(CH_2)_2CH_3$ |
| 3-$CH_3$ | F | F | H | 0 | 4,5-Di-$CH_3$ | $CO_2(CH_2)_2CH_3$ |
| 4-$CH_3$ | F | F | H | 0 | 4,5-Di-$CH_3$ | $CO_2(CH_2)_2CH_3$ |
| 3-$CH_3$ | H | Cl | H | 0 | 4,5-Di-$CH_3$ | $CO_2(CH_2)_2CH_3$ |
| 4-$CH_3$ | H | Cl | H | 0 | 4,5-Di-$CH_3$ | $CO_2(CH_2)_2CH_3$ |
| 3-$CH_3$ | F | Cl | H | 0 | 4,5-Di-$CH_3$ | $CO_2(CH_2)_2CH_3$ |
| 4-$CH_3$ | F | Cl | H | 0 | 4,5-Di-$CH_3$ | $CO_2(CH_2)_2CH_3$ |
| 3-$CH_3$ | H | F | $CH_3$ | 0 | 4,5-Di-$CH_3$ | $CO_2(CH_2)_2CH_3$ |
| 4-$CH_3$ | H | F | $CH_3$ | 0 | 4,5-Di-$CH_3$ | $CO_2(CH_2)_2CH_3$ |
| 3-$CH_3$ | F | F | $CH_3$ | 0 | 4,5-Di-$CH_3$ | $CO_2(CH_2)_2CH_3$ |
| 4-$CH_3$ | F | F | $CH_3$ | 0 | 4,5-Di-$CH_3$ | $CO_2(CH_2)_2CH_3$ |
| 3-$CH_3$ | H | Cl | $CH_3$ | 0 | 4,5-Di-$CH_3$ | $CO_2(CH_2)_2CH_3$ |
| 4-$CH_3$ | H | Cl | $CH_3$ | 0 | 4,5-Di-$CH_3$ | $CO_2(CH_2)_2CH_3$ |
| 3-$CH_3$ | F | Cl | $CH_3$ | 0 | 4,5-Di-$CH_3$ | $CO_2(CH_2)_2CH_3$ |
| 4-$CH_3$ | F | Cl | $CH_3$ | 0 | 4,5-Di-$CH_3$ | $CO_2(CH_2)_2CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 4,5-Di-$CH_3$ | $CO_2CH(CH_3)_2$ |
| 4-$CH_3$ | H | F | H | 0 | 4,5-Di-$CH_3$ | $CO_2CH(CH_3)_2$ |
| 3-$CH_3$ | F | F | H | 0 | 4,5-Di-$CH_3$ | $CO_2CH(CH_3)_2$ |
| 4-$CH_3$ | F | F | H | 0 | 4,5-Di-$CH_3$ | $CO_2CH(CH_3)_2$ |
| 3-$CH_3$ | H | Cl | H | 0 | 4,5-Di-$CH_3$ | $CO_2CH(CH_3)_2$ |
| 4-$CH_3$ | H | Cl | H | 0 | 4,5-Di-$CH_3$ | $CO_2CH(CH_3)_2$ |
| 3-$CH_3$ | F | Cl | H | 0 | 4,5-Di-$CH_3$ | $CO_2CH(CH_3)_2$ |
| 4-$CH_3$ | F | Cl | H | 0 | 4,5-Di-$CH_3$ | $CO_2CH(CH_3)_2$ |
| 3-$CH_3$ | H | F | $CH_3$ | 0 | 4,5-Di-$CH_3$ | $CO_2CH(CH_3)_2$ |
| 4-$CH_3$ | H | F | $CH_3$ | 0 | 4,5-Di-$CH_3$ | $CO_2CH(CH_3)_2$ |
| 3-$CH_3$ | F | F | $CH_3$ | 0 | 4,5-Di-$CH_3$ | $CO_2CH(CH_3)_2$ |
| 4-$CH_3$ | F | F | $CH_3$ | 0 | 4,5-Di-$CH_3$ | $CO_2CH(CH_3)_2$ |
| 3-$CH_3$ | H | Cl | $CH_3$ | 0 | 4,5-Di-$CH_3$ | $CO_2CH(CH_3)_2$ |
| 4-$CH_3$ | H | Cl | $CH_3$ | 0 | 4,5-Di-$CH_3$ | $CO_2CH(CH_3)_2$ |
| 3-$CH_3$ | F | Cl | $CH_3$ | 0 | 4,5-Di-$CH_3$ | $CO_2CH(CH_3)_2$ |
| 4-$CH_3$ | F | Cl | $CH_3$ | 0 | 4,5-Di-$CH_3$ | $CO_2CH(CH_3)_2$ |
| 3-$CH_3$ | H | F | H | 0 | 4,5-Di-$CH_3$ | $CO_2(CH_2)_3CH_3$ |
| 4-$CH_3$ | H | F | H | 0 | 4,5-Di-$CH_3$ | $CO_2(CH_2)_3CH_3$ |
| 3-$CH_3$ | F | F | H | 0 | 4,5-Di-$CH_3$ | $CO_2(CH_2)_3CH_3$ |
| 4-$CH_3$ | F | F | H | 0 | 4,5-Di-$CH_3$ | $CO_2(CH_2)_3CH_3$ |
| 3-$CH_3$ | H | Cl | H | 0 | 4,5-Di-$CH_3$ | $CO_2(CH_2)_3CH_3$ |
| 4-$CH_3$ | H | Cl | H | 0 | 4,5-Di-$CH_3$ | $CO_2(CH_2)_3CH_3$ |
| 3-$CH_3$ | F | Cl | H | 0 | 4,5-Di-$CH_3$ | $CO_2(CH_2)_3CH_3$ |
| 4-$CH_3$ | F | Cl | H | 0 | 4,5-Di-$CH_3$ | $CO_2(CH_2)_3CH_3$ |
| 3-$CH_3$ | H | F | $CH_3$ | 0 | 4,5-Di-$CH_3$ | $CO_2(CH_2)_3CH_3$ |
| 4-$CH_3$ | H | F | $CH_3$ | 0 | 4,5-Di-$CH_3$ | $CO_2(CH_2)_3CH_3$ |
| 3-$CH_3$ | F | F | $CH_3$ | 0 | 4,5-Di-$CH_3$ | $CO_2(CH_2)_3CH_3$ |
| 4-$CH_3$ | F | F | $CH_3$ | 0 | 4,5-Di-$CH_3$ | $CO_2(CH_2)_3CH_3$ |
| 3-$CH_3$ | H | Cl | $CH_3$ | 0 | 4,5-Di-$CH_3$ | $CO_2(CH_2)_3CH_3$ |
| 4-$CH_3$ | H | Cl | $CH_3$ | 0 | 4,5-Di-$CH_3$ | $CO_2(CH_2)_3CH_3$ |
| 3-$CH_3$ | F | Cl | $CH_3$ | 0 | 4,5-Di-$CH_3$ | $CO_2(CH_2)_3CH_3$ |
| 4-$CH_3$ | F | Cl | $CH_3$ | 0 | 4,5-Di-$CH_3$ | $CO_2(CH_2)_3CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 0 | 4,5-Di-$CH_3$ | $CO_2(CH_2)_2OCH_3$ |
| 4-$CH_3$ | H | F | H | 0 | 4,5-Di-$CH_3$ | $CO_2(CH_2)_2OCH_3$ |
| 3-$CH_3$ | F | F | H | 0 | 4,5-Di-$CH_3$ | $CO_2(CH_2)_2OCH_3$ |
| 4-$CH_3$ | F | F | H | 0 | 4,5-Di-$CH_3$ | $CO_2(CH_2)_2OCH_3$ |
| 3-$CH_3$ | H | Cl | H | 0 | 4,5-Di-$CH_3$ | $CO_2(CH_2)_2OCH_3$ |
| 4-$CH_3$ | H | Cl | H | 0 | 4,5-Di-$CH_3$ | $CO_2(CH_2)_2OCH_3$ |
| 3-$CH_3$ | F | Cl | H | 0 | 4,5-Di-$CH_3$ | $CO_2(CH_2)_2OCH_3$ |
| 4-$CH_3$ | F | Cl | H | 0 | 4,5-Di-$CH_3$ | $CO_2(CH_2)_2OCH_3$ |
| 3-$CH_3$ | H | F | $CH_3$ | 0 | 4,5-Di-$CH_3$ | $CO_2(CH_2)_2OCH_3$ |
| 4-$CH_3$ | H | F | $CH_3$ | 0 | 4,5-Di-$CH_3$ | $CO_2(CH_2)_2OCH_3$ |
| 3-$CH_3$ | F | F | $CH_3$ | 0 | 4,5-Di-$CH_3$ | $CO_2(CH_2)_2OCH_3$ |
| 4-$CH_3$ | F | F | $CH_3$ | 0 | 4,5-Di-$CH_3$ | $CO_2(CH_2)_2OCH_3$ |
| 3-$CH_3$ | H | Cl | $CH_3$ | 0 | 4,5-Di-$CH_3$ | $CO_2(CH_2)_2OCH_3$ |
| 4-$CH_3$ | H | Cl | $CH_3$ | 0 | 4,5-Di-$CH_3$ | $CO_2(CH_2)_2OCH_3$ |
| 3-$CH_3$ | F | Cl | $CH_3$ | 0 | 4,5-Di-$CH_3$ | $CO_2(CH_2)_2OCH_3$ |
| 4-$CH_3$ | F | Cl | $CH_3$ | 0 | 4,5-Di-$CH_3$ | $CO_2(CH_2)_2OCH_3$ |
| 3-$CH_3$ | H | F | H | 0 | 4,5-Di-$CH_3$ | $CO_2(CH_2)_2OCH_2CH_3$ |
| 4-$CH_3$ | H | F | H | 0 | 4,5-Di-$CH_3$ | $CO_2(CH_2)_2OCH_2CH_3$ |
| 3-$CH_3$ | F | F | H | 0 | 4,5-Di-$CH_3$ | $CO_2(CH_2)_2OCH_2CH_3$ |
| 4-$CH_3$ | F | F | H | 0 | 4,5-Di-$CH_3$ | $CO_2(CH_2)_2OCH_2CH_3$ |
| 3-$CH_3$ | H | Cl | H | 0 | 4,5-Di-$CH_3$ | $CO_2(CH_2)_2OCH_2CH_3$ |
| 4-$CH_3$ | H | Cl | H | 0 | 4,5-Di-$CH_3$ | $CO_2(CH_2)_2OCH_2CH_3$ |
| 3-$CH_3$ | F | Cl | H | 0 | 4,5-Di-$CH_3$ | $CO_2(CH_2)_2OCH_2CH_3$ |
| 4-$CH_3$ | F | Cl | H | 0 | 4,5-Di-$CH_3$ | $CO_2(CH_2)_2OCH_2CH_3$ |
| 3-$CH_3$ | H | F | $CH_3$ | 0 | 4,5-Di-$CH_3$ | $CO_2(CH_2)_2OCH_2CH_3$ |
| 4-$CH_3$ | H | F | $CH_3$ | 0 | 4,5-Di-$CH_3$ | $CO_2(CH_2)_2OCH_2CH_3$ |
| 3-$CH_3$ | F | F | $CH_3$ | 0 | 4,5-Di-$CH_3$ | $CO_2(CH_2)_2OCH_2CH_3$ |
| 4-$CH_3$ | F | F | $CH_3$ | 0 | 4,5-Di-$CH_3$ | $CO_2(CH_2)_2OCH_2CH_3$ |
| 3-$CH_3$ | H | Cl | $CH_3$ | 0 | 4,5-Di-$CH_3$ | $CO_2(CH_2)_2OCH_2CH_3$ |
| 4-$CH_3$ | H | Cl | $CH_3$ | 0 | 4,5-Di-$CH_3$ | $CO_2(CH_2)_2OCH_2CH_3$ |
| 3-$CH_3$ | F | Cl | $CH_3$ | 0 | 4,5-Di-$CH_3$ | $CO_2(CH_2)_2OCH_2CH_3$ |
| 4-$CH_3$ | F | Cl | $CH_3$ | 0 | 4,5-Di-$CH_3$ | $CO_2(CH_2)_2OCH_2CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | H | $CO_2CH_3$ |
| 4-$CH_3$ | H | F | H | 1 | H | $CO_2CH_3$ |
| 3-$CH_3$ | F | F | H | 1 | H | $CO_2CH_3$ |
| 4-$CH_3$ | F | F | H | 1 | H | $CO_2CH_3$ |
| 3-$CH_3$ | H | Cl | H | 1 | H | $CO_2CH_3$ |
| 4-$CH_3$ | H | Cl | H | 1 | H | $CO_2CH_3$ |
| 3-$CH_3$ | F | Cl | H | 1 | H | $CO_2CH_3$ |
| 4-$CH_3$ | F | Cl | H | 1 | H | $CO_2CH_3$ |
| 3-$CH_3$ | H | F | $CH_3$ | 1 | H | $CO_2CH_3$ |
| 4-$CH_3$ | H | F | $CH_3$ | 1 | H | $CO_2CH_3$ |
| 3-$CH_3$ | F | F | $CH_3$ | 1 | H | $CO_2CH_3$ |
| 4-$CH_3$ | F | F | $CH_3$ | 1 | H | $CO_2CH_3$ |
| 3-$CH_3$ | H | Cl | $CH_3$ | 1 | H | $CO_2CH_3$ |
| 4-$CH_3$ | H | Cl | $CH_3$ | 1 | H | $CO_2CH_3$ |
| 3-$CH_3$ | F | Cl | $CH_3$ | 1 | H | $CO_2CH_3$ |
| 4-$CH_3$ | F | Cl | $CH_3$ | 1 | H | $CO_2CH_3$ |
| 3-$CH_3$ | H | F | H | 1 | H | $CO_2CH_2CH_3$ |
| 4-$CH_3$ | H | F | H | 1 | H | $CO_2CH_2CH_3$ |
| 3-$CH_3$ | F | F | H | 1 | H | $CO_2CH_2CH_3$ |
| 4-$CH_3$ | F | F | H | 1 | H | $CO_2CH_2CH_3$ |
| 3-$CH_3$ | H | Cl | H | 1 | H | $CO_2CH_2CH_3$ |
| 4-$CH_3$ | H | Cl | H | 1 | H | $CO_2CH_2CH_3$ |
| 3-$CH_3$ | F | Cl | H | 1 | H | $CO_2CH_2CH_3$ |
| 4-$CH_3$ | F | Cl | H | 1 | H | $CO_2CH_2CH_3$ |
| 3-$CH_3$ | H | F | $CH_3$ | 1 | H | $CO_2CH_2CH_3$ |
| 4-$CH_3$ | H | F | $CH_3$ | 1 | H | $CO_2CH_2CH_3$ |
| 3-$CH_3$ | F | F | $CH_3$ | 1 | H | $CO_2CH_2CH_3$ |
| 4-$CH_3$ | F | F | $CH_3$ | 1 | H | $CO_2CH_2CH_3$ |
| 3-$CH_3$ | H | Cl | $CH_3$ | 1 | H | $CO_2CH_2CH_3$ |
| 4-$CH_3$ | H | Cl | $CH_3$ | 1 | H | $CO_2CH_2CH_3$ |
| 3-$CH_3$ | F | Cl | $CH_3$ | 1 | H | $CO_2CH_2CH_3$ |
| 4-$CH_3$ | F | Cl | $CH_3$ | 1 | H | $CO_2CH_2CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | H | $CO_2(CH_2)_2CH_3$ |
| 4-$CH_3$ | H | F | H | 1 | H | $CO_2(CH_2)_2CH_3$ |
| 3-$CH_3$ | F | F | H | 1 | H | $CO_2(CH_2)_2CH_3$ |
| 4-$CH_3$ | F | F | H | 1 | H | $CO_2(CH_2)_2CH_3$ |
| 3-$CH_3$ | H | Cl | H | 1 | H | $CO_2(CH_2)_2CH_3$ |
| 4-$CH_3$ | H | Cl | H | 1 | H | $CO_2(CH_2)_2CH_3$ |
| 3-$CH_3$ | F | Cl | H | 1 | H | $CO_2(CH_2)_2CH_3$ |
| 4-$CH_3$ | F | Cl | H | 1 | H | $CO_2(CH_2)_2CH_3$ |
| 3-$CH_3$ | H | F | $CH_3$ | 1 | H | $CO_2(CH_2)_2CH_3$ |
| 4-$CH_3$ | H | F | $CH_3$ | 1 | H | $CO_2(CH_2)_2CH_3$ |
| 3-$CH_3$ | F | F | $CH_3$ | 1 | H | $CO_2(CH_2)_2CH_3$ |
| 4-$CH_3$ | F | F | $CH_3$ | 1 | H | $CO_2(CH_2)_2CH_3$ |
| 3-$CH_3$ | H | Cl | $CH_3$ | 1 | H | $CO_2(CH_2)_2CH_3$ |
| 4-$CH_3$ | H | Cl | $CH_3$ | 1 | H | $CO_2(CH_2)_2CH_3$ |
| 3-$CH_3$ | F | Cl | $CH_3$ | 1 | H | $CO_2(CH_2)_2CH_3$ |
| 4-$CH_3$ | F | Cl | $CH_3$ | 1 | H | $CO_2(CH_2)_2CH_3$ |
| 3-$CH_3$ | H | F | H | 1 | H | $CO_2CH(CH_3)_2$ |
| 4-$CH_3$ | H | F | H | 1 | H | $CO_2CH(CH_3)_2$ |
| 3-$CH_3$ | F | F | H | 1 | H | $CO_2CH(CH_3)_2$ |
| 4-$CH_3$ | F | F | H | 1 | H | $CO_2CH(CH_3)_2$ |
| 3-$CH_3$ | H | Cl | H | 1 | H | $CO_2CH(CH_3)_2$ |
| 4-$CH_3$ | H | Cl | H | 1 | H | $CO_2CH(CH_3)_2$ |
| 3-$CH_3$ | F | Cl | H | 1 | H | $CO_2CH(CH_3)_2$ |
| 4-$CH_3$ | F | Cl | H | 1 | H | $CO_2CH(CH_3)_2$ |
| 3-$CH_3$ | H | F | $CH_3$ | 1 | H | $CO_2CH(CH_3)_2$ |
| 4-$CH_3$ | H | F | $CH_3$ | 1 | H | $CO_2CH(CH_3)_2$ |
| 3-$CH_3$ | F | F | $CH_3$ | 1 | H | $CO_2CH(CH_3)_2$ |
| 4-$CH_3$ | F | F | $CH_3$ | 1 | H | $CO_2CH(CH_3)_2$ |
| 3-$CH_3$ | H | Cl | $CH_3$ | 1 | H | $CO_2CH(CH_3)_2$ |
| 4-$CH_3$ | H | Cl | $CH_3$ | 1 | H | $CO_2CH(CH_3)_2$ |
| 3-$CH_3$ | F | Cl | $CH_3$ | 1 | H | $CO_2CH(CH_3)_2$ |
| 4-$CH_3$ | F | Cl | $CH_3$ | 1 | H | $CO_2CH(CH_3)_2$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | H | $CO_2(CH_2)_3CH_3$ |
| 4-$CH_3$ | H | F | H | 1 | H | $CO_2(CH_2)_3CH_3$ |
| 3-$CH_3$ | F | F | H | 1 | H | $CO_2(CH_2)_3CH_3$ |
| 4-$CH_3$ | F | F | H | 1 | H | $CO_2(CH_2)_3CH_3$ |
| 3-$CH_3$ | H | Cl | H | 1 | H | $CO_2(CH_2)_3CH_3$ |
| 4-$CH_3$ | H | Cl | H | 1 | H | $CO_2(CH_2)_3CH_3$ |
| 3-$CH_3$ | F | Cl | H | 1 | H | $CO_2(CH_2)_3CH_3$ |
| 4-$CH_3$ | F | Cl | H | 1 | H | $CO_2(CH_2)_3CH_3$ |
| 3-$CH_3$ | H | F | $CH_3$ | 1 | H | $CO_2(CH_2)_3CH_3$ |
| 4-$CH_3$ | H | F | $CH_3$ | 1 | H | $CO_2(CH_2)_3CH_3$ |
| 3-$CH_3$ | F | F | $CH_3$ | 1 | H | $CO_2(CH_2)_3CH_3$ |
| 4-$CH_3$ | F | F | $CH_3$ | 1 | H | $CO_2(CH_2)_3CH_3$ |
| 3-$CH_3$ | H | Cl | $CH_3$ | 1 | H | $CO_2(CH_2)_3CH_3$ |
| 4-$CH_3$ | H | Cl | $CH_3$ | 1 | H | $CO_2(CH_2)_3CH_3$ |
| 3-$CH_3$ | F | Cl | $CH_3$ | 1 | H | $CO_2(CH_2)_3CH_3$ |
| 4-$CH_3$ | F | Cl | $CH_3$ | 1 | H | $CO_2(CH_2)_3CH_3$ |
| 3-$CH_3$ | H | F | H | 1 | H | $CO_2(CH_2)_2OCH_3$ |
| 4-$CH_3$ | H | F | H | 1 | H | $CO_2(CH_2)_2OCH_3$ |
| 3-$CH_3$ | F | F | H | 1 | H | $CO_2(CH_2)_2OCH_3$ |
| 4-$CH_3$ | F | F | H | 1 | H | $CO_2(CH_2)_2OCH_3$ |
| 3-$CH_3$ | H | Cl | H | 1 | H | $CO_2(CH_2)_2OCH_3$ |
| 4-$CH_3$ | H | Cl | H | 1 | H | $CO_2(CH_2)_2OCH_3$ |
| 3-$CH_3$ | F | Cl | H | 1 | H | $CO_2(CH_2)_2OCH_3$ |
| 4-$CH_3$ | F | Cl | H | 1 | H | $CO_2(CH_2)_2OCH_3$ |
| 3-$CH_3$ | H | F | $CH_3$ | 1 | H | $CO_2(CH_2)_2OCH_3$ |
| 4-$CH_3$ | H | F | $CH_3$ | 1 | H | $CO_2(CH_2)_2OCH_3$ |
| 3-$CH_3$ | F | F | $CH_3$ | 1 | H | $CO_2(CH_2)_2OCH_3$ |
| 4-$CH_3$ | F | F | $CH_3$ | 1 | H | $CO_2(CH_2)_2OCH_3$ |
| 3-$CH_3$ | H | Cl | $CH_3$ | 1 | H | $CO_2(CH_2)_2OCH_3$ |
| 4-$CH_3$ | H | Cl | $CH_3$ | 1 | H | $CO_2(CH_2)_2OCH_3$ |
| 3-$CH_3$ | F | Cl | $CH_3$ | 1 | H | $CO_2(CH_2)_2OCH_3$ |
| 4-$CH_3$ | F | Cl | $CH_3$ | 1 | H | $CO_2(CH_2)_2OCH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | H | $CO_2(CH_2)_2OCH_2CH_3$ |
| 4-$CH_3$ | H | F | H | 1 | H | $CO_2(CH_2)_2OCH_2CH_3$ |
| 3-$CH_3$ | F | F | H | 1 | H | $CO_2(CH_2)_2OCH_2CH_3$ |
| 4-$CH_3$ | F | F | H | 1 | H | $CO_2(CH_2)_2OCH_2CH_3$ |
| 3-$CH_3$ | H | Cl | H | 1 | H | $CO_2(CH_2)_2OCH_2CH_3$ |
| 4-$CH_3$ | H | Cl | H | 1 | H | $CO_2(CH_2)_2OCH_2CH_3$ |
| 3-$CH_3$ | F | Cl | H | 1 | H | $CO_2(CH_2)_2OCH_2CH_3$ |
| 4-$CH_3$ | F | Cl | H | 1 | H | $CO_2(CH_2)_2OCH_2CH_3$ |
| 3-$CH_3$ | H | F | $CH_3$ | 1 | H | $CO_2(CH_2)_2OCH_2CH_3$ |
| 4-$CH_3$ | H | F | $CH_3$ | 1 | H | $CO_2(CH_2)_2OCH_2CH_3$ |
| 3-$CH_3$ | F | F | $CH_3$ | 1 | H | $CO_2(CH_2)_2OCH_2CH_3$ |
| 4-$CH_3$ | F | F | $CH_3$ | 1 | H | $CO_2(CH_2)_2OCH_2CH_3$ |
| 3-$CH_3$ | H | Cl | $CH_3$ | 1 | H | $CO_2(CH_2)_2OCH_2CH_3$ |
| 4-$CH_3$ | H | Cl | $CH_3$ | 1 | H | $CO_2(CH_2)_2OCH_2CH_3$ |
| 3-$CH_3$ | F | Cl | $CH_3$ | 1 | H | $CO_2(CH_2)_2OCH_2CH_3$ |
| 4-$CH_3$ | F | Cl | $CH_3$ | 1 | H | $CO_2(CH_2)_2OCH_2CH_3$ |
| 3-$CH_3$ | H | F | H | 1 | 4-$CH_3$ | $CO_2CH_3$ |
| 4-$CH_3$ | H | F | H | 1 | 4-$CH_3$ | $CO_2CH_3$ |
| 3-$CH_3$ | F | F | H | 1 | 4-$CH_3$ | $CO_2CH_3$ |
| 4-$CH_3$ | F | F | H | 1 | 4-$CH_3$ | $CO_2CH_3$ |
| 3-$CH_3$ | H | Cl | H | 1 | 4-$CH_3$ | $CO_2CH_3$ |
| 4-$CH_3$ | H | Cl | H | 1 | 4-$CH_3$ | $CO_2CH_3$ |
| 3-$CH_3$ | F | Cl | H | 1 | 4-$CH_3$ | $CO_2CH_3$ |
| 4-$CH_3$ | F | Cl | H | 1 | 4-$CH_3$ | $CO_2CH_3$ |
| 3-$CH_3$ | H | F | $CH_3$ | 1 | 4-$CH_3$ | $CO_2CH_3$ |
| 4-$CH_3$ | H | F | $CH_3$ | 1 | 4-$CH_3$ | $CO_2CH_3$ |
| 3-$CH_3$ | F | F | $CH_3$ | 1 | 4-$CH_3$ | $CO_2CH_3$ |
| 4-$CH_3$ | F | F | $CH_3$ | 1 | 4-$CH_3$ | $CO_2CH_3$ |
| 3-$CH_3$ | H | Cl | $CH_3$ | 1 | 4-$CH_3$ | $CO_2CH_3$ |
| 4-$CH_3$ | H | Cl | $CH_3$ | 1 | 4-$CH_3$ | $CO_2CH_3$ |
| 3-$CH_3$ | F | Cl | $CH_3$ | 1 | 4-$CH_3$ | $CO_2CH_3$ |
| 4-$CH_3$ | F | Cl | $CH_3$ | 1 | 4-$CH_3$ | $CO_2CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4-$CH_3$ | $CO_2CH_2CH_3$ |
| 4-$CH_3$ | H | F | H | 1 | 4-$CH_3$ | $CO_2CH_2CH_3$ |
| 3-$CH_3$ | F | F | H | 1 | 4-$CH_3$ | $CO_2CH_2CH_3$ |
| 4-$CH_3$ | F | F | H | 1 | 4-$CH_3$ | $CO_2CH_2CH_3$ |
| 3-$CH_3$ | H | Cl | H | 1 | 4-$CH_3$ | $CO_2CH_2CH_3$ |
| 4-$CH_3$ | H | Cl | H | 1 | 4-$CH_3$ | $CO_2CH_2CH_3$ |
| 3-$CH_3$ | F | Cl | H | 1 | 4-$CH_3$ | $CO_2CH_2CH_3$ |
| 4-$CH_3$ | F | Cl | H | 1 | 4-$CH_3$ | $CO_2CH_2CH_3$ |
| 3-$CH_3$ | H | F | $CH_3$ | 1 | 4-$CH_3$ | $CO_2CH_2CH_3$ |
| 4-$CH_3$ | H | F | $CH_3$ | 1 | 4-$CH_3$ | $CO_2CH_2CH_3$ |
| 3-$CH_3$ | F | F | $CH_3$ | 1 | 4-$CH_3$ | $CO_2CH_2CH_3$ |
| 4-$CH_3$ | F | F | $CH_3$ | 1 | 4-$CH_3$ | $CO_2CH_2CH_3$ |
| 3-$CH_3$ | H | Cl | $CH_3$ | 1 | 4-$CH_3$ | $CO_2CH_2CH_3$ |
| 4-$CH_3$ | H | Cl | $CH_3$ | 1 | 4-$CH_3$ | $CO_2CH_2CH_3$ |
| 3-$CH_3$ | F | Cl | $CH_3$ | 1 | 4-$CH_3$ | $CO_2CH_2CH_3$ |
| 4-$CH_3$ | F | Cl | $CH_3$ | 1 | 4-$CH_3$ | $CO_2CH_2CH_3$ |
| 3-$CH_3$ | H | F | H | 1 | 4-$CH_3$ | $CO_2(CH_2)_2CH_3$ |
| 4-$CH_3$ | H | F | H | 1 | 4-$CH_3$ | $CO_2(CH_2)_2CH_3$ |
| 3-$CH_3$ | F | F | H | 1 | 4-$CH_3$ | $CO_2(CH_2)_2CH_3$ |
| 4-$CH_3$ | F | F | H | 1 | 4-$CH_3$ | $CO_2(CH_2)_2CH_3$ |
| 3-$CH_3$ | H | Cl | H | 1 | 4-$CH_3$ | $CO_2(CH_2)_2CH_3$ |
| 4-$CH_3$ | H | Cl | H | 1 | 4-$CH_3$ | $CO_2(CH_2)_2CH_3$ |
| 3-$CH_3$ | F | Cl | H | 1 | 4-$CH_3$ | $CO_2(CH_2)_2CH_3$ |
| 4-$CH_3$ | F | Cl | H | 1 | 4-$CH_3$ | $CO_2(CH_2)_2CH_3$ |
| 3-$CH_3$ | H | F | $CH_3$ | 1 | 4-$CH_3$ | $CO_2(CH_2)_2CH_3$ |
| 4-$CH_3$ | H | F | $CH_3$ | 1 | 4-$CH_3$ | $CO_2(CH_2)_2CH_3$ |
| 3-$CH_3$ | F | F | $CH_3$ | 1 | 4-$CH_3$ | $CO_2(CH_2)_2CH_3$ |
| 4-$CH_3$ | F | F | $CH_3$ | 1 | 4-$CH_3$ | $CO_2(CH_2)_2CH_3$ |
| 3-$CH_3$ | H | Cl | $CH_3$ | 1 | 4-$CH_3$ | $CO_2(CH_2)_2CH_3$ |
| 4-$CH_3$ | H | Cl | $CH_3$ | 1 | 4-$CH_3$ | $CO_2(CH_2)_2CH_3$ |
| 3-$CH_3$ | F | Cl | $CH_3$ | 1 | 4-$CH_3$ | $CO_2(CH_2)_2CH_3$ |
| 4-$CH_3$ | F | Cl | $CH_3$ | 1 | 4-$CH_3$ | $CO_2(CH_2)_2CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4-$CH_3$ | $CO_2CH(CH_3)_2$ |
| 4-$CH_3$ | H | F | H | 1 | 4-$CH_3$ | $CO_2CH(CH_3)_2$ |
| 3-$CH_3$ | F | F | H | 1 | 4-$CH_3$ | $CO_2CH(CH_3)_2$ |
| 4-$CH_3$ | F | F | H | 1 | 4-$CH_3$ | $CO_2CH(CH_3)_2$ |
| 3-$CH_3$ | H | Cl | H | 1 | 4-$CH_3$ | $CO_2CH(CH_3)_2$ |
| 4-$CH_3$ | H | Cl | H | 1 | 4-$CH_3$ | $CO_2CH(CH_3)_2$ |
| 3-$CH_3$ | F | Cl | H | 1 | 4-$CH_3$ | $CO_2CH(CH_3)_2$ |
| 4-$CH_3$ | F | Cl | H | 1 | 4-$CH_3$ | $CO_2CH(CH_3)_2$ |
| 3-$CH_3$ | H | F | $CH_3$ | 1 | 4-$CH_3$ | $CO_2CH(CH_3)_2$ |
| 4-$CH_3$ | H | F | $CH_3$ | 1 | 4-$CH_3$ | $CO_2CH(CH_3)_2$ |
| 3-$CH_3$ | F | F | $CH_3$ | 1 | 4-$CH_3$ | $CO_2CH(CH_3)_2$ |
| 4-$CH_3$ | F | F | $CH_3$ | 1 | 4-$CH_3$ | $CO_2CH(CH_3)_2$ |
| 3-$CH_3$ | H | Cl | $CH_3$ | 1 | 4-$CH_3$ | $CO_2CH(CH_3)_2$ |
| 4-$CH_3$ | H | Cl | $CH_3$ | 1 | 4-$CH_3$ | $CO_2CH(CH_3)_2$ |
| 3-$CH_3$ | F | Cl | $CH_3$ | 1 | 4-$CH_3$ | $CO_2CH(CH_3)_2$ |
| 4-$CH_3$ | F | Cl | $CH_3$ | 1 | 4-$CH_3$ | $CO_2CH(CH_3)_2$ |
| 3-$CH_3$ | H | F | H | 1 | 4-$CH_3$ | $CO_2(CH_2)_3CH_3$ |
| 4-$CH_3$ | H | F | H | 1 | 4-$CH_3$ | $CO_2(CH_2)_3CH_3$ |
| 3-$CH_3$ | F | F | H | 1 | 4-$CH_3$ | $CO_2(CH_2)_3CH_3$ |
| 4-$CH_3$ | F | F | H | 1 | 4-$CH_3$ | $CO_2(CH_2)_3CH_3$ |
| 3-$CH_3$ | H | Cl | H | 1 | 4-$CH_3$ | $CO_2(CH_2)_3CH_3$ |
| 4-$CH_3$ | H | Cl | H | 1 | 4-$CH_3$ | $CO_2(CH_2)_3CH_3$ |
| 3-$CH_3$ | F | Cl | H | 1 | 4-$CH_3$ | $CO_2(CH_2)_3CH_3$ |
| 4-$CH_3$ | F | Cl | H | 1 | 4-$CH_3$ | $CO_2(CH_2)_3CH_3$ |
| 3-$CH_3$ | H | F | $CH_3$ | 1 | 4-$CH_3$ | $CO_2(CH_2)_3CH_3$ |
| 4-$CH_3$ | H | F | $CH_3$ | 1 | 4-$CH_3$ | $CO_2(CH_2)_3CH_3$ |
| 3-$CH_3$ | F | F | $CH_3$ | 1 | 4-$CH_3$ | $CO_2(CH_2)_3CH_3$ |
| 4-$CH_3$ | F | F | $CH_3$ | 1 | 4-$CH_3$ | $CO_2(CH_2)_3CH_3$ |
| 3-$CH_3$ | H | Cl | $CH_3$ | 1 | 4-$CH_3$ | $CO_2(CH_2)_3CH_3$ |
| 4-$CH_3$ | H | Cl | $CH_3$ | 1 | 4-$CH_3$ | $CO_2(CH_2)_3CH_3$ |
| 3-$CH_3$ | F | Cl | $CH_3$ | 1 | 4-$CH_3$ | $CO_2(CH_2)_3CH_3$ |
| 4-$CH_3$ | F | Cl | $CH_3$ | 1 | 4-$CH_3$ | $CO_2(CH_2)_3CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4-$CH_3$ | $CO_2(CH_2)_2OCH_3$ |
| 4-$CH_3$ | H | F | H | 1 | 4-$CH_3$ | $CO_2(CH_2)_2OCH_3$ |
| 3-$CH_3$ | F | F | H | 1 | 4-$CH_3$ | $CO_2(CH_2)_2OCH_3$ |
| 4-$CH_3$ | F | F | H | 1 | 4-$CH_3$ | $CO_2(CH_2)_2OCH_3$ |
| 3-$CH_3$ | H | Cl | H | 1 | 4-$CH_3$ | $CO_2(CH_2)_2OCH_3$ |
| 4-$CH_3$ | H | Cl | H | 1 | 4-$CH_3$ | $CO_2(CH_2)_2OCH_3$ |
| 3-$CH_3$ | F | Cl | H | 1 | 4-$CH_3$ | $CO_2(CH_2)_2OCH_3$ |
| 4-$CH_3$ | F | Cl | H | 1 | 4-$CH_3$ | $CO_2(CH_2)_2OCH_3$ |
| 3-$CH_3$ | H | F | $CH_3$ | 1 | 4-$CH_3$ | $CO_2(CH_2)_2OCH_3$ |
| 4-$CH_3$ | H | F | $CH_3$ | 1 | 4-$CH_3$ | $CO_2(CH_2)_2OCH_3$ |
| 3-$CH_3$ | F | F | $CH_3$ | 1 | 4-$CH_3$ | $CO_2(CH_2)_2OCH_3$ |
| 4-$CH_3$ | F | F | $CH_3$ | 1 | 4-$CH_3$ | $CO_2(CH_2)_2OCH_3$ |
| 3-$CH_3$ | H | Cl | $CH_3$ | 1 | 4-$CH_3$ | $CO_2(CH_2)_2OCH_3$ |
| 4-$CH_3$ | H | Cl | $CH_3$ | 1 | 4-$CH_3$ | $CO_2(CH_2)_2OCH_3$ |
| 3-$CH_3$ | F | Cl | $CH_3$ | 1 | 4-$CH_3$ | $CO_2(CH_2)_2OCH_3$ |
| 4-$CH_3$ | F | Cl | $CH_3$ | 1 | 4-$CH_3$ | $CO_2(CH_2)_2OCH_3$ |
| 3-$CH_3$ | H | F | H | 1 | 4-$CH_3$ | $CO_2(CH_2)_2OCH_2CH_3$ |
| 4-$CH_3$ | H | F | H | 1 | 4-$CH_3$ | $CO_2(CH_2)_2OCH_2CH_3$ |
| 3-$CH_3$ | F | F | H | 1 | 4-$CH_3$ | $CO_2(CH_2)_2OCH_2CH_3$ |
| 4-$CH_3$ | F | F | H | 1 | 4-$CH_3$ | $CO_2(CH_2)_2OCH_2CH_3$ |
| 3-$CH_3$ | H | Cl | H | 1 | 4-$CH_3$ | $CO_2(CH_2)_2OCH_2CH_3$ |
| 4-$CH_3$ | H | Cl | H | 1 | 4-$CH_3$ | $CO_2(CH_2)_2OCH_2CH_3$ |
| 3-$CH_3$ | F | Cl | H | 1 | 4-$CH_3$ | $CO_2(CH_2)_2OCH_2CH_3$ |
| 4-$CH_3$ | F | Cl | H | 1 | 4-$CH_3$ | $CO_2(CH_2)_2OCH_2CH_3$ |
| 3-$CH_3$ | H | F | $CH_3$ | 1 | 4-$CH_3$ | $CO_2(CH_2)_2OCH_2CH_3$ |
| 4-$CH_3$ | H | F | $CH_3$ | 1 | 4-$CH_3$ | $CO_2(CH_2)_2OCH_2CH_3$ |
| 3-$CH_3$ | F | F | $CH_3$ | 1 | 4-$CH_3$ | $CO_2(CH_2)_2OCH_2CH_3$ |
| 4-$CH_3$ | F | F | $CH_3$ | 1 | 4-$CH_3$ | $CO_2(CH_2)_2OCH_2CH_3$ |
| 3-$CH_3$ | H | Cl | $CH_3$ | 1 | 4-$CH_3$ | $CO_2(CH_2)_2OCH_2CH_3$ |
| 4-$CH_3$ | H | Cl | $CH_3$ | 1 | 4-$CH_3$ | $CO_2(CH_2)_2OCH_2CH_3$ |
| 3-$CH_3$ | F | Cl | $CH_3$ | 1 | 4-$CH_3$ | $CO_2(CH_2)_2OCH_2CH_3$ |
| 4-$CH_3$ | F | Cl | $CH_3$ | 1 | 4-$CH_3$ | $CO_2(CH_2)_2OCH_2CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 6-$CH_3$ | $CO_2CH_3$ |
| 4-$CH_3$ | H | F | H | 1 | 6-$CH_3$ | $CO_2CH_3$ |
| 3-$CH_3$ | F | F | H | 1 | 6-$CH_3$ | $CO_2CH_3$ |
| 4-$CH_3$ | F | F | H | 1 | 6-$CH_3$ | $CO_2CH_3$ |
| 3-$CH_3$ | H | Cl | H | 1 | 6-$CH_3$ | $CO_2CH_3$ |
| 4-$CH_3$ | H | Cl | H | 1 | 6-$CH_3$ | $CO_2CH_3$ |
| 3-$CH_3$ | F | Cl | H | 1 | 6-$CH_3$ | $CO_2CH_3$ |
| 4-$CH_3$ | F | Cl | H | 1 | 6-$CH_3$ | $CO_2CH_3$ |
| 3-$CH_3$ | H | F | $CH_3$ | 1 | 6-$CH_3$ | $CO_2CH_3$ |
| 4-$CH_3$ | H | F | $CH_3$ | 1 | 6-$CH_3$ | $CO_2CH_3$ |
| 3-$CH_3$ | F | F | $CH_3$ | 1 | 6-$CH_3$ | $CO_2CH_3$ |
| 4-$CH_3$ | F | F | $CH_3$ | 1 | 6-$CH_3$ | $CO_2CH_3$ |
| 3-$CH_3$ | H | Cl | $CH_3$ | 1 | 6-$CH_3$ | $CO_2CH_3$ |
| 4-$CH_3$ | H | Cl | $CH_3$ | 1 | 6-$CH_3$ | $CO_2CH_3$ |
| 3-$CH_3$ | F | Cl | $CH_3$ | 1 | 6-$CH_3$ | $CO_2CH_3$ |
| 4-$CH_3$ | F | Cl | $CH_3$ | 1 | 6-$CH_3$ | $CO_2CH_3$ |
| 3-$CH_3$ | H | F | H | 1 | 6-$CH_3$ | $CO_2CH_2CH_3$ |
| 4-$CH_3$ | H | F | H | 1 | 6-$CH_3$ | $CO_2CH_2CH_3$ |
| 3-$CH_3$ | F | F | H | 1 | 6-$CH_3$ | $CO_2CH_2CH_3$ |
| 4-$CH_3$ | F | F | H | 1 | 6-$CH_3$ | $CO_2CH_2CH_3$ |
| 3-$CH_3$ | H | Cl | H | 1 | 6-$CH_3$ | $CO_2CH_2CH_3$ |
| 4-$CH_3$ | H | Cl | H | 1 | 6-$CH_3$ | $CO_2CH_2CH_3$ |
| 3-$CH_3$ | F | Cl | H | 1 | 6-$CH_3$ | $CO_2CH_2CH_3$ |
| 4-$CH_3$ | F | Cl | H | 1 | 6-$CH_3$ | $CO_2CH_2CH_3$ |
| 3-$CH_3$ | H | F | $CH_3$ | 1 | 6-$CH_3$ | $CO_2CH_2CH_3$ |
| 4-$CH_3$ | H | F | $CH_3$ | 1 | 6-$CH_3$ | $CO_2CH_2CH_3$ |
| 3-$CH_3$ | F | F | $CH_3$ | 1 | 6-$CH_3$ | $CO_2CH_2CH_3$ |
| 4-$CH_3$ | F | F | $CH_3$ | 1 | 6-$CH_3$ | $CO_2CH_2CH_3$ |
| 3-$CH_3$ | H | Cl | $CH_3$ | 1 | 6-$CH_3$ | $CO_2CH_2CH_3$ |
| 4-$CH_3$ | H | Cl | $CH_3$ | 1 | 6-$CH_3$ | $CO_2CH_2CH_3$ |
| 3-$CH_3$ | F | Cl | $CH_3$ | 1 | 6-$CH_3$ | $CO_2CH_2CH_3$ |
| 4-$CH_3$ | F | Cl | $CH_3$ | 1 | 6-$CH_3$ | $CO_2CH_2CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 6-$CH_3$ | $CO_2(CH_2)_2CH_3$ |
| 4-$CH_3$ | H | F | H | 1 | 6-$CH_3$ | $CO_2(CH_2)_2CH_3$ |
| 3-$CH_3$ | F | F | H | 1 | 6-$CH_3$ | $CO_2(CH_2)_2CH_3$ |
| 4-$CH_3$ | F | F | H | 1 | 6-$CH_3$ | $CO_2(CH_2)_2CH_3$ |
| 3-$CH_3$ | H | Cl | H | 1 | 6-$CH_3$ | $CO_2(CH_2)_2CH_3$ |
| 4-$CH_3$ | H | Cl | H | 1 | 6-$CH_3$ | $CO_2(CH_2)_2CH_3$ |
| 3-$CH_3$ | F | Cl | H | 1 | 6-$CH_3$ | $CO_2(CH_2)_2CH_3$ |
| 4-$CH_3$ | F | Cl | H | 1 | 6-$CH_3$ | $CO_2(CH_2)_2CH_3$ |
| 3-$CH_3$ | H | F | $CH_3$ | 1 | 6-$CH_3$ | $CO_2(CH_2)_2CH_3$ |
| 4-$CH_3$ | H | F | $CH_3$ | 1 | 6-$CH_3$ | $CO_2(CH_2)_2CH_3$ |
| 3-$CH_3$ | F | F | $CH_3$ | 1 | 6-$CH_3$ | $CO_2(CH_2)_2CH_3$ |
| 4-$CH_3$ | F | F | $CH_3$ | 1 | 6-$CH_3$ | $CO_2(CH_2)_2CH_3$ |
| 3-$CH_3$ | H | Cl | $CH_3$ | 1 | 6-$CH_3$ | $CO_2(CH_2)_2CH_3$ |
| 4-$CH_3$ | H | Cl | $CH_3$ | 1 | 6-$CH_3$ | $CO_2(CH_2)_2CH_3$ |
| 3-$CH_3$ | F | Cl | $CH_3$ | 1 | 6-$CH_3$ | $CO_2(CH_2)_2CH_3$ |
| 4-$CH_3$ | F | Cl | $CH_3$ | 1 | 6-$CH_3$ | $CO_2(CH_2)_2CH_3$ |
| 3-$CH_3$ | H | F | H | 1 | 6-$CH_3$ | $CO_2CH(CH_3)_2$ |
| 4-$CH_3$ | H | F | H | 1 | 6-$CH_3$ | $CO_2CH(CH_3)_2$ |
| 3-$CH_3$ | F | F | H | 1 | 6-$CH_3$ | $CO_2CH(CH_3)_2$ |
| 4-$CH_3$ | F | F | H | 1 | 6-$CH_3$ | $CO_2CH(CH_3)_2$ |
| 3-$CH_3$ | H | Cl | H | 1 | 6-$CH_3$ | $CO_2CH(CH_3)_2$ |
| 4-$CH_3$ | H | Cl | H | 1 | 6-$CH_3$ | $CO_2CH(CH_3)_2$ |
| 3-$CH_3$ | F | Cl | H | 1 | 6-$CH_3$ | $CO_2CH(CH_3)_2$ |
| 4-$CH_3$ | F | Cl | H | 1 | 6-$CH_3$ | $CO_2CH(CH_3)_2$ |
| 3-$CH_3$ | H | F | $CH_3$ | 1 | 6-$CH_3$ | $CO_2CH(CH_3)_2$ |
| 4-$CH_3$ | H | F | $CH_3$ | 1 | 6-$CH_3$ | $CO_2CH(CH_3)_2$ |
| 3-$CH_3$ | F | F | $CH_3$ | 1 | 6-$CH_3$ | $CO_2CH(CH_3)_2$ |
| 4-$CH_3$ | F | F | $CH_3$ | 1 | 6-$CH_3$ | $CO_2CH(CH_3)_2$ |
| 3-$CH_3$ | H | Cl | $CH_3$ | 1 | 6-$CH_3$ | $CO_2CH(CH_3)_2$ |
| 4-$CH_3$ | H | Cl | $CH_3$ | 1 | 6-$CH_3$ | $CO_2CH(CH_3)_2$ |
| 3-$CH_3$ | F | Cl | $CH_3$ | 1 | 6-$CH_3$ | $CO_2CH(CH_3)_2$ |
| 4-$CH_3$ | F | Cl | $CH_3$ | 1 | 6-$CH_3$ | $CO_2CH(CH_3)_2$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 6-$CH_3$ | $CO_2(CH_2)_3CH_3$ |
| 4-$CH_3$ | H | F | H | 1 | 6-$CH_3$ | $CO_2(CH_2)_3CH_3$ |
| 3-$CH_3$ | F | F | H | 1 | 6-$CH_3$ | $CO_2(CH_2)_3CH_3$ |
| 4-$CH_3$ | F | F | H | 1 | 6-$CH_3$ | $CO_2(CH_2)_3CH_3$ |
| 3-$CH_3$ | H | Cl | H | 1 | 6-$CH_3$ | $CO_2(CH_2)_3CH_3$ |
| 4-$CH_3$ | H | Cl | H | 1 | 6-$CH_3$ | $CO_2(CH_2)_3CH_3$ |
| 3-$CH_3$ | F | Cl | H | 1 | 6-$CH_3$ | $CO_2(CH_2)_3CH_3$ |
| 4-$CH_3$ | F | Cl | H | 1 | 6-$CH_3$ | $CO_2(CH_2)_3CH_3$ |
| 3-$CH_3$ | H | F | $CH_3$ | 1 | 6-$CH_3$ | $CO_2(CH_2)_3CH_3$ |
| 4-$CH_3$ | H | F | $CH_3$ | 1 | 6-$CH_3$ | $CO_2(CH_2)_3CH_3$ |
| 3-$CH_3$ | F | F | $CH_3$ | 1 | 6-$CH_3$ | $CO_2(CH_2)_3CH_3$ |
| 4-$CH_3$ | F | F | $CH_3$ | 1 | 6-$CH_3$ | $CO_2(CH_2)_3CH_3$ |
| 3-$CH_3$ | H | Cl | $CH_3$ | 1 | 6-$CH_3$ | $CO_2(CH_2)_3CH_3$ |
| 4-$CH_3$ | H | Cl | $CH_3$ | 1 | 6-$CH_3$ | $CO_2(CH_2)_3CH_3$ |
| 3-$CH_3$ | F | Cl | $CH_3$ | 1 | 6-$CH_3$ | $CO_2(CH_2)_3CH_3$ |
| 4-$CH_3$ | F | Cl | $CH_3$ | 1 | 6-$CH_3$ | $CO_2(CH_2)_3CH_3$ |
| 3-$CH_3$ | H | F | H | 1 | 6-$CH_3$ | $CO_2(CH_2)_2OCH_3$ |
| 4-$CH_3$ | H | F | H | 1 | 6-$CH_3$ | $CO_2(CH_2)_2OCH_3$ |
| 3-$CH_3$ | F | F | H | 1 | 6-$CH_3$ | $CO_2(CH_2)_2OCH_3$ |
| 4-$CH_3$ | F | F | H | 1 | 6-$CH_3$ | $CO_2(CH_2)_2OCH_3$ |
| 3-$CH_3$ | H | Cl | H | 1 | 6-$CH_3$ | $CO_2(CH_2)_2OCH_3$ |
| 4-$CH_3$ | H | Cl | H | 1 | 6-$CH_3$ | $CO_2(CH_2)_2OCH_3$ |
| 3-$CH_3$ | F | Cl | H | 1 | 6-$CH_3$ | $CO_2(CH_2)_2OCH_3$ |
| 4-$CH_3$ | F | Cl | H | 1 | 6-$CH_3$ | $CO_2(CH_2)_2OCH_3$ |
| 3-$CH_3$ | H | F | $CH_3$ | 1 | 6-$CH_3$ | $CO_2(CH_2)_2OCH_3$ |
| 4-$CH_3$ | H | F | $CH_3$ | 1 | 6-$CH_3$ | $CO_2(CH_2)_2OCH_3$ |
| 3-$CH_3$ | F | F | $CH_3$ | 1 | 6-$CH_3$ | $CO_2(CH_2)_2OCH_3$ |
| 4-$CH_3$ | F | F | $CH_3$ | 1 | 6-$CH_3$ | $CO_2(CH_2)_2OCH_3$ |
| 3-$CH_3$ | H | Cl | $CH_3$ | 1 | 6-$CH_3$ | $CO_2(CH_2)_2OCH_3$ |
| 4-$CH_3$ | H | Cl | $CH_3$ | 1 | 6-$CH_3$ | $CO_2(CH_2)_2OCH_3$ |
| 3-$CH_3$ | F | Cl | $CH_3$ | 1 | 6-$CH_3$ | $CO_2(CH_2)_2OCH_3$ |
| 4-$CH_3$ | F | Cl | $CH_3$ | 1 | 6-$CH_3$ | $CO_2(CH_2)_2OCH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 6-$CH_3$ | $CO_2(CH_2)_2OCH_2CH_3$ |
| 4-$CH_3$ | H | F | H | 1 | 6-$CH_3$ | $CO_2(CH_2)_2OCH_2CH_3$ |
| 3-$CH_3$ | F | F | H | 1 | 6-$CH_3$ | $CO_2(CH_2)_2OCH_2CH_3$ |
| 4-$CH_3$ | F | F | H | 1 | 6-$CH_3$ | $CO_2(CH_2)_2OCH_2CH_3$ |
| 3-$CH_3$ | H | Cl | H | 1 | 6-$CH_3$ | $CO_2(CH_2)_2OCH_2CH_3$ |
| 4-$CH_3$ | H | Cl | H | 1 | 6-$CH_3$ | $CO_2(CH_2)_2OCH_2CH_3$ |
| 3-$CH_3$ | F | Cl | H | 1 | 6-$CH_3$ | $CO_2(CH_2)_2OCH_2CH_3$ |
| 4-$CH_3$ | F | Cl | H | 1 | 6-$CH_3$ | $CO_2(CH_2)_2OCH_2CH_3$ |
| 3-$CH_3$ | H | F | $CH_3$ | 1 | 6-$CH_3$ | $CO_2(CH_2)_2OCH_2CH_3$ |
| 4-$CH_3$ | H | F | $CH_3$ | 1 | 6-$CH_3$ | $CO_2(CH_2)_2OCH_2CH_3$ |
| 3-$CH_3$ | F | F | $CH_3$ | 1 | 6-$CH_3$ | $CO_2(CH_2)_2OCH_2CH_3$ |
| 4-$CH_3$ | F | F | $CH_3$ | 1 | 6-$CH_3$ | $CO_2(CH_2)_2OCH_2CH_3$ |
| 3-$CH_3$ | H | Cl | $CH_3$ | 1 | 6-$CH_3$ | $CO_2(CH_2)_2OCH_2CH_3$ |
| 4-$CH_3$ | H | Cl | $CH_3$ | 1 | 6-$CH_3$ | $CO_2(CH_2)_2OCH_2CH_3$ |
| 3-$CH_3$ | F | Cl | $CH_3$ | 1 | 6-$CH_3$ | $CO_2(CH_2)_2OCH_2CH_3$ |
| 4-$CH_3$ | F | Cl | $CH_3$ | 1 | 6-$CH_3$ | $CO_2(CH_2)_2OCH_2CH_3$ |
| 3-$CH_3$ | H | F | H | 1 | 4,6-Di-$CH_3$ | $CO_2CH_3$ |
| 4-$CH_3$ | H | F | H | 1 | 4,6-Di-$CH_3$ | $CO_2CH_3$ |
| 3-$CH_3$ | F | F | H | 1 | 4,6-Di-$CH_3$ | $CO_2CH_3$ |
| 4-$CH_3$ | F | F | H | 1 | 4,6-Di-$CH_3$ | $CO_2CH_3$ |
| 3-$CH_3$ | H | Cl | H | 1 | 4,6-Di-$CH_3$ | $CO_2CH_3$ |
| 4-$CH_3$ | H | Cl | H | 1 | 4,6-Di-$CH_3$ | $CO_2CH_3$ |
| 3-$CH_3$ | F | Cl | H | 1 | 4,6-Di-$CH_3$ | $CO_2CH_3$ |
| 4-$CH_3$ | F | Cl | H | 1 | 4,6-Di-$CH_3$ | $CO_2CH_3$ |
| 3-$CH_3$ | H | F | $CH_3$ | 1 | 4,6-Di-$CH_3$ | $CO_2CH_3$ |
| 4-$CH_3$ | H | F | $CH_3$ | 1 | 4,6-Di-$CH_3$ | $CO_2CH_3$ |
| 3-$CH_3$ | F | F | $CH_3$ | 1 | 4,6-Di-$CH_3$ | $CO_2CH_3$ |
| 4-$CH_3$ | F | F | $CH_3$ | 1 | 4,6-Di-$CH_3$ | $CO_2CH_3$ |
| 3-$CH_3$ | H | Cl | $CH_3$ | 1 | 4,6-Di-$CH_3$ | $CO_2CH_3$ |
| 4-$CH_3$ | H | Cl | $CH_3$ | 1 | 4,6-Di-$CH_3$ | $CO_2CH_3$ |
| 3-$CH_3$ | F | Cl | $CH_3$ | 1 | 4,6-Di-$CH_3$ | $CO_2CH_3$ |
| 4-$CH_3$ | F | Cl | $CH_3$ | 1 | 4,6-Di-$CH_3$ | $CO_2CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4,6-Di-$CH_3$ | $CO_2CH_2CH_3$ |
| 4-$CH_3$ | H | F | H | 1 | 4,6-Di-$CH_3$ | $CO_2CH_2CH_3$ |
| 3-$CH_3$ | F | F | H | 1 | 4,6-Di-$CH_3$ | $CO_2CH_2CH_3$ |
| 4-$CH_3$ | F | F | H | 1 | 4,6-Di-$CH_3$ | $CO_2CH_2CH_3$ |
| 3-$CH_3$ | H | Cl | H | 1 | 4,6-Di-$CH_3$ | $CO_2CH_2CH_3$ |
| 4-$CH_3$ | H | Cl | H | 1 | 4,6-Di-$CH_3$ | $CO_2CH_2CH_3$ |
| 3-$CH_3$ | F | Cl | H | 1 | 4,6-Di-$CH_3$ | $CO_2CH_2CH_3$ |
| 4-$CH_3$ | F | Cl | H | 1 | 4,6-Di-$CH_3$ | $CO_2CH_2CH_3$ |
| 3-$CH_3$ | H | F | $CH_3$ | 1 | 4,6-Di-$CH_3$ | $CO_2CH_2CH_3$ |
| 4-$CH_3$ | H | F | $CH_3$ | 1 | 4,6-Di-$CH_3$ | $CO_2CH_2CH_3$ |
| 3-$CH_3$ | F | F | $CH_3$ | 1 | 4,6-Di-$CH_3$ | $CO_2CH_2CH_3$ |
| 4-$CH_3$ | F | F | $CH_3$ | 1 | 4,6-Di-$CH_3$ | $CO_2CH_2CH_3$ |
| 3-$CH_3$ | H | Cl | $CH_3$ | 1 | 4,6-Di-$CH_3$ | $CO_2CH_2CH_3$ |
| 4-$CH_3$ | H | Cl | $CH_3$ | 1 | 4,6-Di-$CH_3$ | $CO_2CH_2CH_3$ |
| 3-$CH_3$ | F | Cl | $CH_3$ | 1 | 4,6-Di-$CH_3$ | $CO_2CH_2CH_3$ |
| 4-$CH_3$ | F | Cl | $CH_3$ | 1 | 4,6-Di-$CH_3$ | $CO_2CH_2CH_3$ |
| 3-$CH_3$ | H | F | H | 1 | 4,6-Di-$CH_3$ | $CO_2(CH_2)_2CH_3$ |
| 4-$CH_3$ | H | F | H | 1 | 4,6-Di-$CH_3$ | $CO_2(CH_2)_2CH_3$ |
| 3-$CH_3$ | F | F | H | 1 | 4,6-Di-$CH_3$ | $CO_2(CH_2)_2CH_3$ |
| 4-$CH_3$ | F | F | H | 1 | 4,6-Di-$CH_3$ | $CO_2(CH_2)_2CH_3$ |
| 3-$CH_3$ | H | Cl | H | 1 | 4,6-Di-$CH_3$ | $CO_2(CH_2)_2CH_3$ |
| 4-$CH_3$ | H | Cl | H | 1 | 4,6-Di-$CH_3$ | $CO_2(CH_2)_2CH_3$ |
| 3-$CH_3$ | F | Cl | H | 1 | 4,6-Di-$CH_3$ | $CO_2(CH_2)_2CH_3$ |
| 4-$CH_3$ | F | Cl | H | 1 | 4,6-Di-$CH_3$ | $CO_2(CH_2)_2CH_3$ |
| 3-$CH_3$ | H | F | $CH_3$ | 1 | 4,6-Di-$CH_3$ | $CO_2(CH_2)_2CH_3$ |
| 4-$CH_3$ | H | F | $CH_3$ | 1 | 4,6-Di-$CH_3$ | $CO_2(CH_2)_2CH_3$ |
| 3-$CH_3$ | F | F | $CH_3$ | 1 | 4,6-Di-$CH_3$ | $CO_2(CH_2)_2CH_3$ |
| 4-$CH_3$ | F | F | $CH_3$ | 1 | 4,6-Di-$CH_3$ | $CO_2(CH_2)_2CH_3$ |
| 3-$CH_3$ | H | Cl | $CH_3$ | 1 | 4,6-Di-$CH_3$ | $CO_2(CH_2)_2CH_3$ |
| 4-$CH_3$ | H | Cl | $CH_3$ | 1 | 4,6-Di-$CH_3$ | $CO_2(CH_2)_2CH_3$ |
| 3-$CH_3$ | F | Cl | $CH_3$ | 1 | 4,6-Di-$CH_3$ | $CO_2(CH_2)_2CH_3$ |
| 4-$CH_3$ | F | Cl | $CH_3$ | 1 | 4,6-Di-$CH_3$ | $CO_2(CH_2)_2CH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 3-$CH_3$ | H | F | H | 1 | 4,6-Di-$CH_3$ | $CO_2CH(CH_3)_2$ |
| 4-$CH_3$ | H | F | H | 1 | 4,6-Di-$CH_3$ | $CO_2CH(CH_3)_2$ |
| 3-$CH_3$ | F | F | H | 1 | 4,6-Di-$CH_3$ | $CO_2CH(CH_3)_2$ |
| 4-$CH_3$ | F | F | H | 1 | 4,6-Di-$CH_3$ | $CO_2CH(CH_3)_2$ |
| 3-$CH_3$ | H | Cl | H | 1 | 4,6-Di-$CH_3$ | $CO_2CH(CH_3)_2$ |
| 4-$CH_3$ | H | Cl | H | 1 | 4,6-Di-$CH_3$ | $CO_2CH(CH_3)_2$ |
| 3-$CH_3$ | F | Cl | H | 1 | 4,6-Di-$CH_3$ | $CO_2CH(CH_3)_2$ |
| 4-$CH_3$ | F | Cl | H | 1 | 4,6-Di-$CH_3$ | $CO_2CH(CH_3)_2$ |
| 3-$CH_3$ | H | F | $CH_3$ | 1 | 4,6-Di-$CH_3$ | $CO_2CH(CH_3)_2$ |
| 4-$CH_3$ | H | F | $CH_3$ | 1 | 4,6-Di-$CH_3$ | $CO_2CH(CH_3)_2$ |
| 3-$CH_3$ | F | F | $CH_3$ | 1 | 4,6-Di-$CH_3$ | $CO_2CH(CH_3)_2$ |
| 4-$CH_3$ | F | F | $CH_3$ | 1 | 4,6-Di-$CH_3$ | $CO_2CH(CH_3)_2$ |
| 3-$CH_3$ | H | Cl | $CH_3$ | 1 | 4,6-Di-$CH_3$ | $CO_2CH(CH_3)_2$ |
| 4-$CH_3$ | H | Cl | $CH_3$ | 1 | 4,6-Di-$CH_3$ | $CO_2CH(CH_3)_2$ |
| 3-$CH_3$ | F | Cl | $CH_3$ | 1 | 4,6-Di-$CH_3$ | $CO_2CH(CH_3)_2$ |
| 4-$CH_3$ | F | Cl | $CH_3$ | 1 | 4,6-Di-$CH_3$ | $CO_2CH(CH_3)_2$ |
| 3-$CH_3$ | H | F | H | 1 | 4,6-Di-$CH_3$ | $CO_2(CH_2)_3CH_3$ |
| 4-$CH_3$ | H | F | H | 1 | 4,6-Di-$CH_3$ | $CO_2(CH_2)_3CH_3$ |
| 3-$CH_3$ | F | F | H | 1 | 4,6-Di-$CH_3$ | $CO_2(CH_2)_3CH_3$ |
| 4-$CH_3$ | F | F | H | 1 | 4,6-Di-$CH_3$ | $CO_2(CH_2)_3CH_3$ |
| 3-$CH_3$ | H | Cl | H | 1 | 4,6-Di-$CH_3$ | $CO_2(CH_2)_3CH_3$ |
| 4-$CH_3$ | H | Cl | H | 1 | 4,6-Di-$CH_3$ | $CO_2(CH_2)_3CH_3$ |
| 3-$CH_3$ | F | Cl | H | 1 | 4,6-Di-$CH_3$ | $CO_2(CH_2)_3CH_3$ |
| 4-$CH_3$ | F | Cl | H | 1 | 4,6-Di-$CH_3$ | $CO_2(CH_2)_3CH_3$ |
| 3-$CH_3$ | H | F | $CH_3$ | 1 | 4,6-Di-$CH_3$ | $CO_2(CH_2)_3CH_3$ |
| 4-$CH_3$ | H | F | $CH_3$ | 1 | 4,6-Di-$CH_3$ | $CO_2(CH_2)_3CH_3$ |
| 3-$CH_3$ | F | F | $CH_3$ | 1 | 4,6-Di-$CH_3$ | $CO_2(CH_2)_3CH_3$ |
| 4-$CH_3$ | F | F | $CH_3$ | 1 | 4,6-Di-$CH_3$ | $CO_2(CH_2)_3CH_3$ |
| 3-$CH_3$ | H | Cl | $CH_3$ | 1 | 4,6-Di-$CH_3$ | $CO_2(CH_2)_3CH_3$ |
| 4-$CH_3$ | H | Cl | $CH_3$ | 1 | 4,6-Di-$CH_3$ | $CO_2(CH_2)_3CH_3$ |
| 3-$CH_3$ | F | Cl | $CH_3$ | 1 | 4,6-Di-$CH_3$ | $CO_2(CH_2)_3CH_3$ |
| 4-$CH_3$ | F | Cl | $CH_3$ | 1 | 4,6-Di-$CH_3$ | $CO_2(CH_2)_3CH_3$ |
| 3-$CH_3$ | H | F | H | 1 | 4,6-Di-$CH_3$ | $CO_2(CH_2)_2OCH_3$ |

Tabelle C (Fortsetzung)

| $R_n$ | $R^1$ | $R^2$ | $R^3$ | m | $(R^a)_q$ | $R^4$ |
|---|---|---|---|---|---|---|
| 4-$CH_3$ | H | F | H | 1 | 4,6-Di-$CH_3$ | $CO_2(CH_2)_2OCH_3$ |
| 3-$CH_3$ | F | F | H | 1 | 4,6-Di-$CH_3$ | $CO_2(CH_2)_2OCH_3$ |
| 4-$CH_3$ | F | F | H | 1 | 4,6-Di-$CH_3$ | $CO_2(CH_2)_2OCH_3$ |
| 3-$CH_3$ | H | Cl | H | 1 | 4,6-Di-$CH_3$ | $CO_2(CH_2)_2OCH_3$ |
| 4-$CH_3$ | H | Cl | H | 1 | 4,6-Di-$CH_3$ | $CO_2(CH_2)_2OCH_3$ |
| 3-$CH_3$ | F | Cl | H | 1 | 4,6-Di-$CH_3$ | $CO_2(CH_2)_2OCH_3$ |
| 4-$CH_3$ | F | Cl | H | 1 | 4,6-Di-$CH_3$ | $CO_2(CH_2)_2OCH_3$ |
| 3-$CH_3$ | H | F | $CH_3$ | 1 | 4,6-Di-$CH_3$ | $CO_2(CH_2)_2OCH_3$ |
| 4-$CH_3$ | H | F | $CH_3$ | 1 | 4,6-Di-$CH_3$ | $CO_2(CH_2)_2OCH_3$ |
| 3-$CH_3$ | F | F | $CH_3$ | 1 | 4,6-Di-$CH_3$ | $CO_2(CH_2)_2OCH_3$ |
| 4-$CH_3$ | F | F | $CH_3$ | 1 | 4,6-Di-$CH_3$ | $CO_2(CH_2)_2OCH_3$ |
| 3-$CH_3$ | H | Cl | $CH_3$ | 1 | 4,6-Di-$CH_3$ | $CO_2(CH_2)_2OCH_3$ |
| 4-$CH_3$ | H | Cl | $CH_3$ | 1 | 4,6-Di-$CH_3$ | $CO_2(CH_2)_2OCH_3$ |
| 3-$CH_3$ | F | Cl | $CH_3$ | 1 | 4,6-Di-$CH_3$ | $CO_2(CH_2)_2OCH_3$ |
| 4-$CH_3$ | F | Cl | $CH_3$ | 1 | 4,6-Di-$CH_3$ | $CO_2(CH_2)_2OCH_3$ |
| 3-$CH_3$ | H | F | H | 1 | 4,6-Di-$CH_3$ | $CO_2(CH_2)_2OCH_2CH_3$ |
| 4-$CH_3$ | H | F | H | 1 | 4,6-Di-$CH_3$ | $CO_2(CH_2)_2OCH_2CH_3$ |
| 3-$CH_3$ | F | F | H | 1 | 4,6-Di-$CH_3$ | $CO_2(CH_2)_2OCH_2CH_3$ |
| 4-$CH_3$ | F | F | H | 1 | 6-$CH_3$ | $CO_2(CH_2)_2OCH_2CH_3$ |
| 3-$CH_3$ | H | Cl | H | 1 | 6-$CH_3$ | $CO_2(CH_2)_2OCH_2CH_3$ |
| 4-$CH_3$ | H | Cl | H | 1 | 6-$CH_3$ | $CO_2(CH_2)_2OCH_2CH_3$ |
| 3-$CH_3$ | F | Cl | H | 1 | 6-$CH_3$ | $CO_2(CH_2)_2OCH_2CH_3$ |
| 4-$CH_3$ | F | Cl | H | 1 | 6-$CH_3$ | $CO_2(CH_2)_2OCH_2CH_3$ |
| 3-$CH_3$ | H | F | $CH_3$ | 1 | 6-$CH_3$ | $CO_2(CH_2)_2OCH_2CH_3$ |
| 4-$CH_3$ | H | F | $CH_3$ | 1 | 6-$CH_3$ | $CO_2(CH_2)_2OCH_2CH_3$ |
| 3-$CH_3$ | F | F | $CH_3$ | 1 | 6-$CH_3$ | $CO_2(CH_2)_2OCH_2CH_3$ |
| 4-$CH_3$ | F | F | $CH_3$ | 1 | 6-$CH_3$ | $CO_2(CH_2)_2OCH_2CH_3$ |
| 3-$CH_3$ | H | Cl | $CH_3$ | 1 | 6-$CH_3$ | $CO_2(CH_2)_2OCH_2CH_3$ |
| 4-$CH_3$ | H | Cl | $CH_3$ | 1 | 6-$CH_3$ | $CO_2(CH_2)_2OCH_2CH_3$ |
| 3-$CH_3$ | F | Cl | $CH_3$ | 1 | 6-$CH_3$ | $CO_2(CH_2)_2OCH_2CH_3$ |
| 4-$CH_3$ | F | Cl | $CH_3$ | 1 | 6-$CH_3$ | $CO_2(CH_2)_2OCH_2CH_3$ |

Die N-Phenyltetrahydrophthalimide I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 1.001 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1.003 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.002 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1.002 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 2.001 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 1.001 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1.003 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 2.001 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

In Anbetracht der Vielseitigkeit der Applikationsmethoden können die erfindungsgemäßen Verbindungen bzw. sie enthaltende Mittel noch in einer weiteren zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |

| Botanischer Name | Deutscher Name |
|---|---|
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |

| Botanischer Name | Deutscher Name |
|---|---|
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die N-Phenyltetrahydrophthalimide I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die herbiziden Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt.

Beispiel 1

Herstellung von N-[4-chlorphenyl-3-(4-methyl-1,3-dithiolan-2-yl]-4-methyl-3,4,5,6-tetrahydrophthalimid

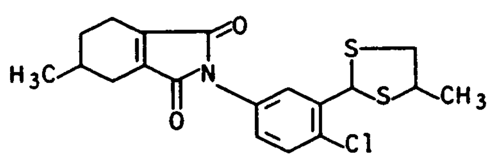

a) Zu 37,1 g (0,2 mol) 2-Chlor-5-nitro-benzaldehyd und 1 g p-Toluolsulfonsäure in 500 ml Toluol wurden 22,8 g (0,21 mol) 1,2-Propandithiol gegeben und 5 Stunden unter Rückfluß am Wasserabscheider gekocht. Nach dem Abkühlen wurde das Lösungsmittel entfernt, der Rückstand mit Perolether verrührt, abfiltriert und getrocknet. Man erhielt 51,8 g (94 %) 4-Chlor-3-(4-methyl-1,3-dithiolan-2-yl)-nitrobenzol (Fp.: 55 °C).

b) zu einer Mischung von 31,5 g (0,56 mol) Eisenpulver in 100 ml Methanol und 150 ml Eisessig wurden unter Rückfluß 51,5 g (0,19 mol) der obigen Nitroverbindung gegeben und 2 Stunden am Rückfluß erhitzt. Nach Einrühren in 400 ml Wasser wurde abgesaugt, das Filtrat mit Essigester extrahiert, die organische Phase mit Wasser gewaschen, getrocknet und eingeengt. Man erhielt 37 g (81 %) 4-Chlor-3-(4-methyl-1,3-dithiolan-2-yl)-anilin (Öl).

c) 4,9 g (0,02 mol) des obigen Anilins und 3,5 g (0,021 mol) 4-Methylcyclohexen-1,2-dicarbonsäureanhydrid wurden in 120 ml Eisessig 24 Stunden auf 60 °C erwärmt und einrotiert. Nach Umkristallisation aus

Methanol erhielt man 3,8 g (48 %) der Titelverbindung (Fp. 128-130 °C) (Bsp. 1.001).

Beispiel 2

Herstellung von N-[3-(5-Methyl-5-n-butyloxycarbonyl-1,3-dioxolan-2-yl)-4-chlor-phenyl]-4-methyl-3,4,5,6-tetrahydrophthalamid

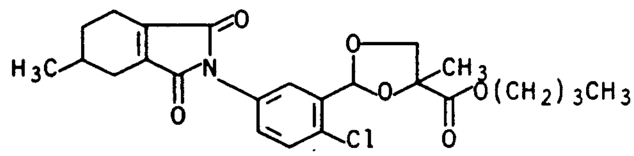

Zu 3,0 g (0,01 mol) N-4-Chlor-3-formylphenyl)-4-methyl-3,4,5,6-tetrahydrophthalimid und 0,1 g p-Toluolsulfonsäure in 200 ml Toluol wurden 1,9 g (0,01 mol) 2,3-Dioxyisobuttersäure-n-butylester gegeben und 5 Stunden unter Rückfluß am Wasserabscheider gekocht. Nach dem Abkühlen wurde mit Wasser gewaschen, getrocknet und eingeengt. Man erhielt 4,5 g (97 %) der Titelverbindung als Öl (Bsp. 2.001).

**Tabelle 1**

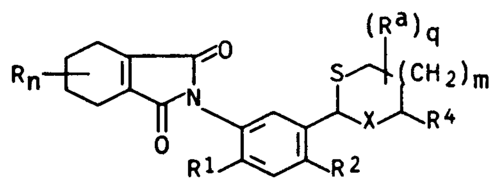

| Nr. | $R^1$ | $R^2$ | $(R^a)_q$ | $R^4$ | $(R)_n$ | X | m | physik. Daten [Fp(°C);IR($cm^{-1}$);NMR(ppm)] |
|---|---|---|---|---|---|---|---|---|
| 1.001 | H | Cl | 5-$CH_3$ | H | 4-$CH_3$ | S | 0 | 128-130 |
| 1.002 | H | Cl | 4-$CH_3$ | H | 4-$CH_3$ | O | 0 | 84- 85 |
| 1.003 | H | Cl | H | H | 4-$CH_3$ | S | 0 | 156-157 |
| 1.004 | F | Cl | 4-$CH_3$ | H | 4-$CH_3$ | S | 0 | 117-118 |
| 1.005 | F | Cl | 4-$CH_3$ | H | 4-$CH_3$ | O | 0 | 151-152 |
| 1.006 | H | Cl | H | H | 4-$CH_3$ | S | 1 | 197-198 |
| 1.007 | H | Cl | H | $CH_2OH$ | 4-$CH_3$ | S | 0 | 76- 78 |

Tabelle 2

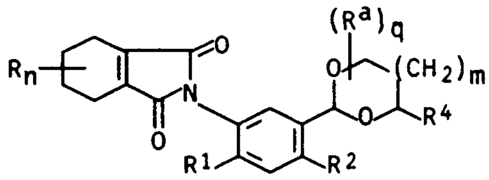

| Nr. | $R^1$ | $R^2$ | $(R^a)_q$ | $R^4$ | $R_n$ | m | physik. Daten [Fp(°C);IR($cm^{-1}$);NMR(ppm)] |
|---|---|---|---|---|---|---|---|
| 2.001 | H | Cl | 4-$CH_3$ | $CO_2(CH_2)_3CH_3$ | 4-$CH_3$ | 0 | 2959, 1715, 1481, 1377 |
| 2.002 | F | Cl | 4-$CH_3$ | $CO_2(CH_2)_2CH_3$ | 4-$CH_3$ | 0 | 1721, 1500, 1422, 1085 |
| 2.003 | H | Cl | 4-$CH_3$ | $CO_2CH_3$ | 4-$CH_3$ | 0 | 1715, 1481, 1377, 1086 |
| 2.004 | H | Cl | 5-$CH_3$ | $CO_2(CH_2)_3CH_3$ | 4-$CH_3$ | 0 | 2958, 1716, 1378, 1104 |

Anwendungsbeispiele

Die herbizide Wirkung der N-Phenyltetrahydrophthalimlde der Formel I ließ sich durch Gewächshausversuche zeigen:

Zur Aufzucht der Testpflanzen dienten Plastikblumentöpfe mit 300 $cm^3$ Inhalt und lehmigem Sand mit etwa 3 % Humus als Substrat. Die Samen der Testpflanzen wurden nach Arten getrennt flach eingesät.

Bei Vorauflaufbehandlung wurden die aufbereiteten Wirkstoffe unmittelbar danach auf die Erdoberfläche aufgebracht. Sie wurden hierbei in Wasser als Verteilungsmittel suspendiert oder emulgiert und mittels fein verteilender Düsen gespritzt. Nach dem Aufbringen der Mittel wurden die Gefäße leicht beregnet, um Keimung und Wachstum in Gang zu bringen. Danach wurden die Gefäße mit durchsichtigen Plastikhauben abgedeckt, bis die Pflanzen angewachsen waren. Diese Abdeckung fördert ein gleichmäßiges Keimen der Testpflanzen, sofern dies nicht durch die Wirkstoffe beeinträchtigt wird.

Zum Zwecke der Nachauflaufbehandlung wurden die Testpflanzen je nach Wuchsform erst bis zu einer Wuchshöhe von 3 bis 15 cm angezüchtet und erst dann mit den in Wasser suspendierten oder emulgierten Wirkstoffen behandelt. Die Testpflanzen wurden dafür entweder direkt gesät und in den gleichen Gefäßen aufgezogen oder sie wurden als Keimpflanzen getrennt gezüchtet und einige Tage vor der Behandlung in die Versuchsgefäße verpflanzt. Die Aufwandmenge betrug 0,03 kg/ha a.S.

Die Versuchsgefäße wurden im Gewächshaus aufgestellt, wobei für wärmeliebende Arten wärmere Bereiche (20 bis 35 °C) und für solche gemäßigter Klimate 10 bis 25 °C bevorzugt wurden. Die versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Galium aparine | Klettenlabkraut | catchweed bedstraw |
| Solanum nigrum | Schwarzer Nachtschatten | black nightshade |
| Stellaria media | Vogelsternmiere | chickweed |
| Zea mays | Mais | Indian corn |

Als Vergleichsmittel wurden Wirkstoffe aus der EP-A 320 677 bzw. aus der EP-A 207 894 verwendet und den neuen Wirkstoffen gegenübergestellt.

Vergleichsverbindungen:

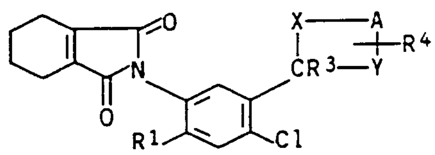

| Vergl. Verbindung | $R^1$ | $R^3$ | $R^4$ | X | Y | A | EP-A |
|---|---|---|---|---|---|---|---|
| A | H | H | 4-$CH_3$ | S | S | $CH_2CH_2$ | 320 677 |
| B | H | $CH_3$ | H | O | O | $CH_2CH_2$ | 207 894 |
| C | F | $CH_3$ | H | O | O | $CH_2CH_2$ | 207 894 |
| D | F | $CH_3$ | H | O | O | $CH_2CH_2CH_2$ | 207 894 |

Mit 0,03 kg/ha a.S. im Nachauflaufverfahren eingesetzt, lassen sich mit dem Beispiel 1.001 breitblättrige unerwünschte Pflanzen sehr gut bekämpfen und zwar mit gleichzeitig deutlicherer Verträglichkeit für Mais als die Vergleichsmittel A-D .

## Patentansprüche

**1.** N-Phenyltetrahydrophthalimide der allgemeinen Formel I,

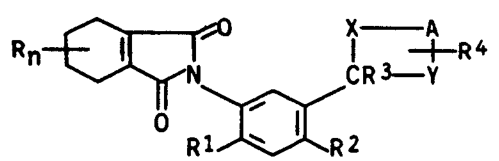

I,

in der die Substituenten und der Index folgende Bedeutung haben:

- R eine $C_1$-$C_4$-Alkylgruppe;
- n 1 oder 2;
- $R^1$ Wasserstoff oder ein Fluoratom;
- $R^2$ ein Halogenatom;
- $R^3$ Wasserstoff oder eine $C_1$-$C_4$-Alkylgruppe;
- $R^4$ Wasserstoff; eine $C_1$-$C_8$-Alkoxycarbonylgruppe, eine $C_3$-$C_6$-Alkenyloxycarbonylgruppe oder eine $C_3$-$C_6$-Alkinyloxycarbonylgruppe, wobei diese Gruppen einen $C_1$-$C_4$-Alkoxyrest tragen können;
- X,Y Sauerstoff oder Schwefel;
- A eine $C_2$-$C_3$-Alkylenkette, welche ein bis drei der folgenden Reste tragen kann: Hydroxyl, Carboxyl, $C_1$-$C_4$-Alkyl und/oder $C_1$-$C_6$-Alkoxycarbonyl, wobei die beiden letztgenannten Reste ihrerseits ein bis fünf Halogenatome und einen der folgenden Reste tragen können: Cyano, Hydroxy, Mercapto, $C_1$-$C_4$-Alkoxy, $C_3$-$C_6$-Alkenyloxy, $C_3$-$C_6$-Alkinyloxy, $C_1$-$C_6$-Alkylcarbonyloxy, $C_1$-$C_4$-Alkylthio, $C_3$-$C_6$-Alkenylthio, $C_3$-$C_6$-Alkinylthio, $C_1$-$C_6$-Alkoxycarbonyl, $C_1$-$C_6$-Alkoxycarbonyl-$C_1$-$C_4$-alkoxy und $C_1$-$C_6$-Alkoxycarbonyl-$C_1$-$C_4$-alkylthio,

wobei $R^4$ nicht Wasserstoff bedeutet, wenn X und Y Sauerstoff bedeuten.

**2.** Verfahren zur Herstellung der Verbindungen I gemäß Anspruch 1, dadurch gekennzeichnet, daß man ein Nitrobenzol der allgemeinen Formel II,

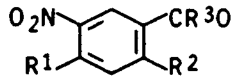

II,

in an sich bekannter Weise mit einer Verbindung III

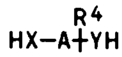

III

acetalisiert, das so erhaltene Acetal bzw. Thioacetal IV

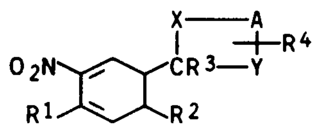

IV

anschließend zum Anilinderivat V

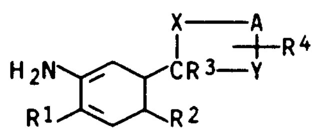

V

reduziert und V mit einem Tetrahydrophthalsäureanhydrid VI

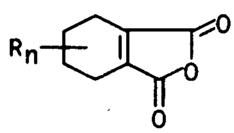

VI

zum Derivat I cyclisiert.

**3.** Verfahren zur Herstellung der Verbindungen I gemäß Anspruch 1, dadurch gekennzeichnet, daß man ein cyclisches Acetal der allgemeinen Formel VII,

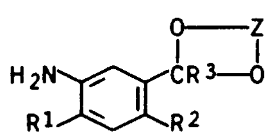

VII

in der Z eine Ethylen- oder Propylenkette bedeutet, welche ein bis drei $C_1$-$C_3$-Alkylgruppen tragen kann, mit einem Tetrahydrophthalsäureanhydrid VI gemäß Anspruch 2 unter Acetalspaltung zum Derivat VIII

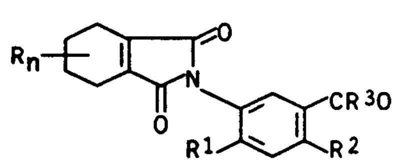

VIII

cyclisiert und VIII anschließend mit einer Verbindung III gemäß Anspruch 2 zu I acetalisiert.

**4.** Herbizide Mittel, enthaltend ein N-Phenyltetrahydrophthalimid der allgemeinen Formel I gemäß Anspruch 1 und inerte Zusatzstoffe.

**5.** Herbizide Mittel gemäß Anspruch 4, enthaltend weitere wirksame Bestandteile.

**6.** Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines N-Phenyltetrahydrophthalimides I gemäß Anspruch 1 behandelt.

## Claims

**1.** An N-phenyltetrahydrophthalimide of the general formula I

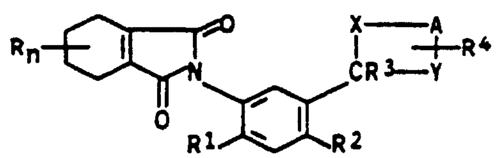

I,

where

| | |
|---|---|
| R | is $C_1$-$C_4$-alkyl; |
| n | is 1 or 2; |
| $R^1$ | is hydrogen or fluorine; |
| $R^2$ | is halogen; |
| $R^3$ | is hydrogen or $C_1$-$C_4$-alkyl; |
| $R^4$ | is hydrogen, $C_1$-$C_8$-alkoxycarbonyl, $C_3$-$C_6$-alkenyloxycarbonyl or $C_3$-$C_6$-alkynyloxycarbonyl, it being possible for each of these groups to carry a $C_1$-$C_4$-alkoxy radical; |
| X and Y | are each oxygen or sulfur; |
| A | is $C_2$-$C_3$-alkylene which can carry one to three of the following: hydroxyl, carboxyl, $C_1$-$C_4$-alkyl and/or $C_1$-$C_6$-alkoxycarbonyl, it being possible for each of the two latter radicals in turn to carry one to five halogens or one of the following: cyano, hydroxyl, mercapto, $C_1$-$C_4$-alkoxy, $C_3$-$C_6$-alkenyloxy, $C_3$-$C_6$-alkynyloxy, $C_1$-$C_6$-alkylcarbonyloxy, $C_1$-$C_4$-alkylthio, $C_3$-$C_6$-alkenylthio, $C_3$-$C_6$-alkynylthio, $C_1$-$C_6$-alkoxycarbonyl, $C_1$-$C_6$-alkoxycarbonyl-$C_1$-$C_4$-alkoxy or $C_1$-$C_6$-alkoxycarbonyl-$C_1$-$C_4$-alkylthio, |

but $R^4$ is not hydrogen when X and Y are both oxygen.

**2.** A process for the preparation of a compound I as claimed in claim 1, wherein a nitrobenzene of the general formula II

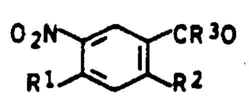

II,

is acetalized in a conventional manner with a compound III

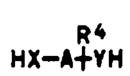

III,

the resulting acetal or thioacetal IV

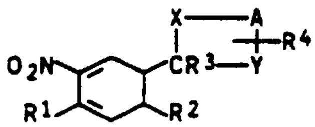

IV

is subsequently reduced to the aniline derivative V

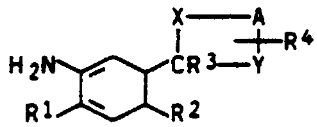

V

and V is cyclized with a tetrahydrophthalic anhydride VI

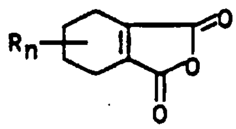

VI

to give the derivative I.

3. A process for the preparation of a compound I as claimed in claim 1, wherein a cyclic acetal of the general formula VII

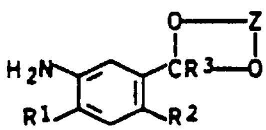

VII,

where Z is ethylene or propylene, each of which can carry one to three $C_1$-$C_3$-alkyl groups, is cyclized with a tetrahydrophthalic anhydride VI as claimed in claim 2, with acetal cleavage, to give the derivative VIII

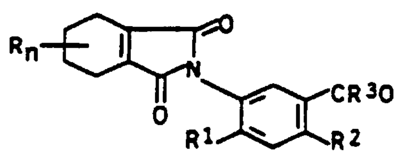

VIII

and VIII is subsequently acetalized with a compound III as claimed in claim 2 to give I.

4. A herbicide containing an N-phenyltetrahydrophthalimide of the general formula I as claimed in claim 1 and inert additives.

5. A herbicide as claimed in claim 4, containing further active components.

6. A method for controlling unwanted plant growth, wherein the unwanted plants and/or their habitat are treated with a herbicidally effective amount of an N-phenyltetrahydrophthalimide I as claimed in claim 1.

## Revendications

**1.** N-phényltétrahydrophtalimides de la formule générale I

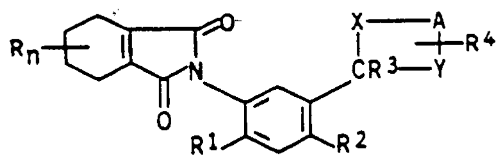

I,

dans laquelle les substituants et les indices ont la signification suivante

- R — un groupe alkyle en C1-C4,
- n — 1 ou 2,
- $R^1$ — hydrogène ou un atome de fluor
- $R^2$ — un atome d'halogène,
- $R^3$ — hydrogène ou un groupe alkyle en C1-C4,
- $R^4$ — hydrogène, un groupe alcoxy en C1-C8-carbonyle, un groupe alcényloxy en C3-C6-carbonyle, ou un groupe alcinyloxy en C3-C6-carbonyle, ces groupes pouvant porter un reste alcoxy en C1-C4,
- X, Y — oxygène ou soufre,
- A — une chaîne alkylène en C2-C3, qui peut porter un à trois des restes suivants : hydroxyle, carboxyle, alkyle en C1-C4 et/ou alcoxy en C1-C6-carbonyle, les deux restes cités en dernier pouvant porter de leur côté, un à cinq atomes d'halogène et un des restes suivants : cyano, hydroxy, mercapto, alcoxy en C1-C4, alcényloxy en C3-C6, alcinyloxy en C3-C6, alkyle en C1-C6-carbonyloxy, alkylthio en C1-C4, alcénylthio en C3-C6, alcinylthio en C3-C6, alcoxy en C1-C6-carbonyle, alcoxy en C1-C6-carbonyle en C1-C4 alcoxy et alcoxy en C1-C6-carbonyle en C1-C4-carbonylthio,
- $R^4$ — ne représentant pas hydrogène, quand X et Y représentent oxygène.

**2.** Procédé de préparation des composés I selon la revendication 1, caractérisé par le fait que l'on acétalise, de manière connue en soi un nitrobenzène de la formule générale II

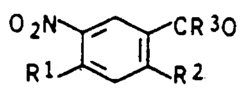

II,

avec un composé III

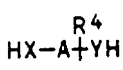

III

on réduit ensuite l'acétal ou thioacétal IV obtenu en dérivé d'aniline V

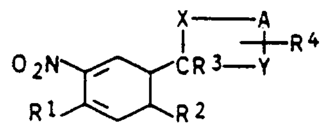

IV

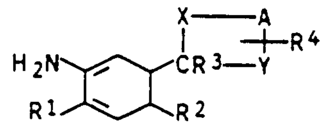

V

et on cyclise V en dérivé I avec un anhydride d'acide tétrahydrophtalique VI

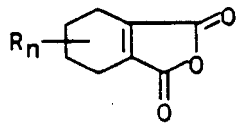

VI

3. Procédé de préparation des composés I selon la revendication 1, caractérisé par le fait que l'on cyclise en dérivé VIII, avec séparation d'acétal, un acétal cyclique de la formule générale VII

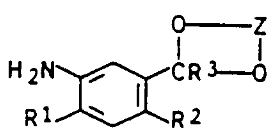

VII

dans laquelle Z représente une chaîne éthylène ou propylène, qui peut porter un à trois groupes alkyle en C1-C3, avec un anhydride d'acide tétrahydrophtalique VI selon la revendication 2

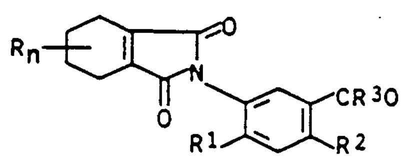

VIII

et on acétalise ensuite VIII en I avec un composé III selon la revendication 2.

4. Herbicide contenant un N-phényltétrahydrophtalimide de la formule générale I selon la revendication 1 et des additifs inertes.

5. Herbicide selon la revendication 4 contenant, en outre, des constituants actifs.

6. Procédé de lutte contre la croissance des plantes indésirables, caractérisé par le fait que l'on traite les plantes indésirables et/ou leur biotope avec une quantité à effet herbicide d'un N-phényltétrahydrophtalimide I selon la revendication 1.